(19) Europäisches Patentamt
European Patent Office
Office européen des brevets

(11) Veröffentlichungsnummer: **0 252 005 B1**

(12) **EUROPÄISCHE PATENTSCHRIFT**

(45) Veröffentlichungstag der Patentschrift: **10.07.91**

(51) Int. Cl.$^5$: **C07D 405/06**, C07D 311/58, C07D 319/20, A61K 31/445

(21) Anmeldenummer: **87810350.6**

(22) Anmeldetag: **22.06.87**

(54) **Hydrierte 1-Benzooxacycloalkyl-pyridincarbonsäureverbindungen.**

(30) Priorität: **26.06.86 CH 2588/86**

(43) Veröffentlichungstag der Anmeldung:
**07.01.88 Patentblatt 88/01**

(45) Bekanntmachung des Hinweises auf die Patenterteilung:
**10.07.91 Patentblatt 91/28**

(84) Benannte Vertragsstaaten:
**AT BE CH DE ES FR GB GR IT LI LU NL SE**

(56) Entgegenhaltungen:
**EP-A- 0 066 456**
**EP-A- 0 076 530**
**FR-A- 1 366 479**
**FR-A- 2 297 618**
**US-A- 4 579 845**

(73) Patentinhaber: **CIBA-GEIGY AG**
**Klybeckstrasse 141**
**CH-4002 Basel(CH)**

(72) Erfinder: **Fröstl, Wolfgang, Dr.**
**St. Johanns-Vorstadt 6/2**
**D-4056 Basel(CH)**

**EP 0 252 005 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue hydrierte 1-Benzooxacycloalkylpyridincarbonsäureverbindungen der Formel

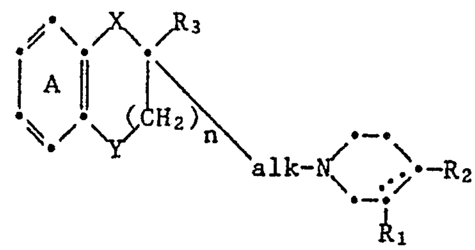

(I),

worin entweder $R_1$ Carboxy, Niederalkoxycarbonyl, Carbamyl, N-Niederalkylcarbamyl, N,N-Diniederalkylcarbamyl oder gegebenenfalls acyliertes Hydroxymethyl bedeutet und $R_2$ Wasserstoff, eine gegebenenfalls verätherte oder acylierte Hydroxygruppe oder eine gegebenenfalls acylierte Aminogruppe darstellt oder $R_1$ Wasserstoff bedeutet und $R_2$ für Carboxy, Niederalkoxycarbonyl, N-Niederalkylcarbamyl, N,N-Diniederalkylcarbamyl oder gegebenenfalls acyliertes Hydroxymethyl steht, $R_3$ Wasserstoff oder Niederalkyl bedeutet, alk Niederalkylen oder Niederalkyliden bedeutet, der Ring A unsubstituiert oder ein- oder mehrfach durch Hydroxy, Niederalkoxy, Niederalkanoyloxy, Halogen, Niederalkyl und/oder Trifluormethyl substituiert ist, die gestrichelte Linie zum Ausdruck bringen soll, dass zwischen den Kohlenstoffatomen, welche die Reste $R_1$ und $R_2$ tragen, eine Einfach- oder eine Doppelbindung vorliegt und entweder X und Y jeweils ein Sauerstoffatom bedeuten und n für 1 steht oder X eine Methylengruppe darstellt, Y ein Sauerstoffatom bedeutet und n für 1 steht oder X ein Sauerstoffatom darstellt, Y eine Methylengruppe bedeutet und n für 1 steht oder X für eine direkte Bindung steht, Y ein Sauerstoffatom darstellt und n für 2 steht, und ihre Tautomeren und/oder Salze, die Verwendung dieser Verbindungen, Verfahren zu ihrer Herstellung und pharmazeutische Zusammensetzungen, enthaltend eine Verbindung der Formel I oder ein Tautomeres und/oder ein pharmazeutisch verwendbares Salz davon.

Veräthertes Hydroxy $R_2$ ist beispielsweise Niederalkoxy oder gegebenenfalls substituiertes Phenylniederalkoxy.

Acyl in acyliertem Hydroxymethyl $R_1$ bzw. $R_2$ sowie in acyliertem Hydroxy bzw. acyliertem Amino $R_2$ ist beispielsweise von einer organischen Carbon- oder Sulfonsäure abgeleitetes Acyl.

Von einer organischen Carbonsäure abgeleitetes Acyl ist beispielsweise der Rest einer aliphatischen oder monocyclisch-aromatischen Carbonsäure, wie Niederalkanoyl oder gegebenenfalls substituiertes Benzoyl, ferner Pyridoyl.

Von einer organischen Sulfonsäure abgeleitetes Acyl ist beispielsweise Niederalkansulfonyl.

Die Erfindung betrifft beispielsweise Verbindungen der Formel I, worin $R_1$ Carboxy, Niederalkoxycarbonyl, Carbamyl, N-Niederalkylcarbamyl oder N,N-Diniederalkylcarbamyl bedeutet, $R_2$ Wasserstoff, eine gegebenenfalls verätherte oder acylierte Hydroxygruppe oder eine gegebenenfalls acylierte Aminogruppe darstellt, $R_3$ Wasserstoff oder Niederalkyl bedeutet, alk Niederalkylen oder Niederalkyliden bedeutet, der Ring A unsubstituiert oder ein- oder mehrfach durch Hydroxy, Niederalkoxy, Niederalkanoyloxy, Halogen, Niederalkyl und/oder Trifluormethyl substituiert ist, die gestrichelte Linie zum Ausdruck bringen soll, dass zwischen den Kohlenstoffatomen, welche die Reste $R_1$ und $R_2$ tragen, eine Einfach- oder eine Doppelbindung vorliegt, X ein Sauerstoffatom oder eine Methylengruppe darstellt, Y ein Sauerstoffatom bedeutet und n für 1 steht, und ihre Tautomeren und/oder Salze, die Verwendung dieser Verbindungen, Verfahren zu ihrer Herstellung und pharmazeutische Zusammensetzungen, enthaltend eine solche Verbindung der Formel I oder ein Tautomeres und/oder ein pharmazeutisch verwendbares Salz davon.

Tautomere Formen von Verbindungen der Formel I existieren z.B. dann, wenn $R_2$ Hydroxy oder Amino bedeutet und die gestrichelte Linie zum Ausdruck bringen soll, dass zwischen den Kohlenstoffatomen, welche die Reste $R_1$ und $R_2$ tragen, eine Doppelbindung vorliegt. D.h., die Enole bzw. Enamine der Formel I stehen im Gleichgewicht mit den entsprechenden Keto- bzw. Ketimin-Tautomeren der Formel

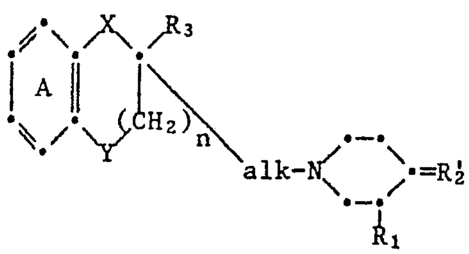

(I'),

worin $R_2'$ Oxo oder Imino bedeutet. Vertreter beider tautomerer Formen können isoliert werden.

Die erfindungsgemässen Verbindungen können ferner in Form von Stereoisomeren vorliegen. Da die Verbindungen der Formel I mindestens ein chirales Kohlenstoffatom (C-Atom) besitzen (das den Rest $R_3$ aufweisende C-Atom), können sie z.B. als reine Enantiomere oder Enantiomerengemische, wie Racemate, und, sofern noch mindestens ein weiteres chirales Zentrum vorhanden ist (z.B. das Atom $C_4$ eines 4-substituierten Piperidinrestes und/oder das Atom $C_3$ eines 3-substituierten Piperidinrestes), auch als Diastereomere, Diastereomerengemische oder Racematgemische vorliegen. So können beispielsweise im Hinblick auf $R_1$ und $R_2$ geometrische Isomere, wie cis- und trans-Isomere, gebildet werden, falls $R_1$ und $R_2$ von Wasserstoff verschieden sind.

Salze von Verbindungen der Formel I bzw. deren Tautomeren sind insbesondere entsprechende Säureadditionssalze, vorzugsweise pharmazeutisch verwendbare Säureadditionssalze. Diese werden beispielsweise mit starken anorganischen Säuren, wie Mineralsäuren, z.B. Schwefelsäure, einer Phosphorsäure oder einer Halogenwasserstoffsäure, mit staken organischen Carbonsäuren, wie Niederalkancarbonsäuren, z.B. Essigsäure, wie gegebenenfalls ungesättigten Dicarbonsäuren, z.B. Malon-, Malein- oder Fumarsäure, oder wie Hydroxycarbonsäuren, z.B. Wein- oder Zitronensäure, oder mit Sulfonsäuren, wie Niederalkan- oder gegebenenfalls substituierten Benzolsulfonsäuren, z.B. Methan- oder p-Toluolsulfonsäure, gebildet. Bedeutet $R_1$ oder $R_2$ beispielsweise Carboxy, können entsprechende Verbindungen Salze mit Basen bilden. Geeignete Salze mit Basen sind beispielsweise entsprechende Alkalimetall- oder Erdalkalimetallsalze, z.B. Natrium-, Kalium- oder Magnesiumsalze, pharmazeutisch verwendbare Uebergangsmetallsalze, wie Zink- oder Kupfersalze, oder Salze mit Ammoniak oder organischen Aminen, wie cyclische Amine, wie Mono-, Di- bzw. Triniederalkylamine, wie Hydroxyniederalkylamine, z.B. Mono-, Di- bzw. Trihydroxyniederalkylamine, Hydroxyniederalkylniederalkyl-amine oder Polyhydroxyniederalkylamine. Cyclische Amine sind z.B. Morpholin, Thiomorpholin, Piperidin oder Pyrrolidin. Als Mononiederalkylamine kommen beispielsweise Aethyl- oder t-Butylamin, als Diniederalkylamine beispielsweise Diäthyl- oder Diisopropylamin, als Triniederalkylamine beispielsweise Trimethyl- oder Triäthylamin in Betracht. Entsprechende Hydroxyniederalkylamine sind z.B. Mono-, Di- bzw. Triäthanolamin; Hydroxyniederalkyl-niederalkyl-amine sind z.B. N,N-Dimethylamino- oder N,N-Diäthylamino-äthanol; als Polyhydroxyniederalkylamin kommt z.B. Glucosamin in Frage.

Umfasst sind ferner für pharmazeutische Verwendungen ungeeignete Salze, da diese beispielsweise für die Isolierung bzw. Reinigung freier erfindungsgemässer Verbindungen sowie deren pharmazeutisch verwendbarer Salze verwendet werden können.

Vor- und nachstehend sind unter mit "Nieder" bezeichneten Resten oder Verbindungen, sofern nicht abweichend definiert, solche zu verstehen, die bis und mit 7, vor allem bis und mit 4, Kohlenstoffatome enthalten.

Niederalkoxy ist $C_1$-$C_4$-Alkoxy, wie Methoxy, Aethoxy, n-Propyloxy, Isopropyloxy, n-Butyloxy, Isobutyloxy oder tert-Butyloxy.

Niederalkyl ist z.B. $C_1$-$C_4$-Alkyl, d.h. Methyl, Aethyl, n-Propyl, Isopropyl, n-Butyl, Isobutyl, sek-Butyl oder tert-Butyl, und umfasst ferner $C_5$-$C_7$-Alkyl-, d.h. Pentyl-, Hexyl- und Heptylreste.

Niederalkylen alk ist $C_1$-$C_4$-Alkylen, welches die beiden Ringsysteme in erster Linie durch bis und mit 3 C-Atome überbrückt, und kann z.B. Methylen, Aethylen oder 1,3-Propylen, aber auch 1,2-Propylen, 1,2- oder 1,3-(2-Methyl)-propylen oder 1,2- oder 1,3-Butylen sein, kann aber ferner die beiden Ringsysteme auch durch 4 C-Atome überbrücken, d.h. 1,4-Butylen darstellen.

Niederalkyliden alk ist $C_1$-$C_4$-Alkyliden und kann z.B. Methylen, Aethyliden, 1,1- oder 2,2-Propyliden oder 1,1- oder 2,2-Butyliden sein.

Niederalkanoyl ist $C_2$-$C_5$-Alkanoyl, wie Acetyl, Propionyl, Butyryl, Isobutyryl oder Pivaloyl.

Niederalkanoyloxy ist $C_2$-$C_5$-Alkanoyloxy, wie Acetyloxy, Propionyloxy, Butyryloxy, Isobutyryloxy oder Pivaloyloxy.

Niederalkoxycarbonyl ist $C_1$-$C_4$-Alkoxycarbonyl, wie Methoxy-, Aethoxy-, n-Propyloxy-, Isopropyloxy-, n-Butyloxy-, Isobutyloxy- oder tert.-Butyloxycarbonyl.

N-Niederalkylcarbamyl ist N-$C_1$-$C_4$-Alkylcarbamyl, wie N-Methyl-, N-Aethyl-, N-(n-Propyl)-, N-Isopropyl-, N-(n-Butyl)-, N-Isobutyl- oder N-tert.-Butylcarbamyl.

N,N-Diniederalkylcarbamyl ist N,N-Di-$C_1$-$C_4$-alkylcarbamyl, wobei jeweils die beiden N-Alkylgruppen gleich oder verschieden sein können, wie N,N-Dimethyl-, N,N-Diäthyl-, N,N-Diisopropyl- oder N-Butyl-N-methyl-carbamyl.

Gegebenenfalls substituiertes Phenylniederalkoxy ist gegebenenfalls im Phenylteil substituiertes Phenyl-$C_1$-$C_4$-alkoxy, wie Benzyloxy, p-Chlorbenzyloxy, 1-Phenyläthoxy oder 1-(p-Bromphenyl)-n-butyloxy.

Gegebenenfalls substituiertes Benzoyl ist z.B. Benzoyl, p-Chlorbenzoyl oder p-Nitrobenzoyl.

Niederalkansulfonyl ist $C_1$-$C_4$-Alkansulfonyl, wie Methan- oder Aethansulfonyl.

Halogen ist insbesondere Halogen mit einer Atomnummer bis und mit 35, d.h. Fluor, Chlor oder Brom, und umfasst ferner Iod.

Die Verbindungen der Formel I und ihre Tautomeren und/oder ihre pharmazeutisch verwendbaren Salze weisen beispielsweise wertvolle pharmakologische, insbesondere nootrope, Eigenschaften auf. So bewirken sie z.B. im Two-Compartment Passive Avoidance-Testmodell nach Mondadori und Classen, Acta Neurol. Scand. 69, Suppl. 99, 125 (1984) in Dosismengen ab etwa 0,1 mg/kg i.p. sowie p.o. bei der Maus eine Reduktion der amnesischen Wirkung eines zerebralen Elektroschocks.

Ebenso besitzen die erfindungsgemässen Verbindungen eine beträchtliche gedächtnisverbessernde Wirkung, die im Step-down Passive Avoidance-Test nach Mondadori und Waser, Psychopharmacol. 63, 297 (1979) ab einer Dosis von etwa 0,1 mg/kg i.p. sowie p.o. an der Maus festzustellen ist.

Entsprechend können die Verbindungen der Formel I und deren Tautomere und/oder ihre pharmazeutisch verwendbaren Salze als Pharmazeutika, z.B. Nootropika, beispielsweise zur therapeutischen und/oder prophylaktischen Behandlung von cerebralen Insuffizienzerscheinungen, insbesondere Gedächtnisstörungen, verwendet werden. Ein weiterer Gegenstand der Erfindung ist somit die Verwendung der Verbindungen der Formel I und ihrer Tautomeren und/oder ihrer pharmazeutisch verwendbaren Salze zur Herstellung von Arzneimitteln, insbesondere von Nootropika, zur Behandlung von cerebralen Insuffizienzerscheinungen,insbesondere Gedächtnisstörungen. Dabei kann auch die gewerbsmässige Herrichtung der Wirksubstanzen eingeschlossen sein.

Die Erfindung betrifft in erster Linie Verbindungen der Formel I, worin entweder $R_1$ Carboxy, Niederalkoxycarbonyl, Carbamyl, N-Niederalkylcarbamyl, N,N-Diniederalkylcarbamyl, Hydroxymethyl, $C_2$-$C_5$-Alkanoyloxymethyl, Niederalkansulfonyloxymethyl, Benzoyloxymethyl oder Pyridoyloxymethyl bedeutet und $R_2$ Wasserstoff, Hydroxy, Niederalkoxy, Benzyloxy, $C_2$-$C_5$-Alkanoyloxy, Niederalkansulfonyloxy, Benzoyloxy, Pyridoyloxy, Amino, $C_2$-$C_5$-Alkanoylamino, Niederalkansulfonylamino, Benzoylamino oder Pyridoylamino darstellt oder $R_1$ Wasserstoff bedeutet und $R_2$ für Carboxy, Niederalkoxycarbonyl, N-Niederalkylcarbamyl, N,N-Diniederalkylcarbamyl, Hydroxymethyl, $C_2$-$C_5$-Alkanoyloxymethyl, Niederalkansulfonyloxymethyl, Benzoyloxymethyl oder Pyridoyloxymethyl steht, $R_3$ Wasserstoff oder $C_1$-$C_7$-Alkyl bedeutet, alk Niederalkylen, das die beiden Ringsysteme durch bis und mit 3 C-Atome überbrückt, oder Niederalkyliden darstellt, der Ring A unsubstituiert ist oder ein-, zwei- oder mehrfach durch Hydroxy, Niederalkoxy, $C_2$-$C_5$-Alkanoyloxy, Halogen mit einer Atomnummer bis und mit 53, $C_1$-$C_7$-Alkyl und/oder Trifluormethyl substituiert ist, die gestrichelte Linie zum Ausdruck bringen soll, dass zwischen den Kohlenstoffatomen, welche die Reste $R_1$ und $R_2$ tragen, eine Einfach- oder eine Doppelbindung vorliegt und entweder X und Y jeweils ein Sauerstoffatom bedeuten und n für 1 steht oder X eine Methylengruppe darstellt, Y ein Sauerstoffatom bedeutet und n für 1 steht oder X ein Sauerstoffatom darstellt, Y eine Methylengruppe bedeutet und n für 1 steht oder X für eine direkte Bindung steht, Y ein Sauerstoffatom darstellt und n für 2 steht, wobei mit "Nieder" bezeichnete Reste 1 bis und mit 4 Kohlenstoffatome aufweisen können, beispielsweise Verbindungen der Formel I, worin $R_1$ Carboxy, Niederalkoxycarbonyl, Carbamyl, N-Niederalkylcarbamyl oder N,N-Diniederalkylcarbamyl bedeutet, $R_2$ Wasserstoff, Hydroxy, Niederalkoxy, Benzyloxy, $C_2$-$C_5$-Alkanoyloxy, Niederalkansulfonyloxy, Benzoyloxy, Pyridoyloxy, Amino, $C_2$-$C_5$-Alkanoylamino, Benzoylamino oder Pyridoylamino darstellt, $R_3$ Wasserstoff oder $C_1$-$C_7$-Alkyl bedeutet, alk Niederalkylen, das die beiden Ringsysteme durch bis und mit 3 C-Atome überbrückt, oder Niederalkyliden darstellt, der Ring A unsubstituiert ist oder ein-, zwei- oder mehrfach durch Hydroxy, Niederalkoxy, $C_2$-$C_5$-Alkanoyloxy, Halogen mit einer Atomnummer bis und mit 53, $C_1$-$C_7$-Alkyl und/oder Trifluormethyl substituiert ist, die gestrichelte Linie zum Ausruck bringen soll, dass zwischen den Kohlenstoffatomen, welche die Reste $R_1$ und $R_2$ tragen, eine Einfach- oder eine Doppelbindung vorliegt, X ein Sauerstoffatom oder eine Methylengruppe darstellt, Y ein Sauerstoffatom bedeutet und n für 1 steht, wobei mit "Nieder" bezeichnete Reste 1 bis und mit 4 Kohlenstoffatome aufweisen können, und ihre Tautomeren und/oder Salze.

Die Erfindung betrifft vor allem Verbindungen der Formel I, worin entweder $R_1$ $C_1$-$C_4$-Alkoxycarbonyl, wie Methoxy- oder Aethoxycarbonyl, Carbamyl, Hydroxymethyl oder $C_2$-$C_5$-Alkanoyloxymethyl, wie Acetoxymethyl, bedeutet und $R_2$ Wasserstoff oder Hydroxy bedeutet oder $R_1$ Wasserstoff darstellt und $R_2$ für $C_1$-

$C_4$-Alkoxycarbonyl, wie Aethoxycarbonyl, steht, $R_3$ Wasserstoff oder $C_1$-$C_4$-Alkyl, wie Methyl, ist, alk für $C_1$-$C_4$-Alkylen, welches die beiden Ringsysteme durch bis und mit 3 C-Atome überbrückt, wie Methylen oder Aethylen, steht, der Ring A unsubstituiert oder, insbesondere in 7-Stellung, durch $C_1$-$C_4$-Alkoxy, wie Methoxy, substituiert ist, die gestrichelte Linie zum Ausdruck bringen soll, dass zwischen den Kohlenstoffatomen, welche die Reste $R_1$ und $R_2$ tragen, eine Einfach- oder eine Doppelbindung vorliegt, und entweder X und Y jeweils ein Sauerstoffatom bedeuten und n für 1 steht oder X eine Methylengruppe darstellt, Y ein Sauerstoffatom bedeutet und n für 1 steht oder X ein Sauerstoffatom darstellt, Y eine Methylengruppe bedeutet und n für 1 steht oder X für eine direkte Bindung steht, Y ein Sauerstoffatom darstellt und n für 2 steht, beispielsweise Verbindungen der Formel I, worin $R_1$ $C_1$-$C_4$-Alkoxycarbonyl, wie Methoxycarbonyl, bedeutet, $R_2$ Wasserstoff oder Hydroxy bedeutet, $R_3$ Wasserstoff oder $C_1$-$C_4$-Alkyl, wie Methyl, ist, alk für $C_1$-$C_4$-Alkylen, welches die beiden Ringsysteme durch bis und mit 3 C-Atome überbrückt, wie Methylen oder Aethylen, steht, der Ring A unsubstituiert ist, die gestrichelte Linie zum Ausdruck bringen soll, dass zwischen den Kohlenstoffatomen, welche die Reste $R_1$ und $R_2$ tragen, eine Einfach-oder eine Doppelbindung vorliegt, X ein Sauerstoffatom oder eine Methylengruppe bedeutet, Y ein Sauerstoffatom darstellt und n für 1 steht, und ihre Tautomeren und/oder Salze.

Die Erfindung betrifft insbesondere Verbindungen der Formel I, worin $R_1$ $C_1$-$C_4$-Alkoxycarbonyl, wie Methoxy- oder Aethoxycarbonyl, bedeutet, $R_2$ Wasserstoff oder Hydroxy darstellt, $R_3$ Wasserstoff ist, alk für Methylen oder Aethylen steht, der Ring A unsubstituiert ist, die gestrichelte Linie zum Ausdruck bringen soll, das zwischen den Kohlenstoffatomen, welche die Reste $R_1$ und $R_2$ tragen, eine Einfach- oder eine Doppelbindung vorliegt, X ein Sauerstoffatom oder eine Methylengruppe bedeutet, Y ein Sauerstoffatom darstellt und n für 1 steht, und ihre Tautomeren und/oder Salze.

Die Erfindung betrifft in allererster Linie Verbindungen der Formel I, worin $R_1$ $C_1$-$C_4$-Alkoxycarbonyl, wie Methoxycarbonyl, bedeutet, $R_2$ Wasserstoff darstellt, $R_3$ Wasserstoff ist, alk für Methylen oder Aethylen steht, der Ring A unsubstituiert ist, die gestrichelte Linie zum Ausdruck bringen soll, dass zwischen den Kohlenstoffatomen, welche die Reste $R_1$ und $R_2$ tragen, eine Einfach- oder eine Doppelbindung vorliegt, X ein Sauerstoffatom oder eine Methylengruppe darstellt, Y ein Sauerstoffatom bedeutet und n für 1 steht, oder worin $R_1$ $C_1$-$C_4$-Alkoxycarbonyl, wie Methoxycarbonyl, bedeutet, $R_2$ Hydroxy darstellt, $R_3$ Wasserstoff ist, alk für Methylen steht, der Ring A unsubstituiert ist, die gestrichelte Linie zum Ausdruck bringen soll, dass zwischen den Kohlenstoffatomen, welche die Reste $R_1$ und $R_2$ tragen, eine Doppelbindung vorliegt, X ein Sauerstoffatom oder eine Methylengruppe darstellt, Y ein Sauerstoffatom bedeutet und n für 1 steht, und jeweils ihre Tautomeren und/oder Salze.

Die Erfindung betrifft namentlich die in den Beispielen genannten neuen Verbindungen der Formel I und ihre Tautomeren und/oder Salze und und Verfahren zu ihrer Herstellung.

Ebenfalls Gegenstand der vorliegenden Erfindung ist ein Verfahren zur Herstellung von Verbindungen der Formel I oder ihrer Tautomeren und/oder ihrer Salze, z.B. dadurch gekennzeichnet, dass man

a) eine Verbindung der Formel

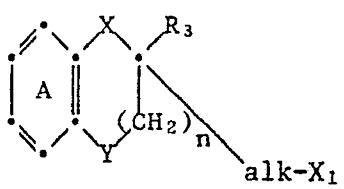

(IIa)

oder ein Salz davon, worin $X_1$ Hydroxy oder reaktionsfähiges verestertes Hydroxy bedeutet, mit einer Verbindung der Formel

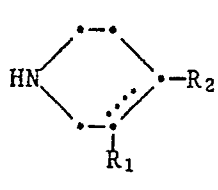

(IIb)

oder einem Tautomeren und/oder Salz davon umsetzt oder

b) in einer Verbindung der Formel

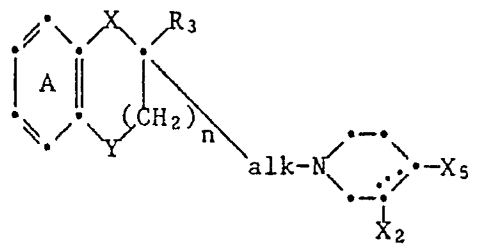

(III)

oder einem Tautomeren und/oder Salz davon, worin $X_2$ einen in von Wasserstoff verschiedenes $R_1$ überführbaren Rest bedeutet und $X_5$ einen Rest $R_a$ darstellt, wobei $R_a$ Wasserstoff, Hydroxy, Niederalkoxy, Benzyloxy, $C_2$-$C_5$-Alkanoyloxy, Niederalkansulfonyloxy, Benzoyloxy, Pyridoyloxy, Amino, $C_2$-$C_5$-Alkanoylamino, Niederalkansulfonylamino, Benzoylamino oder Pyridoylamino ist, $X_2$ in von Wasserstoff verschiedenes $R_1$ überführt oder in einer Verbindung der Formel III, worin $X_2$ Wasserstoff ist und $X_5$ einen in $R_b$ überführbaren Rest bedeutet, wobei $R_b$ für einen von einem Rest $R_a$ verschiedenen Rest $R_2$ steht, $X_5$ in $R_b$ überführt oder

c) zur Herstellung einer Verbindung der Formel I oder eines Tautomeren und/oder Salzes davon, worin $R_2$ Hydroxy oder Amino bedeutet und $R_1$ von Wasserstoff verschieden ist und insbesondere Niederalkoxycarbonyl bedeutet, eine Verbindung der Formel

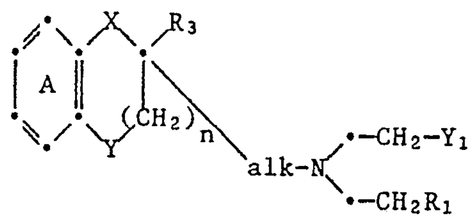

(IV),

worin $Y_1$ eine Gruppe der Formel $-CH=R_2'$, $-C(Y_2)=R_2'$, $-CH(Y_2)-R_2$ oder Cyano darstellt, wobei $R_2'$ Oxo oder Imino bedeutet und $Y_2$ einen abspaltbaren Rest darstellt, oder ein Salz davon cyclisiert oder

d) zur Herstellung einer Verbindung der Formel I' oder eines Tautomeren und/oder Salzes davon, worin $R_2'$ Oxo oder Imino bedeutet und $R_1$ von Wasserstoff verschieden ist und insbesondere Niederalkoxycarbonyl bedeutet, eine Verbindung der Formel

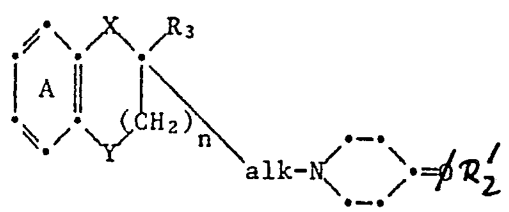

(Va)

oder ein Tautomeres oder ein Salz davon mit einer Verbindung der Formel

$X_3$-$R_1$ (Vb)

oder einem Salz davon, worin $R_1$ von Wasserstoff verschieden ist und $X_3$ Halogen oder Niederalkoxy bedeutet, umsetzt oder

e) zur Herstellung einer Verbindung der Formel I oder eines Tautomeren und/oder Salzes davon, worin $R_2$ Hydroxy, Niederalkoxy, Benzyloxy, $C_2$-$C_5$-Alkanoyloxy, Niederalkansulfonyloxy, Benzoyloxy, Pyridoyloxy, Amino, $C_2$-$C_5$-Alkanoylamino, Niederalkansulfonylamino, Benzoylamino oder Pyridoylamino ist, in einer Verbindung der Formel

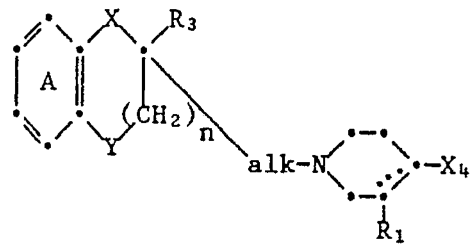

(VI)

oder einem Salz davon, worin $X_4$ einen in $R_2$ überführbaren Rest bedeutet, $X_4$ in $R_2$ überführt oder
f) insbesondere zur Herstellung einer Verbindung der Formel I oder eines Tautomeren und/oder Salzes davon, worin $R_2$ Wasserstoff, Hydroxy, Amino, Carboxy, Niederalkoxycarbonyl, N-Niederalkylcarbamyl, N,N-Diniederalkylcarbamyl oder Hydroxymethyl ist, in einer Verbindung der Formel

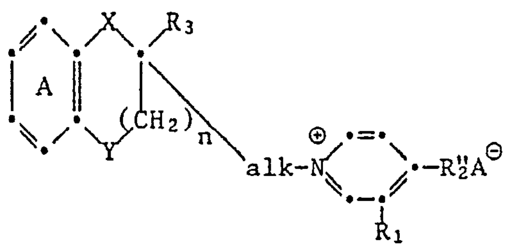

(VII),

worin $A^{\ominus}$ für das Anion einer Säure steht und $R_2''$ Wasserstoff, Niederalkoxy, Benzyloxy, $C_2$-$C_5$-Alkanoyloxy, Niederalkansulfonyloxy, Benzoyloxy, Pyridoyloxy, geschütztes Hydroxy, $C_2$-$C_5$-Alkanoylamino, Niederalkansulfonylamino, Benzoylamino, Pyridoylamino, geschütztes Amino, Carboxy, Niederalkoxycarbonyl, N-Niederalkylcarbamyl, N,N-Diniederalkylcarbamyl, $C_2$-$C_5$-Alkanoyloxymethyl, Niederalkansulfonyloxymethyl, Benzoyloxymethyl, Pyridoyloxymethyl oder veräthertes oder geschütztes Hydroxymethyl bedeutet, die überschüssigen Doppelbindungen zu Einfachbindungen reduziert oder
g) zur Herstellung einer Verbindung der Formel I oder eines Tautomeren und/oder Salzes davon, worin $R_2$ Carboxy, N-Niederalkylcarbamyl, N,N-Diniederalkylcarbamyl oder insbesondere Niederalkoxycarbonyl bedeutet, eine Verbindung der Formel

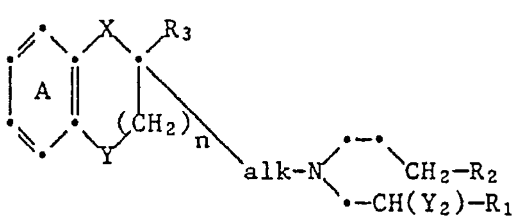

(VIII),

worin $Y_2$ einen abspaltbaren Rest darstellt, oder ein Salz davon cyclisiert und jeweils eine gegebenenfalls vorhandene Schutzgruppe abspaltet und jeweils gewünschtenfalls eine verfahrensgemäss oder auf andere Weise erhältliche Verbindung der Formel I in eine andere Verbindung der Formel I überführt, jeweils ein verfahrensgemäss erhältliches Isomerengemisch in die Komponenten auftrennt, jeweils ein verfahrensgemäss erhältliches Enantiomeren- bzw. Diastereomerengemisch in die Enantiomeren bzw. Diastereomeren aufspaltet und/oder jeweils eine verfahrensgemäss erhältliche freie Verbindung der Formel I in ein Salz überführt oder ein verfahrensgemäss erhältliches Salz in die freie Verbindung der Formel I oder in ein anderes Salz umwandelt, wobei mit "Nieder" bezeichnete Reste 1 bis und mit 4 Kohlenstoffatome aufweisen können.

Die vor- und nachstehend in den Varianten beschriebenen Umsetzungen werden in an sich bekannter Weise durchgeführt, z.B. in Ab- oder üblicherweise in Anwesenheit eines geeigneten Lösungs- oder Verdünnungsmittels oder eines Gemisches derselben, wobei man je nach Bedarf unter Kühlen, bei Raumtemperatur oder unter Erwärmen z.B. in einem Temperaturbereich von etwa -10° bis zur Siedetemperatur des Reaktionsmediums, vorzugsweise von etwa 20° bis etwa 150° C, und, falls erforderlich, in einem geschlossenen Gefäss, unter Druck, in einer Inertgasatmosphäre und/oder unter wasserfreien Bedingungen

arbeitet.

Das vor- und nachstehend aufgeführte Ausgangsmaterial der Formeln IIa und IIb, III, IV, Va und Vb, VI, VII und VIII, das für die Herstellung der Verbindungen der Formel I oder deren Tautomeren und/oder Salzen entwickelt wurde, ist zum Teil bekannt oder kann ebenfalls nach an sich bekannten Methoden, z.B. analog den vorstehend beschriebenen Verfahrensvarianten, hergestellt werden.

Ausgangsmaterial mit basischen Zentren kann z.B. in Form von Säureadditionssalzen, beispielsweise mit den vorstehend aufgeführten Säuren, vorliegen, während Ausgangsverbindungen mit sauren Gruppen, Salze mit Basen, z.B. der vorstehend genannten Art, bilden können. Weiter können Ausgangsverbindungen in Form von Tautomeren vorliegen, insbesondere Verbindungen der Formel IIb, worin $R_2$ Hydroxy bedeutet und die gestrichelte Linie zum Ausdruck bringen soll, dass zwischen den Kohlenstoffatomen, welche die Reste $R_1$ und $R_2$ tragen, eine Doppelbindung vorliegt.

<u>Variante a):</u>

Reaktionsfähiges verestertes Hydroxy bedeutet insbesondere mit einer starken anorganischen Säure oder organischen Sulfonsäure verestertes Hydroxy, beispielsweise Halogen, wie Chlor, Brom oder Iod, Sulfonyloxy, wie Hydroxysulfonyloxy, Halogensulfonyloxy, z.B. Fluorsulfonyloxy, gegebenenfalls, z.B. durch Halogen, substituiertes Niederalkansulfonyloxy, z.B. Methan- oder Trifluormethansulfonyloxy, Cycloalkansulfonyloxy, z.B. Cyclohexansulfonyloxy, oder gegebenenfalls, z.B. durch Niederalkyl oder Halogen, substituiertes Benzolsulfonyloxy, z.B. p-Bromphenyl- oder p-Toluolsulfonyloxy.

Die N-Alkylierung wird insbesondere in Gegenwart eines Kondensationsmittels, wie einer geeigneten Base, durchgeführt. Als Basen kommen beispielsweise Alkalimetallhydroxide, -hydride, -amide, -alkanolate, -carbonate, -triphenylmethylide, -diniederalkylamide, -aminoniederalkylamide oder -niederalkylsilylamide, Naphthalinamine, Niederalkylamine, basische Heterocyclen, Ammoniumhydroxide sowie carbocyclische Amine in Frage. Beispielhaft seien Natriumhydroxid, -hydrid, -amid, -äthylat, Kalium-tert-butylat, -carbonat, Lithiumtriphenylmethylid, Lithium-diisopropylamid, Kalium-3-(aminopropyl)-amid, -bis-(trimethylsilyl)-amid, Dimethylaminonaphthalin, Di- oder Triäthylamin, Pyridin, Benzyl-trimethyl-ammoniumhydroxid, 1,5-Diaza-bicyclo[4.3.0]non-5-en (DBN) sowie 1,5-Diaza-bicyclo-[5.4.0]undec-5-en (DBU) genannt.

Die Ausgangsmaterialien der Formeln IIa und IIb sind teilweise bekannt oder lassen sich in Analogie zu den bekannten Ausgangsmaterialien herstellen.

<u>Variante b):</u>

Ein in von Wasserstoff verschiedenes $R_1$ überführbarer Rest $X_2$ bzw. ein in einen Rest $R_b$ überführbarer Rest $X_5$ ist beispielsweise von $R_1$ bzw. $R_b$ verschiedenes funktionell abgewandeltes Carboxy, wie Cyano, anhydridisiertes Carboxy, gegebenenfalls substituiertes Amidino, gegebenenfalls verestertes oder anhydridisiertes Carboximidoyl, von verestertem oder amidiertem Carboxy $R_1$ verschiedenes verestertes oder amidiertes Carboxy, Triniederalkoxy- oder Trihalogenmethyl.

Anhydridisiertes Carboxy ist beispielsweise mit einer Mineralsäure, wie Halogenwasserstoffsäure, oder mit einer Carbonsäure, wie einer gegebenenfalls substituierten Niederalkan- bzw. Benzoesäure oder einem Kohlensäurehalogenid-niederalkylhalbester anhydridisiertes Carboxy. Als Beispiele seien Halogencarbonyl, wie Chlorcarbonyl, Niederalkanoyloxycarbonyl, wie Acetyloxycarbonyl, oder Niederalkoxycarbonyloxycarbonyl, wie Aethoxycarbonyloxycarbonyl, genannt.

Substituiertes Amidino ist beispielsweise mit einem aliphatischen Rest, z.B. Niederalkyl, substituiertes Amidino, wie Niederalkylamidino, z.B. Aethylamidino.

Unter verestertem oder anhydridisiertem Carboximidoyl ist z.B. Alkoxy- bzw. Halogencarboximidoyl, beispielsweise Niederalkoxy-, wie Aethoxy-, bzw. Chlor-carboximidoyl, zu verstehen.

Triniederalkoxy- bzw. Trihalogenmethyl ist z.B. Trimethoxymethyl bzw. Trichlormethyl.

Reste $R_b$ sind beispielsweise Carboxy-, Niederalkoxycarbonyl-, Carbamyl-, N-Niederalkylcarbamyl-, N,N-Diniederalkylcarbamyl- oder gegebenenfalls acylierte Hydroxymethylreste $R_2$.

$X_2$ kann beispielsweise durch Solvolyse in von Wasserstoff verschiedenes $R_1$ überführt werden, ebenso wie $X_5$ beispielsweise durch Solvolyse in einen Rest $R_b$ überführbar ist. Solvolysemittel sind beispielsweise Wasser, dem gewünschten veresterten Carboxy $R_1$ bzw. $R_2$ entsprechende Niederalkanole, Ammoniak oder der gewünschten amidierten Carboxygruppe $R_1$ bzw. $R_2$ entsprechende Amine. Die Behandlung mit einem entsprechenden Solvolysemittel wird gegebenenfalls in Gegenwart einer Säure oder Base durchgeführt. Als Säuren kommen beispielsweise anorganische oder organische Protonsäuren, wie Mineralsäuren, z.B. Schwefelsäure oder Halogenwasserstoffsäuren, beispielsweise Chlorwasserstoffsäure, wie Sulfonsäuren, z.B. Niederalkan- oder gegebenenfalls substituierte Benzolsulfonsäure, beispielsweise Methan- oder p-Toluolsulfonsäure, oder wie Carbonsäuren, z.B. Niederalkancarbonsäuren, beispielsweise Essigsäure, in Frage, während als Basen beispielsweise die unter Variante a) genannten verwendet werden können, insbesondere Natrium- oder Kaliumhydroxid.

Bei der Solvolyse mit Wasser (Hydrolyse) wird die Cyanogruppe, anhydridisiertes Carboxy, gegebenenfalls substituiertes Amidino, gegebenenfalls verestertes oder anhydridisiertes Carboximidoyl, von verestertem oder amidiertem Carboxy $R_1$ bzw. $R_2$ verschiedenes verestertes oder amidiertes Carboxy, Triniederalkoxy- oder Trihalogenmethyl zu Carboxy hydrolysiert. Dabei können gegebenenfalls am Ring A befindliche Niederalkanoyloxyreste im Verlauf der Hydrolyse zu Hydroxy hydrolysiert werden.

Cyano, anhydridisiertes Carboxy, von verestertem oder amidiertem Carboxy $R_1$ bzw. $R_2$ verschiedenes verestertes oder amidiertes Carboxy werden beispielsweise mit einem geeigneten Niederalkanol zu verestertem Carboxy $R_1$ bzw. $R_2$ alkoholysiert und Cyano und anhydridisiertes Carboxy beispielsweise mit Ammoniak oder einem dem amidierten Carboxy $R_1$ bzw. $R_2$ entsprechenden Amin ammono- bzw. aminolysiert.

Das Ausgangsmaterial der Formel III kann beispielsweise in Analogie zu der unter Variante a) beschriebenen Weise durch Umsetzung einer Verbindung der Formel

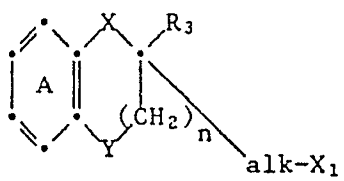

(IIa)

mit einer Verbindung der Formel

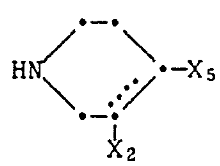

(IIIa),

einem Tautomeren und/oder Salz davon in Gegenwart einer der genannten Basen hergestellt werden.

Verbindungen der Formel III, worin $X_2$ einen in von Wasserstoff verschiedenes $R_1$ überführbaren Rest bedeutet und $X_5$ Hydroxy oder Amino darstellt, können vorteilhaft auch durch Cyclisierung einer Verbindung der Formel

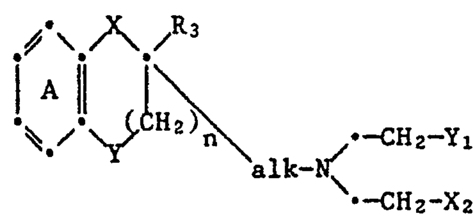

(IVg),

worin $Y_1$ eine Gruppe der Formel $-CH=R_2'$, $-C(Y_2)=R_2'$, $-CH(Y_2)-R_2$ oder Cyano darstellt, wobei $R_2'$ Oxo oder Imino bedeutet, $R_2$ Hydroxy oder Amino ist und $Y_2$ einen abspaltbaren Rest darstellt, oder eines Salzes davon hergestellt werden, wobei z.B. in analoger Weise wie unter der Verfahrensvariante c) angegeben gearbeitet wird.

Variante c):

Abspaltbare Reste $Y_2$ in Gruppen der Formel $-C(Y_2)=R_2'$ oder $-CH(Y_2)-R_2$ sind beispielsweise reaktionsfähige veresterte Hydroxygruppen, wie mit einer starken anorganischen Säure oder organischen Sulfonsäure verestertes Hydroxy, beispielsweise Halogen, wie Chlor, Brom oder Iod, Sulfonyloxy, wie Hydroxysulfonyloxy, Halogensulfonyloxy, z.B. Fluorsulfonyloxy, gegebenenfalls, z.B. durch Halogen, substituiertes Niederalkansulfonyloxy, z.B. Methan- oder Trifluormethansulfonyloxy, Cycloalkansulfonyloxy, z.B. Cyclohexansulfonyloxy, oder gegebenenfalls, z.B. durch Niederalkyl oder Halogen, substituiertes Benzolsulfonyloxy, z.B. p-Bromphenyl- oder p-Toluolsulfonyloxy, oder verätherte Hydroxygruppen, beispielsweise Niederalkoxy oder gegebenenfalls substituiertes Phenylniederalkoxy.

Die Cyclisierung kann beispielsweise in Analogie zur Dieckmann-Reaktion, insbesondere in Gegenwart einer der unter der Variante a) genannten Basen und unter anschliessender hydrolytischer Aufarbeitung, durchgeführt werden.

In einer bevorzugten Ausführungsform kann man beispielsweise eine Verbindung der Formel

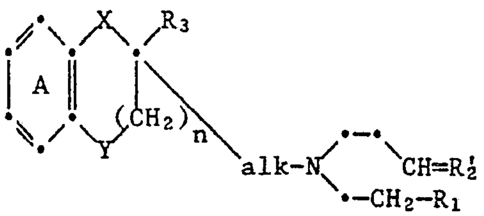

(IVa),

worin $R_2'$ Oxo oder Imino bedeutet, der Behandlung mit einer der genannten Basen, insbesondere mit einem Alkalimetallniederalkanolat, z.B. mit Natriummethanolat oder Natriumäthanolat, unterwerfen. Dabei cyclisiert die Verbindung IVa zu einer Verbindung der Formel I, worin die gestrichelte Linie anzeigt, dass zwischen den Kohlenstoffatomen, welche die Reste $R_1$ und $R_2$ tragen, eine Einfachbindung vorliegt, und worin $R_2$ Hydroxy oder Amino bedeutet. Ausgangsstoffe IVa werden z.B. erhalten, indem man einen reaktionsfähigen Alkylester der Formel

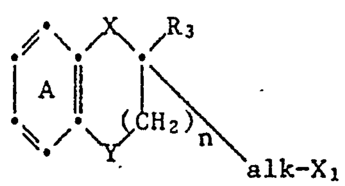

(IIa),

worin $X_1$ reaktionsfähiges verestertes Hydroxy ist, mit einer Verbindung der Formel $H_2N\text{-}CH_2\text{-}CH_2\text{-}R_1$ (IVb) und das erhaltene Zwischenprodukt der Formel

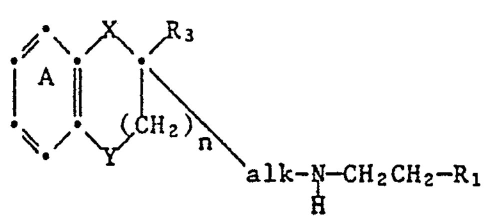

(IVc)

mit Acrolein oder einem gegebenenfalls funktionell abgewandelten Aldehyd der Formel $Y_1\text{-}CH_2\text{-}CH_2\text{-}CH{=}R_2'$ (IVd; $Y_1$ = reaktionsfähiges verestertes Hydroxy; $R_2'$ Oxo oder Imino) umsetzt.

In einer anderen bevorzugten Ausführungsform der Variante c) cyclisiert man eine Verbindung der Formel IV, worin $Y_1$ und $R_1$ Niederalkoxycarbonyl bedeuten, d.h. worin $Y_1$ eine Gruppe der Formel $\text{-}C(Y_2)\text{-}{=}R_2'$ bedeutet, in welcher $R_2'$ Oxo ist und der abspaltbare Rest $Y_2$ als veräthertes Hydroxy eine Niederalkoxygruppe aufweist, zu der entsprechenden Verbindung der Formel I', worin $R_2'$ Oxo ist.

Zur Herstellung der letztgenannten Ausgangsverbindungen der Formel IV kann man beispielsweise von Verbindungen der Formel

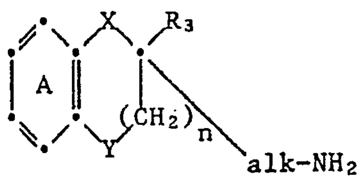

(IVe)

oder deren Salzen ausgehen, welche z.B. durch Reduktion der entsprechenden Nitrile erhältlich sind, und diese mit mindestens 2 Mol einer Verbindung der Formel

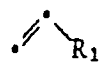

(IVf)

zur Reaktion bringen.

Variante d):

Die verfahrensgemässe C-Acylierung kann insbesondere in Gegenwart einer der unter der Variante a) genannten Basen, besonders vorteilhaft jedoch mittels einer Metallbase, wie Lithiumdiisopropylamin oder n-Butyllithium, gegebenenfalls in Gegenwart von Chlortrimethylsilan, erfolgen.

Die Umsetzung einer Verbindung der Formel

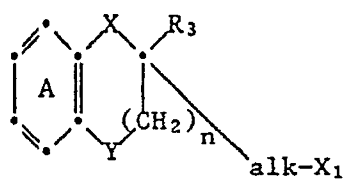

(IIa)

mit einer Verbindung der Formel

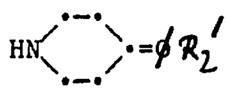

(Vc)

oder einem Salz davon führt in Analogie zu der N-Alkylierung gemäss Variante a) in Gegenwart einer der erwähnten Basen zu dem Ausgangsmaterial der Formel Va.

Variante e):

In $R_2$ überführbare Reste $X_4$ sind beispielsweise durch Solvolyse, d.h. Umsetzung mit einer Verbindung der Formel $R_2H$ oder einem Salz davon, in eine Gruppe $R_2$ überführbare Reste, beispielsweise reaktionsfähige veresterte Hydroxygruppen, wie Halogenatome, z.B. Chlor, Brom oder Iod. In Hydroxy $R_2$ überführbare Reste $X_4$ sind ferner Diazoniumgruppen, z.B. der Formel $-N_2^{\oplus}A^{\ominus}$, worin $A^{\ominus}$ das Anion einer starken Säure, wie einer Mineralsäure, z.B. das Chlorid- oder Sulfation, bedeutet.

Die Solvolyse erfolgt in üblicher Weise, beispielsweise in Gegenwart einer Base, wie eines Alkalimetall- oder Erdalkalimetallhydroxides, z.B. von Natrium- oder Kaliumhydroxid, oder einer tertiären Stickstoffbase, z.B. eines Triniederalkylamins, wie Triäthylamin, oder einer heteroaromatischen Stickstoffbase, wie Pyridin, bzw. eines quaternären Ammoniumhydroxides, wie Benzyl-trimethyl-ammonium-hydroxid, oder indem man die Verbindung $R_2H$ in Form eines Metallsalzes, z.B. der Formel $R_2^{\ominus}M^{\oplus}$ (VIb), worin $M^{\oplus}$ ein Alkalimetallkation, wie das Natriumion, bedeutet, einsetzt. Vorteilhaft arbeitet man in Gegenwart eines Lösungs- oder Verdünnungsmittels, z.B. in einem Ueberschuss der Reaktionskomponente $R_2H$ und/oder einem mit dieser mischbaren inerten Lösungsmittel, erforderlichenfalls unter Kühlen oder Erwärmen, z.B. im Temperaturbereich von etwa 0° bis 120° C, und/oder unter Inertgas, wie Stickstoff.

Die Solvolyse von Resten $X_4$ zu Gruppen $R_2$ kann gegebenenfalls mit der solvolytischen Ueberführung von solvolysierbaren Gruppen $R_1$ in andere erfindungsgemässe Gruppen $R_1$ kombiniert werden, beispiels-

weise können bei der Ammonolyse von Resten $X_4$ zu Amino $R_2$ gegebenenfalls auch Niederalkoxycarbonylgruppen $R_1$ oder andere zu Carbamyl $R_1$ solvolysierbare Gruppen $R_1$ gleichzeitig zu Carbamylgruppen $R_1$ ammonolysiert werden.

Zur Herstellung von Ausgangsverbindungen der Formel VI und deren Salzen geht man beispielsweise von Verbindungen der Formel IIa

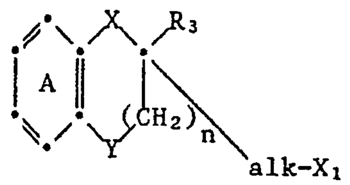

(IIa)

aus und setzt diese mit einer entsprechenden Verbindung der Formel

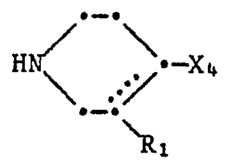

(VIa)

oder einem Salz davon in Gegenwart einer der vorstehend genannten Basen um, wobei z.B. in analoger Weise wie unter der Verfahrens-Variante a) beschrieben verfahren wird.

In einer bevorzugten Ausführungsform erhält man Verbindungen VI, worin $X_4$ für Halogen und die gestrichelte Linie für eine Einfachbindung steht, und deren Salze durch Umsetzung einer Verbindung der Formel I, worin $R_2$ Hydroxy bedeutet und die gestrichelte Linie für eine Einfachbindung steht, oder eines Salzes davon mit einem Halogenierungsmittel, wie Phosphortri- oder -pentachlorid oder Thionylchlorid, wobei die entsprechenden Verbindungen I und ihre Salze beispielsweise in analoger Weise wie unter der Verfahrensvariante a) oder beschrieben erhalten werden können.

Variante f):

Das Anion $A^{\ominus}$ ist beispielsweise das Anion einer starken Protonsäure, z.B. ein Halogenidion, wie Chlorid-, Bromid- oder Iodidion, oder ein Sulfonation, wie ein gegebenenfalls substituiertes Niederalkan- oder Benzolsulfonation, z.B. das Methansulfonat-, Aethansulfonat- oder p-Brombenzolsulfonat- oder p-Toluolsulfonation. $R_2''$ ist insbesondere Wasserstoff, veräthertes Hydroxy $R_2$ oder geschütztes Hydroxy, kann aber auch Carboxy, Niederalkoxycarbonyl, N-Niederalkylcarbamyl, N,N-Diniederalkylcarbamyl $R_2$ oder veräthertes oder geschütztes Hydroxymethyl sein. Geschütztes Hydroxy ist beispielsweise Silyloxy, wie Triniederalkylsilyloxy, z.B. Trimethylsilyloxy, kann aber auch Triphenylniederalkoxy, z.B. Trityloxy, sein. Geschütztes Amino ist beispielsweise Silylamino, wie Triniederalkylsilylamino, z.B. Trimethylsilylamino, kann aber auch Phenyl-, Diphenyl- oder Triphenylniederalkylamino, wie Benzylamino, Diphenylamino oder Tritylamino, sein. Veräthertes Hydroxymethyl ist beispielsweise Niederalkoxymethyl, wie Methoxy- oder Aethoxymethyl, oder gegebenenfalls substituiertes Phenylniederalkoxymethyl, z.B. im Phenylteil substituiertes Phenyl-$C_1$-$C_4$-alkoxymethyl, wie Benzyloxy-, p-Chlorbenzyloxy-, 1-Phenyläthyloxy- oder 1-(p-Bromphenyl)-n-butyloxymethyl. Geschütztes Hydroxymethyl ist beispielsweise Silyloxymethyl, wie Triniederalkylsilyl-, z.B. Trimethylsilyloxymethyl, kann aber auch Triphenylniederalkoxy-, z.B. Trityloxymethyl, sein.

Die Reduktion der überschüssigen Doppelbindungen erfolgt durch Behandeln mit einem geeigneten Reduktionsmittel, beispielsweise durch Hydrierung in Gegenwart eines Hydrierungskatalysators, durch Reduktion mit einem Hydrid-übertragenden Reagenz oder durch Reduktion mit einem metallischen Reduktionssystem aus Metall und Protonen-abspaltendem Mittel.

Als Hydrierungskatalysatoren kommen z.B. Elemente der VIII. Nebengruppe des Periodensystems der Elemente oder deren Derivate in Betracht, wie Palladium, Platin, Platinoxid, Ruthenium, Rhodium, Tris-(triphenylphosphan)-rhodium-I-halogenid, z.B. -chlorid, oder Raney-Nickel, die gegebenenfalls auf einem Trägermaterial, wie Aktivkohle, Alkalimetallcarbonat bzw. -sulfat oder einem Kieselgel aufgezogen sind. Als Hydrid-übertragende Reagenzien kommen beispielsweise geeignete Leichtmetallhydride, insbesondere Alkalimetallaluminiumhydride bzw. -borhydride, wie Lithiumaluminiumhydrid, Lithiumtriäthylborhydrid, Natriumborhydrid, Natriumcyanoborhydrid, oder Zinnhydride, wie Triäthyl- oder Tributylzinnhydrid, oder Diboran in Frage. Der Metallbestandteil des metallischen Reduktionssystems ist beispielsweise ein unedles Metall, wie Alkali- oder Erdalkalimetall, z.B. Lithium, Natrium, Kalium, Magnesium oder Calcium, oder Uebergangsmetall, z.B. Zink, Zinn, Eisen oder Titan, während als Protonen-abspaltende Mittel z.B. Protonsäuren der vorstehend genannten Art, wie Salz- oder Essigsäure, Niederalkanole, wie Aethanol, und/oder Amine bzw. Ammoniak in Frage kommen. Solche Systeme sind beispielsweise Natrium/Ammoniak, Zink/Salz- oder Essigsäure oder Zink/Aethanol.

Die Herstellung von Ausgangsverbindungen der Formel VII erfolgt beispielsweise durch Umsetzung von Verbindungen der Formel

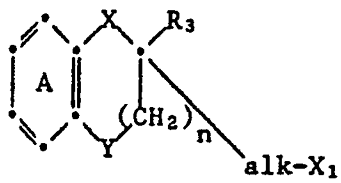

(IIa),

worin $X_1$ dem Anion $A^{\ominus}$ entsprechendes reaktionsfähiges verestertes Hydroxy bedeutet, mit Verbindungen der Formel

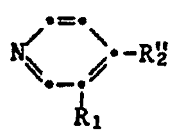

(VIIa)

oder einem Salz davon, wobei z.B. in analoger Weise wie unter der Verfahrensvariante a) beschrieben verfahren wird.

Variante g):

Abspaltbare Reste $Y_2$ in Verbindungen VIII sind beispielsweise reaktionsfähige veresterte Hydroxygruppen, wie mit einer starken anorganischen Säure oder organischen Sulfonsäure verestertes Hydroxy, beispielsweise Halogen, wie Chlor, Brom oder Iod, Sulfonyloxy, wie Hydroxysulfonyloxy, Halogensulfonyloxy, z.B. Fluorsulfonyloxy, gegebenenfalls, z.B. durch Halogen, substituiertes Niederalkansulfonyloxy, z.B. Methan- oder Trifluormethansulfonyloxy, Cycloalkansulfonyloxy, z.B. Cyclohexansulfonyloxy, oder gegebenenfalls, z.B. durch Niederalkyl oder Halogen, substituiertes Benzolsulfonyloxy, z.B. p-Bromphenyl- oder p-Toluolsulfonyloxy, oder verätherte Hydroxygruppen, beispielsweise Niederalkoxy oder gegebenenfalls substituiertes Phenylniederalkoxy.

Die Cyclisierung kann beispielsweise in Gegenwart einer der unter der Variante a) genannten Basen, insbesondere in Gegenwart eines Alkalimetallniederalkanolats, z.B. mit Natriummethanolat oder -äthanolat, durchgeführt werden.

Die Ausgangsstoffe VIII werden beispielsweise erhalten, indem man eine Verbindung der Formel

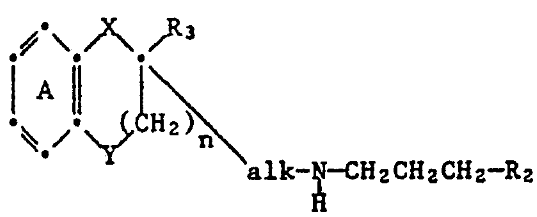

(IVh)

oder ein Salz davon mit einer Verbindung der Formel $Y_2$-$CH_2$-$CH(Y_2)$-$R_1$ (IVi) umsetzt.

In den Ausgangsstoffen der Formeln IIb, III und IIIa kann eine Hydroxygruppe $R_2$ in verätherter bzw. eine Hydroxy- oder Aminogruppe $R_2$ auch in intermediär geschützter Form vorliegen, ebenso wie eine Hydroxymethylgruppe $R_2$ in Verbindungen IIb in verätherter oder intermediär geschützter Form vorliegen

kann. Geschütztes Hydroxy ist beispielsweise Silyloxy, wie Triniederalkylsilyloxy, z.B. Trimethylsilyloxy, kann aber auch Triphenylniederalkoxy, z.B. Trityloxy, sein. Geschütztes Amino ist beispielsweise Silylamino, wie Triniederalkylsilylamino,z.B. Trimethylsilylamino, kann aber auch Phenyl-, Diphenyl- oder Triphenylniederalkylamino, wie Benzylamino, Diphenylmethylamino oder Tritylamino, sein. Veräthertes Hydroxymethyl ist beispielsweise Niederalkoxymethyl, wie Methoxy- oder Aethoxymethyl, oder gegebenenfalls substituiertes Phenylniederalkoxymethyl, z.B. im Phenylteil substituiertes Phenyl-$C_1$-$C_4$-alkoxymethyl, wie Benzyloxy-, p-Chlorbenzyloxy-, 1-Phenyläthyloxy- oder 1-(p-Bromphenyl)-n-butyloxymethyl. Geschütztes Hydroxymethyl ist beispielsweise Silyloxymethyl, wie Triniederalkylsilyl-, z.B. Trimethylsilyloxymethyl, kann aber auch Triphenylniederalkoxy-, z.B. Trityloxymethyl, sein.

Die Freisetzung intermediär geschützter Reste $R_2$, d.h. Abspaltung der intermediären Schutzgruppen, erfolgt in üblicher Weise, beispeilsweise durch Solvolyse, wie milde Hydrolyse, z.B. Behandlung mit Wasser unter neutralen oder schwach sauren Bedingungen, z.B. durch Einwirkung von verdünnt-wässrigen Mineral- oder Carbonsäuren, z.B. von verdünnter Salz- oder Essigsäure. In analoger Weise erfolgt die Freisetzung von intermediär geschützten Hydroxy-, Amino-oder Hydroxymethylgruppen $R_2''$ in Ausgangsstoffen der Formel VII bzw. VIIa.

Verfahrensgemäss oder anderweitig erhältliche Verbindungen der Formel I können in üblicher Weise in andere Verbindungen der Formel I überführt werden.

So kann man z.B. veresterte oder amidierte Carboxygruppen $R_1$ bzw. $R_2$ in üblicher Weise, beispielsweise in Gegenwart eines basischen oder sauren Hydrolysemittels, wie eines Alkalimetallhydroxides oder -carbonates, z.B. von Natriumhydroxid oder Kaliumcarbonat, oder einer Mineralsäure, z.B. von Salzsäure oder Schwefelsäure, zu Carboxy $R_1$ bzw. $R_2$ hydrolysieren. Veresterte Carboxygruppen $R_1$ bzw. $R_2$ können ferner durch Umesterung, d.h. Behandlung mit einem Alkohol in Gegenwart eines sauren oder basischen Solvolysemittels, wie einer Mineralsäure, z.B. von Schwefelsäure, bzw. eines entsprechenden Alkalimetallalkoholates oder eines Alkalimetallhydroxides, in andere veresterte Carboxygruppen $R_1$ bzw. $R_2$ oder durch Umsetzung mit Ammoniak oder einem entsprechenden mindestens ein Wasserstoffatom aufweisenden Amin in amidiertes Carboxy $R_1$ bzw. $R_2$ überführt werden.

Freies Carboxy $R_1$ bzw. $R_2$ kann in üblicher Weise, beispielsweise durch Behandlung mit einem entsprechenden Alkohol in Gegenwart einer Mineralsäure, z.B. von Schwefelsäure, oder durch Ueberführung in ein Halogenid und anschliessende Umsetzung mit einem entsprechenden Alkohol, z.B. in Gegenwart von Pyridin oder Triäthylamin, oder durch Ueberführung in ein Alkalimetallsalz und anschliessende Umsetzung mit einem reaktionsfähigen Ester des entsprechenden Alkohols, wie einem entsprechenden Halogenid, in verestertes Carboxy $R_1$ bzw. $R_2$ überführt werden. Ebenso kann eine Carboxyverbindung unter Verwendung eines Dehydratisierungsmittels, wie N,N'-Dicyclohexylcarbodiimid, mit einem entsprechenden Alkohol verestert werden. Freies oder verestertes Carboxy $R_1$ bzw. $R_2$ kann auch durch Umsetzung mit Ammoniak oder einem mindestens ein Wasserstoffatom aufweisenden Amin und Dehydratisierung des intermediär gebildeten Ammoniumsalzes, z.B. durch Erhitzen oder mittels eines Dehydratisierungsmittels, wie N,N'-Dicyclohexylcarbodiimid, oder durch Ueberführung in das Halogenid und anschliessende Umsetzung mit Ammoniak oder einem mindestens ein Wasserstoffatom aufweisenden Amin in amidiertes Carboxy $R_1$ bzw. $R_2$ überführt werden.

Ferner kann man gegebenenfalls vorhandene Hydroxygruppen verestern, z.B. durch Behandeln mit einem Niederalkancarbonsäureanhydrid bzw. -halogenid in Niederalkanoyloxy überführen, oder durch Umsetzung mit einem reaktionsfähigen Ester, insbesondere Brom- oder Chlor-wasserstoffsäureester, eines Niederalkanols in entsprechendes veräthertes Hydroxy überführen. Umgekehrt kann man aus verestertem oder veräthertem Hydroxy, wie Niederalkanoyloxy oder Niederalkoxy, die Hydroxygruppe(n) solvolytisch freisetzen, vorzugsweise unter sauren Bedingungen. In analoger Weise kann man auch veräthertes oder acyliertes Hydroxy $R_2$ zu Hydroxy hydrolysieren.

Entsprechend kann man weiterhin Hydroxymethyl $R_1$ bzw. $R_2$ verestern, z.B. durch Behandeln mit einem Niederalkancarbonsäureanhydrid bzw. -halogenid in Niederalkanoyloxymethyl $R_1$ bzw. $R_2$ überführen. Umgekehrt kann man aus acyliertem Hydroxymethyl, z.B. Niederalkanoyloxymethyl, $R_1$ bzw. $R_2$ die Hydroxygruppe solvolytisch freisetzen, vorzugsweise unter sauren Bedingungen.

Weiterhin lässt sich Hydroxymethyl $R_1$ bzw. $R_2$ in üblicher Weise in Niederalkoxycarbonyl, N-Niederalkylcarbamyl oder N,N-Diniederalkylcarbamyl $R_1$ bzw. $R_2$ oder in Carbamyl $R_1$ überführen, wobei z.B. in der Weise verfahren wird, dass zunächst Hydroxymethyl $R_1$ bzw. $R_2$ in üblicher Weise, z.B. in Gegenwart eines Oxidationsmittels, wie Kaliumpermanganat oder Kaliumdichromat, zu Carboxy oxidiert wird und anschliessend die Carboxygruppe in üblicher Weise, z.B. durch Behandlung mit einem entsprechenden Alkohol in Gegenwart einer Mineralsäure, z.B. von Schwefelsäure, oder durch Ueberführung in ein Halogenid und anschliessende Umsetzung mit einem entsprechenden Alkohol, z.B. in Gegenwart von Pyridin oder Triäthylamin, oder durch Ueberführung in ein Alkalimetallsalz und anschliessende Umsetzung

mit einem reaktionsfähigen Ester des entsprechenden Alkohols, wie einem entsprechenden Halogenid, oder unter Verwendung eines Dehydratisierungsmittels,wie N,N'-Dicyclohexylcarbodiimid, mit einem entsprechenden Alkohol in Niederalkoxycarbonyl $R_1$ bzw. $R_2$ oder durch Umsetzung mit Ammoniak oder einem mindestens ein Wasserstoffatom aufweisenden Amin und Dehydratisierung des intermediär gebildeten Ammoniumsalzes, z.B. durch Erhitzen oder mittels eines Dehydratisierungsmittels, wie N,N'-Dicyclohexylcarbodiimid, oder durch Ueberführung in das Halogenid und anschliessende Umsetzung mit Ammoniak oder einem mindestens ein Wasserstoffatom aufweisenden Amin in amidiertes Carboxy $R_1$ bzw. $R_2$ überführt wird. Ebenso kann man acyliertes Hydroxymethyl $R_1$ bzw. $R_2$ in verestertes oder amidiertes Carboxy $R_1$ bzw. $R_2$ überführen, indem man zunächst die acylierte Hydroxymethylgruppe solvolytisch freisetzt, z.B. wie vorstehend beschrieben, und dann die erhaltene freie Hydroxymethylgruppe, wie vorstehend erläutert, in eine Carboxylgruppe und letztere weiter in eine veresterte oder amidierte Carboxylgruppe überführt. Umgekehrt lassen sich veresterte oder amidierte Carboxygruppen $R_1$ bzw. $R_2$ in gegebenenfalls acyliertes Hydroxymethyl $R_1$ bzw. $R_2$ überführen, indem man zunächst die veresterte oder amidierte Carboxygruppe $R_1$ bzw. $R_2$ in üblicher Weise, beispielsweise in Gegenwart eines basischen oder sauren Hydrolysemittels, wie eines Alkalimetallhydroxides oder -carbonates, z.B. von Natriumhydroxid oder Kaliumcarbonat, oder einer Mineralsäure, z.B. von Salzsäure oder Schwefelsäure, zu Carboxy hydrolysiert und anschliessend die erhaltene Carboxygruppe in üblicher Weise, z.B. in Gegenwart eines Reduktionsmittels, beispielsweise der vorstehend erwähnten Art, zu Hydroxymethyl $R_1$ bzw. $R_2$ reduziert, wobei gewünschtenfalls letzteres, z.B. wie vorstehend beschrieben, anschliessend noch in acyliertes Hydroxymethyl $R_1$ bzw. $R_2$ überführt werden kann.

Bedeutet die gestrichelte Linie, dass in den erfindungsgemässen Verbindungen zwischen den Kohlenstoffatomen, welche die Reste $R_1$ und $R_2$ tragen, eine Doppelbindung vorliegt, kann diese z.B. in an sich bekannter Weise mit Hilfe eines Reduktionsmittels, z.B. der unter Variante f) aufgeführten Art, zu einer Einfachbindung hydriert werden.

Ferner kann eine erfindungsgemässe Verbindung, in der die gestrichelte Linie für eine Doppelbindung zwischen den Kohlenstoffatomen, welche die Reste $R_1$ und $R_2$ tragen, steht und $R_2$ Wasserstoff bedeutet, z.B. in an sich bekannter Weise durch Addition einer Verbindung $R_2$-H, in der $R_2$ eine gegebenenfalls verätherte oder acylierte Hydroxygruppe oder eine gegebenenfalls acylierte Aminogruppe darstellt, in eine entsprechende erfindungsgemässe Piperidinverbindung überführt werden. Die Addition wird dabei insbesondere in Gegenwart einer geeigneten Base, z.B. der unter Variante a) aufgeführten Art, durchgeführt.

Erfindungsgemässe Verbindungen, in denen die gestrichelte Linie für eine Einfachbindung zwischen den Kohlenstoffatomen, welche die Reste $R_1$ und $R_2$ tragen, steht, können umgekehrt z.B. durch Eliminierung einer Verbindung $R_2$-H, in der $R_2$ eine gegebenenfalls verätherte oder acylierte Hydroxygruppe oder eine gegebenenfalls acylierte Aminogruppe darstellt, in an sich bekannter Weise in entsprechende erfindungsgemässe Tetrahydro-Pyridin-Verbindungen, in denen $R_2$ Wasserstoff darstellt, überführt werden. Für eine Eliminierung schlechter geeignete Abgangsgruppen $R_2$, z.B. Hydroxy, können dabei zuvor, beispielsweise in situ, in besser geeignete Abgangsgruppen $R_2$, z.B. Niederalkansulfonyloxy, wie Methansulfonyloxy, oder Halogen, wie Chlor, Brom oder Iod, umgewandelt werden. Die Eliminierung erfolgt dabei insbesondere in Gegenwart einer geeigneten Base, z.B. der unter Variante a) aufgeführten Art.

Salze von Verbindungen der Formel I bzw. von deren Tautomeren können in an sich bekannter Weise hergestellt werden. So erhält man beispielsweise Säureadditionssalze von Verbindungen der Formel I durch Behandeln mit einer Säure oder einem geeigneten Ionenaustauscherreagens. Salze können in üblicher Weise in die freien Verbindungen der Formel I überführt werden Säureadditionssalze z.B. durch Behandeln mit einem geeigneten basischen Mittel.

Je nach Verfahrensweise bzw. Reaktionsbedingungen können die erfindungsgemässen Verbindungen der Formel I mit salzbildenden, insbesondere basischen Eigenschaften, in freier Form oder in Form von Salzen erhalten werden.

Infolge der engen Beziehung zwischen der neuen Verbindung der Formel I in freier Form und in Form ihrer Salze sind im vorausgegangenen und nachfolgend unter der freien Verbindung der Formel I oder ihren Salzen sinn- und zweckgemäss gegebenenfalls auch die entsprechenden Salze bzw. die freie Verbindung der Formel I zu verstehen.

Die neuen Verbindungen der Formel I einschliesslich ihrer Salze von salzbildenden Verbindungen können auch in Form ihrer Hydrate erhalten werden oder andere, beispielsweise zur Kristallisation von in fester Form vorliegenden Verbindungen verwendete, Lösungsmittel einschliessen.

Die neuen Verbindungen der Formel I können, je nach Wahl der Ausgangsstoffs und Arbeitsweisen, in Form eines der möglichen Isomeren oder als Gemisch derselben vorliegen. Dabei sind, in Abhängigkeit von der Molekülsymmetrie, z.B. je nach Anzahl, absoluter und relativer Konfiguration der Chiralitätszentren, wie asymmetrischen C-Atome, als reine Isomere z.B. reine Enantiomere und/oder reine Diastereomere, wie

reine cis/trans-Isomere oder meso-Verbindungen, erhältlich. Entsprechend können als Isomerengemische z.B. Enantiomerengemische, wie Racemate, Diastereomerengemische oder Racematgemische vorliegen.

Erhaltene Diastereomerengemische und Racematgemische können aufgrund der physikalisch-chemischen Unterschiede der Bestandteile in bekannter Weise in die reinen Diastereomeren oder Racemate aufgetrennt werden, beispielsweise durch fraktionierte Kristallisation.

Erhaltene Enantiomerengemische, wie Racemate, lassen sich nach bekannten Methoden in die Enantiomeren zerlegen, beispielsweise durch Umkristallisation aus einem optisch aktiven Lösungsmittel, Chromatographie an chiralen Adsorbentien, mit Hilfe von geeigneten Mikroorganismen, durch Spaltung mit spezifischen, immobilisierten Enzymen, über die Bildung von Einschlussverbindungen, z.B. unter Verwendung chiraler Kronenäther, wobei nur ein Enantiomeres komplexiert wird, oder durch Ueberführung in diastereomere Salze z.B. durch Umsetzung eines basischen Endstoffracemats mit einer optisch aktiven Säure, wie Carbonsäure, z.B. Wein- oder Aepfelsäure, oder Sulfonsäure, z.B. Camphersulfonsäure, und Trennung des auf diese Weise erhaltenen Diastereomerengemisches, z.B. auf Grund ihrer verschiedenen Löslichkeiten, in die Diastereomeren, aus denen das gewünschte Enantiomere durch Einwirkung geeigneter Mittel freigesetzt werden kann. Vorteilhaft isoliert man das wirksamere Enantiomere.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Derivates bzw. Salzes und/oder seiner Racemate bzw. Enantiomeren verwendet oder insbesondere unter den Reaktionsbedingungen bildet.

Beim Verfahren der vorliegenden Erfindung werden vorzugsweise solche Ausgangsstoffe verwendet, welche zu den eingangs als besonders wertvoll geschilderten Verbindungen der Formel I führen. Neue Ausgangsstoffe, die speziell für die Herstellung der erfindungsgemässen Verbindungen der Formel I entwickelt wurden, ihre Verwendung und Verfahren zu ihrer Herstellung bilden ebenfalls einen Gegenstand der Erfindung, wobei die Variablen $R_1$, $R_2$, $R_3$, X, Y, n und alk sowie die Substituenten des Ringes A und die gestrichelte linie die für die jeweils bevorzugten Verbindungsgruppen der Formel I angegebenen Bedeutungen haben.

In diesem Zusammenhang sind insbesondere Verbindungen der Formel

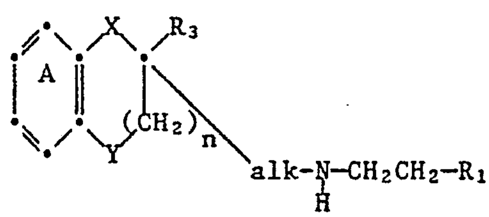

(IVc),

und deren Salze zu erwähnen. Diese besitzen ebenfalls nootrope Eigenschaften in vergleichbarer Wirkungsstärke wie die entsprechenden Verbindungen der Formel I bzw. I' und können ebenfalls als nootrope Arzneimittelwirkstoffe Verwendung finden.

Die Erfindung betrifft dementsprechend ebenfalls pharmazeutische, insbesondere nootrope, Präparate, als Wirkstoff enthaltend eine Verbindung der Formel IVc, worin $R_1$ Carboxy, Niederalkoxycarbonyl, Carbamyl, N-Niederalkylcarbamyl, N,N-Diniederalkylcarbamyl, Hydroxymethyl, $C_2$-$C_5$-Alkanoyloxymethyl, Niederalkansulfonyloxymethyl, Benzoyloxymethyl oder Pyridoyloxymethyl bedeutet, $R_3$ Wasserstoff oder $C_1$-$C_7$-Alkyl darstellt, alk Niederalkylen oder Niederalkyliden bedeutet, der Ring A unsubstituiert oder ein- oder mehrfach durch Hydroxy, Niederalkoxy, $C_2$-$C_5$-Alkanoyloxy, Halogen mit einer Atomnummer bis und mit 53, $C_1$-$C_7$-Alkyl und/oder Trifluormethyl substituiert ist und entweder X und Y jeweils ein Sauerstoffatom bedeuten und n für 1 steht oder X eine Methylengruppe darstellt, Y ein Sauerstoffatom bedeutet und n für 1 steht oder X ein Sauerstoffatom darstellt, Y eine Methylengruppe bedeutet und n für 1 steht oder X für eine direkte Bindung steht, Y ein Sauerstoffatom darstellt und n für 2 steht, oder ein pharmazeutisch verwendbares Salz davon, die Verwendung der genannten Verbindungen der Formel IVc oder ihrer pharmazeutisch verwendbaren Salze zur Herstellung nootroper pharmazeutischer Präparate, ein Verfahren zur Behandlung cerebraler Insuffizienzerscheinungen, dadurch gekennzeichnet, dass man eine der genannten Verbindungen der Formel IVc oder ein pharmazeutisch verwendbares Salz davon verabreicht, sowie Verbindungen der Formel IVc, worin $R_1$ Carboxy, Niederalkoxycarbonyl, Carbamyl, N-Niederalkylcarbamyl, N,N-Diniederalkylcarbamyl,Hydroxymethyl, $C_2$-$C_5$-Alkanoyloxymethyl, Niederalkansulfonyloxymethyl, Benzoyloxymethyl oder Pyridoyloxymethyl bedeutet, $R_3$ Wasserstoff oder $C_1$-$C_7$-Alkyl darstellt, alk Niederalkylen oder Niederalkyliden bedeutet, der Ring A unsubstituiert oder ein- oder mehrfach durch Hydroxy, Niederalkoxy, $C_2$-$C_5$-

Alkanoyloxy, Halogen, mit einer Atomnummer bis und mit 53, $C_1$-$C_7$-Alkyl und/oder Trifluormethyl substituiert ist und entweder X und Y jeweils ein Sauerstoffatom bedeuten und n für 1 steht oder X eine Methylengruppe darstellt, Y ein Sauerstoffatom bedeutet und n für 1 steht oder X ein Sauerstoffatom darstellt, Y eine Methylengruppe bedeutet und n für 1 steht oder X für eine direkte Bindung steht, Y ein Sauerstoffatom darstellt und n für 2 steht, mit der Massgabe, dass in Verbindungen der Formel IVc, worin der Ring A unsubstituiert ist, X und Y jeweils Sauerstoff darstellen, n für 1 steht und $R_3$ Wasserstoff bedeutet, alk von Methylen verschieden ist, wenn $R_1$ Carbamyl oder N-Methyl-, N-Aethyl-, N,N-Dimethyl- oder N,N-Diäthylcarbamyl bedeutet, und ihre Salze sowie ein Verfahren zur Herstellung der letztgenannten, neuen Verbindungen der Formel IVc und ihrer Salze, wobei mit "Nieder" bezeichnete Reste 1 bis und mit 4 Kohlenstoffatome aufweisen können.

Die Erfindung betrifft dementsprechend beispielsweise ebenfalls pharmazeutische, insbesondere nootrope, Präparate, als Wirkstoff enthaltend eine Verbindung der Formel IVc, worin $R_1$ Carboxy, Niederalkoxycarbonyl, Carbamyl, N-Niederalkylcarbamyl oder N,N-Diniederalkylcarbamyl bedeutet, $R_3$ Wasserstoff oder $C_1$-$C_7$-Alkyl darstellt, alk Niederalkylen oder Niederalkyliden bedeutet, der Ring A unsubstituiert oder ein- oder mehrfach durch Hydroxy, Niederalkoxy, $C_2$-$C_5$-Alkanoyloxy, Halogen mit einer Atomnummer bis und mit 53, $C_1$-$C_7$-Alkyl und/oder Trifluormethyl substituiert ist, X ein Sauerstoffatom oder eine Methylengruppe darstellt, Y ein Sauerstoffatom bedeutet und n für 1 steht, oder ein pharmazeutisch verwendbares Salz davon, die Verwendung der genannten Verbindungen der Formel IVc oder ihrer pharmazeutisch verwendbaren Salze zur Herstellung nootroper pharmazeutischer Präparate, ein Verfahren zur Behandlung cerebraler Insuffizienzerscheinungen, dadurch gekennzeichnet, dass man eine der genannten Verbindungen der Formel IVc oder ein pharmazeutisch verwendbares Salz davon verabreicht, sowie Verbindungen der Formel IVc, worin $R_1$ Carboxy, Niederalkoxycarbonyl, Carbamyl, N-Niederalkylcarbamyl oder N,N-Diniedercarbamyl bedeutet, $R_3$ Wasserstoff oder $C_1$-$C_7$-Alkyl darstellt, alk Niederalkylen oder Niederalkyliden bedeutet, der Ring A unsubstituiert oder ein- oder mehrfach durch Hydroxy, Niederalkoxy, $C_2$-$C_5$-Alkanoyloxy, Halogen mit einer Atomnummer bis und mit 53, $C_1$-$C_7$-Alkyl und/oder Trifluormethyl substituiert ist, X ein Sauerstoffatom oder eine Methylengruppe darstellt, Y ein Sauerstoffatom bedeutet und n für 1 steht, mit der Massgabe, dass in Verbindungen der Formel IVc, worin der Ring A unsubstituiert ist, X und Y jeweils Sauerstoff darstellen, n für 1 steht und $R_3$ Wasserstoff bedeutet, alk von Methylen verschieden ist, wenn $R_1$ Carbamyl oder N-Methyl-, N-Aethyl-, N,N-Dimethyl- oder N,N-Diäthylcarbamyl bedeutet, und ihre Salze sowie ein Verfahren zur Herstellung der letztgenannten, neuen Verbindungen der Formel IVc und ihrer Salze, wobei mit "Nieder" bezeichnete Reste 1 bis und mit 4 Kohlenstoffatome aufweisen können.

Die Variablen der Formel IVc haben dabei beispielsweise die unter Formel I angegebenen bevorzugten Bedeutungen.

Die Erfindung betrifft diesbezüglich in erster Linie pharmazeutische, insbesondere nootrope, Präparate bzw. die Herstellung derselben und Behandlungsverfahren, dadurch gekennzeichnet, dass man eine Verbindung der Formel IVc, worin $R_1$ Carboxy, Niederalkoxycarbonyl, Carbamyl, N-Niederalkylcarbamyl, N,N-Diniederalkylcarbamyl, Hydroxymethyl, $C_2$-$C_5$-Alkanoyloxymethyl, Niederalkansulfonyloxymethyl, Benzoyloxymethyl oder Pyridoyloxymethyl bedeutet, $R_3$ Wasserstoff oder $C_1$-$C_7$-Alkyl darstellt, alk Niederalkylen, welches das Ringsystem mit der in Formel IVc eingezeichneten NH-Gruppe durch bis und mit 3 C-Atome verknüpft, oder Niederalkyliden darstellt, der Ring A unsubstituiert ist oder ein-, zwei-oder mehrfach durch Hydroxy, Niederalkoxy, $C_2$-$C_5$-Alkanoyloxy, Halogen mit einer Atomnummer bis und mit 53, $C_1$-$C_7$-Alkyl und/oder Trifluormethyl substituiert ist und entweder X und Y jeweils ein Sauerstoffatom bedeuten und n für 1 steht oder X eine Methylengruppe darstellt, Y ein Sauerstoffatom bedeutet und n für 1 steht oder X ein Sauerstoffatom darstellt, Y eine Methylengruppe bedeutet und n für 1 steht oder X für eine direkte Bindung steht, Y ein Sauerstoffatom darstellt und n für 2 steht, oder eines ihrer pharmazeutisch verwendbaren Salze wählt, sowie Verbindungen der Formel IVc, worin $R_1$ Carboxy, Niederalkoxycarbonyl, Carbamyl, N-Niederalkylcarbamyl, N,N-Diniederalkylcarbamyl, Hydroxymethyl, $C_2$-$C_5$-Alkanoyloxymethyl, Niederalkansulfonyloxymethyl, Benzoyloxymethyl oder Pyridoyloxymethyl bedeutet, $R_3$ Wasserstoff oder $C_1$-$C_7$-Alkyl darstellt, alk Niederalkylen, welches das Ringsystem mit der in Formel IVc eingezeichneten NH-Gruppe durch bis und mit 3 C-Atome verknüpft, oder Niederalkyliden darstellt, der Ring A unsubstituiert ist oder ein-, zwei-oder mehrfach durch Hydroxy, Niederalkoxy, $C_2$-$C_5$-Alkanoyloxy, Halogen mit einer Atomnummer bis und mit 53, $C_1$-$C_7$-Alkyl und/oder Trifluormethyl subtituiert ist und entweder X und Y jeweils ein Sauerstoffatom bedeuten und n für 1 steht oder X eine Methylengruppe darstellt, Y ein Sauerstoffatom bedeutet und n für 1 steht oder X ein Sauerstoffatom darstellt, Y eine Methylengruppe bedeutet und n für 1 steht oder X für eine direkte Bindung steht, Y ein Sauerstoffatom darstellt und n für 2 steht, mit der Massgabe, dass in Verbindungen der Formel IVc, worin der Ring A unsubstituiert ist, X und Y jeweils Sauerstoff darstellen, n für 1 steht und $R_3$ Wasserstoff bedeutet, alk von Methylen verschieden ist, wenn $R_1$ Carbamyl oder N-Methyl-, N-Aethyl-, N,N-Dimethyl- oder N,N-Diäthylcarbamyl bedeutet, und ihre Salze

sowie ein Verfahren zur Herstellung der letztgenannten, neuen Verbindungen der Formel IVc und ihrer Salze, wobei mit "Nieder" bezeichnete Reste 1 bis und mit 4 Kohlenstoffatome aufweisen können, beispielsweise pharmazeutische, insbesondere nootrope, Präparate bzw. die Herstellung derselben und Behandlungsverfahren, dadurch gekennzeichnet, dass man eine Verbindung der Formel IVc, worin $R_1$ Carboxy, Niederalkoxycarbonyl, Carbamyl, N-Niederalkylcarbamyl oder N,N-Diniederalkylcarbamyl bedeutet, $R_3$ Wasserstoff oder $C_1$-$C_7$-Alkyl darstellt, alk Niederalkylen, welches das Ringsystem mit der in Formel IVc eingezeichneten NH-Gruppe durch bis und mit 3 C-Atome verknüpft, oder Niederalkyliden darstellt, der Ring A unsubstituiert ist oder ein-, zwei- oder mehrfach durch Hydroxy, Niederalkoxy, $C_2$-$C_5$-Alkanoyloxy, Halogen mit einer Atomnummer bis und mit 53, $C_1$-$C_7$-Alkyl und/oder Trifluormethyl substituiert ist, X ein Sauerstoffatom oder eine Methylengruppe darstellt, Y ein Sauerstoffatom bedeutet und n für 1 steht, oder eines ihrer pharmazeutisch verwendbaren Salze wählt, sowie Verbindungen der Formel IVc, worin $R_1$ Carboxy, Niederalkoxycarbonyl, Carbamyl, N-Niederalkylcarbamyl oder N,N-Diniederalkylcarbamyl bedeutet, $R_3$ Wasserstoff oder $C_1$-$C_7$-Alkyl darstellt, alk Niederalkylen, welches das Ringsystem mit der in Formel IVc eingezeichneten NH-Gruppe durch bis und mit 3 C-Atome verknüpft, oder Niederalkyliden darstellt, der Ring A unsubstituiert ist oder ein-, zwei-oder mehrfach durch Hydroxy, Niederalkoxy, $C_2$-$C_5$-Alkanoyloxy, Halogen mit einer Atomnummer bis und mit 53, $C_1$-$C_7$-Alkyl und/oder Trifluormethyl substituiert ist, X ein Sauerstoffatom oder eine Methylengruppe darstellt, Y ein Sauerstoffatom bedeutet und n für 1 steht, mit der Massgabe, dass in Verbindungen der Formel IVc, worin der Ring A unsubstituiert ist, X und Y jeweils Sauerstoff darstellen, n für 1 steht und $R_3$ Wasserstoff bedeutet, alk von Methylen verschieden ist, wenn $R_1$ Carbamyl oder N-Methyl-, N-Aethyl-, N,N-Dimethyl- oder N,N-Diäthylcarbamyl bedeutet, und ihre Salze sowie ein Verfahren zur Herstellung der letztgenannten, neuen Verbindungen der Formel IVc und ihrer Salze, wobei mit "Nieder" bezeichnete Reste 1 bis und mit 4 Kohlenstoffatome aufweisen können.

Die Erfindung betrifft diesbezüglich vor allem pharmazeutische, insbesondere nootrope, Präparate bzw. die Herstellung derselben und Behandlungsverfahren, dadurch gekennzeichnet, dass man eine Verbindung der Formel IVc, worin $R_1$ Carboxy, Hydroxymethyl, $C_2$-$C_5$-Alkanoyloxymethyl, wie Acetoxymethyl, $C_1$-$C_4$-Alkoxycarbonyl, wie Methoxycarbonyl, oder Carbamyl bedeutet, $R_3$ Wasserstoff oder $C_1$-$C_4$-Alkyl, wie Methyl, ist, alk für $C_1$-$C_4$-Alkylen, welches das Ringsystem mit der in Formel IVc eingezeichneten NH-Gruppe durch bis und mit 3 C-Atome verknüpft, wie Methylen oder Aethylen, steht, der Ring A unsubstituiert oder, insbesondere in 7-Stellung, durch $C_1$-$C_4$-Alkoxy, wie Methoxy, substituiert ist und entweder X und Y jeweils ein Sauerstoffatom bedeuten und n für 1 steht oder X eine Methylengruppe darstellt, Y ein Sauerstoffatom bedeutet und n für 1 steht oder X ein Sauerstoffatom darstellt, Y eine Methylengruppe bedeutet und n für 1 steht oder X für eine direkte Bindung steht, Y ein Sauerstoffatom darstellt und n für 2 steht, oder eines ihrer pharmazeutisch verwendbaren Salze wählt, sowie Verbindungen der Formel IVc, worin $R_1$ Carboxy, Hydroxymethyl, $C_2$-$C_5$-Alkanoyloxymethyl, wie Acetoxymethyl, $C_1$-$C_4$-Alkoxycarbonyl, wie Methoxycarbonyl, oder Carbamyl bedeutet, $R_3$ Wasserstoff oder $C_1$-$C_4$-Alkyl, wie Methyl, ist, alk für $C_1$-$C_4$-Alkylen, welches das Ringsystem mit der in Formel IVc eingezeichneten NH-Gruppe durch bis und mit 3 C-Atome verknüpft, wie Methylen oder Aethylen, steht, der Ring A unsubstituiert oder, insbesondere in 7-Stellung, durch $C_1$-$C_4$-Alkoxy, wie Methoxy, substituiert ist und entweder X und Y jeweils ein Sauerstoffatom bedeuten und n für 1 steht oder X eine Methylengruppe darstellt, Y ein Sauerstoffatom bedeutet und n für 1 steht oder X ein Sauerstoffatom darstellt, Y eine Methylengruppe bedeutet und n für 1 steht oder X für eine direkte Bindung steht, Y ein Sauerstoffatom darstellt und n für 2 steht, mit der Massgabe, dass in Verbindungen der Formel IVc, worin der Ring A unsubstituiert ist, X und Y jeweils Sauerstoff darstellen, n für 1 steht und $R_3$ Wasserstoff bedeutet, alk von Methylen verschieden ist, wenn $R_1$ Carbamyl bedeutet, und ihre Salze sowie ein Verfahren zur Herstellung der letztgenannten, neuen Verbindungen der Formel IVc und ihrer Salze, beispielsweise pharmazeutische, insbesondere nootrope, Präparate bzw. die Herstellung derselben und Behandlungsverfahren, dadurch gekennzeichnet, dass man eine Verbindung der Formel IVc, worin $R_1$ $C_1$-$C_4$-Alkoxycarbonyl, wie Methoxycarbonyl, bedeutet, $R_3$ Wasserstoff oder $C_1$-$C_4$-Alkyl, wie Methyl, ist, alk für $C_1$-$C_4$-Alkylen, welches das Ringsystem mit der in Formel IVc eingezeichneten NH-Gruppe durch bis und mit 3 C-Atome verknüpft, wie Methylen oder Aethylen, steht, der Ring A unsubstituiert ist, X ein Sauerstoffatom oder eine Methylengruppe darstellt, Y ein Sauerstoffatom bedeutet und n für 1 steht, oder eines ihrer pharmazeutisch verwendbaren Salze wählt, sowie Verbindungen der Formel IVc, worin $R_1$ $C_1$-$C_4$-Alkoxycarbonyl, wie Methoxycarbonyl, bedeutet, $R_3$ Wasserstoff oder $C_1$-$C_4$-Alkyl, wie Methyl, ist, alk für $C_1$-$C_4$-Alkylen, welches das Ringsystem mit der in Formel IVc eingezeichneten NH-Gruppe durch bis und mit 3 C-Atome verknüpft, wie Methylen oder Aethylen, steht, der Ring A unsubstituiert ist, X ein Sauerstoffatom oder eine Methylengruppe darstellt, Y ein Sauerstoffatom bedeutet und n für 1 steht, und ihre Salze sowie ein Verfahren zur Herstellung der letztgenannten, neuen Verbindungen der Formel IVc und ihrer Salze.

Die Erfindung betrifft diesbezüglich in allererster Linie pharmazeutische, insbesondere nootrope, Präparate bzw. die Herstellung derselben und Behandlungsverfahren, dadurch gekennzeichnet, dass man eine Verbindung der Formel IVc, worin $R_1$ $C_1$-$C_4$-Alkoxycarbonyl, wie Methoxycarbonyl, bedeutet, $R_3$ Wasserstoff ist, alk für Methylen oder Aethylen steht, der Ring A unsubstituiert ist, X ein Sauerstoffatom oder eine Methylengruppe darstellt, Y ein Sauerstoffatom bedeutet und n für 1 steht, oder eines ihrer pharmazeutisch verwendbaren Salze wählt, sowie Verbindungen der Formel IVc, worin $R_1$ $C_1$-$C_4$-Alkoxycarbonyl, wie Methoxycarbonyl, bedeutet, $R_3$ Wasserstoff ist, alk für Methylen oder Aethylen steht, der Ring A unsubstituiert ist, X ein Sauerstoffatom oder eine Methylengruppe darstellt, Y ein Sauerstoffatom bedeutet und n für 1 steht, und ihre Salze sowie ein Verfahren zur Herstellung der letztgenannten, neuen Verbindungen der Formel IVc und ihrer Salze.

Die Erfindung betrifft diesbezüglich namentlich pharmazeutische, insbesondere nootrope, Präparate bzw. die Herstellung derselben und Behandlungsverfahren, dadurch gekennzeichnet, dass man eine der in den Beispielen genannten neuen Verbindungen der Formel IVc oder eines ihrer pharmazeutisch verwendbaren Salze wählt, sowie die in den Beispielen genannten neuen Verbindungen der Formel IVc und ihre Salze sowie Verfahren zur Herstellung der letztgenannten, neuen Verbindungen der Formel IVc und ihrer Salze.

Ebenfalls Gegenstand der vorliegenden Erfindung ist ein Verfahren zur Herstellung von Verbindungen der Formel IVc und ihrer Salze, z.B. dadurch gekennzeichnet, dass man

**h) Verbindungen der Formeln**

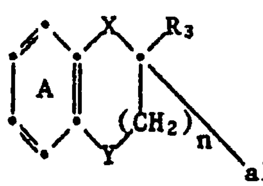 (VIIIa) und $Z_2$–$CH_2$–$CH(Z_3)$–$R_1$ (VIIIb),

Die Umsetzung wird in diesem Fall insbesondere in Gegenwart eines Kondensationsmittels, wie einer geeigneten Base, durchgeführt. Als Basen kommen beispielsweise Alkalimetallhydroxide, -hydride, -amide, -alkanolate, -carbonate, -triphenylmethylide, -diniederalkylamide, -aminoniederalkylamide oder -niederalkylsilylamide, Naphthalinamine, Niederalkylamine, basische Heterocyclen, Ammoniumhydroxide sowie carbocyclische Amine in Frage. Beispielhaft seien Natriumhydroxid, -hydrid, -amid, -äthylat, Kalium-tert-butylat, -carbonat, Lithiumtriphenylmethylid, Lithium-diisopropylamid, Kalium-3-(aminopropyl)-amid, -bis-(trimethylsilyl)-amid, Dimethylaminonaphthalin, Di- oder Triäthylamin, Pyridin, Benzyl-trimethyl-ammoniumhydroxid, 1,5-Diaza-bicyclo[4.3.0]non-5-en (DBN) sowie 1,5-Diaza-bicyclo[5.4.0]undec-5-en (DBU) genannt. Die Umsetzung von Aminen VIIIa ($Z_1$ = Amino) mit Acrylsäureverbindungen VIIIb ($Z_2$ + $Z_3$ = Bindung) erfolgt beispielsweise unter Erwärmen, z.B. auf etwa 60-120 °C.

Das Ausgangsmaterial der Formeln VIIIa und VIIIb ist bekannt oder kann in Analogie zu bekannten Methoden hergestellt werden.

Variante i):

Ein in $R_1$ überführbarer Rest $X_6$ ist beispielsweise von $R_1$ verschiedenes funktionell abgewandeltes Carboxy, wie Cyano, anhydridisiertes Carboxy, gegebenenfalls substituiertes Amidino, gegebenenfalls verestertes oder anhydridisiertes Carboximidoyl, vcn verestertem oder amidiertem Carboxy $R_1$ verschiedenes verestertes oder amidiertes Carboxy, Triniederalkoxy- oder Trihalogenmethyl.

Anhydridisiertes Carboxy ist beispielsweise mit einer Mineralsäure, wie Halogenwasserstoffsäure, oder mit einer Carbonsäure, wie einer gegebenenfalls substituierten Niederalkan- bzw. Benzoesäure oder einem Kohlensäurehalogenid-niederalkylhalbester anhydridisiertes Carboxy. Als Beispiele seien Halogencarbonyl, wie Chlorcarbonyl, Niederalkanoyloxycarbonyl, wie Acetyloxycarbonyl, oder Niederalkoxycarbonyloxycarbonyl, wie Aethoxycarbonyloxycarbonyl, genannt.

Substituiertes Amidino ist beispielsweise mit einem aliphatischen Rest, z.B. Niederalkyl, substituiertes Amidino, wie Niederalkylamidino, z.B. Aethylamidino.

Unter verestertem oder anhydridisiertem Carboximidoyl ist z.B. Alkoxy- bzw. Halogencarboximidoyl, beispielsweise Niederalkoxy-, wie Aethoxy-, bzw. Chlorcarboximidoyl, zu verstehen.

Triniederalkoxy- bzw. Trihalogenmethyl ist z.B. Trimethoxymethyl bzw. Trichlormethyl.

$X_6$ kann beispielsweise durch Solvolyse in $R_1$ überführt werden. Solvolysemittel sind beispielsweise Wasser, dem gewünschten veresterten Carboxy $R_1$ entsprechende Niederalkanole, Ammoniak oder der

gewünschten amidierten Carboxygruppe $R_1$ entsprechende Amine. Die Behandlung mit einem entsprechenden Solvolysemittel wird gegebenenfalls in Gegenwart einer Säure oder Base durchgeführt. Als Säuren kommen beispielsweise anorganische oder organische Protonsäuren, wie Mineralsäuren, z.B. Schwefelsäure oder Halogenwasserstoffsäuren, beispielsweise Chlorwasserstoffsäure, wie Sulfonsäuren, z.B. Niederalkan- oder gegebenenfalls substituierte Benzolsulfonsäure, beispielsweise Methan- oder p-Toluolsulfonsäure, oder wie Carbonsäuren, z.B. Niederalkancarbonsäuren, beispielsweise Essigsäure, in Frage, während als Basen beispielsweise die unter Variante h) genannten verwendet werden können, insbesondere Natrium- oder Kaliumhydroxid.

Bei der Solvolyse mit Wasser (Hydrolyse) wird die Cyanogruppe, anhydridisiertes Carboxy, gegebenenfalls substituiertes Amidino, gegebenenfalls verestertes oder anhydridisiertes Carboximidoyl, von verestertem oder amidiertem Carboxy $R_1$ verschiedenes verestertes oder amidiertes Carboxy, Triniederalkoxy- oder Trihalogenmethyl zu Carboxy hydrolysiert. Dabei können gegebenenfalls am Ring A befindliche Niederalkanoyloxyreste im Verlauf der Hydrolyse zu Hydroxy hydrolysiert werden.

Cyano, anhydridisiertes Carboxy, von verestertem oder amidiertem Carboxy $R_1$ verschiedenes verestertes oder amidiertes Carboxy werden beispielsweise mit einem geeigneten Niederalkanol zu verestertem Carboxy $R_1$ alkoholysiert und Cyano und anhydridisiertes Carboxy beispielsweise mit Ammoniak oder einem dem amidierten Carboxy $R_1$ entsprechenden Amin ammono- bzw. aminolysiert.

Das Ausgangsmaterial der Formel IX kann z.B. durch Umsetzung von Verbindungen der Formeln

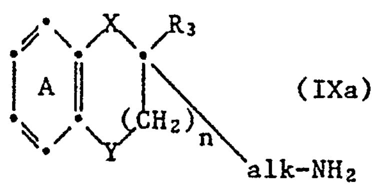 (IXa) und $Z_2-CH_2-CH(Z_3)-X_6$ (IXb),

worin $Z_2$ reaktionsfähiges verestertes Hydroxy ist und $Z_3$ Wasserstoff darstellt oder $Z_2$ und $Z_3$ gemeinsam für eine zusätzliche Bindung stehen, oder gegebenenfalls Salzen dieser Verbindungen erhalten werden, wobei in analoger Weise wie unter der Verfahrensvariante c) beschrieben, beispielsweise in Gegenwart eines basischen Mittels, gearbeitet wird.

An verfahrensgemäss erhältlichen Verbindungen IVc gewünschtenfalls vorzunehmende Nachoperationen sind insbesondere analoge Umwandlungsreaktionen von $R_1$ und von Substituenten des Ringes A, Enantiomeren-sowie Diastereomerentrennungen und gegenseitige Umwandlungen von Salzen und freien Verbindungen wie für die Verbindungen der Formel I angegeben, die auch analog durchgeführt werden.

Die Erfindung betrifft ebenfalls die Verwendung von Verbindungen der Formel I bzw. IVc und gegebenenfalls deren Tautomeren und/oder von pharmazeutisch verwendbaren Salzen von solchen Verbindungen mit salzbildenden Eigenschaften, insbesondere als pharmakologische, in erster Linie nootrop wirksame, Wirksubstanzen. Dabei kann man sie, vorzugsweise in Form von pharmazeutisch verwendbaren Zubereitungen, in einem Verfahren zur prophylaktischen und/oder therapeutischen Behandlung des tierischen oder menschlichen Körpers, insbesondere als Nootropika, z.B. zur Behandlung von cerebralen Insuffizienzerscheinungen, insbesondere Gedächtnisstörungen, anwenden.

Die Erfindung betrifft gleichfalls pharmazeutische Präparate, die eine Verbindung der Formel I bzw. IVc oder gegebenenfalls ein Tautomeres und/oder pharmazeutisch verwendbares Salz davon als Wirkstoff enthalten, sowie Verfahren zu ihrer Herstellung.

Bei den erfindungsgemässen pharmazeutischen Präparaten, welche eine Verbindung der Formel I bzw. IVc oder gegebenenfalls ein Tautomeres und/oder pharmazeutisch verwendbares Salz davon enthalten, handelt es sich um solche zur enteralen, wie oralen, ferner rektalen, und parenteralen Verabreichung an Warmblüter, wobei der pharmakologische Wirkstoff allein oder zusammen mit üblichen pharmazeutischen Hilfsstoffen enthalten ist.

Die neuen pharmazeutischen Präparate enthalten z.B. von etwa 10 % bis etwa 80 %, vorzugsweise von etwa 20 % bis etwa 60 %, des Wirkstoffs. Erfindungsgemässe pharmazeutische Präparate zur enteralen bzw. parenteralen Verabreichung sind z.B. solche in Dosiseinheitsformen, wie Dragées, Tabletten, Kapseln oder Suppositorien, ferner Ampullen. Diese werden in an sich bekannter Weise, z.B. mittels konventioneller Misch-, Granulier-, Dragier-, Lösungs-oder Lyophilisierungsverfahren hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphat, z.B. Tricalciumphosphat oder Calciumhydrogenphosphat, ferner Bindemittel, wie Stärkekleister, unter Verwendung z.B. von Mais-, Weizen-, Reis- oder Kartoffelstärke, Gelatine, Tragaganth, Methylcellulose und/oder Polyvinylpyrrolidon, wenn erwünscht, Sprengmittel, wie die obengenannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier- und Schmiermitel, z.B. Kieselsäure, Talk, Stearinsäure oder Salze davon, wie Magnesium- oder Calciumstearat, und/oder Polyäthylenglykol. Dragée-Kerne werden mit geeigneten, gegebenenfalls Magensaft-resistenten Ueberzügen versehen, wobei man u.a. konzentrierte Zuckerlösungen, welche gegebenfalls arabischen Gummi, Talk, Polyvinylpyrrolidon, Polyäthylenglykol und/oder Titandioxid enthalten, Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbitol. Die Steckkapseln können den Wirkstoff in Form eines Granulats, z.B. im Gemisch mit Füllstoffen, wie Lactose, Bindemitteln, wie Stärken, und/oder Gleitmitteln, wie Talk oder Magnesiumstearat, und gegebenenfalls von Stabilisatoren, enthalten. In weichen Kapseln ist der Wirkstoff vorzugsweise in geeigneten Flüssigkeiten, wie fetten Oelen, Paraffinöl oder flüssigen Polyäthylenglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyäthylenglykole oder höhere Alkanole. Ferner können auch Gelatine-Rektalkapseln verwendet werden, die eine Kombination des Wirkstoffs mit einem Grundmassenstoff enthalten. Als Grundmassenstoffe kommen z.B. flüssige Triglyceride, Polyäthylenglykole oder Paraffinkohlenwasserstoffe in Frage.

Zur parenteralen Verabreichung eignen sich in erster Linie wässrige Lösungen eines Wirkstoffs in wasserlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Oele, z.B. Sesamöl, oder synthetische Fettsäureester, z.B. Aethyloleat oder Triglyceride, verwendet oder wässerige Injektionssuspensionen, welche viskositätserhöhende Stoffe, z.B. Natrium-carboxymethylcellulose, Sorbit und/oder Dextran, und gegebenenfalls auch Stabilisatoren enthalten.

Die Dosierung des Wirkstoffs kann von verschiedenen Faktoren, wie Applikationsweise, Warmblüter-Spezies, Alter und/oder individuellem Zustand abhängen. Im Normalfall ist für einen etwa 75 kg schweren Warmblüter bei oraler Applikation eine ungefähre Tagesdosis von etwa 20 bis etwa 500 mg, insbesondere von etwa 25 bis etwa 250 mg, vorteilhaft in mehreren gleichen Teildosen, zu veranschlagen.

Die nachfolgenden Beispiele illustrieren die oben beschriebene Erfindung; sie sollen jedoch diese in ihrem Umfang in keiner Weise einschränken. Temperaturen sind in Celsiusgraden angegeben.

Infolge der engen Beziehung zwischen einer Verbindung der Formel I und der entsprechenden tautomeren Verbindung der Formel I' ist in den Beispielen unter einer Verbindung der Formel I sinn- und zweckgemäss gegebenenfalls auch die tautomere Verbindung der Formel I' zu verstehen. Analoges gilt für eine Verbindung der Formel I' sowie für Salze von Verbindungen der Formeln I und I'.

Beispiel 1:

Eine Lösung von 7,96 g (25 mmol) 3-(p-Toluolsulfonyloxymethyl)chroman in 100 ml Dimethylformamid wird zuerst mit 5,55 g (25 mmol) 1,2,5,6-Tetrahydro-pyridin-3-carbonsäuremethylesterhydrobromid (Guvacolin-hydrobromid) und anschliessend mit 11,31 g (87,5 mmol) N-Aethyl-N,N-diisopropyl-amin versetzt. Das Gemisch wird 15 Stunden bei 50° gerührt und anschliessend im Hochvakuum eingedampft. Der Rückstand wird mit Wasser versetzt und mit Diäthyläther extrahiert. Die organischen Phasen werden mit Wasser gewaschen und mit 2H-Salzsäure ausgezogen. Die Salzsäure-Extrakte werden vereinigt, kalt mit Natronlauge (30 %) alkalisch gestellt und mit Dichlormethan extrahiert. Die vereinigten organischen Phasen werden über Natriumsulfat getrocknet und im Vakuum eingedampft. Man erhält 5,92 g (83 %) 1-(Chroman-3-ylmethyl)-1,2,5,6-tetrahydro-pyridin-3-carbonsäuremethylester als hellgelbes Oel. Das hieraus mit Salzsäure in Diäthyläther hergestellte 1-(Chroman-3-ylmethyl)-1,2,5,6-tetrahydro-pyridin-3-carbonsäuremethylester-hydrochlorid schmilzt nach Kristallisation aus Methanol/Diäthyläther bei 158-159°.

3-(p-Toluolsulfonyloxymethyl)chroman kann z.B. wie folgt hergestellt werden:
Zu einer Suspension von 9,86 g (260 mmol) Lithiumaluminiumhydrid in 300 ml absolutem Diäthyläther wird innert 40 Minuten unter Rühren bei Raumtemperatur eine Lösung von 50,0 g (260 mmol) 3-Methoxycarbonylchroman (US-4,178,380) in 200 ml absolutem Tetrahydrofuran zugetropft. Nach 16 Stunden Rühren bei Raumtemperatur wird das Reaktionsgemisch mit 9,9 ml Wasser, 9,9 ml Natronlauge (15 %) und 30 ml Wasser zusetzt. Der ausgefallene Niederschlag wird abgesaugt und das Filtrat im Vakuum zur Trockene eingedampft. Der ölige Rückstand wird in Diäthyläther gelöst und die Lösung mit Wasser gewaschen, über Natriumsulfat getrocknet und zur Trockne eingedampft. Man erhält 36,71 g (86 %) öliges 3-Hydroxymethylchroman, das aus Diäthyläther/Pentan kristallisiert und bei 60-61° schmilzt.

Eine Lösung von 36,12 g (220 mmol) 3-Hydroxymethylchroman in 100 ml absolutem Pyridin wird unter Rühren bei Raumtemperatur mit 46,14 g (242 mmol) p-Toluolsulfonsäurechlorid versetzt, wobei die leicht exotherme Reaktion mit einem Eisbad bei Raumtemperatur gehalten wird. Das Reaktionsgemisch wird 3 Stunden bei Raumtemperatur nachgerührt und dann auf Eiswasser gegossen. Die ausgefallenen Kristalle werden abgesaugt, mit Wasser gewaschen und im Vakuum getrocknet. Man erhält 65,84 g (94 %) 3-(p-Toluolsulfonyloxymethyl)chroman vom Smp. 86-87°.

Beispiel 2:

In eine Lösung von 4,36 g (13 mmol) N,N-bis(2-methoxycarbonyläthyl)-N-(chroman-3-ylmethyl)-amin in 50 ml absolutem Dimethylformamid werden bei Raumtemperatur unter Rühren innert 30 Minuten 0,75 g (15,6 mmol) Natriumhydrid-Dispersion in Mineralöl (50 %) eingetragen. Das Reaktionsgemisch wird 1 Stunde bei Raumtemperatur nachgerührt und dann im Hochvakuum eingedampft. Der erhaltene Rückstand wird mit Diäthyläther versetzt und mit kalter 2N-Salzsäure extrahiert. Die vereinigten salzsauren Extrakte werden mit Dichlormethan ausgeschüttelt und die vereinigten Dichlormethan-Extrakte über Natriumsulfat getrocknet und eingedampft. Man erhält 3,8 g (85,8 %) kristallines 4-Hydroxy-1-(chroman-3-yl-methyl)-1,2,5,6-tetrahydro-pyridin-3-carbonsäuremethylester-hydrochlorid bzw. 1-(Chroman-3-ylmethyl)-4-oxo-piperidin-3-carbonsäuremethylester-hydrochlorid, das nach Umkristallisation aus Methanol/Diäthyläther einen Zersetzungspunkt von 167-168° aufweist.

N,N-Bis(2-methoxycarbonyläthyl)-N-(chroman-3-ylmethyl)-amin lässt sich z.B. auf folgende Weise herstellen:
Zu einer Suspension von 3,04 g (80 mmol) Lithiumaluminiumhydrid in 100 ml absolutem Diäthyläther werden zuerst unter Rühren bei Raumtemperatur 1,77 g (13,32 mmol) Aluminiumchlorid in 50 ml absolutem Diäthyläther zugetropft. Anschliessend werden 6,29 g (40 mmol) 3-Cyanochromen [R.C. Gupta et al., Ind.J.Chem 21B, 344 (1982)] in 50 ml absolutem Tetrahydrofuran innert 20 Minuten zugetropft. Das Reaktionsgemisch wird 16 Stunden unter Rückfluss gekocht. Nach dem Erkalten wird das Reaktionsgemisch vorsichtig mit 3,1 ml Wasser, 3,1 ml Natronlauge (15 %) und 9,3 ml Wasser zersetzt. Der ausgefallene Niederschlag wird abgesaugt, das Filtrat im Vakuum eingedampft und der ölige Rückstand in Diäthyläther gelöst. Die organische Phase wird mit Wasser gewaschen und mit 2N-Salzsäure extrahiert. Die vereinigten salzsauren Extrakte werden kalt mit Natronlauge (30 %) alkalisch gestellt und mit Dichlormethan ausgeschüttelt. Nach Trocknung der vereinigten organischen Phasen über Natriumsulfat und Einengen im Vakuum erhält man 3,5 g (53,6 %) 3-Aminomethylchroman als gelbes Oel. Das hieraus mit Salzsäure in Diäthyläther hergestellte 3-Aminomethylchroman-hydrochlorid wird aus Methanol/Diäthyläther umkristallisiert und schmilzt bei 218-219°.

Eine Lösung von 2,61 g (16 mmol) 3-Aminomethylchroman in 20 ml Methanol wird mit 3,03 g (35,2 mmol) Acrylsäuremethylester versetzt. Die Reaktionslösung wird 16 Stunden bei 50° gerührt und nach dem Erkalten im Vakuum eingedampft. Man erhält 5,1 g (95 %) N,N-Bis-(2-methoxycarbonyläthyl)-N-(chroman-3-ylmethyl)-amin in Form eines rötlichen Oels.

Beispiel 3:

Eine Lösung von 10,48 g (30 mmol) N-[2-(Chroman-3-yl)äthyl]-N,N-bis(2-methoxycarbonyläthyl)-amin in 35 ml absolutem Dimethylformamid wird bei Raumtemperatur unter Rühren zu einer Suspension von 2,16 g (40 mmol) Natriummethanolat in 25 ml Dimethylformamid innert 15 Minuten zugetropft. Das Reaktionsgemisch wird 16 Stunden bei Raumtemperatur gerührt und danach im Hochvakuum zur Trockne eingedampft. Der Rückstand wird mit Diäthyläther versetzt und mit kalter 2N-Salzsäure extrahiert. Die vereinigten salzsauren Extrakte werden mit Dichlormethan ausgeschüttelt und die Dichlormethan-Phasen über Natriumsulfat getrocknet und im Vakuum eingedampft. Man erhält 4,25 g (40 %) 1-[2-(Chroman-3-yl)äthyl]-4-hydroxy-1,2,5,6-tetrahydro-pyridin-3-carbonsäuremethylester-hydrochlorid bzw. 1-[2-(Chroman-3-yl)äthyl]-4-oxo-piperidin-3-carbonsäuremethylester-hydrochlorid, das nach Umkristallisation aus Methanol/Diäthyläther einen Zersetzungspunkt von 175-177° besitzt.

N-[2-(Chroman-3-yl)äthyl]-N,N-bis(2-methoxycarbonyläthyl)-amin kann z.B. auf folgende Weise hergestellt werden:
Eine Lösung von 55,72 g (175 mmol) 3-(p-Toluolsulfonyloxymethyl)chroman (Herstellung siehe Beispiel 1) in 300 ml Dimethylsulfoxid wird bei Raumtemperatur mit 12,53 g (192,5 mmol) Kaliumcyanid versetzt und unter Rühren auf 60° erwärmt. Nach 3 Stunden wird das Reaktionsgemisch mit Eiswasser versetzt, mit Diäthyläther extrahiert und gründlich mit Wasser gewaschen. Die vereinigten organischen Phasen werden über Natriumsulfat getrocknet und im Vakuum eingedampft. Man erhält 26,75 g (88,3 %) 3-Cyanomethylchroman in Form eines hellgelben Oels, das aus Diäthyläther/Pentan kristallisiert. Die Kristalle schmelzen bei 63°.

Zu einer Suspension von 7,59 g (200 mmol) Lithiumaluminiumhydrid in 300 ml absolutem Diäthyläther werden zuerst unter Rühren bei Raumtemperatur 4,44 g (33,3 mmol) Aluminiumchlorid in 150 ml absolutem Diäthyläther zugetropft. Anschliessend werden 17,32 g (100 mmol) 3-Cyanomethylchroman, gelöst in 200 ml Tetrahydrofuran, innert 15 Minuten zugetropft. Das Reaktionsgemisch wird 16 Stunden bei Raumtemperatur gerührt und danach mit 7,6 ml Wasser, 7,6 ml Natronlauge (15 %) und 22,8 ml Wasser zersetzt. Der ausgefallene Niederschlag wird abgesaugt und das Filtrat im Vakuum eingedampft. Der ölige Rückstand wird in Diäthyläther gelöst und mit Wasser gewaschen. Anschliessend wird die organische Phase mit 2N-Salzsäure ausgeschüttelt. Die vereinigten salzsauren Extrakte werden kalt mit Natronlauge (30 %) alkalisch gestellt und mit Dichlormethan extrahiert. Die vereinigten organischen Phasen werden über Natriumsulfat getrocknet und im Vakuum eingedampft. Man erhält 15,95 g (90 %) 3-(2-Aminoäthyl)chroman als farbloses Oel. Das hieraus mit Salzsäure in Diäthyläther hergestellte 3-(2-Aminoäthyl)chroman-hydrochlorid kristallisiert aus Methanol/Diäthyläther und weist einen Smp. von 244-245° auf.

Eine Lösung von 6,2 g (35 mmol) 3-(2-Aminoäthyl)chroman in 50 ml Methanol wird bei Raumtemperatur mit 6,63 g (77 mmol) Acrylsäuremethylester versetzt und 16 Stunden bei Raumtemperatur gerührt. Das Reaktionsgemisch wird anschliessend im Vakuum eingedampft und liefert 12,23 g (100 %) N-[2-(Chroman-3-yl)äthyl-N,N-bis(2-methoxycarbonyläthyl)-amin als rötliches Oel.

Beispiel 4:

In analoger Weise wie in Beispiel 3 beschrieben kann durch Umsetzung von 3-(2-Aminoäthyl)chroman mit 1 Aequivalent Acrylsäuremethylester das entsprechende N-[2-(Chroman-3-yl)äthyl]-N-(2-methoxycarbonyläthyl)-amin hergestellt werden, dessen Hydrochlorid bei 190-192° schmilzt.

Beispiel 5:

In analoger Weise wie in den Beispielen 3 und 4 beschrieben, kann man durch Umsetzung von 3-Aminomethylchroman mit 1 Aequivalent Acrylsäuremethylester das N-(2-Methoxycarbonyläthyl)-N-(chroman-3-ylmethyl)-amin bzw. dessen Hydrochlorid herstellen.

Beispiel 6:

Eine Lösung von 4,5 g (13,5 mmol) 3-[2-(p-Toluolsulfonyloxy)äthyl]chromanin 70 ml absolutem Dimethylformamid wird zuerst mit 3,3 g (14,8 mmol) 1,2,5,6-Tetrahydro-pyridin-3-carbonsäuremethylester-hydrobromid (Guvacolin-hydrobromid) und anschliessend mit 6,1 g (47,3 mmol) N-Aethyl-N,N-diisopropyl-amin versetzt. Das Gemisch wird 16 Stunden bei 60° gerührt und nach dem Erkalten im Hochvakuum eingedampft. Der ölige Rückstand wird mit Wasser versetzt und mit Diäthyläther extrahiert. Die vereinigten organischen Phasen werden mit 2N-Salzsäure extrahiert. Die vereinigten salzsauren Extrakte werden kalt mit Natronlauge (30 %) alkalisch gestellt und mit Diohlormethan extrahiert. Die vereinigten Dichlormethan-Phasen werden über Natriumsulfat getrocknet und im Vakuum eingedampft. Man erhält 3,97 g (97,7 %) 1-[2-(Chroman-3-yl)äthyl]-1,2,5,6-tetrahydro-pyridin-3-carbonsäuremethylester als gelbes Oel. Das hieraus mit Salzsäure in Diäthyläther hergestellte 1-[2-(Chroman-3-yl)äthyl]-1,2,5,6-tetrahydro-pyridin-3-carbonsäuremethylester-hydrochlorid kristallisiert aus Methanol/Diäthyläther und schmilzt bei 175-177°.

3-[2-(p-Toluolsulfonyloxy)äthyl]chroman kann man z.B. wie folgt herstellen:
Eine Lösung von 7,8 g (45 mmol) 3-Cyanomethylchroman in 150 ml Aethanol wird mit 50 ml 2N-Natronlauge versetzt und 16 Stunden unter Rückfluss gekocht. Nach dem Abkühlen wird das Reaktionsgemisch im Vakuum eingedampft. Der Rückstand wird in Wasser gelöst und mit Diäthyläther extrahiert. Die wässrige Phase wird mit Salzsäure (36 %) angesäuert und mit Dichlormethan ausgeschüttelt. Die vereinigten Dichlormethan-Extrakte werden über Natriumsulfat getrocknet und im Vakuum eingedampft. Man erhält 8,3 g (96 %) 3-Carboxymethylchroman in Form von farblosen Kristallen, die bei 106-107° schmelzen.

Eine Lösung von 7,69 g (40 mmol) 3-Carboxymethylchroman in 150 ml Methanol wird mit 1,5 ml Schwefelsäure (100 %) versetzt und 3 Stunden unter Rückfluss gekocht. Nach Erkalten wird das Reaktionsgemisch im Vakuum eingedampft. Der Rückstand wird in Diäthyläther gelöst und mit Wasser, Natriumhydrogencarbonat und nochmals Wasser kalt gewaschen. Die vereinigten organischen Phasen werden über Natriumsulfat getrocknet und im Vakuum eingedampft. Man erhält 8,08 g (98 %) 3-Methoxycarbonylmethylchroman als hellgelbes Oel.

Zu einer Suspension von 1,33 g (35 mmol) Lithiumaluminiumhydrid in 50 ml absolutem Diäthyläther wird bei Raumtemperatur eine Lösung von 7,22 g (35 mmol) 3-Methoxycarbonylmethylchroman in 50 ml absolutem Tetrahydrofuran innert 30 Minuten unter Rühren zugetropft. Es wird 16 Stunden bei Raumtemperatur nachgerührt und vorsichtig mit 1,33 ml Wasser, 1,33 ml Natronlauge (15 %) und 4,0 ml Wasser zersetzt. Der ausgefallene Niederschlag wird abgesaugt und das Filtrat im Vakuum eingedampft. Der ölige Rückstand wird in Diäthyläther gelöst. Die Lösung wird mit Wasser gewaschen, über Natriumsulfat getrocknet und im Vakuum eingedampft. Man erhält 6,23 g (100 %) 3-(2-Hydroxyäthyl)chroman als gelbes Oel.

Eine Lösung von 5,35 g (30 mmol) 3-(2-Hydroxyäthyl)chroman in 30 ml absolutem Pyridin wird bei Raumtemperatur unter Rühren mit 6,29 g (33 mmol) p-Toluolsulfonsäurechlorid versetzt, wobei die leicht exotherme Reaktion mit einem Eisbad bei Raumtemperatur gehalten wird. Nach 3 Stunden Nachrühren bei Raumtemperatur wird das Reaktionsgemisch auf Eiswasser gegossen. Die ausgefallenen Kristalle werden abgesaugt, mit Wasser gewaschen und im Vakuum getrocknet. Man erhält 5,9 g (59,2 %) 3-[2-(p-Toluolsulfonyloxy)äthyl]chroman, das bei 91-93° schmilzt.

Beispiel 7:

Eine Lösung von 11,45 g (50 mmol) 2-Brommethyl-benzo-1,4-dioxan (US-2,366,102) in 100 ml absolutem Dimethylformamid wird zuerst mit 11,1 g (50 mmol) 1,2,5,6-Tetrahydro-pyridin-3-carbonsäuremethylester-hydrobromid (Guvacolin-hydrobromid) und anschliessend mit 22,6 g (175 mmol) N-Aethyl-N,N-diisopropyl-amin versetzt. Das Gemisch wird 16 Stunden bei 50° gerührt und sodann im Hochvakuum eingedampft. Der Rückstand wird mit Wasser versetzt und mit Diäthyläther extrahiert. Die organischen Phasen werden mit Wasser gewaschen und mit 2N-Salzsäure ausgeschüttelt. Die vereinigten salzsauren Extrakte werden kalt mit Natronlauge (30 %) alkalisch gestellt und mit Dichlormethan extrahiert. Die vereinigten Dichlormethan-Phasen werden über Natriumsulfat getrocknet und im Vakuum eingedampft. Man erhält 10,75 g (74,4 %) 1-(Benzo-1,4-dioxan-2-ylmethyl)-1,2,5,6-tetrahydro-pyridin-3-carbonsäuremethylester in Form eines gelben Oels. Das hieraus mit Salzsäure in Diäthyläther hergestellte 1-(Benzo-1,4-dioxan-2-ylmethyl)-1,2,5,6-tetrahydro-pyridin-3-carbonsäuremethylester-hydrochlorid kristallisiert aus Methanol/Diäthyläther und zersetzt sich bei 215-217°.

Beispiel 8:

Eine Lösung von 4,88 g (15 mmol) 1-(Benzo-1,4-dioxan-2-ylmethyl)-1,2,5,6-tetrahydro-pyridin-3-carbonsäuremethylester-hydrochlorid in 100 ml Methanol wird mit 0,5 g Palladium auf Kohle (5 %) versetzt und bei Raumtemperatur und Normaldruck während 6 Stunden hydriert. Danach wird der Katalysator abgesaugt und das Filtrat im Vakuum eingedampft. Der ölige Rückstand wird in heissem Aceton gelöst und bis zur Trübung mit Diäthyläther versetzt. Es kristallisieren 4,08 g (83 %) 1-(Benzo-1,4-dioxan-2-ylmethyl)-piperidin-3-carbonsäuremethylester-hydrochlorid vom Smp. 186-188° aus.

Beispiel 9:

Eine Lösung von 10,12 g (30 mmol) N,N-Bis(2-methoxycarbonyläthyl)-N-(benzo-1,4-dioxan-2-ylmethyl)-amin in 25 ml absolutem Dimethylformamid wird bei Raumtemperatur unter Rühren zu einer Suspension von 2,16 g (40 mmol) Natriummethanolat in 25 ml Dimethylformamid innert 15 Minuten zugetropft. Das Reaktionsgemisch wird 3 Stunden bei Raumtemperatur nachgerührt und dann im Hochvakuum zur Trockne eingedampft. Der Rückstand wird mit Diäthyläther versetzt und mit kalter 2N-Salzsäure ausgeschüttelt. Die vereinigten salzsauren Extrakte werden mit Dichlormethan extrahiert und die Dichlormethan-Phasen über Natriumsulfat getrocknet und im Vakuum eingedampft. Man erhält 4,5 g (44,4 %) 4-Hydroxy-1-(benzo-1,4-dioxan-2-ylmethyl)-1,2,5,6-tetrahydro-pyridin-3-carbonsäuremethylester-hydrochlorid bzw. 1-(Benzo-1,4-dioxan-2-ylmethyl)-4-oxo-piperidin-3-carbonsäuremethylester-hydrochlorid, das aus Methanol/Diäthyläther

umkristallisiert wird und einen Zersetzungspunkt von 185-187° aufweist.

N,N-Bis(2-methoxycarbonyläthyl)-N-(benzo-1,4-dioxan-2-ylmethyl)-amin ist z.B. folgendermassen herstellbar:

Eine Lösung von 6,61 g (40 mmol) 2-Aminomethyl-benzo-1,4-dioxan [J. Augustin et al., J.Med.Chem. 8, 446 (1965)] in 80 ml Methanol wird mit 7,57 g (88 mmol) Acrylsäuremethylester versetzt und 16 Stunden bei 50° gerührt. Nach Erkalten wird das Reaktionsgemisch im Vakuum eingedampft. Man erhält 12,82 g (95 %) N,N-Bis(2-methoxycarbonyläthyl)-N-(benzo-1,4-dioxan-2-ylmethyl)-amin als rötliches Oel.

Beispiel 10:

In analoger Weise wie in Beispiel 9 beschrieben kann durch Umsetzung von 2-Aminomethyl-benzo-1,4-dioxan mit 1 Aequivalent Acrylsäuremethylester das entsprechende N-(2-Methoxycarbonyläthyl)-N-(benzo-1,4-dioxan-2-ylmethyl)-amin hergestellt werden, dessen Hydrochlorid bei 153-155° schmilzt.

Beispiel 11:

In analoger Weise wie in Beispiel 9 beschrieben kann man auch das 4-Hydroxy-1-(2-methyl-benzo-1,4-dioxan-2-ylmethyl)-1,2,5,6-tetrahydro-pyridin-3-carbonsäuremethylester-hydrochlorid bzw. das 1-(2-Methyl-benzo-1,4-dioxan-2-ylmethyl)-4-oxo-piperidin-3-carbonsäuremethylester-hydrochlorid herstellen.

Beispiel 12:

In analoger Weise wie in den Beispielen 9 und 10 beschrieben kann man durch Umsetzung von 2-(2-Aminoäthyl)-benzo-1,4-dioxanmit 1 Aequivalent Acrylsäuremethylester das N-[2-(Benzo-1,4-dioxan-2-yl)-äthyl]-N-(2-methoxycarbonyläthyl)-amin bzw. dessen Hydrochlorid und durch Umsetzung von 2-Aminomethyl-2-methyl-benzo-1,4-dioxan mit 1 Aequivalent Acrylsäuremethylester das N-(2-Methoxycarbonyläthyl)-N-(2-methyl-benzo-1,4-dioxan-2-ylmethyl)-amin bzw. dessen Hydrochlorid herstellen.

Beispiel 13:

Eine Lösung von 52,7 g (0.15 mol) N,N-Bis(2-methoxycarbonyläthyl)-N-[2-(benzo-1,4-dioxan-2-yl)äthyl]-amin in 150 ml absolutem Dimethylformamid wird bei Raumtemperatur unter Rühren zu einer Suspension von 10,8 g (0,20 mol) Natriummethanolat in 100 ml absolutem Dimethylformamid innert 10 Minuten zugetropft. Das Reaktionsgemisch wird 15 Stunden bei Raumtemperatur nachgerührt und dann im Hochvakuum zur Trockne eingedampft. Der erhaltene Rückstand wird mit Diäthyläther versetzt und mit kalter 2N-Salzsäure extrahiert. Die vereinigten salzsauren Extrakte werden mit Dichlormethan ausgeschüttelt. Die Dichlormethan-Phasen werden über Natriumsulfat getrocknet und im Vakuum eingedampft. Man erhält 34,0 g (63,7 %) 1-[2-(Benzo-1,4-dioxan-2-yl)äthyl]-4-hydroxy-1,2,5,6-tetrahydro-pyridin-3-carbonsäuremethylester-hydrochlorid bzw. 1-[2-(Benzo-1,4-dioxan-2-yl)äthyl]-4-oxo-piperidin-3-carbonsäuremethylester-hydrochlorid, das aus Methanol/Diäthyläther umkristallisiert wird und einen Zersetzungspunkt von 165-166° aufweist.

N,N-Bis(2-methoxycarbonyläthyl)-N-[2-(benzo-1,4-2-yl)äthyl]-amin ist z.B. auf folgende Weise herstellbar:

Eine Lösung von 32,25 g (0,18 mol) 2-(2-Aminoäthyl)-benzo-1,4-dioxan [J. Augustin et al., J.Med.Chem. 8, 446 (1965)] in 250 ml Methanol wird bei Raumtemperatur mit 34,1 g (0,396 mol) Acrylsäuremethylester versetzt. Das Reaktionsgemisch wird 6 Stunden bei 50° gerührt und nach Erkalten im Vakuum eingedampft. Man erhält 57,9 g (91,5 %) N,N-Bis(2-methoxycarbonyläthyl)-N-[2-(benzo-1,4-dioxan-2-yl)äthyl]-amin als rotes Oel.

Beispiel 14:

Eine Lösung von 12,45 g (35 mmol) 1-[2-Benzo-1,4-dioxan-2-yl)äthyl]-4-hydroxy-1,2,5,6-tetrahydropyridin-3-carbonsäuremethylester-hydrochloridbzw. 1-[2-(Benzo-1,4-dioxan-2-yl)äthyl]-4-oxo-piperidin-3-carbonsäuremethylester-hydrochlorid in 250 ml Methanol wird mit 1,25 g Platinoxid versetzt und bei Raumtemperatur und Normaldruck hydriert. Nach Aufnahme der theoretisch notwendigen Menge Wasserstoff wird der Katalysator abgetrennt, das Filtrat im Vakuum eingedampft und der ölige Rückstand in heissem Aceton gelöst. Nach Abkühlen kristallisieren 4,87 g (38,9 %) cis-1-[2-(Benzo-1,4-dioxan-2-yl)äthyl]-4-hydroxy-piperidin-3-carbonsäuremethylester-hydrochlorid vom Smp. 182-185° aus.

Beispiel 15:

Eine Suspension von 12,45 g (35 mmol) 1-[2-(Benzo-1,4-dioxan-2-yl)äthyl]-4-hydroxy-1,2,5,6-tetrahydropyridin-3-carbonsäuremethylester-hydrochlorid bzw. 1-[2-(Benzo-1,4-dioxan-2-yl)-äthyl]-4-oxo-piperidin-3-carbonsäuremethylester-hydrochlorid in 250 ml Methanol wird während 1 Stunde unter Rühren bei -15° portionsweise mit 2,65 g (70 mmol) Natriumborhydrid versetzt. Es wird noch 4 Stunden bei -10° nachgerührt und danach das Reaktionsgemisch im Vakuum eingedampft und in Wasser/Aethylacetat aufgenommen. Die Aethylacetat-Extrakte werden mit Wasser gewaschen, über Natriumsulfat getrocknet und eingedampft. Man erhält 11,2 g (100 %) Rohprodukt, das über 560 g Keiselgel (0,040-0,063 mm) mit Aethylacetat als Laufmittel chromatographiert wird. Man erhält 5,41 g (48,2 %) trans-1-[2-(Benzo-1,4-dioxan-2-yl)äthyl]-4-hydroxypiperidin-3-carbonsäuremethylester als hellgelbes Oel. Das hieraus mit Fumarsäure hergestellte trans-1-[2-(Benzo-1,4-dioxan-2-yl)äthyl]-4-hydroxy-piperidin-3-carbonsäuremethylester-fumarat kristallisiert aus Methanol/Diäthyläther als Hemihydrat mit einem Smp. von 150-152°.

Beispiel 16:

Eine Lösung von 8,03 g (25 mmol) eines Gemisches von cis- und trans-1-[2-(Benzo-1,4-dioxan-2-yl)-äthyl]-4-hydroxy-piperidin-3-carbonsäuremethylester in 100 ml Toluol wird zunächst mit 19,03 g (125 mmol) 1,5-Diazabicyclo[5.4.0)undec-5-en und anschliessend unter Rühren bei 0-5° tropfenweise mit einer Lösung von 3,44 g (30 mmol) Methansulfonsäurechlorid in 20 ml Toluol versetzt. Danach lässt man auf Raumtemperatur erwärmen und rührt weitere 16 Stunden nach. Das Reaktionsgemisch wird dann mit Eiswasser versetzt und die organische Phase mit 2N-Salzsäure extrahiert. Die vereinigten salzsauren Extrakte werden kalt mit Natronlauge (30 %) alkalisch gestellt und mit Dichlormethan ausgeschüttelt. Die vereinigten organischen Phasen werden mit Wasser gewaschen, über Natriumsulfat getrocknet und im Vakuum eingedampft. Man erhält 5,8 g (76,5 %) Rohprodukt, das über 300 g Kieselgel (0,040-0,063 mm) mit Toluol/Aethylacetat (1:1) als Laufmittel chromatographiert wird. Man erhält 4,13 g (54,5 %) 1-[2-(Benzo-1,4-dioxan-2-yl)äthyl]-1,2,5,6-tetrahydro-pyridin-3-carbonsäuremethylester als gelbes Oel. Das hieraus mit Salzsäure in Diäthyläther hergestellte 1-[2-(Benzo-1,4-dioxan-2-yl)äthyl]-1,2,5,6-tetrahydro-pyridin-3-carbonsäuremethylester-hydrohlorid kristallisiert aus Methanol/Diäthyläther und zersetzt sich bei 205-206°.

Beispiel 17:

Eine Lösung von 3,32 g (10 mmol) 3-[2-(p-Toluolsulfonyloxy)äthyl]chroman in 50 ml absolutem Dimethylformamid wird zuerst mit 2,1 g (11 mmol) 1,2,5,6-Tetrahydro-pyridin-3-carbonsäureäthylester-hydrochlorid und anschliessend mit 4,53 g (35 mmol) N-Aethyl-N,N-diisopropyl-amin versetzt. Das Gemisch wird 16 Stunden bei 60° gerührt und nach dem Erkalten im Hochvakuum eingedampft. Der ölige Rückstand wird mit Wasser versetzt und mit Diäthyläther extrahiert. Die vereinigten organischen Phasen werden mit 2N-Salzsäure extrahiert. Die vereinigten salzsauren Extrakte werden kalt mit Natronlauge (30 %) alkalisch gestellt und mit Dichlormethan extrahiert. Die vereinigten Dichlormethan-Phasen werden über Natriumsulfat getrocknet und im Vakuum eingedampft. Man erhält 1,65 g (52,3 %) 1-[2-(Chroman-3-yl)-äthyl]-1,2,5,6-tetrahydro-pyridin-3-carbonsäureäthylester als gelbes Oel. Das hieraus mit Salzsäure in Diäthyläther hergestellte 1-[2-(Chroman-3-yl)äthyl]-1,2,5,6-tetrahydro-pyridin-3-carbonsäureäthylester-hydrochlorid kristallisiert aus Aceton/Diäthyläther und schmilzt bei 177 - 178°.

Beispiel 18:

Eine Lösung von 2,54 g (7,5 mmol) 1-[2-(Chroman-3-yl)äthyl]-1,2,5,6-tetrahydro-pyridin-3-carbonsäuremethylester-hydrochlorid in 170 ml absolutem Aethanol wird mit 3,4 ml Schwefelsäure (100 %) versetzt und 35 Stunden unter Rückfluss gekocht. Nach dem Erkalten wird das Reaktionsgemisch im Vakuum eingedampft. Der Rückstand wird kalt in Wasser gelöst und mit Diäthyläther extrahiert. Die wässrige Phase wird kalt mit Natronlauge (30 %) alkalisch gestellt und mit Dichlormethan extrahiert. Die vereinigten Dichlormethan-Extrakte werden über Natriumsulfat getrocknet und im Vakuum eingedampft. Man erhält 2,30 g (97,4 %) 1-[2-(Chroman-3-yl)äthyl]-1,2,5,6-tetrahydro-pyridin-3-carbonsäureäthylester als gelbes Oel. Das hieraus mit Salzsäure in Diäthyläther hergestellte 1-[2-(Chroman-3-yl)äthyl]-1,2,5,6-tetrahydro-pyridin-3-carbonsäureäthylester-hydrochlorid kristallisiert aus Aceton/Diäthyläther und schmilzt bei 177-178°.

Beispiel 19:

Eine Lösung von 3,98 g (12 mmol) 3-[2-(p-Toluolsulfonyloxy)äthyl]chroman in 50 ml absolutem Dimethylformamid wird zuerst mit 3,76 g (24 mmol) Piperidin-3-carbonsäureäthylester und anschliessend mit 3,1 g (24 mmol) N-Aethyl-N,N-diisopropyl-amin versetzt. Das Gemisch wird 16 Stunden bei 60° gerührt und nach dem Erkalten im Hochvakuum eingedampft. Der ölige Rückstand wird mit Wasser versetzt und mit Diäthyläther extrahiert. Die vereinigten organischen Phasen werden mit 2 N-Salzsäure extrahiert. Die vereinigten salzsauren Extrakte werden kalt mit Natronlauge (30 %) alkalisch gestellt und mit Dichlormethan extrahiert. Die vereinigten Dichlormethan-Phasen werden über Natriumsulfat getrocknet und im Vakuum eingedampft. Man erhält 3,7 g (97,3 %) 1-[2-(Chroman-3-yl)äthyl]-piperidin-3-carbonsäureäthylester als gelbes Oel. Das mit Salzsäure in Diäthyläther hieraus hergestellte 1-[2-(Chroman-3-yl)äthyl]-piperidin-3-carbonsäureäthylester-hydrochlorid kristallisiert aus Aceton/Diäthyläther mit 0,18 Aequivalenten Kristallwasser und schmilzt bei 140 -143°.

Beispiel 20:

Eine Lösung von 3,98 g (12 mmol) 3-[2-(p-Toluolsulfonyloxy)äthyl]chroman in 50 ml absolutem Dimethylformamid wird zuerst mit 3,76 g (24 mmol) Piperidin-4-carbonsäureäthylester und anschliessend mit 3,1 g (24 mmol) N-Aethyl-N,N-diisopropyl-amin versetzt. Das Gemisch wird 16 Stunden bei 60° gerührt und nach dem Erkalten im Hochvakuum eingedampft. Der ölige Rückstand wird mit Wasser versetzt und mit Diäthyläther ausgeschüttelt. Die vereinigten organischen Phasen werden mit 2N-Salzsäure extrahiert. Die vereinigten salzsauren Extrakte werden kalt mit Natronlauge (30 %) alkalisch gestllt und mit Dichlormethan extrahiert. Die vereinigten Dichlormethan-Phasen werden über Natriumsulfat getrocknet und im Vakuum eingedampft. Man erhält 3,8 g (100 %) Rohprodukt, das über 200 g Kieselgel (0,040 - 0,063 mm) mit Aethylacetat als Laufmittel chromatographiert wird. Danach erhält man 3,7 g (97,3 %) 1-[2-(Chroman-3-yl)äthyl]-piperidin-4-carbonsäureäthylester als farbloses Oel. Das hieraus mit Salzsäure in Diäthyläther hergestellte 1-[2-(Chroman-3-yl)äthyl]-piperidin-4-carbonsäureäthylester-hydrochlorid kristallisiert aus Aethanol/Diäthyläther und schmilzt bei 182 - 186°.

Beispiel 21:

Eine Lösung von 3,34 g (10 mmol) 2-[2-(p-Toluolsulfonyloxy)äthyl]-benzo-1,4,-dioxan in 50 ml absolutem Dimethylformamid wird zuerst mit 1,96 g (12,5 mmol) Piperidin-3-carbonsäureäthylester und anschliessend mit 2,58 g (20 mmol) N-Aethyl-N,N-diisopropyl-amin versetzt. Das Gemisch wird 16 Stunden bei 60° gerührt und nach dem Abkühlen im Hochvakuum eingedampft. Der ölige Rückstand wird mit Wasser versetzt und mit Diäthyläther extrahiert. Die vereinigten organischen Phasen werden mit Wasser gewaschen und mit 2 N-Salzsäure extrahiert. Die vereinigten Salzsäure-Extrakte werden kalt mit Natronlauge (30 %) alkalisch gestellt und mit Dichlormethan extrahiert. Die vereinigten Dichlormethan-Phasen werden über Natriumsulfat getrocknet und im Vakuum eingedampft. Man erhält 3,82 g (100 %) Rohprodukt, das über 190 g Kieselgel (0,040 - 0,063 mm) mit Aethylacetat als Laufmittel filtriert wird. Danach erhält man 3,70 g (96,6 %) 1-[2-(Benzo-1,4-dioxan-2-yl)äthyl]-piperidin-3-carbonsäureäthylester als hellgelbes Oel. Das hieraus mit Salzsäure in Diäthyläther hergestellte 1-[2-(Benzo-1,4-dioxan-2-yl)äthyl]-piperidin-3-carbonsäureäthylester-hydrochlorid kristallisiert aus Aethanol/Diäthyläther und schmilzt bei 162 - 165°.

Das 2-[2-(p-Toluolsulfonyloxy)äthyl]-benzo-1,4-dioxan lässt sich z.B. folgendermassen erhalten:
In eine Lösung von 15,76 g (90 mmol) 2-Cyanomethyl-benzo-1,4-dioxan (BE-613,211) in 200 ml absolutem Methanol wird bei 5 - 10° unter Rühren Chlorwasserstoff bis zur Sättigung eingeleitet. Anschliessend lässt man das Reaktionsgemisch auf Raumtemperatur auftauen und rührt 16 Stunden bei Raumtemperatur nach. Das Reaktionsgemisch wird sodann 2 Stunden unter Rückfluss gekocht. Nach dem Erkalten wird das Gemisch im Vakuum eingedampft. Der ölige Rückstand wird mit Eiswasser versetzt und mit Diäthyläther extrahiert. Die vereinigten organischen Phasen werden kalt mit Wasser, gesättigter Natriumhydrogencarbonatlösung und nochmals Wasser gewaschen, über Natriumsulfat getrocknet und im Vakuum eingedampft. Man erhält 18,2 g (97,3 %) 2-Methoxycarbonylmethyl-benzo-1,4-dioxan als hellgelbes Oel.

Zu einer Suspension von 2,85 g (75 mmol) Lithiumaluminiumhydrid in 120 ml absolutem Diäthyläther wird eine Lösung von 15,61 g (75 mmol) 2-Methoxycarbonylmethyl-benzo-1,4-dioxan in 120 ml absolutem Tetrahydrofuran innert 30 Minuten unter Rühren bei Raumtemperatur zugetropft. Das Reaktionsgemisch wird 2 Stunden bei Raumtemperatur nachgerührt. Anschliessend wird das Reaktionsgemisch vorsichtig mit 2,85 ml Wasser, 2,85 ml Natronlauge (15 %) und 8,55 ml Wasser zersetzt. Der ausgefallene Niederschlag wird abgesaugt und das Filtrat im Vakuum eingedampft. Der ölige Rückstand wird in Diäthyläther gelöst. Die Lösung wird gründlich mit Wasser gewaschen, über Natriumsulfat getrocknet und im Vakuum eingedampft. Man erhält 12,37 g (91,6 %) 2-(2-Hydroxyäthyl)-benzo-1,4-dioxan als farbloses Oel.

Eine Lösung von 10,81 g (60 mmol) 2-(2-Hydroxyäthyl)-benzo-1,4-dioxan in 35 ml absolutem Pyridin wird bei Raumtemperatur unter Rühren mit 12,20 g (64 mmol) p-Toluolsulfonsäurechlorid versetzt, wobei die leicht exotherme Reaktion mit einem Eisbad bei Raumtemperatur gehalten wird. Das Reaktionsgemisch wird 3 Stunden bei Raumtemperatur nachgerührt und dann auf Eiswasser gegossen. Die ausgefallenen Kristalle werden abgesaugt, mit Wasser gewaschen und im Vakuum getrocknet. Man erhält 15,50 g (77,2 %) 2-[2-(p-Toluolsulfonyloxy)äthyl]-benzo-1,4-dioxan, das bei 82 - 84° schmilzt.

Beispiel 22:

Eine Lösung von 3,34 g (10 mmol) 2-[2-(p-Toluolsulfonyloxy)äthyl]-benzo-1,4-dioxan in 50 ml absolutem Dimethylformamid wird zuerst mit 1,96 g (12,5 mmol) Piperidin-4-carbonsäureäthylester und anschliessend mit 2,58 g (20 mmol) N-Aethyl-N,N-diisopropyl-amin versetzt. Das Gemisch wird 16 Stunden bei 60° gerührt und nach dem Abkühlen im Hockvakuum eingedampft. Der ölige Rückstand wird mit Wasser versetzt und mit Diäthyläther extrahiert. Die vereinigten organischen Phasen werden mit Wasser gewaschen und mit 2N-Salzsäure extrahiert. Die vereinigten Salzsäure-Extrakte werden kalt mit Natronlauge (30 %) alkalisch gestellt und mit Dichlormethan extrahiert. Die vereinigten Dichlormethan Phasen werden über Natriumsulfat getrocknet und im Vakuum eingedampft. Man erhält 3,80 g (99,2 %) Rohprodukt, das über 190 g Kieselgel (0,040 - 0,063 mm) mit Aethylacetat als Laufmittel filtriert wird. Danach erhält man 3,7 g (96,6 %) 1-[2-(Benzo-1,4-dioxan-2-yl)äthyl]-piperidin-4-carbonsäureäthylester als gelbes Oel. Das hieraus mit Salzsäure in Diäthyläther hergestellte 1-[2-(Benzo-1,4-dioxan-2-yl)äthyl]-piperidin-4-carbonsäureäthylester-hydrochlorid kristallisiert aus Aethanol/Diäthyläther und schmilzt bei 165 - 168°.

Beispiel 23:

Eine Lösung von 2,99 g (10 mmol) N-[2-(Chroman-3-yl)äthyl]-N-(2-methoxycarbonyläthyl)-amin-hydrochlorid in 60 ml Methanol wird bei Raumtemperatur unter Rühren mit 21 ml (42 mmol) 2N-Natronlauge versetzt. Nach 5 Minuten setzt man dem Reaktionsgemisch 40 ml Wasser zu und rührt das Gemisch sodann für 30 Minuten bei 50 - 60°. Nach dem Erkalten wird das Gemisch im Vakuum eingedampft. Der Rückstand wird in 30 ml Wasser gelöst, mit 10 ml Salzsäure (36 %) versetzt und im Eisbad abgekühlt. Die ausgefallenen Kristalle werden abgesaugt. Man erhält 2,5 g (87,5 %) N-[2-(Chroman-3-yl)äthyl]-N-(2-carboxyäthyl)-amin-hydrochlorid (Smp. 186 - 188°).

Das N-[2-(Chroman-3-yl)äthyl]-N-(2-methoxycarbonyläthyl)-amin-hydrochlorid kann z.B. wie in Beispiel 4 beschrieben hergestellt werden.

Beispiel 24:

Eine Lösung von 2,66 g (8 mmol) 2-[2-(p-Toluolsulfonyloxy)äthyl]chroman in 35 ml absolutem Dimethylformamid wird zuerst mit 1,57 g (10 mmol) Piperidin-3-carbonsäureäthylester und anschliessend mit 2,07 g (16 mmol) N-Aethyl-N,N-diisopropyl-amin versetzt. Das Gemisch wird 16 Stunden bei 60° gerührt und nach dem Abkühlen im Hochvakuum eingedampft. Der ölige Rückstand wird mit Wasser versetzt und mit Diäthyläther extrahiert. Die vereinigten organischen Phasen werden mit Wasser gewaschen und mit 2N-Salzsäure extrahiert. Die vereinigten Salzsäure-Extrakte werden kalt mit Natronlauge (30 %) alkalisch gestellt und mit Dichlormethan extrahiert. Die vereinigten Dichlormethan-Phasen werden über Natriumsulfat getrocknet und im Vakuum eingedampft. Man erhält 2,28 g (90,1 %) Rohprodukt, das über 120 g Kieselgel (0,040 - 0,063 mm) mit Aethylacetat als Laufmittel chromatographiert wird. Danach erhält man 1,82 g (72,2 %) 1-[2-(Chroman-2-yl)äthyl]-piperidin-3-carbonsäureäthylester als gelbes Oel. Das hieraus mit Salzsäure in Diäthyläther hergestellte 1-[2-(Chroman-2-yl)äthyl]-piperidin-3-carbonsäureäthylester-hydrochlorid kristallisiert aus Aethanol/Diäthyläther und schmilzt bei 148 - 151°.

2-[2-(p-Toluolsulfonyloxy)äthyl]chroman kann z.B. auf folgende Weise hergestellt werden:

Eine Lösung von 70,34 g (0,39 mol) 2-Carboxychroman in 1400 ml Methanol wird mit 14,2 ml Schwefelsäure (100 %) versetzt und während 4 Stunden unter Rückfluss gekocht. Nach dem Erkalten wird das Reaktionsgemisch im Vakuum eingedampft und der Rückstand in Diäthyläther gelöst und mit Wasser, kalter gesättigter Natriumhydrogencarbonatlösung und nochmals Wasser gewaschen. Die ätherische Phase wird über Natriumsulfat getrocknet und im Vakuum eingedampft. Man erhält 72,8 g (96 %) 2-Methoxycarbonylchroman als hellgelbes Oel.

Zu einer Suspension von 7,2 g (0,19 mol) Lithiumaluminiumhydrid in 400 ml absolutem Diäthyläther wird eine Lösung von 36,4 g (0,19 mol) 2-Methoxycarbonylchroman in 400 ml absolutem Tetrahydrofuran innert 1 Stunde unter Rühren bei Raumtemperatur zugetropft. Nach 16-stündigem Nachrühren bei Raumtemperatur wird das Reaktionsgemisch vorsichtig mit 7,2 ml Wasser, 7,2 ml Natronlauge (15 %) und 21,6 ml Wasser zersetzt. Der ausgefallene Niederschlag wird abgesaugt und das Filtrat im Vakuum eingedampft. Der ölige Rückstand wird in Diäthyläther gelöst. Die ätherische Lösung wird mit Wasser gewaschen, über Natriumsulfat getrocknet und im Vakuum eingedampft. Man erhält so 31 g (99,3 %) 2-Hydroxymethylchroman in Form eines farblosen Oels.

Eine Lösung von 31 g (0,189 mol) 2-Hydroxymethylchroman in 110 ml absolutem Pyridin wird bei Raumtemperatur unter Rühren mit 38,16 g (0,2 mol) p-Toluolsulfonsäurechlorid versetzt, wobei die leicht exotherme Reaktion mit einem Eisbad bei Raumtemperatur gehalten wird. Das Reaktionsgemisch wird 3 Stunden bei Raumtemperatur nachgerührt und dann auf Eiswasser gegossen. Das abgeschiedene Oel wird durch Abdekantieren der wässrigen Phase abgetrennt, in Diäthyläther gelöst und mit eiskalter 2N-Salzsäure und Eiswasser gewaschen. Die ätherischen Phasen werden über Natriumsulfat getrocknet und im Vakuum eingedampft. Man erhält 58,15 g (96,6 %) 2-(p-Toluolsulfonyloxymethyl)chroman als farbloses Oel.

Eine Lösung von 57,31 g (0,18 mol) 2-(p-Toluolsulfonyloxymethyl)chroman in 800 ml absolutem Dimethylformamid wird mit 10,6 g (0,216 mol) Natriumcyanid versetzt und unter Rühren auf 60° erwärmt. Nach 10 Stunden wird das Reaktionsgemisch mit Eiswasser versetzt und mit Diäthyläther extrahiert. Die vereinigten ätherischen Phasen werden gründlich mit Wasser gewaschen, über Natriumsulfat getrocknet und im Vakuum eingedampft. Man erhält 30,0 g (96,2 %) Rohprodukt, das über 1000 g Kieselgel (0,040 - 0,063 mm) mit Toluol als Laufmittel chromatographiert wird. Auf diese Weise erhält man 18,16 g (58,2 %) 2-Cyanomethylchroman als gelbes Oel.

In eine Lösung von 17,32 g (0,1 mol) 2-Cyanomethylchroman in 200 ml absolutem Methanol wird bei 5 - 10° Chlorwasserstoffgas bis zur Sättigung eingeleitet. Anschliessend lässt man das Reaktionsgemisch auf Raumtemperatur auftauen und rührt 16 Stunden bei Raumtemperatur nach. Danach wird das Reaktionsgemisch 2 Stunden unter Rückfluss gekocht. Anschliessend lässt man erkalten und dampft das Gemisch im Vakuum ein. Der Rückstand wird mit Eiswasser versetzt und mit Diäthyläther extrahiert. Die vereinigten organischen Phasen werden in der Kälte mit Wasser, Natriumhydrogencarbonatlösung und nochmals Wasser gewaschen, über Natriumsulfat getrocknet und im Vakuum eingedampft. Man erhält 18,58 g (90,1 %) Rohprodukt, das über 460 g Kieselgel (0,040 - 0,063 mm) mit Toluol als Laufmittel filtriert wird. Man erhält 17,80 g (86,3 %) 2-Methoxycarbonylmethylchroman als hellgelbes Oel.

Zu einer Suspension von 3,11 g (82 mmol) Lithiumaluminiumhydrid in 150 ml absolutem Diäthyläther wird bei Raumtemperatur eine Lösung von 16,91 g (82 mmol) 2-Methoxycarbonylmethylchroman in 150 ml absolutem Tetrahydrofuran innert 30 Minuten unter Rühren zugetropft. Es wird 16 Stunden bei Raumtemperatur nachgerührt und dann vorsichtig mit 3,1 ml Wasser, 3,1 ml Natronlauge (15 %) und 9,3 ml Wasser zersetzt. Der ausgefallene Niederschlage wird abgesaugt und das Filtrat im Vakuum eingedampft. Der ölige Rückstand wird in Diäthyläther gelöst. Die ätherische Lösung wird mit Wasser gewaschen, über Natriumsulfat getrocknet und im Vakuum eingedampft. Man erhält 14,36 g (98,3 %) 2-(2-Hydroxyäthyl)chroman in Form eines farblosen Oels.

Eine Lösung von 13,36 g (75 mmol) 2-(2-Hydroxyäthyl)chroman in 90 ml absolutem Pyridin wird bei Raumtemperatur unter Rühren mit 15,73 g (82,5 mmol) p-Toluolsulfonsäurechloriod versetzt, wobei die leicht exotherme Reaktion mit einem Eisbad bei Raumtemperatur gehalten wird. Nach-3-stündigem Nachrühren bei Raumtemperatur wird das Reaktionsgemisch auf Eiswasser gegossen. Die ausgefallenen Kristalle werden abgesaugt, mit Wasser gewaschen und im Vakuum getrocknet. Man erhält 8,68 g (34,7 %) 2-[2-(p-Toluolsulfonyloxy)äthyl]chroman, das bei 57 - 59° schmilzt.

Beispiel 25:

Eine Lösung von 2,66 g (8 mmol) 2-[2-(p-Toluolsulfonyloxy)äthyl]chroman in 35 ml absolutem Dimethylformamid wird zuerst mit 1,57 g (10 mmol) Piperidin-4-carbonsäureäthylester und anschliessend mit 2,07 g (16 mmol) N-äthyl-N,N-diisopropyl-amin versetzt. Das Gemisch wird 16 Stunden bei 60° gerührt und dann nach dem Abkühlen im Hochvakuum eingedampft. Der ölige Rückstand wird mit Wasser versetzt und mit Diäthyläther extrahiert. die vereinigten organischen Phasen werden mit Wasser gewaschen und mit 2 N-Salzsäure extrahiert. Die vereinigten Salzsäure-Extrakte werden kalt mit Natronlauge (30 %) alkalisch gestellt und mit Dichlormethan extrahiert. Die vereinigten Dichlormethan-Phasen werden über Natriumsulfat getrocknet und im Vakuum eingedampft. Man erhält 2,17 g (85,7 %) Rohprodukt, das über 110 g Kieselgel (0,040 - 0,063 mm) mit Aethylacetat als Laufmittel chromatographiert wird. Auf diese Weise erhält man 1,90 g (75,1 %) 1-[2-(Chroman-2-yl)äthyl]-piperidin-4-carbonsäureäthylester als gelbes Oel. Das hieraus mit Salzsäure in Diäthyläther hergestellte 1-[2-(Chroman-2-yl)äthyl]-piperidin-4-carbonsäureäthylester-hydrochlorid kristallisiert aus Aethanol/Diäthyläther und schmilzt bei 190 - 192°.

Beispiel 26:

Eine Lösung von 6,2 g (35 mmol) 2-(2-Aminoäthyl)-chroman in 200 ml Methanol wird bei 0 - 5° unter Rühren mit 3,01 g (35 mmol) Acrylsäuremethylester versetzt. Man rührt 16 Stunden bei 0 - 5° nach und dampft das Gemisch dann im Vakuum ein. Man erhält 8,76 g (95,2 %) Rohprodukt, das über 250 g Kieselgel (0,040 - 0,063 mm) mit Aethylacetat als Laufmittel chromatographiert wird. Man erhält auf diese Weise 5,10 g (55,4 %) N-[2-(Chroman-2-yl)äthyl]-N-(2-methoxycarbonyläthyl)-amin als gelbes Oel. Das hieraus mit Salzsäure in Diäthyläther hergestellte N-[2-(Chroman-2-yl)äthyl]-N-(2-methoxycarbonyläthyl)-amin-hydrochlorid kristallisiert aus Methanol/Diäthyläther und schmilzt bei 152 - 153°.

2-(2-Aminoäthyl)chroman kann man z.B. folgendermassen herstellen:
Zu einer Suspension von 3,8 g (100 mmol) Lithiumaluminiumhydrid in 150 ml absolutem Diäthyläther werden zuerst unter Rühren bei Raumtemperatur 2,2 g (16,5 mmol) Aluminiumchlorid in 70 ml absolutem Diäthyläther zugetropft. Anschliessend werden 8,66 g (50 mmol) 2-Cyanomethylchroman in 70 ml absolutem Tetrahydrofuran innert 20 Minuten zugetropft. Das Reaktionsgemisch wird 16 Stunden bei Raumtemperatur nachgerührt und dann vorsichtig mit 3,8 ml Wasser, 3,8 ml Natronlauge (15 %) und 11,4 ml Wasser zersetzt. Der ausgefallene Niederschlag wird abgesaugt und das Filtrat im Vakuum eingedampft. Der ölige Rückstand wird in Diäthyläther gelöst. Die ätherische Phase wird mit Wasser gewaschen, über Natriumsulfat getrocknet und eingedampft. Man erhält 8,75 g (98,8 %) 2-(2-Aminoäthyl)chroman als farbloses Oel.

Beispiel 27:

Eine Lösung von 4,81 g (0.015 mol) 1-[2-(Chroman-3-yl)äthyl]-3-cyano-4-hydroxy-1,2,5,6-tetrahydropyridin-hydrochlorid bzw. 1-[2-(Chroman-3-yl)äthyl]-3-cyano-4-oxo-piperidin-hydrochlorid in 100 ml Methanol (95 %) wird mit 1,5 ml konzentrierter Salzsäure versetzt und 15 Stunden unter Rückfluss gekocht. Nach dem Erkalten wird das Reaktionsgemisch unter vermindertem Druck auf ein Volumen von etwa 30 ml eingeengt und diese Lösung in ein Gemisch von 80 ml 5N-Salzsäure und 20 ml Toluol gegossen, woraufhin unter Rühren und Abkühlen 1-[2-(Chroman-3-yl)äthyl]-4-hydroxy-1,2,5,6-tetrahydropyridin-3-carbonsäuremethylester-hydrochlorid bzw. 1-[2-(Chroman-3-yl)äthyl]-4-oxo-piperidin-3-carbonsäu remethylester-hydrochlorid vom Smp. 175-177° (Zers.) auskristallisiert.

Das 1-[2-(Chroman-3-yl)äthyl]-3-cyano-4-hydroxy-1,2,5,6-tetrahydropyridin-hydrochlorid bzw. 1-[2-(Chroman-3-yl)äthyl]-3-cyano-4-oxo-piperidin-hydrochlorid kann man z.B. folgendermassen herstellen:
30 g (0.1 mol) N-[2-(Chroman-3-yl)äthyl]-N-(2-methoxycarbonyläthyl)-amin-hydrochlorid werden in 100 ml Methanol gelöst und die Lösung mit 10.5 g (0.1 mol) Triäthylamin und 5.84 g (0.11 mol) Acrylnitril versetzt und 15 Stunden bei Raumtemperatur gerührt. Danach wird das Reaktionsgemisch im Wasserstrahlvakuum eingeengt, der Rückstand in Diäthyläther aufgenommen und die ätherische Lösung mit Eiswasser neutral gewaschen. Die ätherische Phase wird über Kaliumcarbonat getrocknet und eingedampft. Man erhält so das N-[2-(Chroman-3-yl)äthyl]-N-(2-cyanoäthyl)-N-(2-methoxycarbonyläthyl)-amin in Form eines gelben Oeles.

Eine Suspension von 5,73 g Natriumhydrid (55 % Suspension in Mineralöl) in 100 ml Tetrahydrofuran wird unter einer Stickstoffatmosphäre tropfenweise mit einer Lösung von 13,07 g (41,3 mmol) N-[2-(Chroman-3-yl)äthyl]-N-(2-cyanoäthyl)-N-(2-methoxycarbonyläthyl)-amin in 200 ml Tetrahydrofuran versetzt und 16 Stunden bei Raumtemperatur gerührt. Nach Zusatz von 70 ml 2N-Schwefelsäure erhält man eine gelbe Lösung. Man versetzt diese mit 300 ml Diäthyläther und 100 ml Wasser, wobei zwei Phasen entstehen. Die wässrige Phase wird dreimal mit je 100 ml Diäthyläther extrahiert. Die vereinigten organischen Phasen werden über Natriumsulfat getrocknet, unter vermindertem Druck auf etwa 100 ml eingeengt und sodann in ein Gemisch von 80 ml 5N-Salzsäure und 20 ml Toluol gegossen, woraufhin unter Rühren und Abkühlen das 1-[2-(Chroman-3-yl)äthyl]-3-cyano-4-hydroxy-1,2,5,6-tetrahydro-pyridin-hydrochlorid bzw. das 1-[2-(Chroman-3-yl)äthyl]-3-cyano-4-oxo-piperidin-hydrochlorid auskristallisiert.

Beispiel 28:

Eine Lösung von 2,81 g Diisopropylamin in 30 ml trockenem Tetrahydrofuran wird bei 0-5° mit 17,4 ml n-Butyllithium in Hexan versetzt. Man lässt 30 Minuten bei Raumtemperatur rühren, kühlt dann auf -15° ab und setzt eine Lösung von 6,24 g (25 mmol) 1-[2-(Chroman-3-yl)äthyl]-4-oxo-piperidin in 30 ml Tetrahydrofuran zu. Nach 15 Minuten tropft man eine Lösung von 3,05 g (28 mmol) Chlortrimethylsilan in 15 ml Tetrahydrofuran zu. Man lässt über Nacht bei Raumtemperatur rühren, filtriert die Lösung und dampft das Filtrat unter vermindertem Druck zur Trockne ein. Man erhält so das 1-[2-(Chroman-3-yl)äthyl]-4-trimethylsilyloxy-1,2,5,6-tetrahydropyridin in Form eines hellgelben Oeles. 6,63 g (20 mmol) des erhaltenen 1-[2-(Chroman-3-yl)äthyl]-4-trimethylsilyloxy-1,2,5,6-tetrahydropyridin werden in 50 ml Dichlormethan gelöst

und diese Lösung zu einer auf 0° gekühlten Lösung von 2,3 g (24 mmol) Chlorameisensäuremethylester und 60 mg (2,4 mmol) wasserfreiem Zinkbromid in 50 ml absolutem Dichlormethan zugetropft. Nach Erwärmen auf Raumtemperatur wird die Reaktionslösung 1 Stunde nachgerührt und dann auf 150 ml gesättigte Natriumhydrogencarbonatlösung gegossen. Man extrahiert mit Dichlormethan, trocknet die vereinigten organischen Phasen über Natriumsulfat und dampft sie ein. Der Rückstand wird in 70 ml Aethanol gelöst und die Lösung mit äthanolischer Salzsäure angesäuert. Nach Versetzen mit Diäthyläther und Abkühlen kristallisiert das 1-[2-(Chroman-3-yl)äthyl]-4-hydroxy-1,2,5,6-tetrahydro-pyridin-3-carbonsäuremethylester-hydrochlorid bzw. das 1-[2-(Chroman-3-yl)äthyl]-4-oxo-piperidin-3-carbonsäuremethylester-hydrochlorid vom Smp. 175-177° aus.

Das 1-[2-(Chroman-3-yl)äthyl]-4-oxo-piperidin kann man z.B. folgendermassen herstellen.

Eine Lösung von 16,62 g (50 mmol) 3-[2-(p-Toluolsulfonyloxy)äthyl]-chroman in 100 ml Dimethylformamid wird zuerst mit 8,45 g (55 mmol) Piperidon-hydrochlorid-monohydrat und anschliessend mit 22,62 g (175 mmol) N-Aethyl-N,N-diisopropyl-amin versetzt. Das Gemisch wird 18 Stunden bei 80°C gerührt und nach Erkalten unter vermindertem Druck zur Trockne eingedampft. Der Rückstand wird in Diäthyläther gelöst und mit Wasser gewaschen. Die organische Phase wird abgetrennt und mit 2N-Salzsäure extrahiert. Die Salzsäure-Extrakte werden vereinigt, kalt mit konzentrierter Natronlauge alkalisch gestellt und mit Dichlormethan extrahiert. Die Dichlormethan-Phasen werden vereinigt, über Natriumsulfat getrocknet und unter vermindertem Druck zur Trockne eingedampft. Man erhält ein dunkelbraunes Harz, welches mit Toluol/Aethylacetat (1:1) als Laufmittel an 350 g Kieselgel (0,040-0,063 mm) chromatographisch gereinigt wird. Man erhält das 1-[2-(Chroman-3-yl)äthyl]-4-oxo-piperidin in Form eines hellgelben Oeles.

Beispiel 29:

Eine Suspension von 1,2 g Natriumhydrid (50%ige Suspension in Mineralöl) in 25 ml trockenem Tetrahydrofuran wird mit einer Lösung von 2,7 g (25 mmol) Benzylalkohol in 25 ml Tetrahydrofuran versetzt und nach Abklingen der Gasentwicklung 30 Minuten unter Rückfluss erhitzt. Nach dem Erkalten fügt man tropfenweise eine Lösung von 8,5 g (25 mmol) 1-[2-(Benzo-1,4-dioxan-2-yl)äthyl]-1,2,5,6-tetrahydro-pyridin-3-carbonsäuremethylester in 50 ml Tetrahydrofuran hinzu und erhitzt erneut 5 Stunden unter Rückfluss. Nach dem Erkalten wird das Lösungsmittel entfernt. Man erhält ein Gemisch von cis- und trans-1-[2-(Benzo-1,4-dioxan-2-yl)äthyl]-4-benzyloxy-piperidin-3-carbonsäuremethylester in Form eines Oeles.

Beispiel 30:

In eine Suspension von 8,45 g (18 mmol) 1-[2-(Chroman-3-yl)äthyl]-3-methoxycarbonyl-pyridinium-p-toluolsulfonat in 43 ml Methanol werden unter Rühren bei -10° innerhalb von 90 Minuten 1,41 g Natriumborhydrid eingetragen. Man lässt 1 Stunde bei 0° und 2 Stunden bei Raumtemperatur nachrühren, versetzt dann das Reaktionsgemisch mit 50 ml Wasser und schüttelt zweimal mit je 100 ml Dichlormethan aus. Die Dichlormethanphasen werden vereinigt, über Magnesiumsulfat getrocknet und zur Trockne eingedampft. Das Rohprodukt wird an 150 g Kieselgel (0,063-0,2 mm) mit Aethylacetat als Laufmittel chromatographisch gereinigt. Durch Behandeln des eingedampften Haupteluates mit ätherischer Salzsäure erhält man das 1-[2-(Chroman-3-yl)äthyl]-1,2,5,6-tetrahydro-pyridin-3-carbonsäuremethylester-hydrochlorid vom Smp. 175-177°.

Das 1-[2-(Chroman-3-yl)äthyl]-3-methoxycarbonyl-pyridinium-p-toluolsulfonat kann z.B. folgendermassen hergestellt werden.

16,6 g (50 mmol) 3-[2-(p-Toluolsulfonyloxy)äthyl]chroman und 9,3 g (67,5 mmol) Pyridin-3-carbonsäuremethylester werden in 50 ml Butan-2-on suspendiert und die Suspension 72 Stunden unter Rühren zum Sieden erhitzt. Man lässt abkühlen, engt das Reaktionsgemisch unter vermindertem Druck ein und erhält so das 1-[2-(Chroman-3-yl)äthyl]-3-methoxycarbonyl-pyridinium-p-toluolsulfonat in Form eines weissen Schaumes.

Beispiel 31:

33,2 g (0,1 mol) 3-[2-(p-Toluolsulfonyloxy)äthyl]-chroman, 14,0 g N-(2-Methoxycarbonyläthyl)amin-hydrochlorid und 39 g N-Aethyl-N,N-diisopropyl-amin werden unter Stickstoff in 750 ml Dimethylformamid gelöst und die Lösung 16 Stunden bei Raumtemperatur gerührt. Danach wird das Reaktionsgemisch unter vermindertem Druck auf etwa 200 ml eingeengt, sodann mit 500 ml Waser versetzt und dreimal mit je 150 ml Dichlormethan ausgeschüttelt. Die vereinigten organischen Phasen werden über Natriumsulfat getrocknet und zur Trockne eingedampft. Durch Versetzen mit äthanolischer Salzsäure und Abkühlen erhält man das

N-[2-(Chroman-3-yl)äthyl]-N-(2-methoxycarbonyläthyl)-amin-hydrochlorid vom Smp. 190-192°.

Beispiel 32:

Eine Lösung von 11,5 g (0,05 mmol) N-[2-(Chroman-3-yl)äthyl]-N-(2-cyanoäthyl)-amin in 100 ml Methanol wird mit 6 ml konzentrierter Salzsäure versetzt. Das Reaktionsgemisch wird 15 Stunden unter Rückfluss gekocht. Nach dem Erhalten wird das Lösungsmittel unter vermindertem Druck entfernt und der Rückstand aus Methanol/Aceton kristallisiert. Nach dem Umkristallisieren aus Methanol/Aceton erhält man das N-[2-(Chroman-3-yl)äthyl]-N-(2-methoxycarbonyläthyl)-amin-hydrochlorid vom Smp. 190-192° (Ausbeute: 82 %).

Das N-[2-(Chroman-3-yl)äthyl]-N-(2-cyanoäthyl)-amin kann man z.B. folgendermassen herstellen.

17,7 g (0,1 mol) 3-(2-Aminoäthyl)chroman werden in 100 ml Methanol gelöst und die Lösung mit 10,5 g (0,1 mol) Triäthylamin und 5,84 g (0,11 mol) Acrylnitril versetzt. Man rührt. 15 Stunden bei Raumtemperatur und engt das Reaktionsgemisch dann im Wasserstrahlvakuum ein. Der Rückstand wird in Diäthyläther aufgenommen und mit Eiswasser neutral gewaschen. Die ätherische Phase wird über Kaliumcarbonat getrocknet und eingedampft. Man erhält so das N-[2-(Chroman-3-yl)äthyl]-N-(2-cyanoäthyl)-amin in Form eines hellgelben Oeles.

Beispiel 33:

5,2 g eines Gemisches von cis- und trans-1-[2-(Benzo-1,4-dioxan-2-yl)äthyl]-4-benzyloxy-piperidin-3-carbonsäuremethylester werden in 100 ml Methanol gelöst, mit 2 g Palladium auf Kohle (10 %) versetzt und in einer Parr-Apparatur 12 Stunden bei Raumtemperatur hydriert. Das Reaktionsgemisch wird sodann über Diatomeenerde filtriert und das Filtrat zur Trockne eingedampft. Der rohe ölige Eindampfrückstand wird über Kieselgel mit Toluol/Aethylacetat (9:1) als Laufmittel chromatographiert. Zunächst wird der trans-1-[2-(Benzo-1,4-dioxan-2-yl)äthyl]-4-hydroxy-piperidin-3-carbonsäuremethylester und dann der cis-1-[2-(Benzo-1,4-dioxan-2-yl)äthyl]-4-hydroxy-piperidin-3-carbonsäuremethylester eluiert. Die gereinigten Fraktionen werden jeweils vereinigt und eingedampft. Der Rückstand, der das trans-Produkt enthält, wird mit Fumarsäure in Methanol/Diäthyläther behandelt und ergibt so das trans-1-[2-(Benzo-1,4-dioxan-2-yl)äthyl]-4-hydroxy-piperidin-3-carbonsäuremethylester-fumarat, das als Hemihydrat auskristallisiert und bei 150-152° schmilzt. Der Rückstand, welcher das cis-Prdodukt enthält, wird mit ätherischer Salzsäure behandelt und liefert so das cis-1-[2-(Benzo-1,4-dioxan-2-yl)äthyl]-4-hydroxy-piperidin-3-carbonsäuremethylester-hydrochlorid vom Smp. 182-185°.

Beispiel 34:

6,8 g (20 mmol) eines Gemisches von cis- und trans-1-[2-(Benzo-1,4-dioxan-2-yl)äthyl]-4-chlor-piperidin-3-carbonsäuremethylester werden in 20 ml Methanol gelöst. Bei Raumtemperatur werden 40 ml (140 mmol) einer 3,5N-Lösung von Ammoniak in Methanol zugetropft. Das Gemisch wird 24 Std. bei Raumtemperatur stehengelassen. Sodann wird das Lösungsmittel unter vermindertem Druck entfernt. Der erhaltene Rückstand wird in Dichlormethan gelöst, die Lösung mit 2N-Salzsäure ausgeschüttelt und die saure wässrige Phase abgetrennt, mit Natriumhydrogencarbonat alkalisch gestellt und mit Diäthyläther/Dichlormethan (2:1) extrahiert. Die organischen Extrakte werden mit gesättigter Natriumchloridlösung gewaschen, über Magnesiumsulfat getrocknet und unter vermindertem Druck vom Lösungsmittel befreit. Der erhaltene Rückstand wird an basischem Kieselgel mit Dichlormethan/Methanol (99:1) als Laufmittel chromatographiert. Die Eluate werden vereinigt und zur Trockne eingedampft. Der ölige Rückstand besteht aus reinem 4-Amino-1-[2-benzo-1,4-dioxan-2-yl)äthyl]-piperidin-3-carbonsäureamid (cis/trans-Gemisch).

Ein Gemisch von cis- und trans-1-[2-(Benzo-1,4-dioxan-2-yl)äthyl]-4-chlor-piperidin-3-carbonsäuremethylester kann man beispielsweise wie folgt erhalten:

9,7 g (30 mmol) eines Gemisches von cis- und trans-1-[2-(Benzo-1,4-dioxan-2-yl)äthyl]-4-hydroxy-piperidin-3-carbonsäuremethylester (Herstellung siehe Beispiel 33) und 3,6 g (36 mmol) Triäthylamin werden in 100 ml Dichlormethan gelöst. Bei Raumtemperatur werden unter Rühren 3,92 g (33 mmol) Thionylchlorid zugetropft. Das Gemisch wird 4 Std. bei Raumtemperatur gerührt. Sodann wird vom ausgefallenen Triäthylaminhydrochlorid abfiltriert und das Filtrat kalt mit gesättigter Natriumhydrogencarbonatlösung ausgeschüttelt. Die organische Phase wird mit gesättigter Natriumchloridlösung gewaschen, über Magnesiumsulfat getrocknet und unter vermindertem Druck vom Lösungsmittel befreit. Das erhaltene Gemisch von cis- und trans-1-[2-(Benzo-1,4-dioxan-2-yl)äthyl]-4-chlor-piperidin-3-carbonsäuremethylester wird roh weiterverwendet.

Beispiel 35:

Eine Lösung von 11,94 g (30 mmol) N-(3-Aethoxycarbonylpropyl)-N-(2-bromäthyl)-N-[2-(chroman-3-yl)-äthyl]-amin in 40 ml absolutem Dimethylformamid wird unter Rühren bei Raumtemperatur zu einer Suspension von 2,72 g (40 mmol) Natriumäthanolat in 30 ml Dimethylformamid innert 20 Minuten zugetropft. Das Reaktionsgemisch wird 16 Stunden bei Raumtemperatur gerührt und danach im Hockvakuum zur Trockne eingedampft. Der Rückstand wird mit Diäthyläther versetzt und mit kalter 2N-Salzsäure extrahiert. Die vereinigten salzsauren Extrakte werden mit Dichlormethan ausgeschüttelt und die Dichlormethan-Phasen über Natriumsulfat getrocknet und im Vakuum eingedampft. Man erhält als Rückstand das rohe Produkt, welches über 500 g Kieselgel (0,040-0,063 mm) mit Aethylacetat als Laufmittel chromatographiert wird. Nach Eindampfen des Eluats erhält man den 1-[2-(Chroman-3-yl)äthyl]-piperidin-4-carbonsäureäthylester als farbloses Oel. Das hieraus mit Salzsäure in Diäthyläther hergestellte 1-[2-(Chroman-3-yl)äthyl]-piperidin-4-carbonsäureäthylester-hydrochlorid kristallisiert aus Aethanol/Diäthyläther und schmilzt bei 182 - 186°.

Das N-(3-Aethoxycarbonylpropyl)-N-(2-bromäthyl)-N-[2-(chroman-3-yl)äthyl]-amin kann man z.B. folgendermassen herstellen.

17,7 g (0,1 mol) 3-(2-Aminoäthyl)chroman werden in 100 ml Methanol gelöst und die Lösung mit 10,5 g (0,1 mol) Triäthylamin und 19,5 g (0,1 mol) 4-Brombuttersäureäthylester versetzt. Man rührt 15 Stunden bei Raumtemperatur und engt das Reaktionsgemisch dann im Wasserstrahlvakuum ein. Der Rückstand wird in Diäthyläther aufgenommen und mit Eiswasser neutral gewaschen. Die ätherische Phase wird über Kaliumcarbonat getrocknet und eingedampft. Man erhält so das ölige N-(3-Aethoxycarbonylpropyl)-N-[2-(chroman-3-yl)äthyl]-amin, das in roher Form weiterverwendet werden kann.

29,1 g (0,1 mol) N-(3-Aethoxycarbonylpropyl)-N-[2-(chroman-3-yl)äthyl]-amin werden in 200 ml Methanol gelöst und die Lösung mit 10,5 (0,1 mol) Triäthylamin und 18,8 g (0,1 mol) 1,2-Dibromäthan versetzt. Man rührt 16 Stunden bei Raumtemperatur und engt das Reaktionsgemisch anschliessend im Wasserstrahlvakuum ein. Der Rückstand wird in Diäthyläther aufgenommen und mit Eiswasser neutral gewaschen. Die ätherische Phase wird über Kaliumcarbonat getrocknet und eingedampft. Man erhält so das N-(3-Aethoxycarbonylpropyl)-N-(2-bromäthyl)-N-[2-(chroman-3-yl)äthyl]-amin in Form eines Oeles, welches roh weiterverwendet werden kann.

Beispiel 36:

In analoger Weise wie in den Beispielen 4, 5, 10, 12, 23, 26, 31 und 32 beschrieben kann man auch das N-[2-(Chroman-4-yl)äthyl]-N-(2-methoxycarbonyläthyl)-amin und dessen Hydrochlorid erhalten.

Beispiel 37:

In analoger Weise sie in den Beispielen 1 bis 3, 6 bis 9, 11, 13 bis 22, 24, 25, 27 bis 30 und 33 bis 35 beschrieben kann man auch den 1-[2-(Chroman-4-yl)äthyl]-1,2,5,6-tetrahydropyridin-3-carbonsäuremethylester und dessen Hydrochlorid sowie den 1-[2-(Chroman-4-yl)äthyl]-piperidin-3-carbonsäuremethylester und dessen Hydrochlorid erhalten.

Beispiel 38:

Tabletten, enthaltend 25 mg Wirkstoff, z.B. 1-(Benzo-1,4-dioxan-2-ylmethyl)-piperidin-3-carbonsäuremethylester-hydrochlorid, können folgendermassen hergestellt werden:

Bestandteile (für 1000 Tabletten):
Wirkstoff 25,0 g
Lactose 100,7 g
Weizenstärke 7,5 g
Polyäthylenglykol 6000 5,0 g
Talkum 5,0 g
Magnesiumstearat 1,8 g
entmineralisiertes Wasser q.s.

Herstellung:

Sämtliche festen Ingredienzien werden zunächst durch ein Sieb mit 0,6 mm Maschenweite getrieben. Dann werden der Wirkstoff, die Lactose, das Talkum, das Magnesiumstearat und die Hälfte der Stärke vermischt. Die andere Hälfte der Stärke wird in 40 ml Wasser suspendiert und diese Suspension zu einer siedenden Lösung des Polyäthylenglykols in 100 ml Wasser hinzugegeben. Der erhaltene Stärkekleister wird zu der Hauptmenge hinzugegeben und das Gemisch, wenn nötig unter Hinzufügen von Wasser, granuliert. Das Granulat wird über Nacht bei 35° getrocknet, durch ein Sieb mit 1,2 mm Maschenweite getrieben und zu beidseitig konkaven Tabletten von etwa 6 mm Durchmesser verpresst.

Beispiel 39:

Tabletten, enthaltend 50 mg des Wirkstoffs, z.B. 1-(Benzo-1,4-dioxan-2-ylmethyl)-piperidin-3-carbonsäuremethylester-hydrochlorid, werden wie folgt hergestellt:

Zusammensetzung (für 10 000 Tabletten):
Wirkstoff 500,00 g
Lactose 140,80 g
Kartoffelstärke 274,70 g
Stearinsäure 10,00 g
Talk 50,00 g
Magnesiumstearat 2,50 g
Kolloidales Siliciumdioxid 32,00 g
Aethanol q.s.

Ein Gemisch des Wirkstoffs, der Lactose und 194,70 g Kartoffelstärke wird mit einer äthanolischen Lösung der Stearinsäure befeuchtet und durch ein Sieb granuliert. Nach dem Trocknen mischt man die restliche Kartoffelstärke, den Talk, das Magnesiumstearat und das kolloidale Siliciumdioxid zu und presst die Mischung zu Tabletten von je 0,1 g Gewicht, die gewünschtenfalls mit Teilkerben zur feineren Anpassung der Dosierung versehen sein können.

In analoger Weise können 100 mg Wirkstoff eingearbeitet werden.

Beispiel 40:

Kapseln, enthaltend 0,025 g des Wirkstoffs, z.B. 1-(Benzo-1,4-dioxan-2-ylmethyl)-piperidin-3-carbonsäuremethylester-hydrochlorid, können wie folgt hergestellt werden:

Zusammensetzung (für 1000 Kapseln):
Wirkstoff 25,00 g
Lactose 249,00 g
Gelatine 2,00 g
Maisstärke 10,00 g
Talk 15,00 g
Wasser q.s.

Man mischt den Wirkstoff mit der Lactose, befeuchtet die Mischung gleichmässig mit einer wässrigen Lösung der Gelatine und granuliert sie durch ein Sieb mit einer Maschenweite von 1,2 bis 1,5 mm. Das Granulat mischt man mit der getrockneten Maisstärke und dem Talk und füllt Portionen von 300 mg in Hartgelatinekapseln (Grösse 1) ab.

Beispiel 41:

In analoger Weise wie in den Beispielen 38 bis 40 beschrieben, können auch pharmazeutische Präparate, als Wirkstoff enthaltend das N-[2-(Chroman-3-yl)äthyl]-N-(2-methoxycarbonyläthyl)-amin-hydrochlorid, hergestellt werden.

Beispiel 42:

In analoger Weise wie in den Beispielen 38 bis 41 beschrieben können auch pharmazeutische Präparate, als Wirkstoff enthaltend eine andere Verbindung der Formel I oder ein Tautomeres und/oder ein pharmazeutisch verwendbares Salz davon oder eine andere Verbindung der Formel IVc oder ein pharmazeutisch verwendbares Salz davon, beispielsweise gemäss den Beispielen 1 bis 37, hergestellt werden.

## Ansprüche

**Patentansprüche für folgende Vertragsstaaten : BE, CH, DE, FR, GB, IT, LI, LU, NL, SE**

1. Eine Verbindung der Formel

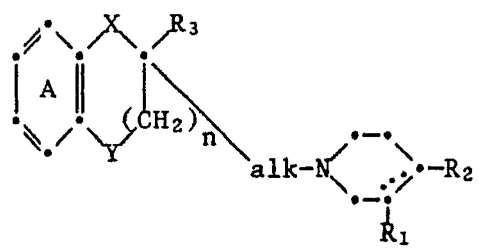

(I),

worin entweder $R_1$ Carboxy, Niederalkoxycarbonyl, Carbamyl, N-Niederalkylcarbamyl, N,N-Diniederalkylcarbamyl, Hydroxymethyl, $C_2$-$C_5$-Alkanoyloxymethyl, Niederalkansulfonyloxymethyl, Benzoyloxymethyl oder Pyridoyloxymethyl bedeutet und $R_2$ Wasserstoff, Hydroxy, Niederalkoxy, Benzyloxy, $C_2$-$C_5$-Alkanoyloxy, Niederalkansulfonyloxy, Benzoyloxy, Pyridoyloxy, Amino, $C_2$-$C_5$-Alkanoylamino, Niederalkansulfonylamino, Benzoylamino oder Pyridoylamino darstellt oder $R_1$ Wasserstoff bedeutet und $R_2$ für Carboxy, Niederalkoxycarbonyl, N-Niederalkylcarbamyl, N,N-Diniederalkylcarbamyl, Hydroxymethyl, $C_2$-$C_5$-Alkanoyloxymethyl, Niederalkansulfonyloxymethyl, Benzoyloxymethyl oder Pyridoyloxymethyl steht, $R_3$ Wasserstoff oder $C_1$-$C_7$-Alkyl bedeutet, alk Niederalkylen, das die beiden Ringsysteme durch bis und mit 3 C-Atome überbrückt, oder Niederalkyliden darstellt, der Ring A unsubstituiert ist oder ein-, zwei- oder mehrfach durch Hydroxy, Niederalkoxy, $C_2$-$C_5$-Alkanoyloxy, Halogen mit einer Atomnummer bis und mit 53, $C_1$-$C_7$-Alkyl und/oder Trifluormethyl substituiert ist, die gestrichelte Linie zum Ausdruck bringen soll, dass zwischen den Kohlenstoffatomen, welche die Reste $R_1$ und $R_2$ tragen, eine Einfach- oder eine Doppelbindung vorliegt, und entweder X und Y jeweils ein Sauerstoffatom bedeuten und n für 1 steht oder X eine Methylengruppe darstellt, Y ein Sauerstoffatom bedeutet und n für 1 steht oder X ein Sauerstoffatom darstellt, Y eine Methylengruppe bedeutet und n für 1 steht oder X für eine direkte Bindung steht, Y ein Sauerstoffatom darstellt und n für 2 steht, wobei mit "Nieder" bezeichnete Reste 1 bis und mit 4 Kohlenstoffatome aufweisen können, oder ein Tautomeres und/oder Salz davon.

2. Eine Verbindung der Formel I gemäss Anspruch 1, worin $R_1$ Carboxy, Niederalkoxycarbonyl, Carbamyl, N-Niederalkylcarbamyl oder N,N-Diniederalkylcarbamyl bedeutet, $R_2$ Wasserstoff, Hydroxy, Niederalkoxy, Benzyloxy, $C_2$-$C_5$-Alkanoyloxy, Niederalkansulfonyloxy, Benzoyloxy, Pyridoyloxy, Amino, $C_2$-$C_5$-Alkanoylamino, Benzoylamino oder Pyridoylamino darstellt, $R_3$ Wasserstoff oder $C_1$-$C_7$-Alkyl bedeutet, alk Niederalkylen, das die beiden Ringsysteme durch bis und mit 3 C-Atome überbrückt, oder Niederalkyliden darstellt, der Ring A unsubstituiert ist oder ein-, zwei- oder mehrfach durch Hydroxy, Niederalkoxy, $C_2$-$C_5$-Alkanoyloxy, Halogen mit einer Atomnummer bis und mit 53, $C_1$-$C_7$-Alkyl und/oder Trifluormethyl substituiert ist, die gestrichelte Linie zum Ausdruck bringen soll, dass zwischen den Kohlenstoffatomen, welche die Reste $R_1$ und $R_2$ tragen, eine Einfach- oder eine Doppelbindung vorliegt, X ein Sauerstoffatom oder eine Methylengruppe darstellt, Y ein Sauerstoffatom bedeutet und n für 1 steht, wobei mit "Nieder" bezeichnete Reste 1 bis und mit 4 Kohlenstoffatome aufweisen können, oder ein Tautomeres und/oder Salz davon.

3. Eine Verbindung der Formel I gemäss Anspruch 1, worin entweder $R_1$ $C_1$-$C_4$-Alkoxycarbonyl, Carbamyl, Hydroxymethyl oder $C_2$-$C_5$-Alkanoyloxymethyl bedeutet und $R_2$ Wasserstoff oder Hydroxy bedeutet oder $R_1$ Wasserstoff darstellt und $R_2$ für $C_1$-$C_4$-Alkoxycarbonyl steht, $R_3$ Wasserstoff oder $C_1$-$C_4$-Alkyl ist, alk für $C_1$-$C_4$-Alkylen, welches die beiden Ringsysteme durch bis und mit 3 C-Atome überbrückt, steht, der Ring A unsubstituiert oder, insbesondere in 7-Stellung, durch $C_1$-$C_4$-Alkoxy substituiert ist, die gestrichelte Linie zum Ausdruck bringen soll, dass zwischen den Kohlenstoffatomen, welche die Reste $R_1$ und $R_2$ tragen, eine Einfach- oder eine Doppelbindung vorliegt, und entweder X

und Y jeweils ein Sauerstoffatom bedeuten und n für 1 steht oder X eine Methylengruppe darstellt, Y ein Sauerstoffatom bedeutet und n für 1 steht oder X ein Sauerstoffatom darstellt, Y eine Methylengruppe bedeutet und n für 1 steht oder X für eine direkte Bindung steht, Y ein Sauerstoffatom darstellt und n für 2 steht, oder ein Tautomeres und/oder Salz davon.

4. Eine Verbindung der Formel I gemäss Anspruch 1, worin $R_1$ $C_1$-$C_4$-Alkoxycarbonyl bedeutet, $R_2$ Wasserstoff oder Hydroxy bedeutet, $R_3$ Wasserstoff oder $C_1$-$C_4$-Alkyl ist, alk für $C_1$-$C_4$-Alkylen, welches die beiden Ringsysteme durch bis und mit 3 C-Atome überbrückt, steht, der Ring A unsubstituiert ist, die gestrichelte Linie zum Ausdruck bringen soll, dass zwischen den Kohlenstoffatomen, welche die Reste $R_1$ und $R_2$ tragen, eine Einfach- oder eine Doppelbindung vorliegt, X ein Sauerstoffatom oder eine Methylengruppe bedeutet, Y ein Sauerstoffatom darstellt und n für 1 steht, oder ein Tautomeres und/oder Salz davon.

5. Eine Verbindung der Formel I gemäss Anspruch 1, worin $R_1$ $C_1$-$C_4$-Alkoxycarbonyl bedeutet, $R_2$ Wasserstoff oder Hydroxy darstellt, $R_3$ Wasserstoff ist, alk für Methylen oder Aethylen steht, der Ring A unsubstituiert ist, die gestrichelte Linie zum Ausdruck bringen soll, dass zwischen den Kohlenstoffatomen, welche die Reste $R_1$ und $R_2$ tragen, eine Einfach- oder eine Doppelbindung vorliegt, X ein Sauerstoffatom oder eine Methylengruppe bedeutet, Y ein Sauerstoffatom darstellt und n für 1 steht, oder ein Tautomeres und/oder Salz davon.

6. 4-Hydroxy-1-(chroman-3-ylmethyl)-1,2,5,6-tetrahydro-pyridin-3-carbonsäuremethylester bzw. 1-(Chroman-3-ylmethyl)-4-oxo-piperidin-3-carbonsäuremethylester, 1-[2-(Chroman-3-yl)äthyl]-1,2,5,6-tetrahydro-pyridin-3-carbonsäuremethylester, 1-(Benzo-1,4-dioxan-2-ylmethyl)-piperidin-3-carbonsäuremethylester oder 4-Hydroxy-1-(benzo-1,4-dioxan-2-ylmethyl)-1,2,5,6-tetrahydro-pyridin-3-carbonsäuremethylester bzw. 1-(Benzo-1,4-dioxan-2-ylmethyl)-4-oxo-piperidin-3-carbonsäuremethylester oder jeweils ein Salz davon.

7. 1-[2-(Chroman-3-yl)äthyl]-1,2,5,6-tetrahydro-pyridin-3-carbonsäureäthylester oder ein Salz davon.

8. 1-(Chroman-3-ylmethyl)-1,2,5,6-tetrahydro-pyridin-3-carbonsäuremethylester, 1-[2-(Chroman-3-yl)äthyl]-4-hydroxy-1,2,5,6-tetrahydro-pyridin-3-carbonsäuremethylester bzw. 1-[2-(Chroman-3-yl)äthyl]-4-oxo-piperidin-3-carbonsäuremethylester, 1-(Benzo-1,4-dioxan-2-ylmethyl)-1,2,5,6-tetrahydro-pyridin-3-carbonsäuremethylester, 4-Hydroxy-1-(2-methyl-benzo-1,4-dioxan-2-ylmethyl)-1,2,5,6-tetrahydro-pyridin-3-carbonsäuremethylester bzw. 1-(2-Methyl-benzo-1,4-dioxan-2-ylmethyl)-4-oxo-piperidin-3-carbonsäuremethylester, 1-[2-(Benzo-1,4-dioxan-2-yl)äthyl]-4-hydroxy-1,2,5,6-tetrahydropyridin-3-carbonsäuremethylester bzw. 1-[2-(Benzo-1,4-dioxan-2-yl)äthyl]-4-oxo-piperidin-3-carbonsäuremethylester, cis-1-[2-(Benzo-1,4-dioxan-2-yl)äthyl]-4-hydroxy-piperidin-3-carbonsäuremethylester, trans-1-[2-(Benzo-1,4-dioxan-2-yl)äthyl]-4-hydroxy-piperidin-3-carbonsäuremethylester oder 1-[2-(Benzo-1,4-dioxan-2-yl)-äthyl]-1,2,5,6-tetrahydro-pyridin-3-carbonsäuremethylester oder jeweils ein Salz davon.

9. 1-[2-(Chroman-3-yl)äthyl]-piperidin-3-carbonsäureäthylester, 1-[2-(Chroman-3-yl)äthyl]-piperidin-4-carbonsäuremethylester, 1-[2-(Benzo-1,4-dioxan-2-yl)äthyl]-piperidin-3-carbonsäureäthylester, 1-[2-(Benzo-1,4-dioxan-2-yl)äthyl]-piperidin-4-carbonsäureäthylester, 1-[2-(Chroman-2-yl)äthyl]-piperidin-3-carbonsäureäthylester, 1-[2-(Chroman-2-yl)äthyl]-piperidin-4-carbonsäureäthylester, cis-1-[2-(Benzo-1,4-dioxan-2-yl)äthyl]-4-benzyloxy-piperidin-3-carbonsäuremethylester, trans-1-[2-(Benzo-1,4-dioxan-2-yl)-äthyl]-4-benzyloxy-piperidin-3-carbonsäuremethylester, cis-4-Amino-1-[2-(benzo-1,4,-dioxan-2-yl)äthyl]-piperidin-3-carbonsäureamid, trans-4-Amino-1-[2-(benzo-1,4-dioxan-2-yl)äthyl]-piperidin-3-carbonsäure amid 1-[2-(Chroman-4-yl)äthyl]-1,2,5,6-tetrahydro-pyridin-3-carbonsäuremethylester oder 1-[2-(Chroman-4-yl)äthyl]-piperidin-3-carbonsäureäthylester oder jeweils ein Salz davon.

10. Verfahren zur Herstellung einer Verbindung der Formel I oder eines Tautomeren und/oder Salzes davon gemäss einem der Ansprüche 1 bis 9, dadurch gekennzeichnet, dass man

a) eine Verbindung der Formel

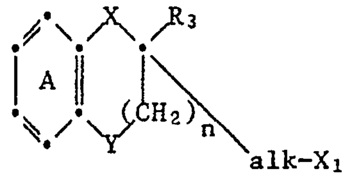

(IIa)

oder ein Salz davon, worin $X_1$ Hydroxy oder reaktionsfähiges verestertes Hydroxy bedeutet, mit einer Verbindung der Formel

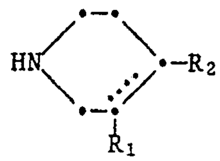

(IIb)

oder einem Tautomeren und/oder Salz davon umsetzt oder
b) in einer Verbindung der Formel

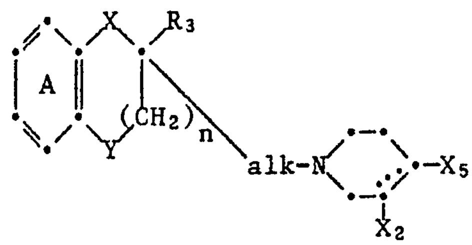

(III)

oder einem Tautomeren und/oder Salz davon, worin $X_2$ einen in von Wasserstoff verschiedenes $R_1$ überführbaren Rest bedeutet und $X_5$ einen Rest $R_a$ darstellt, wobei $R_a$ Wasserstoff, Hydroxy, Niederalkoxy, Benzyloxy, $C_2$-$C_5$-Alkanoyloxy, Niederalkansulfonyloxy, Benzoyloxy, Pyridoyloxy, Amino, $C_2$-$C_5$-Alkanoylamino, Niederalkansulfonylamino, Benzoylamino oder Pyridoylamino ist, $X_2$ in von Wasserstoff verschiedenes $R_1$ überführt oder in einer Verbindung der Formel III, worin $X_2$ Wasserstoff ist und $X_5$ einen in $R_b$ überführbaren Rest bedeutet, wobei $R_b$ für einen von einem Rest $R_a$ verschiedenen Rest $R_2$ steht, $X_5$ in $R_b$ überführt oder
c) zur Herstellung einer Verbindung der Formel I oder eines Tautomeren und/oder Salzes davon, worin $R_2$ Hydroxy oder Amino bedeutet und $R_1$ von Wasserstoff verschieden ist und insbesondere Niederalkoxycarbonyl bedeutet, eine Verbindung der Formel

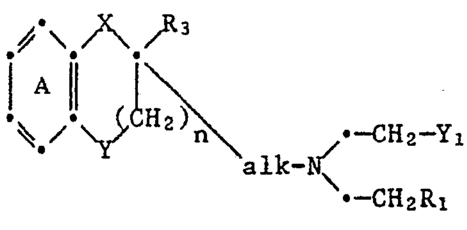

(IV),

worin $Y_1$ eine Gruppe der Formel $-CH=R_2'$, $-C(Y_2)=R_2'$, $-CH(Y_2)-R_2$ oder Cyano darstellt, wobei $R_2'$ Oxo oder Imino bedeutet und $Y_2$ einen abspaltbaren Rest darstellt, oder ein Salz davon cyclisiert oder
d) zur Herstellung einer Verbindung der Formel I' oder eines Tautomeren und/oder Salzes davon, worin $R_2'$ Oxo oder Imino bedeutet und $R_1$ von Wasserstoff verschieden ist und insbesondere Niederalkoxycarbonyl bedeutet, eine Verbindung der Formel

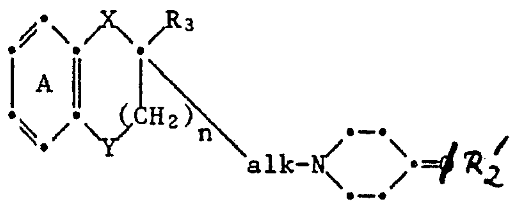

(Va)

oder ein Tautomeres oder jeweils ein Salz davon mit einer Verbindung der Formel

$X_3$-$R_1$ (Vb)

oder einem Salz davon, worin $R_1$ von Wasserstoff verschieden ist und $X_3$ Halogen oder Niederalkoxy bedeutet, umsetzt oder

e) zur Herstellung einer Verbindung der Formel I oder eines Tautomeren und/oder Salzes davon, worin $R_2$ Hydroxy, Niederalkoxy, Benzyloxy, $C_2$-$C_5$-Alkanoyloxy, Niederalkansulfonyloxy, Benzoyloxy, Pyridoyloxy, Amino, $C_2$-$C_5$-Alkanoylamino, Niederalkansulfonylamino, Benzoylamino oder Pyridoylamino ist, in einer Verbindung der Formel

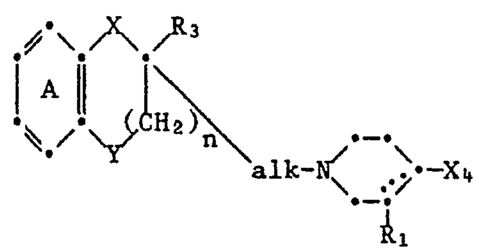

(VI)

oder einem Salz davon, worin $X_4$ einen in $R_2$ überführbaren Rest bedeutet, $X_4$ in $R_2$ überführt oder

f) insbesondere zur Herstellung einer Verbindung der Formel I oder eines Tautomeren und/oder Salzes davon, worin $R_2$ Wasserstoff, Hydroxy, Amino, Carboxy, Niederalkoxycarbonyl, N-Niederalkylcarbamyl, N,N-Diniederalkylcarbamyl oder Hydroxymethyl ist, in einer Verbindung der Formel

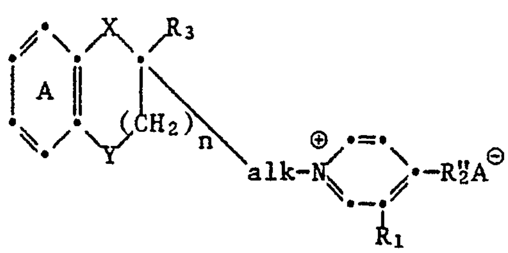

(VII),

worin $A^{\ominus}$ für das Anion einer Säure steht und $R_2''$ Wasserstoff, Niederalkoxy, Benzyloxy, $C_2$-$C_5$-Alkanoyloxy, Niederalkansulfonyloxy, Benzoyloxy, Pyridoyloxy, geschütztes Hydroxy, $C_2$-$C_5$-Alkanoylamino, Niederalkansulfonylamino, Benzoylamino, Pyridoylamino, geschütztes Amino, Carboxy, Niederalkoxycarbonyl, N-Niederalkylcarbamyl, N,N-Diniederalkylcarbamyl, $C_2$-$C_5$-Alkanoyloxymethyl, Niederalkansulfonyloxymethyl, Benzoyloxymethyl, Pyridoyloxymethyl oder veräthertes oder geschütztes Hydroxymethyl bedeutet, die überschüssigen Doppelbindungen zu Einfachbindungen reduziert oder

g) zur Herstellung einer Verbindung der Formel I oder eines Tautomeren und/oder Salzes davon, worin $R_2$ Carboxy, N-Niederalkylcarbamyl, N-N-Diniederalkylcarbamyl oder insbesondere Niederalkoxycarbonyl bedeutet, eine Verbindung der Formel

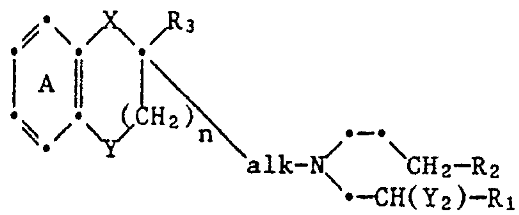

(VIII),

worin $Y_2$ einen abspaltbaren Rest darstellt, oder ein Salz davon cyclisiert und jeweils eine gegebenenfalls vorhandene Schutzgruppe abspaltet und jeweils gewünschtenfalls eine verfahrensgemäss oder auf andere Weise erhältliche Verbindung der Formel I in eine andere Verbindung der Formel I überführt, jeweils ein verfahrensgemäss erhältliches Isomerengemisch in die Komponenten auftrennt, jeweils ein verfahrensgemäss erhältliches Enantiomeren- bzw. Diastereomerengemisch in die Enantiomeren bzw. Diastereomeren aufspaltet und/oder jeweils eine verfahrensgemäss erhältliche freie Verbindung der Formel I in ein Salz überführt oder ein verfahrensgemäss erhältliches Salz in die freie Verbindung der Formel I oder in ein anderes Salz umwandelt, wobei mit "Nieder" bezeichnete Reste 1 bis und mit 4 Kohlenstoffatome aufweisen können.

**11.** Eine Verbindung der Formel

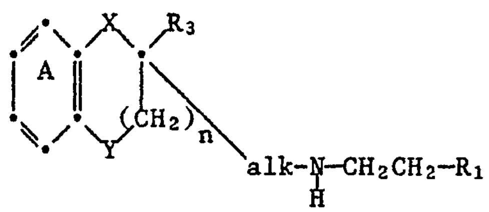

(IVc),

worin $R_1$ Carboxy, Niederalkoxycarbonyl, Carbamyl, N-Niederalkylcarbamyl, N,N-Diniederalkylcarbamyl, Hydroxymethyl, $C_2$-$C_5$-Alkanoyloxymethyl, Niederalkansulfonyloxymethyl, Benzoyloxymethyl oder Pyridoyloxymethyl bedeutet, $R_3$ Wasserstoff oder $C_1$-$C_7$-Alkyl darstellt, alk Niederalkylen oder Niederalkyliden bedeutet, der Ring A unsubstituiert oder ein- oder mehrfach durch Hydroxy, Niederalkoxy, $C_2$-$C_5$-Alkanoyloxy, Halogen mit einer Atomnummer bis und mit 53, $C_1$-$C_7$-Alkyl und/oder Trifluormethyl substituiert ist und entweder X und Y jeweils ein Sauerstoffatom bedeuten und n für 1 steht oder X eine Methylengruppe darstellt, Y ein Sauerstoffatom bedeutet und n für 1 steht oder X ein Sauerstoffatom darstellt, Y eine Methylengruppe bedeutet und n für 1 steht oder X für eine direkte Bindung steht, Y ein Sauerstoffatom darstellt und n für 2 steht, mit der Massgabe, dass in Verbindungen der Formel IVc, worin der Ring A unsubstituiert ist, X und Y jeweils Sauerstoff darstellen, n für 1 steht und $R_3$ Wasserstoff bedeutet, alk von Methylen verschieden ist, wenn $R_1$ Carbamyl oder N-Methyl-, N-Aethyl-, N,N-Dimethyl- oder N,N-Diäthylcarbamyl bedeutet, wobei mit "Nieder" bezeichnete Reste 1 bis und mit 4 Kohlenstoffatome aufweisen können, oder ein Salz davon.

**12.** Eine Verbindung der Formel IVc gemäss Anspruch 11, worin $R_1$ Carboxy, Niederalkoxycarbonyl, Carbamyl, N-Niederalkylcarbamyl oder N,N-Diniederalkylcarbamyl bedeutet, $R_3$ Wasserstoff oder $C_1$-$C_7$-Alkyl darstellt, alk Niederalkylen oder Niederalkyliden bedeutet, der Ring A unsubstituiert oder ein- oder mehrfach durch Hydroxy, Niederalkoxy, $C_2$-$C_5$-Alkanoyloxy, Halogen mit einer Atomnummer bis und mit 53, $C_1$-$C_7$-Alkyl und/oder Trifluormethyl substituiert ist, X ein Sauerstoffatom oder eine Methylengruppe darstellt, Y ein Sauerstoffatom bedeutet und n für 1 steht, mit der Massgabe, dass in Verbindung der Formel IVc, worin der Ring A unsubstituiert ist, X und Y jeweils Sauerstoff darstellen, n für 1 steht und $R_3$ Wasserstoff bedeutet, alk von Methylen verschieden ist, wenn $R_1$ Carbamyl oder N-Methyl-, N-Aethyl-, N,N-Dimethyl- oder N,N-Diäthylcarbamyl bedeutet, wobei mit "Nieder" bezeichnete Reste 1 bis und mit 4 Kohlenstoffatome aufweisen können, oder ein Salz davon.

**13.** Eine Verbindung der Formel IVc gemäss Anspruch 11, worin $R_1$ Carboxy, Niederalkoxycarbonyl, Carbamyl, N-Niederalkylcarbamyl, N,N-Diniederalkylcarbamyl, Hydroxymethyl, $C_2$-$C_5$-Alkanoyloxymethyl, Niederalkansulfonyloxymethyl, Benzoyloxymethyl oder Pyridoyloxymethyl bedeutet, $R_3$

Wasserstoff oder $C_1$-$C_7$-Alkyl darstellt, alk Niederalkylen, welches das Ringsystem mit der in Formel IVc eingezeichneten NH-Gruppe durch bis und mit 3 C-Atome verknüpft, oder Niederalkyliden darstellt, der Ring A unsubstituiert ist oder ein-, zwei- oder mehrfach durch Hydroxy, Niederalkoxy, $C_2$-$C_5$-Alkanoyloxy, Halogen mit einer Atomnummer bis und mit 53, $C_1$-$C_7$-Alkyl und/oder Trifluormethyl substituiert ist und entweder X und Y jeweils ein Sauerstoffatom bedeuten und n für 1 steht oder X eine Methylengruppe darstellt, Y ein Sauerstoffatom bedeutet und n für 1 steht oder X ein Sauerstoffatom darstellt, Y eine Methylengruppe bedeutet und n für 1 steht oder X für eine direkte Bindung steht, Y ein Sauerstoffatom darstellt und n für 2 steht, mit der Massgabe, dass in Verbindungen der Formel IVc, worin der Ring A unsubstituiert ist, X und Y jeweils Sauerstoff darstellen, n für 1 steht und $R_3$ Wasserstoff bedeutet, alk von Methylen verschieden ist, wenn $R_1$ Carbamyl oder N-Methyl-, N-Aethyl-, N,N-Dimethyl- oder N,N-Diäthylcarbamyl bedeutet, wobei mit "Nieder" bezeichnete Reste 1 bis und mit 4 Kohlenstoffatome aufweisen können, oder ein Salz davon.

**14.** Eine Verbindung der Formel IVc gemäss Anspruch 11, worin $R_1$ Carboxy, Niederalkoxycarbonyl, Carbamyl, N-Niederalkylcarbamyl oder N,N-Diniederalkylcarbamyl bedeutet, $R_3$ Wasserstoff oder $C_1$-$C_7$-Alkyl darstellt, alk Niederalkylen, welches das Ringsystem mit der in Formel IVc eingezeichneten NH-Gruppe durch bis und mit 3 C-Atome verknüpft, oder Niederalkyliden darstellt, der Ring A unsubstituiert ist oder ein-, zwei- oder mehrfach durch Hydroxy, Niederalkoxy, $C_2$-$C_5$-Alkanoyloxy, Halogen mit ein Atomnummer bis und mit 53, $C_1$-$C_7$-Alkyl und/oder Trifluormethyl substituiert ist, X ein Sauerstoffatom oder eine Methylengruppe darstellt, Y ein Sauerstoffatom bedeutet und n für 1 steht, mit der Massgabe, dass in Verbindungen der Formel IVc, worin der Ring A unsubstituiert ist, X und Y jeweils Sauerstoff darstellen, n für 1 steht und $R_3$ Wasserstoff bedeutet, alk von Methylen verschieden ist, wenn $R_1$ Carbamyl oder N-Methyl-, N-Aethyl-, N,N-Dimethyl- oder N,N-Diäthylcarbamyl bedeutet, wobei mit "Nieder" bezeichnete Reste 1 bis und mit 4 Kohlenstoffatome aufweisen können, oder ein Salz davon.

**15.** Eine Verbindung der Formel IVc gemäss Anspruch 11, worin $R_1$ Carboxy, Hydroxymethyl, $C_2$-$C_5$-Alkanoyloxymethyl, $C_1$-$C_4$-Alkoxycarbonyl oder Carbamyl bedeutet, $R_3$ Wasserstoff oder $C_1$-$C_4$-Alkyl ist, alk für $C_1$-$C_4$-Alkylen, welches das Ringsystem mit der in Formel IVc eingezeichneten NH-Gruppe durch bis und mit 3 C-Atome verknüpft, steht, der Ring A unsubstituiert oder, insbesondere in 7-Stellung, durch $C_1$-$C_4$-Alkoxy substituiert ist und entweder X und Y jeweils ein Sauerstoffatom bedeuten und n für 1 steht oder X eine Methylengruppe darstellt, Y ein Sauerstoffatom bedeutet und n für 1 steht oder X ein Sauerstoffatom darstellt, Y eine Methylengruppe bedeutet und n für 1 steht oder X für eine direkte Bindung steht, Y ein Sauerstoffatom darstellt und n für 2 steht, mit der Massgabe, dass in Verbindungen der Formel IVc, worin der Ring A unsubstituiert ist, X und Y jeweils Sauerstoff darstellen, n für 1 steht und $R_3$ Wasserstoff bedeutet, alk von Methylen verschieden ist, wenn $R_1$ Carbamyl bedeutet, oder ein Salz davon.

**16.** Eine Verbindung der Formel IVc gemäss Anspruch 11, worin $R_1$ $C_1$-$C_4$-Alkoxycarbonyl bedeutet, $R_3$ Wasserstoff oder $C_1$-$C_4$-Alkyl ist, alk für $C_1$-$C_4$-Alkylen, welches das Ringsystem mit der in Formel IVc eingezeichneten NH-Gruppe durch bis und mit 3 C-Atome verknüpft, steht, der Ring A unsubstituiert ist, X ein Sauerstoffatom oder eine Methylengruppe darstellt, Y ein Sauerstoffatom bedeutet und n für 1 steht, oder ein Salz davon.

**17.** Eine Verbindung der Formel IVc gemäss Anspruch 11, worin $R_1$ $C_1$-$C_4$-Alkoxycarbonyl bedeutet, $R_3$ Wasserstoff ist, alk für Methylen oder Aethylen steht, der Ring A unsubstituiert ist, X ein Sauerstoffatom oder eine Methylengruppe darstellt, Y ein Sauerstoffatom bedeutet und n für 1 steht, oder ein Salz davon.

**18.** N-[2-(Chroman-3-yl)äthyl]-N-(2-methoxycarbonyläthyl)-amin oder ein Salz davon.

**19.** N-(2-Methoxycarbonyläthyl)-N-(benzo-1,4-dioxan-2-ylmethyl)-amin oder ein Salz davon.

**20.** N-(2-Methoxycarbonyläthyl)-N-(chroman-3-ylmethyl)-amin, N-[2-(Benzo-1,4-dioxan-2-yl)äthyl]-N-(2-methoxycarbonyläthyl)-amin oder N-(2-Methoxycarbonyläthyl)-N-(2-methyl-benzo-1,4-dioxan-2-ylmethyl)-amin oder jeweils ein Salz davon.

**21.** N-[2-(Chroman-3-yl)äthyl]-N-(2-carboxyäthyl)-amin, N-[2-(Chroman-2-yl)äthyl]-N-(2-methoxycarbonyl-

äthyl)-amin oder N-[2-(Chroman-4-yl)äthyl]-N-(2-methoxycarbonyläthyl)-amin oder jeweils ein Salz davon.

**22.** Verfahren zur Herstellung einer Verbindung der Formel IVc oder eines Salzes davon gemäss einem der Ansprüche 11 bis 21, dadurch gekennzeichnet, dass man

h) Verbindungen der Formeln

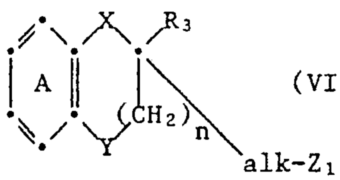 (VIIIa) und $Z_2-CH_2-CH(Z_3)-R_1$ (VIIIb),

worin einer der Reste $Z_1$ und $Z_2$ reaktionsfähiges verestertes Hydroxy, der andere Amino und $Z_3$ Wasserstoff bedeutet oder $Z_1$ Amino ist und $Z_2$ sowie $Z_3$ gemeinsam eine zusätzliche Bindung darstellen, oder gegebenenfalls Salze dieser Verbindungen untereinander umsetzt oder

**i) in einer Verbindung der Formel**

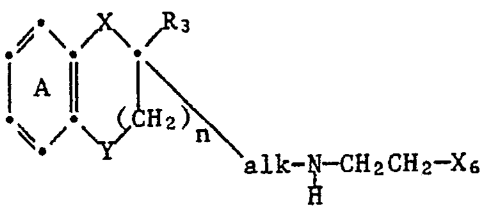 (IX),

worin $X_6$ einen in $R_1$ überführbaren Rest bedeutet, oder einem Salz davon $X_6$ in $R_1$ überführt und gewünschtenfalls jeweils eine verfahrensgemäss oder auf andere Weise erhältliche Verbindung der Formel IVc in eine andere Verbindung der Formel IVc überführt, jeweils ein verfahrensgemäss erhältliches Isomerengemisch in die Komponenten auftrennt, jeweils ein verfahrensgemäss erhältliches Enantiomeren- bzw. Diastereomerengemisch in die Enantiomeren bzw. Diastereomeren aufspaltet und/oder jeweils eine verfahrensgemäss erhältliche freie Verbindung der Formel IVc in ein Salz überführt oder ein verfahrensgemäss erhältliches Salz in die freie Verbindung der Formel IVc oder in ein anderes Salz umwandelt.

**23.** Eine Verbindung der Formel

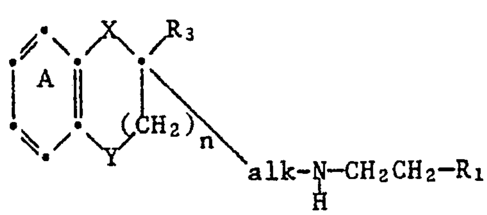 (IVc),

worin $R_1$ Carboxy, Niederalkoxycarbonyl, Carbamyl, N-Niederalkylcarbamyl, N,N-Diniederalkylcarbamyl, Hydroxymethyl, $C_2$-$C_5$-Alkanoyloxymethyl, Niederalkansulfonyloxymethyl, Benzoyloxymethyl oder Pyridoyloxymethyl bedeutet, $R_3$ Wasserstoff oder $C_1$-$C_7$-Alkyl darstellt, alk Niederalkylen oder Niederalkyliden bedeutet, der Ring A unsubstituiert oder ein- oder mehrfach durch Hydroxy, Niederalkoxy, $C_2$-$C_5$-Alkanoyloxy, Halogen mit einer Atomnummer bis und mit 53, $C_1$-$C_7$-Alkyl und/oder Trifluormethyl substituiert ist, und entweder X und Y jeweils ein Sauerstoffatom bedeuten und n für 1 steht oder X eine Methylengruppe darstellt, Y ein Sauerstoffatom bedeutet und n für 1 steht oder X ein Sauerstoffatom darstellt, Y eine Methylengruppe bedeutet und n für 1 steht oder X für eine direkte Bindung steht, Y ein Sauerstoffatom darstellt und n für 2 steht, wobei mit "Nieder" bezeichnete Reste 1 bis und mit 4

Kohlenstoffatome aufweisen können, oder ein pharmazeutisch verwendbares Salz davon zur Anwendung in einem Verfahren zur therapeutischen und/oder prophylaktischen Behandlung des menschlichen oder tierischen Körpers.

**24.** Eine Verbindung der Formel

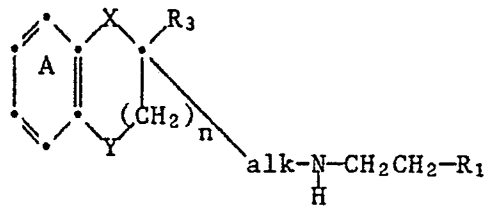

(IVc),

worin $R_1$ Carboxy, Niederalkoxycarbonyl, Carbamyl, N-Niederalkylcarbamyl oder N,N-Diniederalkylcarbamyl bedeutet, $R_3$ Wasserstoff oder $C_1$-$C_7$-Alkyl darstellt, alk Niederalkylen oder Niederalkyliden bedeutet, der Ring A unsubstituiert oder ein- oder mehrfach durch Hydroxy, Niederalkoxy, $C_2$-$C_5$-Alkanoyloxy, Halogen mit einer Atomnummer bis und mit 53, $C_1$-$C_7$-Alkyl und/oder Trifluormethyl substituiert ist, X ein Sauerstoffatom oder eine Methylengruppe darstellt, Y ein Sauerstoffatom bedeutet und n für 1 steht, wobei mit "Nieder" bezeichnete Reste 1 bis und 4 Kohlenstoffatome aufweisen können, oder ein pharmazeutisch verwendbares Salz davon zur Anwendung in einem Verfahren zur therapeutischen und/oder prophylaktischen Behandlung des menschlichen oder tierischen Körpers.

**25.** Eine Verbindung gemäss einem der Ansprüche 1 bis 9 und 11 bis 21 oder gegebenenfalls ein Tautomeres und/oder ein pharmazeutisch verwendbares Salz davon zur Anwendung in einem Verfahren zur therapeutischen und/oder prophylaktischen Behandlung des menschlichen oder tierischen Körpers.

**26.** Eine Verbindung gemäss einem der Ansprüche 2, 4, 6, 8, 12, 14 und 16 bis 20 oder gegebenenfalls ein Tautomeres und/oder ein pharmazeutisch verwendbares Salz davon zur Anwendung in einem Verfahren zur therapeutischen und/oder prophylaktischen Behandlung des menschlichen oder tierischen Körpers.

**27.** Eine Verbindung der Formel IVc gemäss Anspruch 23, worin $R_1$ Carbamyl oder N-Methyl-, N-Aethyl-, N,N-Dimethyl- oder N,N-Diäthylcarbamyl bedeutet, $R_3$ Wasserstoff darstellt, alk Methylen bedeutet, der Ring A unsubstituiert ist, X und Y jeweils ein Sauerstoffatom bedeuten und n für 1 steht, oder ein pharmazeutisch verwendbares Salz davon zur Anwendung in einem Verfahren zur therapeutischen und/oder prophylaktischen Behandlung des menschlichen oder tierischen Körpers.

**28.** Ein pharmazeutisches Präparat, als Wirkstoff enthaltend eine Verbindung gemäss einem der Ansprüche 1 bis 9, 11 bis 21, 23 und 25 oder gegebenenfalls ein Tautomeres und/oder ein pharmazeutisch verwendbares Salz davon gegebenenfalls neben üblichen pharmazeutischen Hilfsstoffen.

**29.** Ein pharmazeutisches Präparat, als Wirkstoff enthaltend eine Verbindung gemäss einem der Ansprüche 2, 4, 6, 8, 12, 14, 16 bis 20, 24, 26 und 27 oder gegebenenfalls ein Tautomeres und/oder ein pharmazeutisch verwendbares Salz davon gegebenenfalls neben üblichen pharmazeutischen Hilfsstoffen.

**30.** Verwendung einer Verbindung gemäss einem der Ansprüche 1 bis 9, 11 bis 21, 23 und 25 oder gegebenenfalls eines Tautomeren und/oder eines pharmazeutisch verwendbaren Salzes davon zur Herstellung eines pharmazeutischen Präparats, z.B. eines Nootropikums.

**Patentansprüche für folgende Vertragsstaaten : AT, ES, GR**

**1.** Verfahren zur Herstellung einer Verbindung der Formel

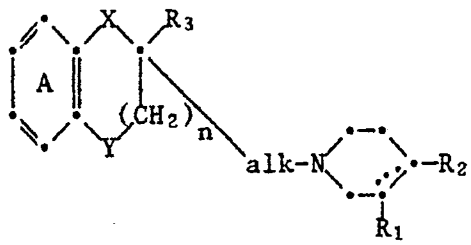

(I),

worin entweder $R_1$ Carboxy, Niederalkoxycarbonyl, Carbamyl, N-Niederalkylcarbamyl, N,N-Diniederalkylcarbamyl, Hydroxymethyl, $C_2$-$C_5$-Alkanoyloxymethyl, Niederalkansulfonyloxymethyl, Benzoyloxymethyl oder Pyridoyloxymethyl bedeutet und $R_2$ Wasserstoff, Hydroxy, Niederalkoxy, Benzyloxy, $C_2$-$C_5$-Alkanoyloxy, Niederalkansulfonyloxy, Benzoyloxy, Pyridoyloxy, Amino, $C_2$-$C_5$-Alkanoylamino, Niederalkansulfonylamino, Benzoylamino oder Pyridoylamino darstellt oder $R_1$ Wasserstoff bedeutet und $R_2$ für Carboxy, Niederalkoxycarbonyl, N-Niederalkylcarbamyl, N,N-Diniederalkylcarbamyl, Hydroxymethyl, $C_2$-$C_5$-Alkanoyloxymethyl, Niederalkansulfonyloxymethyl, Benzoyloxymethyl oder Pyridoyloxymethyl steht, $R_3$ Wasserstoff oder $C_1$-$C_7$-Alkyl bedeutet, alk Niederalkylen, das die beiden Ringsysteme durch bis und mit 3 C-Atome überbrückt, oder Niederalkyliden bedeutet, der Ring A unsubstituiert oder ein-, zwei- oder mehrfach durch Hydroxy, Niederalkoxy, $C_2$-$C_5$-Alkanoyloxy, Halogen mit einer Atomnummer bis und mit 53, $C_1$-$C_7$-Alkyl und/oder Trifluormethyl substituiert ist, die gestrichelte Linie zum Ausdruck bringen soll, dass zwischen den Kohlenstoffatomen, welche die Reste $R_1$ und $R_2$ tragen, eine Einfach- oder eine Doppelbindung vorliegt und entweder X und Y jeweils ein Sauerstoffatom bedeuten und n für 1 steht oder X eine Methylengruppe darstellt, Y ein Sauerstoffatom bedeutet und n für 1 steht oder X ein Sauerstoffatom darstellt, Y eine Methylengruppe bedeutet und n für 1 steht oder X für eine direkte Bindung steht, Y ein Sauerstoffatom darstellt und n für 2 steht, oder eines Tautomeren und/oder Salzes davon, dadurch gekennzeichnet, dass man

a) eine Verbindung der Formel

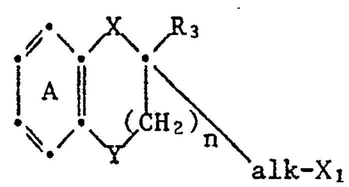

(IIa)

oder ein Salz davon, worin $X_1$ Hydroxy oder reaktionsfähiges verestertes Hydroxy bedeutet, mit einer Verbindung der Formel

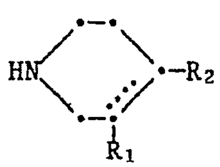

(IIb)·

oder einem Tautomeren und/oder Salz davon umsetzt oder

b) in einer Verbindung der Formel

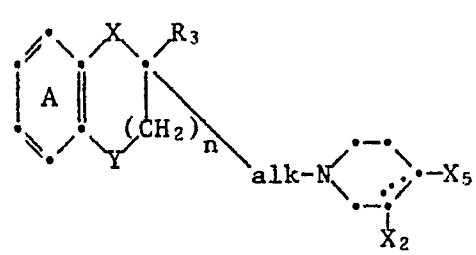

(III)·

oder einem Tautomeren und/oder Salz davon, worin $X_2$ einen in von Wasserstoff verschiedenes $R_1$ überführbaren Rest bedeutet und $X_5$ einen Rest $R_a$ darstellt, wobei $R_a$ Wasserstoff, Hydroxy, Niederalkoxy, Benzyloxy, $C_2$-$C_5$-Alkanoyloxy, Niederalkansulfonyloxy, Benzoyloxy, Pyridoyloxy, Amino, $C_2$-$C_5$-Alkanoylamino, Niederalkansulfonylamino, Benzoylamino oder Pyridoylamino ist, $X_2$ in von Wasserstoff verschiedenes $R_1$ überführt oder in einer Verbindung der Formel III, worin $X_2$ Wasserstoff ist und $X_5$ einen in $R_b$ überführbaren Rest bedeutet, wobei $R_b$ für einen von einem Rest $R_a$ verschiedenen Rest $R_2$ steht, $X_5$ in $R_b$ überführt oder

c) zur Herstellung einer Verbindung der Formel I oder eines Tautomeren und/oder Salzes davon, worin $R_2$ Hydroxy oder Amino bedeutet und $R_1$ von Wasserstoff verschieden ist und insbesondere Niederalkoxycarbonyl bedeutet, eine Verbindung der Formel

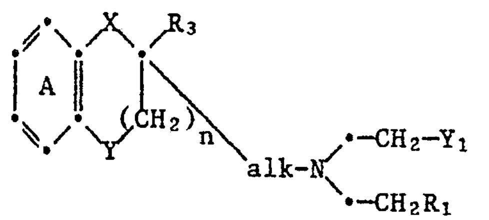

(IV),

worin $Y_1$ eine Gruppe der Formel $-CH=R_2'$, $-C(Y_2)=R_2'$, $-CH(Y_2)-R_2$ oder Cyano darstellt, wobei $R_2'$ Oxo oder Imino bedeutet und $Y_2$ einen abspaltbaren Rest darstellt, oder ein Salz davon cyclisiert oder

d) zur Herstellung einer Verbindung der Formel I' oder eines Tautomeren und/oder Salzes davon, worin $R_2'$ Oxo oder Imino bedeutet und $R_1$ von Wasserstoff verschieden ist und insbesondere Niederalkoxycarbonyl bedeutet, eine Verbindung der Formel

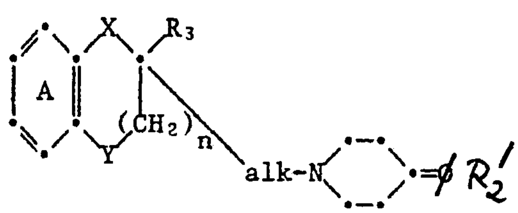

(Va)

oder ein Tautomeres oder jeweils ein Salz davon mit einer Verbindung der Formel

$X_3$-$R_1$ (Vb)

oder einem Salz davon, worin $R_1$ von Wasserstoff verschieden ist und $X_3$ Halogen oder Niederalkoxy bedeutet, umsetzt oder

e) zur Herstellung einer Verbindung der Formel I oder eines Tautomeren und/oder Salzes davon, worin $R_2$ Hydroxy, Niederalkoxy, Benzyloxy, $C_2$-$C_5$-Alkanoyloxy, Niederalkansulfonyloxy, Benzoyloxy, Pyridoyloxy, Amino, $C_2$-$C_5$-Alkanoylamino, Niederalkansulfonylamino, Benzoylamino oder Pyridoylamino ist, in einer Verbindung der Formel

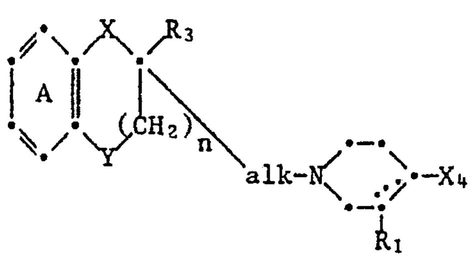

(VI)

oder einem Salz davon, worin $X_4$ einen in $R_2$ überführbaren Rest bedeutet, $X_4$ in $R_2$ überführt oder

f) insbesondere zur Herstellung einer Verbindung der Formel I oder eines Tautomeren und/oder Salzes davon, worin $R_2$ Wasserstoff, Hydroxy, Amino, Carboxy, Niederalkoxycarbonyl, N-Niederalkylcarbamyl, N,N-Diniederalkylcarbamyl oder Hydroxymethyl ist, in einer Verbindung der Formel

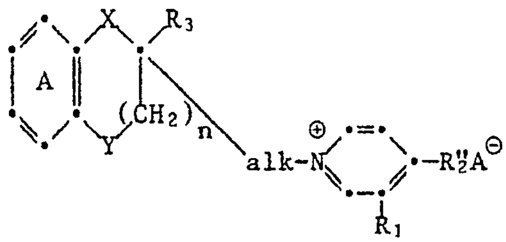

(VII),

worin $A^{\ominus}$ für das Anion einer Säure steht und $R_2''$ Wasserstoff, Niederalkoxy, Benzyloxy, $C_2$-$C_5$-Alkanoyloxy, Niederalkansulfonyloxy, Benzoyloxy, Pyridoyloxy, geschütztes Hydroxy, $C_2$-$C_5$-Alkanoylamino, Niederalkansulfonylamino, Benzoylamino, Pyridoylamino, geschütztes Amino, Carboxy, Niederalkoxycarbonyl, N-Niederalkylcarbamyl, N,N-Diniederalkylcarbamyl, $C_2$-$C_5$-Alkanoyloxymethyl, Niederalkansulfonyloxymethyl, Benzoyloxymethyl, Pyridoyloxymethyl oder veräthertes oder geschütztes Hydroxymethyl bedeutet, die überschüssigen Doppelbindungen zu Einfachbindungen reduziert oder

g) zur Herstellung einer Verbindung der Formel I oder eines Tautomeren und/oder Salzes davon, worin $R_2$ Carboxy, N-Niederalkylcarbamyl, N,N-Diniederalkylcarbamyl oder insbesondere Niederalkoxycarbonyl bedeutet, eine Verbindung der Formel

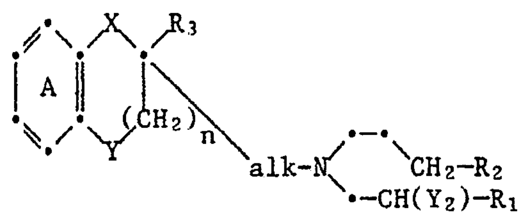

(VIII),

worin $Y_2$ einen abspaltbaren Rest darstellt, oder ein Salz davon cyclisiert und jeweils eine gegebenenfalls vorhandene Schutzgruppe abspaltet und jeweils gewünschtenfalls eine verfahrensgemäss oder auf andere Weise erhältliche Verbindung der Formel I in eine andere Verbindung der Formel I überführt, jeweils ein verfahrensgemäss erhältliches Isomerengemisch in die Komponenten auftrennt, jeweils ein verfahrensgemäss erhältliches Enantiomeren- bzw. Diastereomerengemisch in die Enantiomeren bzw. Diastereomeren aufspaltet und/oder jeweils ein verfahrensgemäss erhältliche freie Verbindung der Formel I in ein Salz überführt oder ein verfahrensgemäss erhältliches Salz in die freie Verbindung der Formel I oder in ein anderes Salz umwandelt, wobei mit "Nieder" bezeichnete Reste 1 bis und mit 4 Kohlenstoffatome aufweisen können.

**2.** Verfahren gemäss Anspruch 1 zur Herstellung einer Verbindung der Formel I, worin $R_1$ Carboxy, Niederalkoxycarbonyl, Carbamyl, N-Niederalkylcarbamyl oder N,N-Diniederalkylcarbamyl bedeutet, $R_2$ Wasserstoff, Hydroxy, Niederalkoxy, Benzyloxy, $C_2$-$C_5$-Alkanoyloxy, Niederalkansulfonyloxy, Benzoyloxy, Pyridoyloxy, Amino, $C_2$-$C_5$-Alkanoylamino, Benzoylamino oder Pyridoylamino darstellt, $R_3$ Wasserstoff oder $C_1$-$C_7$-Alkyl bedeutet, alk Niederalkylen, das die beiden Ringsysteme durch bis und mit 3 C-Atome überbrückt, oder Niederalkyliden darstellt, der Ring A unsubstituiert ist oder ein-, zwei- oder mehrfach durch Hydroxy, Niederalkoxy, $C_2$-$C_5$-Alkanoyloxy, Halogen mit einer Atomnummer bis und mit 53, $C_1$-$C_7$-Alkyl und/oder Trifluormethyl substituiert ist, die gestrichelte Linie zum Ausdruck bringen soll, dass zwischen den Kohlenstoffatomen, welche die Reste $R_1$ und $R_2$ tragen, eine Einfach- oder eine Doppelbindung vorliegt, X ein Sauerstoffatom oder eine Methylengruppe darstellt, Y ein Sauerstoffatom bedeutet und n für 1 steht, wobei mit "Nieder" bezeichnete Reste 1 bis und mit 4 Kohlenstoffatome aufweisen können, oder eines Tautomeren und/oder Salzes davon.

**3.** Verfahren gemäss Anspruch 1 zur Herstellung einer Verbindung der Formel I, worin entweder $R_1$ $C_1$-$C_4$-Alkoxycarbonyl, Carbamyl, Hydroxymethyl oder $C_2$-$C_5$-Alkanoyloxymethyl bedeutet und $R_2$ Wasserstoff oder Hydroxy bedeutet oder $R_1$ Wasserstoff darstellt und $R_2$ für $C_1$-$C_4$-Alkoxycarbonyl steht, $R_3$

Wasserstoff oder $C_1$-$C_4$-Alkyl ist, alk für $C_1$-$C_4$-Alkylen, welches die beiden Ringsysteme durch bis und mit 3 C-Atome überbrückt, steht, der Ring A unsubstituiert oder, insbesondere in 7-Stellung, durch $C_1$-$C_4$-Alkoxy substituiert ist, die gestrichelte Linie zum Ausdruck bringen soll, dass zwischen den Kohlenstoffatomen, welche die Reste $R_1$ und $R_2$ tragen, eine Einfach-oder eine Doppelbindung vorliegt, und entweder X und Y jeweils ein Sauerstoffatom bedeuten und n für 1 steht oder X eine Methylengruppe darstellt, Y ein Sauerstoffatom bedeutet und n für 1 steht oder X ein Sauerstoffatom darstellt, Y eine Methylengruppe bedeutet und n für 1 steht oder X für eine direkte Bindung steht, Y ein Sauerstoffatom darstellt und n für 2 steht, oder eines Tautomeren und/oder Salzes davon.

**4.** Verfahren gemäss Anspruch 1 zur Herstellung einer Verbindung der Formel I, worin $R_1$ $C_1$-$C_4$-Alkoxycarbonyl bedeutet, $R_2$ Wasserstoff oder Hydroxy bedeutet, $R_3$ Wasserstoff oder $C_1$-$C_4$-Alkyl ist, alk für $C_1$-$C_4$-Alkylen, welches die beiden Ringsysteme durch bis und mit 3 C-Atome überbrückt, steht, der Ring A unsubstituiert ist, die gestrichelte Linie zum Ausdruck bringen soll, dass zwischen den Kohlenstoffatomen, welche die Reste $R_1$ und $R_2$ tragen, eine Einfach- oder eine Doppelbindung vorliegt, X ein Sauerstoffatom oder eine Methylengruppe bedeutet, Y ein Sauerstoffatom darstellt und n für 1 steht, oder eines Tautomeren und/oder Salzes davon.

**5.** Verfahren gemäss Anspruch 1 zur Herstellung einer Verbindung der Formel I, worin $R_1$ $C_1$-$C_4$-Alkoxycarbonyl bedeutet, $R_2$ Wasserstoff oder Hydroxy darstellt, $R_3$ Wasserstoff ist, alk für Methylen oder Aethylen steht, der Ring A unsubstituiert ist, die gestrichelte Linie zum Ausdruck bringen soll, dass zwischen den Kohlenstoffatomen, welche die Reste $R_1$ und $R_2$ tragen, eine Einfach- oder eine Doppelbindung vorliegt, X ein Sauerstoffatom oder eine Methylengruppe bedeutet, Y ein Sauerstoffatom darstellt und n für 1 steht, oder eines Tautomeren und/oder Salzes davon.

**6.** Verfahren gemäss Anspruch 1 oder 2 zur Herstellung von 4-Hydroxy-1-(chroman-3-ylmethyl)-1,2,5,6-tetrahydro-pyridin-3-carbonsäuremethylester bzw. 1-(Chroman-3-ylmethyl)-4-oxo-piperidin-3-carbonsäuremethylester, 1-[2-(Chroman-3-yl)äthyl]-1,2,5,6-tetrahydro-pyridin-3-carbonsäuremethylester, 1-(Benzo-1,4-dioxan-2-ylmethyl)-piperidin-3-carbonsäuremethylester oder 4-Hydroxy-1-(benzo-1,4-dioxan-2-ylmethyl)-1,2,5,6-tetrahydro-pyridin-3-carbonsäuremethylester bzw. 1-(Benzo-1,4-dioxan-2-ylmethyl)-4-oxo-piperidin-3-carbonsäuremethylester oder jeweils eines Salzes davon.

**7.** Verfahren gemäss Anspruch 1 zur Herstellung von 1-[2-(Chroman-3-yl)äthyl]-1,2,5,6-tetrahydro-pyridin-3-carbonsäureäthylester oder eines Salzes davon.

**8.** Verfahren gemäss Anspruch 1 oder 2 zur Herstellung von 1-(Chroman-3-ylmethyl)-1,2,5,6-tetrahydro-pyridin-3-carbonsäuremethylester, 1-[2-(Chroman-3-yl)äthyl]-4-hydroxy-1,2,5,6-tetrahydropyridin-3-carbonsäuremethylester bzw. 1-[2-(Chroman-3-yl)äthyl]-4-oxo-piperidin-3-carbonsäuremethylester, 1-(Benzo-1,4-dioxan-2-ylmethyl)-1,2,5,6-tetrahydro-pyridin-3carbonsäuremethylester, 4-Hydroxy-1-(2-methyl-benzo-1,4-dioxan-2-ylmethyl)-1,2,5,6-tetrahydro-pyridin-3-carbonsäuremethylester bzw. 1-(2-Methyl-benzo-1,4-dioxan-2-ylmethyl)-4-oxo-piperidin-3-carbonsäuremethylester, 1-[2-(Benzo-1,4,-dioxan-2-yl)äthyl]-4-hydroxy-1,2,5,6-tetrahydropyridin-3-carbonsäuremethylester bzw. 1-[2-(Benzo-1,4-dioxan-2-yl)äthyl]-4-oxo-piperidin-3-carbonsäuremethylester, cis-1-[2-(Benzo-1,4-dioxan-2-yl)äthyl]-4-hydroxy-piperidin-3-carbonsäuremethylester, trans-1-[2-(Benzo-1,4-dioxan-2-yl)äthyl]-4-hydroxy-piperidin-3-carbonsäuremethylester oder 1-[2-(Benzo-1,4,-dioxan-2-yl)äthyl]-1,2,5,6-tetrahydro-pyridin-3-carbonsäuremethylester oder jeweils eines Salzes davon.

**9.** Verfahren gemäss Anspruch 1 zur Herstellung von 1-[2-(Chroman-3-yl)äthyl]-piperidin-3-carbonsäuremethylester, 1-[2-(Chroman-3-yl)äthyl]-piperidin-4-carbonsäureäthylester, 1-[2-(Benzo-,1,4-dioxan-2-yl)-äthyl]-piperidin-3-carbonsäureäthylester, 1-[2-(Benzo-1,4-dioxan-2-yl)äthyl]-piperidin-4-carbonsäureäthylester, 1-[2-(Chroman-2-yl)äthyl]-piperidin-3-carbonsäureäthylester, 1-[2-(Chroman-2-yl)-äthyl]-piperidin-4-carbonsäureäthylester, cis-1-[2-(Benzo-1,4-dioxan-2-yl)äthyl]-4-benzyloxy-piperidin-3-carbonsäuremethylester, trans-1-[2-Benzo-1,4-dioxan-2-yl)äthyl]-4-benzyloxy-piperidin-3-carbonsäuremethylester, cis-4-Amino-1-[2-(benzo-1,4-dioxan-2-yl)äthyl]-piperidin-3-carbonsäureamid, trans-4-Amino-1-[2-(benzo-1,4-dioxan-2-yl)äthyl]-piperidin-3-carbonsäureamid, 1-[2-(Chroman-4-yl)äthyl]-1,2,5,6-tetrahydro-pyridin-3-carbonsäuremethylester oder 1-[2-(Chroman-4-yl)äthyl]-piperidin-3-carbonsäureäthylester oder jeweils eines Salzes davon.

**10.** Verfahren zur Herstellung einer Verbindung der Formel

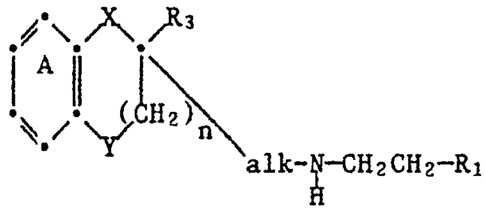

(IVc),

worin $R_1$ Carboxy, Niederalkoxycarbonyl, Carbamyl, N-Niederalkylcarbamyl, N,N-Diniederalkylcarbamyl, Hydroxymethyl, $C_2$-$C_5$-Alkanoyloxymethyl, Niederalkansulfonyloxymethyl, Benzoyloxymethyl oder Pyridoyloxymethyl bedeutet, $R_3$ Wasserstoff oder $C_1$-$C_7$-Alkyl darstellt, alk Niederalkylen oder Niederalkyliden bedeutet, der Ring A unsubstituiert oder ein- oder mehrfach durch Hydroxy, Niederalkoxy, $C_2$-$C_5$-Alkanoyloxy, Halogen mit einer Atomnummer bis und mit 53, $C_1$-$C_7$-Alkyl und/oder Trifluormethyl substituiert ist und entweder X und Y jeweils ein Sauerstoffatom bedeuten und n für 1 steht oder X eine Methylengruppe darstellt, Y ein Sauerstoffatom bedeutet und n für 1 steht oder X ein Sauerstoffatom darstellt, Y eine Methylengruppe bedeutet und n für 1 steht oder X für eine direkte Bindung steht, Y ein Sauerstoffatom darstellt und n für 2 steht, mit der Massgabe, dass in Verbindungen der Formel IVc, worin der Ring A unsubstituiert ist, X und Y jeweils Sauerstoff darstellen, n für 1 steht und $R_3$ Wasserstoff bedeutet, alk von Methylen verschieden ist, wenn $R_1$ Carbamyl oder N-Methyl-, N-Aethyl-, N,N-Dimethyl- oder N,N-Diäthylcarbamyl bedeutet, wobei mit "Nieder" bezeichnete Reste 1 bis und mit 4 Kohlenstoffatome aufweisen können, oder eines Salzes davon, dadurch gekennzeichnet, dass man

h) Verbindungen der Formeln

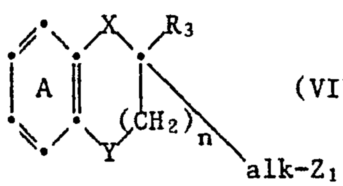

(VIIIa) und $Z_2$—$CH_2$—$CH(Z_3)$—$R_1$ (VIIIb),

worin einer der Reste $Z_1$ und $Z_2$ reaktionsfähiges verestertes Hydroxy, der andere Amino und $Z_3$ Wasserstoff bedeutet oder $Z_1$ Amino ist und $Z_2$ sowie $Z_3$ gemeinsam eine zusätzliche Bindung darstellen, oder gegebenenfalls Salze dieser Verbindungen untereinander umsetzt oder

i) in einer Verbindung der Formel

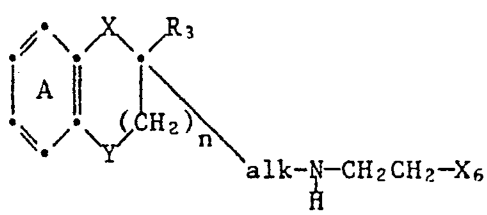

(IX),

worin $X_6$ einen in $R_1$ überführbaren Rest bedeutet, oder einem Salz davon $X_6$ in $R_1$ überführt und gewünschtenfalls jeweils eine verfahrensgemäss oder auf andere Weise erhältliche Verbindung der Formel IVc in eine andere Verbindung der Formel IVc überführt, jeweils ein verfahrensgemäss erhältliches Isomerengemisch in die Komponenten auftrennt, jeweils ein verfahrensgemäss erhältliches Enantiomeren- bzw. Diastereomerengemisch in die Enantiomeren bzw. Diastereomeren aufspaltet und/oder jeweils eine verfahrensgemäss erhältliche freie Verbindung der Formel IVc in ein Salz überführt oder ein verfahrensgemäss erhältliches Salz in die freie Verbindung der Formel IVc oder in ein anderes Salz umwandelt.

**11.** Verfahren gemäss Anspruch 10 zur Herstellung einer Verbindung der Formel IVc, worin $R_1$ Carboxy,

Niederalkoxycarbonyl, Carbamyl, N-Niederalkyl-carbamyl oder N,N-Diniederalkylcarbamyl bedeutet, $R_3$ Wasserstoff oder $C_1$-$C_7$-Alkyl darstellt, alk Niederalkylen oder Niederalkyliden bedeutet, der Ring A unsubstituiert oder ein- oder mehrfach durch Hydroxy, Niederalkoxy, $C_2$-$C_5$-Alkanoyloxy, Halogen mit einer Atomnummer bis und mit 53, $C_1$-$C_7$-Alkyl und/oder Trifluormethyl substituiert ist, X ein Sauerstoffatom oder eine Methylengruppe darstellt, Y ein Sauerstoffatom bedeutet und n für 1 steht, mit der Massgabe, dass in Verbindungen der Formel IVc, worin der Ring A unsubstituiert ist, X und Y jeweils Sauerstoff darstellen, n für 1 steht und $R_3$ Wasserstoff bedeutet, alk von Methylen verschieden ist, wenn $R_1$ Carbamyl oder N-Methyl-, N-Aethyl-, N,N-Dimethyl- oder N,N-Diäthylcarbamyl bedeutet, wobei mit "Nieder" bezeichnete Reste 1 bis und mit 4 Kohlenstoffatome aufweisen können, oder eines Salzes davon.

**12.** Verfahren gemäss Anspruch 10 zur Herstellung einer Verbindung der Formel IVc, worin $R_1$ Carboxy, Niederalkoxycarbonyl, Carbamyl, N-Niederalkylcarbamyl, N,N-Diniederalkylcarbamyl, Hydroxymethyl, $C_2$-$C_5$-Alkanoyloxymethyl, Niederalkansulfonyloxymethyl, Benzoyloxymethyl oder Pyridoyloxymethyl bedeutet, $R_3$ Wasserstoff oder $C_1$-$C_7$-Alkyl darstellt, alk Niederalkylen, welches das Ringsystem mit der in Formel IVc eingezeichneten NH-Gruppe durch bis und mit 3 C-Atome verknüpft, oder Niederalkyliden darstellt, der Ring A unsubstituiert ist oder ein-, zwei- oder mehrfach durch Hydroxy, Niederalkoxy, $C_2$-$C_5$-Alkanoyloxy, Halogen mit einer Atomnummer bis und mit 53, $C_1$-$C_7$-Alkyl und/oder Trifluormethyl substituiert ist und entweder X und Y jeweils ein Sauerstoffatom bedeuten und n für 1 steht oder X eine Methylengruppe darstellt, Y ein Sauerstoffatom bedeutet und n für 1 steht oder X ein Sauerstoffatom darstellt, Y eine Methylengruppe bedeutet und n für 1 steht oder X für eine direkte Bindung steht, Y ein Sauerstoffatom darstellt und n für 2 steht, mit der Massgabe, dass in Verbindungen der Formel IVc, worin der Ring A unsubstituiert ist, X und Y jeweils Sauerstoff darstellen, n für 1 steht und $R_3$ Wasserstoff bedeutet, alk von Methylen verschieden ist, wenn $R_1$ Carbamyl oder N-Methyl-, N-Aethyl-, N,N-Dimethyl- oder N,N-Diäthylcarbamyl bedeutet, wobei mit "Nieder" bezeichnete Reste 1 bis und mit 4 Kohlenstoffatome aufweisen können, oder eines Salzes davon.

**13.** Verfahren gemäss Anspruch 10 zur Herstellung einer Verbindung der Formel IVc, worin $R_1$ Carboxy, Niederalkoxycarbonyl, Carbamyl, N-Niederalkylcarbamyl oder N,N-Diniederalkylcarbamyl bedeutet, $R_3$ Wasserstoff oder $C_1$-$C_7$-Alkyl darstellt, alk Niederalkylen, welches das Ringsystem mit der in Formel IVc eingezeichneten NH-Gruppe durch bis und mit 3 C-Atome verknüpft, oder Niederalkyliden darstellt, der Ring A unsubstituiert ist oder ein-, zwei-oder mehrfach durch Hydroxy, Niederalkoxy, $C_2$-$C_5$-Alkanoyloxy, Halogen mit einer Atomnummer bis und mit 53, $C_1$-$C_7$-Alkyl und/oder Trifluormethyl substituiert ist, X ein Sauerstoffatom oder eine Methylengruppe darstellt, Y ein Sauerstoffatom bedeutet und n für 1 steht, mit der Massgabe, dass in Verbindungen der Formel IVc, worin der Ring A unsubstituiert ist, X und Y jeweils Sauerstoff darstellen, n für 1 steht und $R_3$ Wasserstoff bedeutet, alk von Methylen verschieden ist, wenn $R_1$ Carbamyl oder N-Methyl-, N-Aethyl-, N,N-Dimethyl- oder N,N-Diäthylcarbamyl bedeutet, wobei mit "Nieder" bezeichnete Reste 1 bis und mit 4 Kohlenstoffatome aufweisen können, oder eines Salzes davon.

**14.** Verfahren gemäss Anspruch 10 zur Herstellung einer Verbindung der Formel IVc, worin $R_1$ Carboxy, Hydroxymethyl, $C_2$-$C_5$-Alkanoyloxymethyl, $C_1$-$C_4$-Alkoxycarbonyl oder Carbamyl bedeutet, $R_3$ Wasserstoff oder $C_1$-$C_4$-Alkyl ist, alk für $C_1$-$C_4$-Alkylen, welches das Ringsystem mit der in Formel IVc eingezeichneten NH-Gruppe durch bis und mit 3 C-Atome verknüpft, steht, der Ring A unsubstituiert oder, insbesondere in 7-Stellung, durch $C_1$-$C_4$-Alkoxy substituiert ist und entweder X und Y jeweils ein Sauerstoffatom bedeuten und n für 1 steht oder X eine Methylengruppe darstellt, Y ein Sauerstoffatom bedeutet und n für 1 steht oder X ein Sauerstoffatom darstellt, Y eine Methylengruppe bedeutet und n für 1 steht oder X für eine direkte Bindung steht, Y ein Sauerstoffatom darstellt und n für 2 steht, mit der Massgabe, dass in Verbindung der Formel IVc, worin der Ring A unsubstituiert ist, X und Y jeweils Sauerstoff darstellen, n für 1 steht und $R_3$ Wasserstoff bedeutet, alk von Methylen verschieden ist, wenn $R_1$ Carbamyl bedeutet, oder eines Salzes davon.

**15.** Verfahren gemäss Anspruch 10 zur Herstellung einer Verbindung der Formel IVc, worin $R_1$ $C_1$-$C_4$-Alkoxycarbonyl bedeutet, $R_3$ Wasserstoff oder $C_1$-$C_4$-Alkyl ist, alk für $C_1$-$C_4$-Alkylen, welches das Ringsystem mit der in Formel IVc eingezeichneten NH-Gruppe durch bis und mit 3 C-Atome verknüpft, steht, der Ring A unsubstituiert ist, X ein Sauerstoffatom oder eine Methylengruppe darstellt, Y ein Sauerstoffatom bedeutet und n für 1 steht, oder eines Salzes davon.

**16.** Verfahren gemäss Anspruch 10 zur Herstellung einer Verbindung der Formel IVc, worin $R_1$ $C_1$-$C_4$-Alkoxycarbonyl bedeutet, $R_3$ Wasserstoff ist, alk für Methylen oder Aethylen steht, der Ring A unsubstituiert ist, X ein Sauerstoffatom oder eine Methylengruppe darstellt, Y ein Sauerstoffatom bedeutet und n für 1 steht, oder eines Salzes davon.

**17.** Verfahren zur Herstellung einer Verbindung der Formel

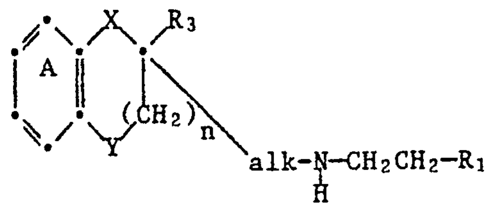

(IVc),

worin $R_1$ Carboxy, Niederalkoxycarbonyl, Carbamyl, N-Niederalkylcarbamyl oder N,N-Diniederalkylcarbamyl bedeutet, $R_3$ Wasserstoff oder $C_1$-$C_7$-Alkyl darstellt, alk Niederalkylen oder Niederalkyliden bedeutet, der Ring A unsubstituiert oder ein- oder mehrfach durch Hydroxy, Niederalkoxy, $C_2$-$C_5$-Alkanoyloxy, Halogen mit einer Atomnummer bis und mit 53, $C_1$-$C_7$-Alkyl und/oder Trifluormethyl substituiert ist, X ein Sauerstoffatom oder eine Methylengruppe darstellt, Y ein Sauerstoffatom bedeutet und n für 1 steht, mit der Massgabe, dass in Verbindungen der Formel IVc, worin der Ring A unsubstituiert ist, X und Y jeweils Sauerstoff darstellen, n für 1 steht und $R_3$ Wasserstoff bedeutet, alk von Methylen verschieden ist, wenn $R_1$ Carbamyl oder N-Methyl-, N-Aethyl-, N,N-Dimethyl- oder N,N-Diäthylcarbamyl bedeutet, wobei mit "Nieder" bezeichnete Reste 1 bis und mit 4 Kohlenstoffatome aufweisen können, oder eine Salzes davon, dadurch gekennzeichnet, dass man Verbindungen der Formeln

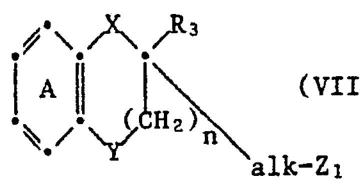

(VIIIa) und $Z_2-CH_2-CH(Z_3)-R_1$ (VIIIb),

worin einer der Reste $Z_1$ und $Z_2$ reaktionsfähiges verestertes Hydroxy, der andere Amino und $Z_3$ Wasserstoff bedeutet oder $Z_1$ Amino ist und $Z_2$ sowie $Z_3$ gemeinsam eine zusätzliche Bindung darstellen, oder gegebenenfalls Salze dieser Verbindungen untereinander umsetzt und gewünschtenfalls eine verfahrensgemäss oder auf andere Weise erhältliche Verbindung der Formel IVc in eine andere Verbindung der Formel IVc überführt, ein verfahrensgemäss erhältliches Isomerengemisch in die Komponenten auftrennt, ein verfahrensgemäss erhältliches Enantiomeren- bzw. Diastereomerengemisch in die Enantiomeren bzw. Diastereomeren aufspaltet und/oder eine verfahrensgemäss erhältliche freie Verbindung der Formel IVc in ein Salz überführt oder ein verfahrensgemäss erhältliches Salz in die freie Verbindung der Formel IVc oder in ein anderes Salz umwandelt.

**18.** Verfahren gemäss Anspruch 17 zur Herstellung einer Verbindung der Formel IVc, worin $R_1$ Carboxy, Niederalkoxycarbonyl, Carbamyl, N-Niederalkylcarbamyl oder N,N-Diniederalkylcarbamyl bedeutet, $R_3$ Wasserstoff oder $C_1$-$C_7$-Alkyl darstellt, alk Niederalkylen, welches das Ringsystem mit der in Formel IVc eingezeichneten NH-Gruppe durch bis und mit 3 C-Atome verknüpft, oder Niederalkyliden darstellt, der Ring A unsubstituiert ist oder ein-, zwei-oder mehrfach durch Hydroxy, Niederalkoxy, $C_2$-$C_5$-Alkanoyloxy, Halogen mit einer Atomnummer bis und mit 53, $C_1$-$C_7$-Alkyl und/oder Trifluormethyl substituiert ist, X ein Sauerstoffatom oder eine Methylengruppe darstellt, Y ein Sauerstoffatom bedeutet und n für 1 steht, mit der Massgabe, dass in Verbindungen der Formel IVc, worin der Ring A unsubstituiert ist, X und Y jeweils Sauerstoff darstellen, n für 1 steht und $R_3$ Wasserstoff bedeutet, alk von Methylen verschieden ist, wenn $R_1$ Carbamyl oder N-Methyl-, N-Aethyl-, N,N-Dimethyl- oder N,N-Diäthylcarbamyl bedeutet, wobei mit "Nieder" bezeichnete Reste 1 bis und mit 4 Kohlenstoffatome aufweisen können, oder eines Salzes davon.

19. Verfahren gemäss Anspruch 17 zur Herstellung einer Verbindung der Formel IVc, worin $R_1$ $C_1$-$C_4$-Alkoxycarbonyl bedeutet, $R_3$ Wasserstoff oder $C_1$-$C_4$-Alkyl ist, alk für $C_1$-$C_4$-Alkylen, welches das Ringsystem mit der in Formel IVc eingezeichneten NH-Gruppe durch bis und mit 3 C-Atome verknüpft, steht, der Ring A unsubstituiert ist, X ein Sauerstoffatom oder eine Methylengruppe darstellt, Y ein Sauerstoffatom bedeutet und n für 1 steht, oder eines Salzes davon.

20. Verfahren gemäss Anspruch 17 zur Herstellung einer Verbindung der Formel IVc, worin $R_1$ $C_1$-$C_4$-Alkoxycarbonyl bedeutet, $R_3$ Wasserstoff ist, alk für Methylen oder Aethylen steht, der Ring A unsubstituiert ist, X ein Sauerstoffatom oder eine Methylengruppe darstellt, Y ein Sauerstoffatom bedeutet und n für 1 steht, oder eines Salzes davon.

21. Verfahren gemäss Anspruch 10 oder 17 zur Herstellung von N-[2-(Chroman-3-yl)äthyl]-N-(2-methoxycarbonyläthyl)-amin oder eines Salzes davon.

22. Verfahren gemäss Anspruch 10 oder 17 zur Herstellung von N-(2-Methoxycarbonyläthyl)-N-(benzo-1,4-dioxan-2-ylmethyl)-amin oder eines Salzes davon.

23. Verfahren gemäss Anspruch 10 oder 17 zur Herstellung von N-(2-Methoxycarbonyläthyl)-N-(chroman-3-ylmethyl)-amin, N-[2-(Benzo-1,4-dioxan-2-yl)äthyl]-N-(2-methoxycarbonyläthyl)-amin oder N-(2-Methoxycarbonyläthyl)-N-(2-methyl-benzo-1,4-dioxan-2-ylmethyl)-amin oder jeweils eines Salzes davon.

24. Verfahren gemäss Anspruch 10 zur Herstellung von N-[2-(Chroman-3-yl)äthyl]-N-(2-carboxyäthyl)-amin, N-[2-(Chroman-2-yl)äthyl]-N-(2-methoxycarbonyläthyl)-amin oder N-[2-(Chroman-4-yl)äthyl]-N-(2-methoxycarbonyläthyl)-amin oder jeweils eines Salzes davon.

25. Verfahren zur Herstellung eines pharmazeutischen Präparates, dadurch gekennzeichnet, dass man
a) eine Verbindung der Formel

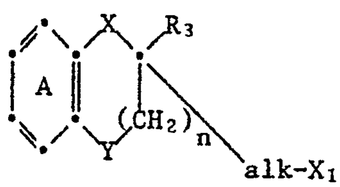

(IIa)

oder ein Salz davon, worin $X_1$ Hydroxy oder reaktionsfähiges verestertes Hydroxy bedeutet, mit einer Verbindung der Formel

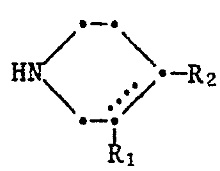

(IIb)

oder einem Tautomeren und/oder Salz davon umsetzt oder
b) in einer Verbindung der Formel

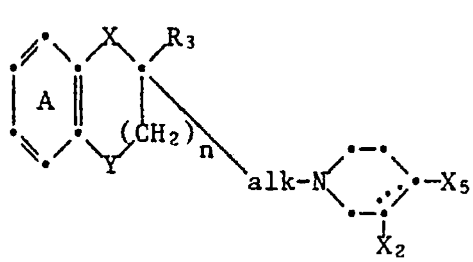

(III)

oder einem Tautomeren und/oder Salz davon, worin $X_2$ einen in von Wasserstoff verschiedenes $R_1$ überführbaren Rest bedeutet und $X_5$ einen Rest $R_a$ darstellt, wobei $R_a$ Wasserstoff, Hydroxy, Niederalkoxy, Benzyloxy, $C_2$-$C_5$-Alkanoyloxy, Niederalkansulfonyloxy, Benzoyloxy, Pyridoyloxy, Amino, $C_2$-$C_5$-Alkanoylamino, Niederalkansulfonylamino, Benzoylamino oder Pyridoylamino ist, $X_2$ in von Wasserstoff verschiedenes $R_1$ überführt oder in einer Verbindung der Formel III, worin $X_2$ Wasserstoff ist und $X_5$ einen in $R_b$ überführbaren Rest bedeutet, wobei $R_b$ für einen von einem Rest $R_a$ verschiedenen Rest $R_2$ steht, $X_5$ in $R_b$ überführt oder

c) zur Herstellung einer Verbindung der Formel I oder eines Tautomeren und/oder Salzes davon, worin $R_2$ Hydroxy oder Amino bedeutet und $R_1$ von Wasserstoff verschieden ist und insbesondere Niederalkoxycarbonyl bedeutet, eine Verbindung der Formel

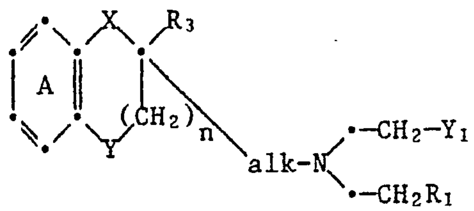

(IV),

worin $Y_1$ eine Gruppe der Formel $-CH=R_2'$, $-C(Y_2)=R_2'$, $-CH(Y_2)-R_2$ oder Cyano darstellt, wobei $R_2'$ Oxo oder Imino bedeutet und $Y_2$ einen abspaltbaren Rest darstellt, oder ein Salz davon cyclisiert oder

d) zur Herstellung einer Verbindung der Formel I' oder eines Tautomeren und/oder Salzes davon, worin $R_2'$ Oxo oder Imino bedeutet und $R_1$ von Wasserstoff verschieden ist und insbesondere Niederalkoxycarbonyl bedeutet, eine Verbindung der Formel

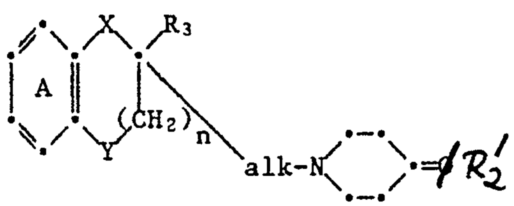

(Va)

oder ein Tautomeres oder jeweils ein Salz davon mit einer Verbindung der Formel

$X_3$-$R_1$ (Vb)

oder einem Salz davon, worin $R_1$ von Wasserstoff verschieden ist und $X_3$ Halogen oder Niederalkoxy bedeutet, umsetzt oder

e) zur Herstellung einer Verbindung der Formel I oder eines Tautomeren und/oder Salzes davon, worin $R_2$ Hydroxy, Niederalkoxy, Benzyloxy, $C_2$-$C_5$-Alkanoyloxy, Niederalkansulfonyloxy, Benzoyloxy, Pyridoyloxy, Amino, $C_2$-$C_5$-Alkanoylamino, Niederalkansulfonylamino, Benzoylamino oder Pyridoylamino ist, in einer Verbindung der Formel

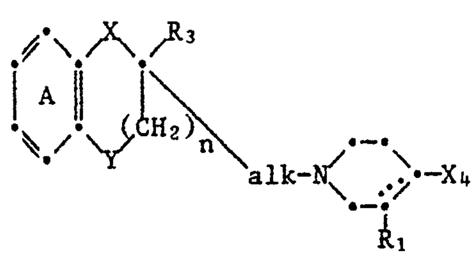

(VI)

oder einem Salz davon, worin $X_4$ einen in $R_2$ überführbaren Rest bedeutet, $X_4$ in $R_2$ überführt oder

f) insbesondere zur Herstellung einer Verbindung der Formel I oder eines Tautomeren und/oder Salzes davon, worin $R_2$ Wasserstoff, Hydroxy, Amino, Carboxy, Niederalkoxycarbonyl, N-Niederalkylcarbamyl, N,N-Diniederalkylcarbamyl oder Hydroxymethyl ist, in einer Verbindung der Formel

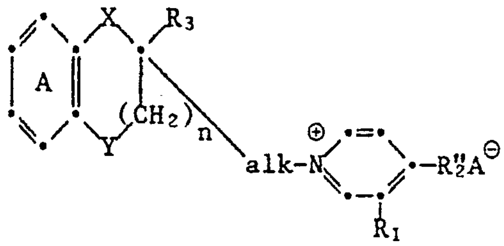

(VII),

worin $A^{\ominus}$ für das Anion einer Säure steht und $R_2''$ Wasserstoff, Niederalkoxy, Benzyloxy, $C_2$-$C_5$-Alkanoyloxy, Niederalkansulfonyloxy, Benzoyloxy, Pyridoyloxy, geschütztes Hydroxy, $C_2$-$C_5$-Alkanoylamino, Niederalkansulfonylamino, Benzoylamino, Pyridoylamino, geschütztes Amino, Carboxy, Niederalkoxycarbonyl, N-Niederalkylcarbamyl, N,N-Diniederalkylcarbamyl, $C_2$-$C_5$-Alkanoyloxymethyl, Niederalkansulfonyloxymethyl, Benzoyloxymethyl, Pyridoyloxymethyl oder veräthertes oder geschütztes Hydroxymethyl bedeutet, die überschüssigen Doppelbindungen zu Einfachbindungen reduziert oder

g) zur Herstellung einer Verbindung der Formel I oder eines Tautomeren und/oder Salzes davon, worin $R_2$ Carboxy, N-Niederalkylcarbamyl, N,N-Diniederalkylcarbamyl oder insbesondere Niederalkoxycarbonyl bedeutet, eine Verbindung der Formel

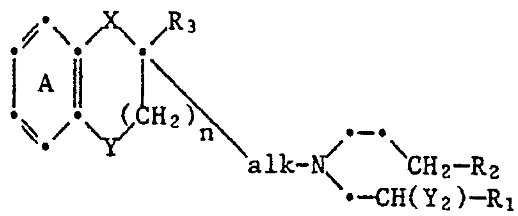

(VIII),

worin $Y_2$ einen abspaltbaren Rest darstellt, oder ein Salz davon cyclisiert und jeweils eine gegebenenfalls vorhandene Schutzgruppe abspaltet und jeweils gewünschtenfalls eine verfahrensgemäss oder auf andere Weise erhältliche Verbindung der Formel I in eine andere Verbindung der Formel I überführt, jeweils ein verfahrensgemäss erhältliches Isomerengemisch in die Komponente auftrennt, jeweils ein verfahrensgemäss erhältliches Enantiomeren- bzw. Diastereomerengemisch in die Enantiomeren bzw. Diastereomeren aufspaltet und/oder jeweils eine verfahrensgemäss erhältliche freie Verbindung der Formel I in ein Salz überführt oder ein verfahrensgemäss erhältliches Salz in die freie Verbindung der Formel I oder in ein anderes Salz umwandelt und eine jeweils erhaltene Verbindung der Formel

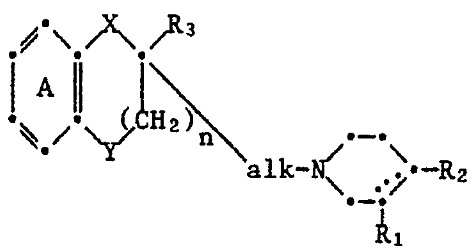

(I),

worin entweder $R_1$ Carboxy, Niederalkoxycarbonyl, Carbamyl, N-Niederalkylcarbamyl, N,N-Diniederalkylcarbamyl, Hydroxymethyl, $C_2$-$C_5$-Alkanoyloxymethyl, Niederalkansulfonyloxymethyl, Benzoyloxymethyl oder Pyridoyloxymethyl bedeutet und $R_2$ Wasserstoff, Hydroxy, Niederalkoxy, Benzyloxy, $C_2$-$C_5$-Alkanoyloxy, Niederalkansulfonyloxy, Benzoyloxy, Pyridoyloxy, Amino, $C_2$-$C_5$-Alkanoylamino, Niederalkansulfonylamino, Benzoylamino oder Pyridoylamino darstellt oder $R_1$ Wasserstoff bedeutet und $R_2$ für Carboxy, Niederalkoxycarbonyl, N-Niederalkylcarbamyl, N,N-Diniederalkylcarbamyl, Hydroxymethyl, $C_2$-$C_5$-Alkanoyloxymethyl, Niederalkansulfonyloxymethyl, Benzoyloxymethyl oder Pyridoyloxymethyl steht, $R_3$ Wasserstoff oder $C_1$-$C_7$-Alkyl bedeutet, alk Niederalkylen, das die beiden

Ringsysteme durch bis und mit 3 C-Atome überbrückt, oder Niederalkyliden bedeutet, der Ring A unsubstituiert oder ein-, zwei- oder mehrfach durch Hydroxy, Niederalkoxy, $C_2$-$C_5$-Alkanoyloxy, Halogen mit einer Atomnummer bis und mit 53, $C_1$-$C_7$-Alkyl und/oder Trifluormethyl substituiert ist, die gestrichelte Linie zum Ausdruck bringen soll, dass zwischen den Kohlenstoffatomen, welche die Reste $R_1$ und $R_2$ tragen, eine Einfach- oder eine Doppelbindung vorliegt und entweder X und Y jeweils ein Sauerstoffatom bedeuten und n für 1 steht oder X eine Methylengruppe darstellt, Y ein Sauerstoffatom bedeutet und n für 1 steht oder X ein Sauerstoffatom darstellt, Y eine Methylengruppe bedeutet und n für 1 steht oder X für eine direkte Bindung steht, Y ein Sauerstoffatom darstellt und n für 2 steht, oder ein jeweils erhaltenes Tautomeres und/oder ein jeweils erhaltenes pharmazeutisch verwendbares Salz davon mit üblichen pharmazeutischen Hilfsstoffen vermischt, wobei mit "Nieder" bezeichnete Reste 1 bis und mit 4 Kohlenstoffatome aufweisen können.

**26.** Verfahren zur Herstellung eines pharmazeutischen Präparates, dadurch gekennzeichnet, dass man

h) Verbindungen der Formeln

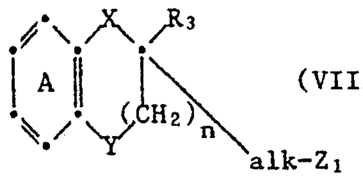

(VIIIa) und $Z_2-CH_2-CH(Z_3)-R_1$ (VIIIb),

worin einer der Reste $Z_1$ und $Z_2$ reaktionsfähiges verestertes Hydroxy, der andere Amino und $Z_3$ Wasserstoff bedeutet oder $Z_1$ Amino ist und $Z_2$ sowie $Z_3$ gemeinsam eine zusätzliche Bindung darstellen, oder gegebenenfalls Salze dieser Verbindungen untereinander umsetzt oder

i) in einer Verbindung der Formel

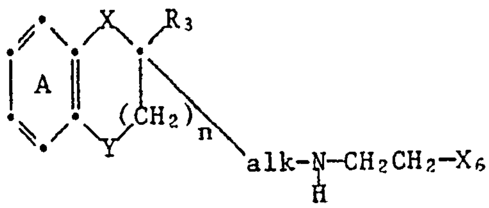

(IX),

worin $X_6$ einen in $R_1$ überführbaren Rest bedeutet, oder einem Salz davon $X_6$ in $R_1$ überführt und gewünschtenfalls jeweils eine verfahrensgemäss oder auf andere Weise erhältliche Verbindung der Formel IVc in eine andere Verbindung der Formel IVc überführt, jeweils ein verfahrensgemäss erhältliches Isomerengemisch in die Komponente auftrennt, jeweils ein verfahrensgemäss erhältliches Enantiomeren- bzw. Diastereomerengemisch in die Enantiomeren bzw. Diastereomeren aufspaltet und/oder jeweils eine verfahrensgemäss erhältliche freie Verbindung der Formel IVc in ein Salz überführt oder ein verfahrensgemäss erhältliches Salz in die freie Verbindung der Formel IVc oder in ein anderes Salz umwandelt und eine jeweils erhaltene Verbindung der Formel

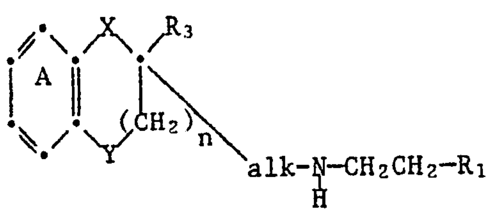

(IVc),

worin $R_1$ Carboxy, Niederalkoxycarbonyl, Carbamyl, N-Niederalkylcarbamyl, N,N-Diniederalkylcarbamyl,

Hydroxymethyl, $C_2$-$C_5$-Alkanoyloxymethyl, Niederalkansulfonyloxymethyl, Benzoyloxymethyl oder Pyridoyloxymethyl bedeutet, $R_3$ Wasserstoff oder $C_1$-$C_7$-Alkyl darstellt, alk Niederalkylen oder Niederalkyliden bedeutet, der Ring A unsubstituiert oder ein- oder mehrfach durch Hydroxy, Niederalkoxy, $C_2$-$C_5$-Alkanoyloxy, Halogen mit einer Atomnummer bis und mit 53, $C_1$-$C_7$-Alkyl und/oder Trifluormethyl substituiert ist, und entweder X und Y jeweils ein Sauerstoffatom bedeuten und n für 1 steht oder X eine Methylengruppe darstellt, Y ein Sauerstoffatom bedeutet und n für 1 steht oder X ein Sauerstoffatom darstellt, Y eine Methylengruppe bedeutet und n für 1 steht oder X für eine direkte Bindung steht, Y ein Sauerstoffatom darstellt und n für 2 steht, oder ein jeweils erhaltenes pharmazeutisch verwendbares Salz davon mit üblichen pharmazeutischen Hilfstoffen vermischt, wobei mit "Nieder" bezeichnete Reste 1 bis und mit 4 Kohlenstoffatome aufweisen können.

**27.** Verfahren zur Herstellung eines pharmazeutischen Präparats, dadurch gekennzeichnet, dass man Verbindungen der Formeln

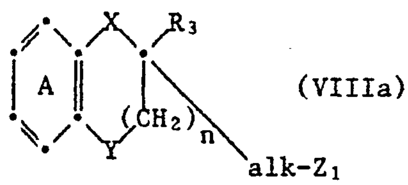

(VIIIa) und $Z_2{-}CH_2{-}CH(Z_3){-}R_1$ (VIIIb),

worin einer der Reste $Z_1$ und $Z_2$ reaktionsfähiges verestertes Hydroxy, der andere Amino und $Z_3$ Wasserstoff bedeutet oder $Z_1$ Amino ist und $Z_2$ sowie $Z_3$ gemeinsam eine zusätzliche Bindung darstellen, oder gegebenenfalls Salze dieser Verbindungen untereinander umsetzt und gewünschtenfalls eine verfahrensgemäss oder auf andere Weise erhältliche Verbindung der Formel IVc in eine andere Verbindung der Formel IVc überführt, ein verfahrensgemäss erhältliches Isomerengemisch in die Komponenten auftrennt, die verfahrensgemäss erhältliches Enantiomeren- bzw. Diastereomerengemisch in die Enantiomeren bzw. Diastereomeren aufspaltet und/oder eine verfahrensgemäss erhältliche freie Verbindung der Formel IVc in ein Salz überführt oder ein verfahrensgemäss erhältliches Salz in die freie Verbindung der Formel IVc oder in ein anderes Salz umwandelt und eine jeweils erhaltene Verbindung der Formel

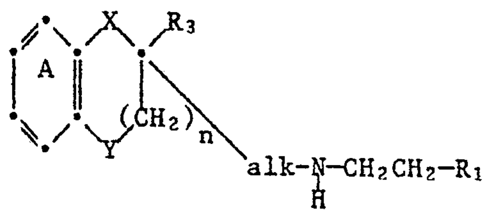

(IVc),

worin $R_1$ Carboxy, Niederalkoxycarbonyl, Carbamyl, N-Niederalkylcarbamyl oder N,N-Diniederalkylcarbamyl bedeutet, $R_3$ Wasserstoff oder $C_1$-$C_7$-Alkyl darstellt, alk Niederalkylen oder Niederalkyliden bedeutet, der Ring A unsubstituiert oder ein- oder mehrfach durch Hydroxy, Niederalkoxy, $C_2$-$C_5$-Alkanoyloxy, Halogen mit einer Atomnummer bis und mit 53, $C_1$-$C_7$-Alkyl und/oder Trifluormethyl substituiert ist, X ein Sauerstoffatom oder eine Methylengruppe darstellt, Y ein Sauerstoffatom bedeutet und n für 1 steht, oder ein jeweils erhaltenes pharmazeutisch verwendbares Salz davon mit üblichen pharmazeutischen Hilfsstoffen vermischt, wobei mit "Nieder" bezeichnete Reste 1 bis und mit 4 Kohlenstoffatome aufweisen können.

**28.** Verfahren zur Herstellung eines pharmazeutischen Präparates, dadurch gekennziechnet, dass man eine Verbindung, erhältlich gemäss einem der Ansprüche 1 bis 24, oder gegebenenfalls ein Tautomeres und/oder ein pharmazeutisch verwendbares Salz davon mit üblichen pharmazeutischen Hilfsstoffen vermischt.

**29.** Verfahren zur Herstellung eines pharmazeutischen Präparates, dadurch gekennzeichnet, dass man eine Verbindung, erhältlich gemäss einem der Ansprüche 2, 4, 6, 8 und 17 bis 23, oder gegebenenfalls ein

Tautomeres und/oder ein pharmazeutisch verwendbares Salz davon mit üblichen pharmazeutischen Hilfsstoffen vermischt.

**30.** Verfahren zur Herstellung eines pharmazeutischen Präparates, dadurch gekennzeichnet, dass man eine Verbindung der Formel IVc, worin $R_1$ Carbamyl oder N-Methyl-, N-Aethyl-, N,N-Dimethyl- oder N,N-Diäthylcarbamyl bedeutet, $R_3$ Wasserstoff darstellt, alk Methylen bedeutet, der Ring A unsubstituiert ist, X und Y jeweils ein Sauerstoffatom bedeuten und n für 1 steht, oder ein pharmazeutisch verwendbares Salz davon mit üblichen pharmazeutischen Hilfsstoffen vermischt.

**31.** Verwendung einer Verbindung, erhältlich gemäss einem der Ansprüche 1 bis 24, oder gegebenenfalls eines Tautomeren und/oder eines pharmazeutisch verwendbaren Salzes davon zur Herstellung eines pharmazeutischen Präparats, z.B. eines Nootropikums.

## Claims

## Claims for the following Contracting States : BE, CH, DE, FR, GB, IT, LI, LU, NL, SE

**1.** A compound of the formula

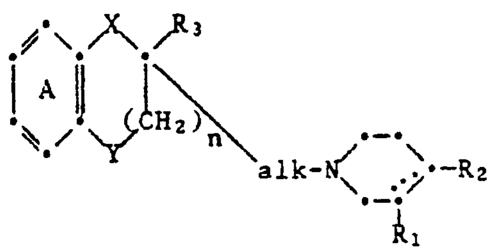

(I),

in which either $R_1$ represents carboxy, lower alkoxycarbonyl, carbamoyl, N-lower alkylcarbamoyl, N,N-di-lower alkylcarbamoyl, hydroxymethyl, $C_2$-$C_5$alkanoyloxymethyl, lower alkanesulphonyloxymethyl, benzoyloxymethyl or pyridoyloxymethyl and $R_2$ represents hydrogen, hydroxy, lower alkoxy, benzyloxy, $C_2$-$C_5$alkanoyloxy, lower alkanesulphonyloxy, benzoyloxy, pyridoyloxy, amino, $C_2$-$C_5$-alkanoylamino, lower alkanesulphonylamino, benzoylamino or pyridoylamino, or $R_1$ represents hydrogen and $R_2$ represents carboxy, lower alkoxycarbonyl, N-lower alkylcarbamoyl, N,N-di-lower alkylcarbamoyl, hydroxymethyl, $C_2$-$C_5$alkanoyloxymethyl, lower alkanesulphonyloxymethyl, benzoyloxymethyl or pyridoyloxymethyl, $R_3$ represents hydrogen or $C_1$-$C_7$alkyl, alk represents lower alkylene that bridges the two ring systems by up to and including 3 carbon atoms, or alk represents lower alkylidene, the ring A is unsubstituted or is mono-, di-or poly-substituted by hydroxy, lower alkoxy, $C_2$-$C_5$-alkanoyloxy, halogen having an atomic number of up to and including 53, $C_1$-$C_7$alkyl and/or by trifluoromethyl, the dotted line is intended to indicate the presence of a single or a double bond between the carbon atoms carrying the radicals $R_1$ and $R_2$, and either each of X and Y represents an oxygen atom and n represents 1, or X represents a methylene group, Y represents an oxygen atom and n represents 1, or X represents an oxygen atom, Y represents a methylene group and n represents 1, or X represents a direct bond, Y represents an oxygen atom and n represents 2, radicals designated "lower" containing from 1 up to and including 4 carbon atoms, or a tautomer and/or salt thereof.

**2.** A compound of the formula I according to claim 1 in which $R_1$ represents carboxy, lower alkoxycarbonyl, carbamoyl, N-lower alkylcarbamoyl or N,N-di-lower alkylcarbamoyl, $R_2$ represents hydrogen, hydroxy, lower alkoxy, benzyloxy, $C_2$-$C_5$alkanoyloxy, lower alkanesulphonyloxy, benzoyloxy, pyridoyloxy, amino, $C_2$-$C_5$-alkanoylamino, benzoylamino or pyridoylamino, $R_3$ represents hydrogen or $C_1$-$C_7$alkyl, alk represents lower alkylene that bridges the two ring systems by up to and including 3 carbon atoms, or alk represents lower alkylidene, the ring A is unsubstituted or is mono-, di-or poly-substituted by hydroxy, lower alkoxy, $C_2$-$C_5$-alkanoyloxy, halogen having an atomic number of up to and including 53, $C_1$-$C_7$alkyl and/or by trifluoromethyl, the dotted line is intended to indicate the presence of a single or a double bond between the carbon atoms carrying the radicals $R_1$ and $R_2$, X represents an oxygen atom or a methylene group, Y represents an oxygen atom and n represents 1, radicals designated "lower" containing from 1 up to and including 4 carbon atoms, or a tautomer and/or salt thereof.

3. A compound of the formula I according to claim 1 in which either $R_1$ represents $C_1$-$C_4$alkoxycarbonyl, carbamoyl, hydroxymethyl or $C_2$-$C_5$alkanoyloxymethyl and $R_2$ represents hydrogen or hydroxy, or $R_1$ represents hydrogen and $R_2$ represents $C_1$-$C_4$alkoxycarbonyl, $R_3$ represents hydrogen or $C_1$-$C_4$alkyl, alk represents $C_1$-$C_4$alkylene that bridges the two ring systems by up to and including 3 carbon atoms, the ring A is unsubstituted or is substituted, especially in the 7-position, by $C_1$-$C_4$alkoxy, the dotted line is intended to indicate the presence of a single or a double bond between the carbon atoms carrying the radicals $R_1$ and $R_2$, and either each of X and Y represents an oxygen atom and n represents 1, or X represents a methylene group, Y represents an oxygen atom and n represents 1, or X represents an oxygen atom, Y represents a methylene group and n represents 1, or X represents a direct bond, Y represents an oxygen atom and n represents 2, or a tautomer and/or salt thereof.

4. A compound of the formula I according to claim 1 in which $R_1$ represents $C_1$-$C_4$alkoxycarbonyl, $R_2$ represents hydrogen or hydroxy, $R_3$ represents hydrogen or $C_1$-$C_4$-alkyl, alk represents $C_1$-$C_4$alkylene that bridges the two ring systems by up to and including 3 carbon atoms, the ring A is unsubstituted, the dotted line is intended to indicate the presence of a single or a double bond between the carbon atoms carrying the radicals $R_1$ and $R_2$, X represents an oxygen atom or a methylene group, Y represents an oxygen atom and n represents 1, or a tautomer and/or salt thereof.

5. A compound of the formula I according to claim 1 in which $R_1$ represents $C_1$-$C_4$alkoxycarbonyl, $R_2$ represents hydrogen or hydroxy, $R_3$ represents hydrogen, alk represents methylene or ethylene, the ring A is unsubstituted, the dotted line is intended to indicate the presence of a single or a double bond between the carbon atoms carrying the radicals $R_1$ and $R_2$, X represents an oxygen atom or a methylene group, Y represents an oxygen atom and n represents 1, or a tautomer and/or salt thereof.

6. 4-Hydroxy-1-(chroman-3-ylmethyl)-1,2,5,6-tetrahydropyridine-3-carboxylic acid methyl ester or 1-(chroman-3-ylmethyl)-4-oxo-piperidine-3-carboxylic acid methyl ester, 1-[2-(chroman-3-yl)-ethyl]-1,2,5,6-tetrahydropyridine-3-carboxylic acid methyl ester, 1-(benzo-1,4-dioxan-2-ylmethyl)piperidine-3-carboxylic acid methyl ester or 4-hydroxy-1-(benzo-1,4-dioxan-2-ylmethyl)-1,2,5,6-tetrahydropyridine-3-carboxylic acid methyl ester or 1-(benzo-1,4-dioxan-2-ylmethyl)-4-oxopiperidine-3-carboxylic acid methyl ester or, respectively, a salt thereof.

7. 1-[2-(Chroman-3-yl)-ethyl]-1,2,5,6-tetrahydropyridine-3-carboxylic acid ethyl ester or a salt thereof.

8. 1-(Chroman-3-ylmethyl)-1,2,5,6-tetrahydropyridine-3-carboxylic acid methyl ester, 1-[2-(chroman-3-yl)-ethyl]-4-hydroxy-1,2,5,6-tetrahydropyridine-3-carboxylic acid methyl ester or 1-[2-(chroman-3-yl)-ethyl]-4-oxopiperidine-3-carboxylic acid methyl ester, 1-(benzo-1,4-dioxan-2-ylmethyl)-1,2,5,6-tetrahy dropyridine-3-carboxylic acid methyl ester, 4-hydroxy-1-(2-methyl-benzo-1,4-dioxan-2-ylmethyl)-1,2,5,6-tetrahydropyridine-3-carboxylic acid methyl ester or 1-(2-methyl-benzo-1,4-dioxan-2-ylm ethyl)-4-oxopiperidine-3-carboxylic acid methyl ester, 1-[2-(benzo-1,4-dioxan-2-yl)-ethyl]-4-hydroxy-1,2,5,6-tetrahydropyridine-3-carboxylic acid methyl ester or 1-[2-(benzo-1,4-dioxan-2-yl)-ethyl]-4-oxopiperidine-3-carboxylic acid methyl ester, cis-1-[2-(benzo-1,4-dioxan-2-yl)-ethyl]-4-hydroxypiperidine-3-carboxylic acid methyl ester, trans-1-[2-(benzo-1,4-dioxan-2-yl)-ethyl]-4-hydroxypiperidine-3-carboxylic acid methyl ester or 1-[2-(benzo-1,4-dioxan-2-yl)-ethyl]-1,2,5,6-tetrahydropyridine-3-carboxylic acid methyl ester or, respectively, a salt thereof.

9. 1-[2-(Chroman-3-yl)-ethyl]-piperidine-3-carboxylic acid ethyl ester, 1-[2-(chroman-3-yl)-ethyl]-piperidine-4-carboxylic acid ethyl ester, 1-[2-(benzo-1,4-dioxan-2-yl)-ethyl]-piperidine-3-carboxylic acid ethyl ester, 1-[2-(benzo-1,4-dioxan-2-yl)-ethyl]-piperidine-4-carboxylic acid ethyl ester, 1-[2-(chroman-2-yl)-ethyl]-piperidine-3-carboxylic acid ethyl ester, 1-[2-(chroman-2-yl)-ethyl]-piperidine-4-carboxylic acid ethyl ester, cis-1-[2-(benzo-1,4-dioxan-2-yl)-ethyl]-4-benzyloxy-piperidine-3-carboxylic acid methyl ester, trans-1-[2-(benzo-1,4-dioxan-2-yl)-ethyl]-4-benzyloxy-piperidine-3-carboxylic acid methyl ester, cis-4-amino-1-[2-(benzo-1,4-dioxan-2-yl)-ethyl]-piperidine-3-carboxylic acid amide, trans-4-amino-1-[2-(benzo-1,4-dioxan-2-yl)-ethyl]-piperidine-3-carboxylic acid amide, 1-[2-(chroman-4-yl)-ethyl]-1,2,5,6-tetrahydropyridine-3-carboxylic acid methyl ester or 1-[2-(chroman-4-yl)-ethyl]-piperidine-3-carboxylic acid ethyl ester or, respectively, a salt thereof.

10. A process for the manufacture of a compound of the formula I, or a tautomer and/or salt thereof, according to any one of claims 1 to 9, characterised in that

a) a compound of the formula

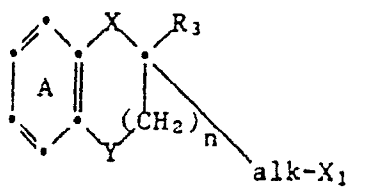

(IIa)

or a salt thereof, in which $X_1$ represents hydroxy or reactive esterified hydroxy, is reacted with a compound of the formula

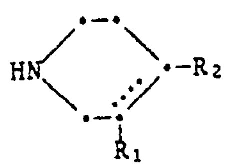

(IIb)

or a tautomer and/or salt thereof, or
b) in a compound of the formula

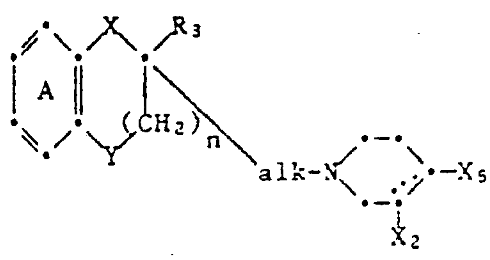

(III)

or a tautomer and/or salt thereof, in which $X_2$ represents a radical that can be converted into $R_1$ that is other than hydrogen, and $X_5$ represents a radical $R_a$, and $R_a$ represents hydrogen, hydroxy, lower alkoxy, benzyloxy, $C_2$-$C_5$alkanoyloxy, lower alkanesulphonyloxy, benzoyloxy, pyridoyloxy, amino, $C_2$-$C_5$alkanoylamino, lower alkanesulphonylamino, benzoylamino or pyridoylamino, $X_2$ is converted into $R_1$ that is other than hydrogen, or in a compound of the formula III in which $X_2$ represents hydrogen and $x_5$ represents a radical that can be converted into $R_b$, and $R_b$ represents a radical $R_2$ other than a radical $R_a$, $X_5$ is converted into $R_b$, or
c) for the manufacture of a compound of the formula I or a tautomer and/or salt thereof, in which $R_2$ represents hydroxy or amino and $R_1$ is other than hydrogen and represents especially lower alkoxycarbonyl, a compound of the formula

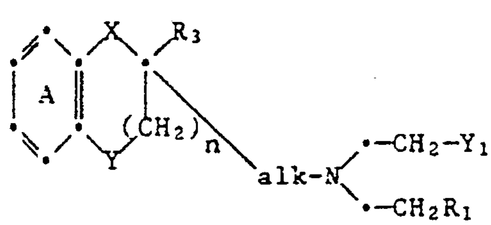

(IV),

in which $Y_1$ represents a group of the formula $-CH=R_2'$ , $-C(Y_2)=R_2'$ , $-CH(Y_2)-R_2$ or cyano, wherein $R_2'$ represents oxo or imino and $Y_2$ represents a removable radical, or a salt thereof, is cyclised, or
d) for the manufacture of a compound of the formula I' or a tautomer and/or salt thereof, in which $R_2'$ represents oxo or imino and $R_1$ is other than hydrogen and represents especially lower alkoxycarbonyl, a compound of the formula

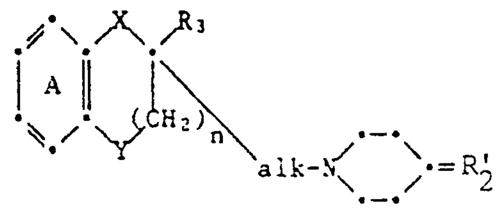

(Va)

or a tautomer, or in each case a salt thereof, is reacted with a compound of the formula

$X_3$-$R_1$ (Vb)

or a salt thereof, in which $R_1$ is other than hydrogen and $X_3$ represents halogen or lower alkoxy, or
e) for the manufacture of a compound of the formula I or a tautomer and/or salt thereof, in which $R_2$ represents hydroxy, lower alkoxy, benzyloxy, $C_2$-$C_5$alkanoyloxy, lower alkanesulphonyloxy, benzoyloxy, pyridoyloxy, amino, $C_2$-$C_5$alkanoylamino, lower alkanesulphonylamino, benzoylamino or pyridoylamino, in a compound of the formula

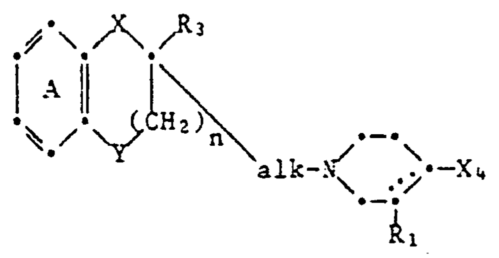

(VI)

or in a salt thereof, in which $X_4$ represents a radical that can be converted into $R_2$, $X_4$ is converted into $R_2$, or
f) especially for the manufacture of a compound of the formula I or a tautomer and/or salt thereof, in which $R_2$ is hydrogen, hydroxy, amino, carboxy, lower alkoxycarbonyl, N-lower alkylcarbamoyl, N,N-di-lower alkylcarbamoyl or hydroxymethyl, in a compound of the formula

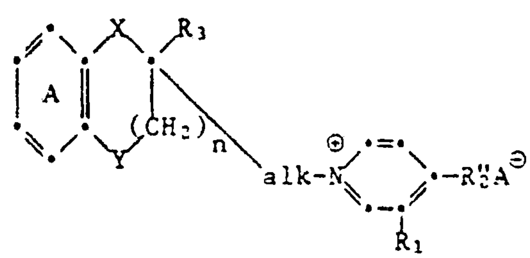

(VII),

in which $A^{\ominus}$ represents the anion of an acid and $R_2''$ represents hydrogen, lower alkoxy, benzyloxy, $C_2$-$C_5$-alkanoyloxy, lower alkanesulphonyloxy, benzoyloxy, pyridoyloxy, protected hydroxy, $C_2$-$C_5$-alkanoylamino, lower alkanesulphonylamino, benzoylamino, pyridoylamino, protected amino, carboxy, lower alkoxycarbonyl, N-lower alkylcarbamoyl, N,N-di-lower alkylcarbamoyl, $C_2$-$C_5$-alkanoyloxymethyl, lower alkanesulphonyloxymethyl, benzoyloxymethyl, pyridoyloxymethyl or etherified or protected hydroxymethyl, the excess double bonds are reduced to single bonds, or
g) for the manufacture of a compound of the formula I or a tautomer and/or salt thereof, in which $R_2$ represents carboxy, N-lower alkylcarbamoyl, N,N-di-lower alkylcarbamoyl or especially lower alkoxycarbonyl, a compound of the formula

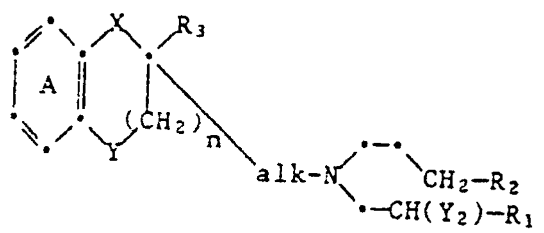

(VIII),

in which $Y_2$ represents a removable radical, or a salt thereof, is cyclised, and in the case of each of processes a) to g), a protecting group which may be present is removed, and, if desired, a compound of the formula I obtainable in accordance with the process or by other means is converted into a different compound of the formula I, an isomeric mixture obtainable in accordance with the process is separated into the components, an enantiomeric or diastereoisomeric mixture obtainable in accordance with the process is separated into the enantiomers or diastereoisomers, respectively, and/or a free compound of the formula I obtainable in accordance with the process is converted into a salt or a salt obtainable in accordance with the process is converted into the free compound of the formula I or into a different salt, radicals designated "lower" containing from 1 up to and including 4 carbon atoms.

**11.** A compound of the formula

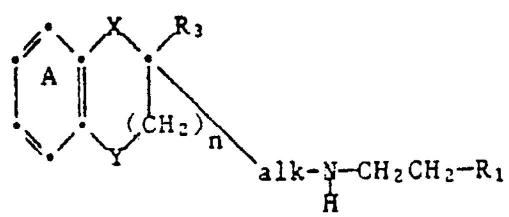

(IVc),

in which $R_1$ represents carboxy, lower alkoxycarbonyl, carbamoyl, N-lower alkylcarbamoyl, N,N-di-lower alkylcarbamoyl, hydroxymethyl, $C_2$-$C_5$alkanoyloxymethyl, lower alkanesulphonyloxymethyl, benzoyloxymethyl or pyridoyloxymethyl, $R_3$ represents hydrogen or $C_1$-$C_7$alkyl, alk represents lower alkylene or lower alkylidene, the ring A is unsubstituted or is mono- or poly-substituted by hydroxy, lower alkoxy, $C_2$-$C_5$alkanoyloxy, halogen having an atomic number of up to and including 53, $C_1$-$C_7$alkyl and/or by trifluoromethyl, and either each of X and Y represents an oxygen atom and n represents 1, or X represents a methylene group, Y represents an oxygen atom and n represents 1, or X represents an oxygen atom, Y represents a methylene group and n represents 1, or X represents a direct bond, Y represents an oxygen atom and n represents 2, with the proviso that in compounds of the formula IVc in which the ring A is unsubstituted, each of X and Y represents oxygen, n represents 1 and $R_3$ represents hydrogen, alk is other than methylene if $R_1$ represents carbamoyl or N-methyl-, N-ethyl-, N,N-dimethyl- or N,N-diethyl-carbamoyl, radicals designated "lower" containing from 1 up to and including 4 carbon atoms, or a salt thereof.

**12.** A compound of the formula IVc according to claim 11 in which $R_1$ represents carboxy, lower alkoxycarbonyl, carbamoyl, N-lower alkylcarbamoyl or N,N-di-lower alkylcarbamoyl, $R_3$ represents hydrogen or $C_1$-$C_7$alkyl, alk represents lower alkylene or lower alkylidene, the ring A is unsubstituted or is mono- or poly-substituted by hydroxy, lower alkoxy, $C_2$-$C_5$alkanoyloxy, halogen having an atomic number of up to and including 53, $C_1$-$C_7$alkyl and/or by trifluoromethyl, X represents an oxygen atom or a methylene group, Y represents an oxygen atom and n represents 1, with the proviso that in compounds of the formula IVc in which the ring A is unsubstituted, each of X and Y represents oxygen, n represents 1 and $R_3$ represents hydrogen, alk is other than methylene if $R_1$ represents carbamoyl or N-methyl-, N-ethyl-, N,N-dimethyl- or N,N-diethyl-carbamoyl, radicals designated "lower" containing from 1 up to and including 4 carbon atoms, or a salt thereof.

**13.** A compound of the formula IVc according to claim 11 in which $R_1$ represents carboxy, lower alkoxycarbonyl, carbamoyl, N-lower alkylcarbamoyl, N,N-di-lower alkylcarbamoyl, hydroxymethyl, $C_2$-$C_5$alkanoyloxymethyl, lower alkanesulphonyloxymethyl, benzoyloxymethyl or pyridoyloxymethyl, $R_3$

represents hydrogen or $C_1$-$C_7$alkyl, alk represents lower alkylene that links the ring system with the NH group shown in formula IVc by up to and including 3 carbon atoms, or alk represents lower alkylidene, the ring A is unsubstituted or is mono-, di- or poly-substituted by hydroxy, lower alkoxy, $C_2$-$C_5$alkanoyloxy, halogen having an atomic number of up to and including 53, $C_1$-$C_7$alkyl and/or by trifluoromethyl, and either each of X and Y represents an oxygen atom and n represents 1, or X represents a methylene group, Y represents an oxygen atom and n represents 1, or X represents an oxygen atom, Y represents a methylene group and n represents 1, or X represents a direct bond, Y represents an oxygen atom and n represents 2, with the proviso that in compounds of the formula IVc in which the ring A is unsubstituted, each of X and Y represents oxygen, n represents l and $R_3$ represents hydrogen, alk is other than methylene if $R_1$ represents carbamoyl or N-methyl-, N-ethyl-, N,N-dimethyl- or N,N-diethyl-carbamoyl, radicals designated "lower" containing from 1 up to and including 4 carbon atoms, or a salt thereof.

**14.** A compound of the formula IVc according to claim 11 in which $R_1$ represents carboxy, lower alkoxycarbonyl, carbamoyl, N-lower alkylcarbamoyl or N,N-di-lower alkylcarbamoyl, $R_3$ represents hydrogen or $C_1$-$C_7$alkyl, alk represents lower alkylene that links the ring system with the NH group shown in formula IVc by up to and including 3 carbon atoms, or alk represents lower alkylidene, the ring A is unsubstituted or is mono-, di- or poly-substituted by hydroxy, lower alkoxy, $C_2$-$C_5$alkanoyloxy, halogen having an atomic number of up to and including 53, $C_1$-$C_7$alkyl and/or by trifluoromethyl, X represents an oxygen atom or a methylene group, Y represents an oxygen atom and n represents 1, with the proviso that in compounds of the formula IVc in which the ring A is unsubstituted, each of X and Y represents oxygen, n represents 1 and $R_3$ represents hydrogen, alk is other than methylene if $R_1$ represents carbamoyl or N-methyl-, N-ethyl-, N,N-dimethyl- or N,N-diethyl-carbamoyl, radicals designated "lower" containing from 1 up to and including 4 carbon atoms, or a salt thereof.

**15.** A compound of the formula IVc according to claim 11 in which $R_1$ represents carboxy, hydroxymethyl, $C_2$-$C_5$-alkanoyloxymethyl, $C_1$-$C_4$alkoxycarbonyl or carbamoyl, $R_3$ represents hydrogen or $C_1$-$C_4$alkyl, alk represents $C_1$-$C_4$alkylene that links the ring system with the NH group shown in formula IVc by up to and including 3 carbon atoms, the ring A is unsubstituted or is substituted, especially in the 7-position, by $C_1$-$C_4$alkoxy, and either each of X and Y represents an oxygen atom and n represents 1, or X represents a methylene group, Y represents an oxygen atom and n represents 1, or X represents an oxygen atom, Y represents a methylene group and n represents 1, or x represents a direct bond, Y represents an oxygen atom and n represents 2, with the proviso that in compounds of the formula IVc in which the ring A is unsubstituted, each of X and Y represents oxygen, n represents 1 and $R_3$ represents hydrogen, alk is other than methylene if $R_1$ represents carbamoyl, or a salt thereof.

**16.** A compound of the formula IVc according to claim 11 in which $R_1$ represents $C_1$-$C_4$alkoxycarbonyl, $R_3$ represents hydrogen or $C_1$-$C_4$alkyl, alk represents $C_1$-$C_4$alkylene that links the ring system with the NH group shown in formula IVc by up to and including 3 carbon atoms, the ring A is unsubstituted, X represents an oxygen atom or a methylene group, Y represents an oxygen atom and n represents 1, or a salt thereof.

**17.** A compound of the formula IVc according to claim 11 in which $R_1$ represents $C_1$-$C_4$alkoxycarbonyl, $R_3$ represents hydrogen, alk represents methylene or ethylene, the ring A is unsubstituted, X represents an oxygen atom or a methylene group, Y represents an oxygen atom and n represents 1, or a salt thereof.

**18.** N-[2-(Chroman-3-yl)-ethyl]-N-(2-methoxycarbonylethyl)-amine or a salt thereof.

**19.** N-(2-Methoxycarbonylethyl)-N-(benzo-1,4-dioxan-2-ylmethyl)-amine or a salt thereof.

**20.** N-(2-Methoxycarbonylethyl)-N-(chroman-3-ylmethyl)-amine, N-[2-(benzo-1,4-dioxan-2-yl)-ethyl]-N-(2-methoxycarbonylethyl)-amine or N-(2-methoxycarbonylethyl)-N-(2-methylbenzo-1,4-dioxan-2-ylmethyl)-amine or, respectively, a salt thereof.

**21.** N-[2-(Chroman-3-yl)-ethyl]-N-(2-carboxyethyl)-amine, N-[2-(chroman-2-yl)-ethyl]-N-(2-methoxycarbonylethyl)-amine or N-[2-(chroman-4-yl)-ethyl]-N-(2-methoxycarbonylethyl)-amine or, respectively, a salt thereof.

**22.** A process for the manufacture of a compound of the formula IVc, or a salt thereof, according to any one of claims 11 to 21, characterised in that

h) compounds of the formulae

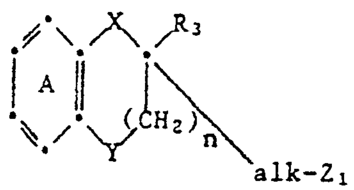

(VIIIa)

and $Z_2-CH_2-CH(Z_3)-R_1$ (VIIIb),

in which one of the radicals $Z_1$ and $Z_2$ represents reactive esterified hydroxy and the other represents amino and $Z_3$ represents hydrogen, or $Z_1$ represents amino and $Z_2$ and $Z_3$ together represent an additional bond, or optionally salts of these compounds, are reacted with one another, or

i) in a compound of the formula

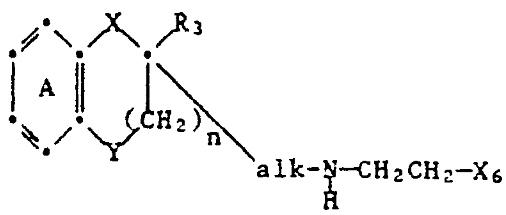

(IX)

in which $X_6$ represents a radical that can be converted into $R_1$, or in a salt thereof, $X_6$ is converted into $R_1$, and, if desired, in the case of each of processes h) and i), a compound of the formula IVc obtainable in accordance with the process or by other means is converted into a different compound of the formula IVc, an isomeric mixture obtainable in accordance with the process is separated into the components, an enantiomeric or diastereoisomeric mixture obtainable in accordance with the process is separated into the enantiomers or diastereoisomers, respectively, and/or a free compound of the formula IVc obtainable in accordance with the process is converted into a salt or a salt obtainable in accordance with the process is converted into the free compound of the formula IVc or into a different salt.

**23.** A compound of the formula

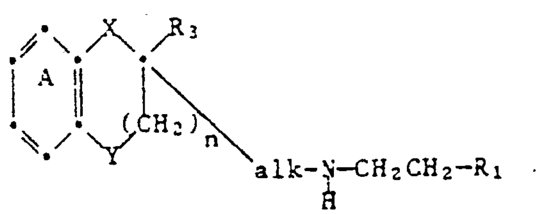

(IVc)

in which $R_1$ represents carboxy, lower alkoxycarbonyl, carbamoyl, N-lower alkylcarbamoyl, N,N-di-lower alkylcarbamoyl, hydroxymethyl, $C_2$-$C_5$alkanoyloxymethyl, lower alkanesulphonyloxymethyl, benzoyloxymethyl or pyridoyloxymethyl, $R_3$ represents hydrogen or $C_1$-$C_7$alkyl, alk represents lower alkylene or lower alkylidene, the ring A is unsubstituted or is mono- or poly-substituted by hydroxy, lower alkoxy, $C_2$-$C_5$alkanoyloxy, halogen having an atomic number of up to and including 53, $C_1$-$C_7$alkyl and/or by trifluoromethyl, and either each of X and Y represents an oxygen atom and n represents 1, or X represents a methylene group, Y represents an oxygen atom and n represents 1, or X represents an oxygen atom, Y represents a methylene group and n represents 1, or X represents a direct bond, Y represents an oxygen atom and n represents 2, radicals designated "lower" containing from 1 up to

and including 4 carbon atoms, or a pharmaceutically acceptable salt thereof, for use in a method for the therapeutic and/or prophylactic treatment of the human or animal body.

**24.** A compound of the formula

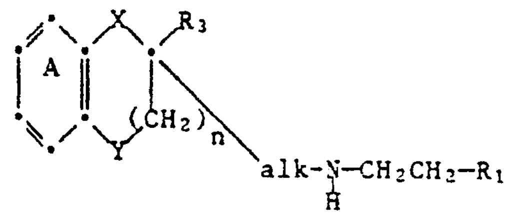

(IVc),

in which $R_1$ represents carboxy, lower alkoxycarbonyl, carbamoyl, N-lower alkylcarbamoyl or N,N-di-lower alkylcarbamoyl, $R_3$ represents hydrogen or $C_1$-$C_7$alkyl, alk represents lower alkylene or lower alkylidene, the ring A is unsubstituted or is mono- or poly-substituted by hydroxy, lower alkoxy, $C_2$-$C_5$alkanoyloxy, halogen having an atomic number of up to and including 53, $C_1$-$C_7$alkyl and/or by trifluoromethyl, x represents an oxygen atom or a methylene group, Y represents an oxygen atom and n represents 1, radicals designated "lower" containing from 1 up to and including 4 carbon atoms, or a pharmaceutically acceptable salt thereof, for use in a method for the therapeutic and/or prophylactic treatment of the human or animal body.

**25.** A compound according to any one of claims 1 to 9 and 11 to 21 or if appropriate a tautomer and/or a pharmaceutically acceptable salt thereof for use in a method for the therapeutic and/or prophylactic treatment of the human or animal body.

**26.** A compound according to any one of claims 2, 4, 6, 8, 12, 14 and 16 to 20 or if appropriate a tautomer and/or a pharmaceutically acceptable salt thereof for use in a method for the therapeutic and/or prophylactic treatment of the human or animal body.

**27.** A compound of the formula IVc according to claim 23 in which $R_1$ represents carbamoyl or N-methyl-, N-ethyl-, N,N-dimethyl or N,N-diethyl-carbamoyl, $R_3$ represents hydrogen, alk represents methylene, the ring A is unsubstituted, each of X and Y represents an oxygen atom and n represents 1, or a pharmaceutically acceptable salt thereof, for use in a method for the therapeutic and/or prophylactic treatment of the human or animal body.

**28.** A pharmaceutical preparation comprising as active ingredient a compound according to any one of claims 1 to 9, 11 to 21, 23 and 25 or if appropriate a tautomer and/or a pharmaceutically acceptable salt thereof, optionally together with customary pharmaceutical adjuncts.

**29.** A pharmaceutical preparation comprising as active ingredient a compound according to any one of claims 2, 4, 6, 8, 12, 14, 16 to 20, 24, 26 and 27 or if appropriate a tautomer and/or a pharmaceutically acceptable salt thereof, optionally together with customary pharmaceutical adjuncts.

**30.** The use of a compound according to any one of claims 1 to 9, 11 to 21, 23 and 25, or if appropriate a tautomer and/or a pharmaceutically acceptable salt thereof, for the manufacture of a pharmaceutical preparation, for example a nootropic.

**Claims for the following Contracting States : AT, ES, GR**

**1.** A process for the manufacture of a compound of the formula

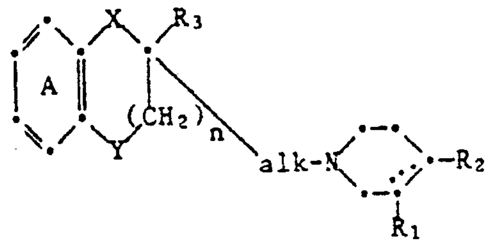

(I),

in which either $R_1$ represents carboxy, lower alkoxycarbonyl, carbamoyl, N-lower alkylcarbamoyl, N,N-di-lower alkylcarbamoyl, hydroxymethyl, $C_2$-$C_5$alkanoyloxymethyl, lower alkanesulphonyloxymethyl, benzoyloxymethyl or pyridoyloxymethyl and $R_2$ represents hydrogen, hydroxy, lower alkoxy, benzyloxy, $C_2$-$C_5$alkanoyloxy, lower alkanesulphonyloxy, benzoyloxy, pyridoyloxy, amino, $C_2$-$C_5$-alkanoylamino, lower alkanesulphonylamino, benzoylamino or pyridoylamino, or $R_1$ represents hydrogen and $R_2$ represents carboxy, lower alkoxycarbonyl, N-lower alkylcarbamoyl, N,N-di-lower alkylcarbamoyl, hydroxymethyl, $C_2$-$C_5$alkanoyloxymethyl, lower alkanesulphonyloxymethyl, benzoyloxymethyl or pyridoyloxymethyl, $R_3$ represents hydrogen or $C_1$-$C_7$alkyl, alk represents lower alkylene that bridges the two ring systems by up to and including 3 carbon atoms, or alk represents lower alkylidene, the ring A is unsubstituted or is mono-, di-or poly-substituted by hydroxy, lower alkoxy, $C_2$-$C_5$-alkanoyloxy, halogen having an atomic number of up to and including 53, $C_1$-$C_7$alkyl and/or by trifluoromethyl, the dotted line is intended to indicate the presence of a single or a double bond between the carbon atoms carrying the radicals $R_1$ and $R_2$, and either each of X and Y represents an oxygen atom and n represents 1, or X represents a methylene group, Y represents an oxygen atom and n represents 1, or X represents an oxygen atom, Y represents a methylene group and n represents 1, or X represents a direct bond, Y represents an oxygen atom and n represents 2, or a tautomer and/or salt thereof, characterised in that

a) a compound of the formula

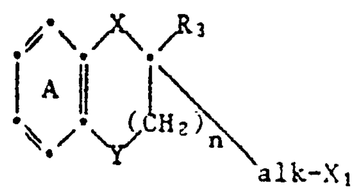

(IIa)

or a salt thereof, in which $X_1$ represents hydroxy or reactive esterified hydroxy, is reacted with a compound of the formula

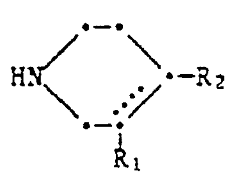

(IIb)

or a tautomer and/or salt thereof, or

b) in a compound of the formula

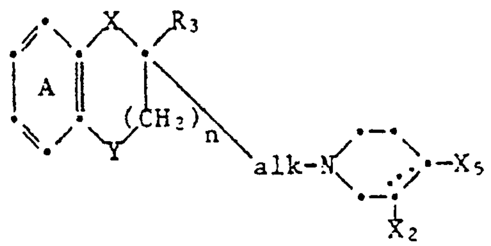

(III)

or a tautomer and/or salt thereof, in which $X_2$ represents a radical that can be converted into $R_1$ that is other than hydrogen, and $x_5$ represents a radical $R_a$, and $R_a$ represents hydrogen, hydroxy, lower alkoxy, benzyloxy, $C_2$-$C_5$alkanoyloxy, lower alkanesulphonyloxy, benzoyloxy, pyridoyloxy, amino, $C_2$-$C_5$alkanoylamino, lower alkanesulphonylamino, benzoylamino or pyridoylamino, $X_2$ is converted into $R_1$ that is other than hydrogen, or in a compound of the formula III in which $X_2$ represents hydrogen and $X_5$ represents a radical that can be converted into $R_b$, and $R_b$ represents a radical $R_2$ other than a radical $R_a$, $X_5$ is converted into $R_b$, or

c) for the manufacture of a compound of the formula I or a tautomer and/or salt thereof, in which $R_2$ represents hydroxy or amino and $R_1$ is other than hydrogen and represents especially lower alkoxycarbonyl, a compound of the formula

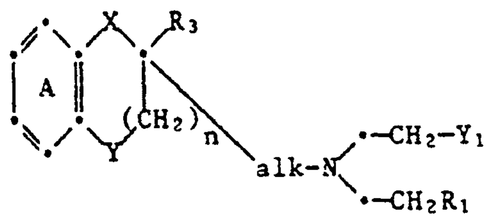

(IV),

in which $Y_1$ represents a group of the formula $-CH=R_2'$, $-C(Y_2)=R_2'$, $-CH(Y_2)-R_2$ or cyano, wherein $R_2'$ represents oxo or imino and $Y_2$ represents a removable radical, or a salt thereof, is cyclised, or

d) for the manufacture of a compound of the formula I' or a tautomer and/or salt thereof, in which $R_2'$ represents oxo or imino and $R_1$ is other than hydrogen and represents especially lower alkoxycarbonyl, a compound of the formula

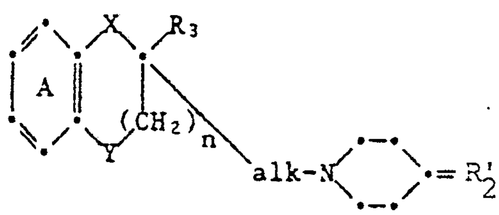

(Va)

or a tautomer, or in each case a salt thereof, is reacted with a compound of the formula

$X_3$-$R_1$ (Vb)

or a salt thereof, in which $R_1$ is other than hydrogen and $X_3$ represents halogen or lower alkoxy, or

e) for the manufacture of a compound of the formula I or a tautomer and/or salt thereof, in which $R_2$ represents hydroxy, lower alkoxy, benzyloxy, $C_2$-$C_5$alkanoyloxy, lower alkanesulphonyloxy, benzoyloxy, pyridoyloxy, amino, $C_2$-$C_5$alkanoylamino, lower alkanesulphonylamino benzoylamino or pyridoylamino, in a compound of the formula

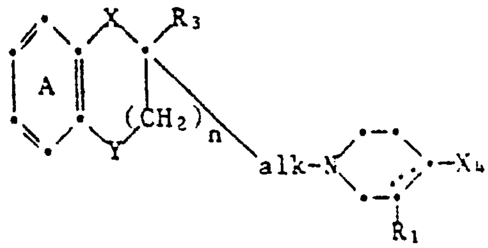

(VI)

or in a salt thereof, in which $X_4$ represents a radical that can be converted into $R_2$, $X_4$ is converted into $R_2$, or

f) especially for the manufacture of a compound of the formula I or a tautomer and/or salt thereof, in which $R_2$ is hydrogen, hydroxy, amino, carboxy, lower alkoxycarbonyl, N-lower alkylcarbamoyl, N,N-di-lower alkylcarbamoyl or hydroxymethyl, in a compound of the formula

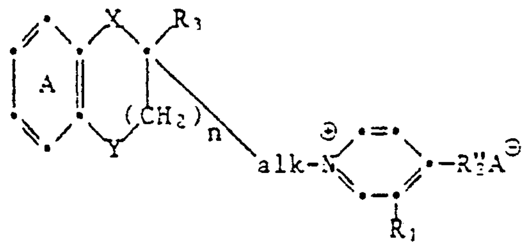

(VII),

in which $A^{\ominus}$ represents the anion of an acid and $R_2''$ represents hydrogen, lower alkoxy, benzyloxy, $C_2$-$C_5$-alkanoyloxy, lower alkanesulphonyloxy, benzoyloxy, pyridoyloxy, protected hydroxy, $C_2$-$C_5$-alkanoylamino, lower alkanesulphonylamino, benzoylamino, pyridoylamino, protected amino, carboxy, lower alkoxycarbonyl, N-lower alkylcarbamoyl, N,N-di-lower alkylcarbamoyl, $C_2$-$C_5$-alkanoyloxymethyl, lower alkanesulphonyloxymethyl, benzoyloxymethyl, pyridoyloxymethyl or etherified or protected hydroxymethyl, the excess double bonds are reduced to single bonds, or

g) for the manufacture of a compound of the formula I or a tautomer and/or salt thereof, in which $R_2$ represents carboxy, N-lower alkylcarbamoyl, N,N-di-lower alkylcarbamoyl or especially lower alkoxycarbonyl, a compound of the formula

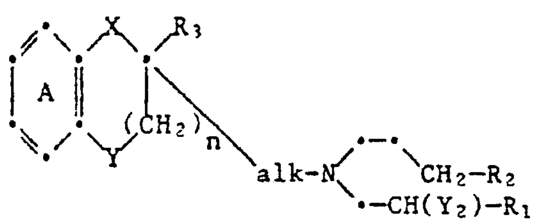

(VIII),

in which $Y_2$ represents a removable radical, or a salt thereof, is cyclised, and in the case of each of processes a) to g), a protecting group which may be present is removed, and, if desired, a compound of the formula I obtainable in accordance with the process or by other means is converted into a different compound of the formula I, an isomeric mixture obtainable in accordance with the process is separated into the components, an enantiomeric or diastereoisomeric mixture obtainable in accordance with the process is separated into the enantiomers or diastereoisomers, respectively, and/or a free compound of the formula I obtainable in accordance with the process is converted into a salt or a salt obtainable in accordance with the process is converted into the free compound of the formula I or into a different salt, radicals designated "lower" containing from 1 up to and including 4 carbon atoms.

2. A process according to claim 1 for the manufacture of a compound of the formula I in which $R_1$ represents carboxy, lower alkoxycarbonyl, carbamoyl, N-lower alkylcarbamoyl or N,N-di-lower alkylcar-

bamoyl, $R_2$ represents hydrogen, hydroxy, lower alkoxy, benzyloxy, $C_2$-$C_5$alkanoyloxy, lower alkanesulphonyloxy, benzoyloxy, pyridoyloxy, amino, $C_2$-$C_5$alkanoylamino, benzoylamino or pyridoylamino, $R_3$ represents hydrogen or $C_1$-$C_7$alkyl, alk represents lower alkylene that bridges the two ring systems by up to and including 3 carbon atoms, or alk represents lower alkylidene, the ring A is unsubstituted or is mono-, di- or poly-substituted by hydroxy, lower alkoxy, $C_2$-$C_5$alkanoyloxy, halogen having an atomic number of up to and including 53, $C_1$-$C_7$alkyl and/or by trifluoromethyl, the dotted line is intended to indicate the presence of a single or a double bond between the carbon atoms carrying the radicals $R_1$ and $R_2$, X represents an oxygen atom or a methylene group, Y represents an oxygen atom and n represents 1, radicals designated "lower" containing from 1 up to and including 4 carbon atoms, or a tautomer and/or salt thereof.

3. A process according to claim 1 for the manufacture of a compound of the formula I in which either $R_1$ represents $C_1$-$C_4$alkoxycarbonyl, carbamoyl, hydroxymethyl or $C_2$-$C_5$-alkanoyloxymethyl and $R_2$ represents hydrogen or hydroxy, or $R_1$ represents hydrogen and $R_2$ represents $C_1$-$C_4$alkoxycarbonyl, $R_3$ represents hydrogen or $C_1$-$C_4$alkyl, alk represents $C_1$-$C_4$alkylene that bridges the two ring systems by up to and including 3 carbon atoms, the ring A is unsubstituted or is substituted, especially in the 7-position, by $C_1$-$C_4$alkoxy, the dotted line is intended to indicate the presence of a single or a double bond between the carbon atoms carrying the radicals $R_1$ and $R_2$, and either each of X and Y represents an oxygen atom and n represents 1, or X represents a methylene group, Y represents an oxygen atom and n represents 1, or X represents an oxygen atom, Y represents a methylene group and n represents 1, or X represents a direct bond, Y represents an oxygen atom and n represents 2, or a tautomer and/or salt thereof.

4. A process according to claim 1 for the manufacture of a compound of the formula I in which $R_1$ represents $C_1$-$C_4$-alkoxycarbonyl, $R_2$ represents hydrogen or hydroxy, $R_3$ represents hydrogen or $C_1$-$C_4$-alkyl, alk represents $C_1$-$C_4$alkylene that bridges the two ring systems by up to and including 3 carbon atoms, the ring A is unsubstituted, the dotted line is intended to indicate the presence of a single or a double bond between the carbon atoms carrying the radicals $R_1$ and $R_2$, X represents an oxygen atom or a methylene group, Y represents an oxygen atom and n represents 1, or a tautomer and/or salt thereof.

5. A process according to claim 1 for the manufacture of a compound of the formula I in which $R_1$ represents $C_1$-$C_4$-alkoxycarbonyl, $R_2$ represents hydrogen or hydroxy, $R_3$ represents hydrogen, alk represents methylene or ethylene, the ring A is unsubstituted, the dotted line is intended to indicate the presence of a single or a double bond between the carbon atoms carrying the radicals $R_1$ and $R_2$, X represents an oxygen atom or a methylene group, Y represents an oxygen atom and n represents 1, or a tautomer and/or salt thereof.

6. A process according to either claim 1 or claim 2 for the manufacture of 4-hydroxy-1-(chroman-3-ylmethyl)-1,2,5,6-tetrahydropyridine-3-carboxylic acid methyl ester or 1(chroman-3-ylmethyl)-4-oxopiperidine-3-carboxylic acid methyl ester, 1-[2-(chroman-3-yl)-ethyl]-1,2,5,6-tetrahydropyridine-3-carboxylic acid methyl ester, 1-(benzo-1,4-dioxan-2-ylmethyl)-piperidine-3-carboxylic acid methyl ester or 4-hydroxy-1-(benzo-1,4-dioxan-2-ylmethyl)-1,2,5,6-tetrahydropyridine-3-carboxylic acid methyl ester or 1-(benzo-1,4-dioxan-2-ylmethyl)-4-oxopiperidine-3-carboxylic acid methyl ester or, respectively, a salt thereof.

7. A process according to claim 1 for the manufacture of 1-[2-(chroman-3-yl)-ethyl]-1,2,5,6-tetrahydropyridine-3-carboxylic acid ethyl ester or a salt thereof.

8. A process according to either claim 1 or claim 2 for the manufacture of 1-(chroman-3-ylmethyl)-1,2,5,6-tetrahydropyridine-3-carboxylic acid methyl ester, 1-[2-(chroman-3-yl)-ethyl]-4-hydroxy-1,2,5,6-tetrahydropyridine-3-carboxylic acid methyl ester or 1-[2-(chroman-3-yl)ethyl]-4-oxopiperidine-3-carboxylic acid methyl ester, 1-(benzo-1,4-dioxan-2-ylmethyl)-1,2,5,6-tetrahydropyridine-3-carboxylic acid methyl ester, 4-hydroxy-1-(2-methyl-benzo-1,4-dioxan-2-ylmethyl)-1,2,5,6-tetrahydropyridine-3-carboxylic acid methyl ester or 1-(2-methyl-benzo-1,4-dioxan-2-ylmethyl)-4-oxopiperidine-3-carboxylic acid methyl ester, 1[2-(benzo-1,4-dioxan-2-yl)-ethyl]-4-hydroxy-1,2,5,6-tetrahydropyridine-3-carboxylic acid methyl ester or 1-[2-(benzo-1,4-dioxan-2-yl)-ethyl]-4-ox opiperidine-3-carboxylic acid methyl ester, cis-1-[2-(benzo-1,4-dioxan-2-yl)-ethyl]-4-hydroxypiperidine-3-carboxylic acid methyl ester, trans-1-[2-(benzo-1,4-dioxan-2-yl)-ethyl]-4-hydroxypiperidine-3-carboxylic acid methyl ester or 1-[2-(benzo-1,4-dioxan-2-yl)-ethyl]-1,2,5,6-tetrahydropyridine-3-carboxylic acid methyl ester or, respectively, a salt thereof.

**9.** A process according to claim 1 for the manufacture of 1-[2-(chroman-3-yl)-ethyl]-piperidine-3-carboxylic acid ethyl ester, 1-[2-(chroman-3-yl)-ethyl]-piperidine-4-carboxylic acid ethyl ester, 1-[2-(benzo-1,4-dioxan-2-yl)-ethyl]-piperidine-3-carboxylic acid ethyl ester, 1-[2- (benzo-1,4-dioxan-2-yl)-ethyl]-piperidine-4-carboxylic acid ethyl ester, 1-[2-(chroman-2-yl)-ethyl]-piperidine-3-carboxylic acid ethyl ester, 1-[2-(chroman-2-yl)-ethyl]-piperidine-4-carboxylic acid ethyl ester, cis-1-[2-(benzo-1,4-dioxan-2-yl)-ethyl]-4-benzyloxy-piperidine-3-carboxylic acid methyl ester, trans-1-[2-(benzo-1,4-dioxan-2-yl)-ethyl]-4-benzyloxy-piperidine-3-carboxylic acid methyl ester, cis-4-amino-1-[2(benzo-1,4-dioxan-2-yl)-ethyl]-piperidine-3-carboxylic acid amide, trans-4-amino-1-[2-(benzo-1,4-dioxan-2-yl)-ethyl]-piperidine-3-carboxylic acid amide, 1-[2-(chroman-4-yl)-ethyl]-1,2,5,6-tetrahydropyridine-3-carboxylic acid methyl ester or 1-[2-(chroman-4-yl)-ethyl]-piperidine-3-carboxylic acid ethyl ester or, respectively, a salt thereof.

**10.** A process for the manufacture of a compound of the formula

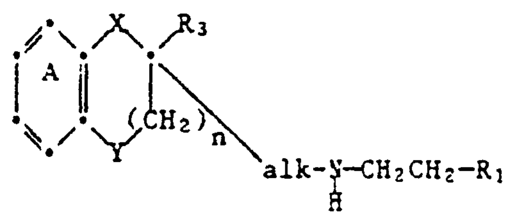

(IVc),

in which $R_1$ represents carboxy, lower alkoxycarbonyl, carbamoyl, N-lower alkylcarbamoyl, N,N-di-lower alkylcarbamoyl, hydroxymethyl, $C_2$-$C_5$alkanoyloxymethyl, lower alkanesulphonyloxymethyl, benzoyloxymethyl or pyridoyloxymethyl, $R_3$ represents hydrogen or $C_1$-$C_7$alkyl, alk represents lower alkylene or lower alkylidene, the ring A is unsubstituted or is mono- or poly-substituted by hydroxy, lower alkoxy, $C_2$-$C_5$alkanoyloxy, halogen having an atomic number of up to and including 53, $C_1$-$C_7$alkyl and/or by trifluoromethyl, and either each of X and Y represents an oxygen atom and n represents 1, or X represents a methylene group, Y represents an oxygen atom and n represents 1, or X represents an oxygen atom, Y represents a methylene group and n represents 1, or X represents a direct bond, Y represents an oxygen atom and n represents 2, with the proviso that in compounds of the formula IVc in which the ring A is unsubstituted, each of X and Y represents oxygen, n represents 1 and $R_3$ represents hydrogen, alk is other than methylene if $R_1$ represents carbamoyl or N-methyl-, N-ethyl-, N,N-dimethyl- or N,N-diethyl-carbamoyl, radicals designated "lower" containing from 1 up to and including 4 carbon atoms, or a salt thereof, characterised in that

h) compounds of the formulae

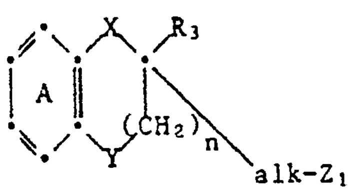

and $Z_2$-$CH_2$-$CH(Z_3)$-$R_1$

(VIIIa) (VIIIb),

in which one of the radicals $Z_1$ and $Z_2$ represents reactive esterified hydroxy and the other represents amino and $z_3$ represents hydrogen, or $z_1$ represents amino and $z_2$ and $z_3$ together represent an additional bond, or optionally salts of these compounds, are reacted with one another, or

i) in a compound of the formula

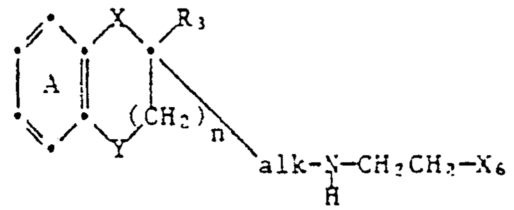

(IX),

in which $X_6$ represents a radical that can be converted into $R_1$, or in a salt thereof, $X_6$ is converted into $R_1$, and, if desired, in the case of each of processes h) and i), a compound of the formula IVc obtainable in accordance with the process or by other means is converted into a different compound of the formula IVc, an isomeric mixture obtainable in accordance with the process is separated into the components, an enantiomeric or diastereoisomeric mixture obtainable in accordance with the process is separated into the enantiomers or diastereoisomers, respectively, and/or a free compound of the formula IVc obtainable in accordance with the process is converted into a salt or a salt obtainable in accordance with the process is converted into the free compound of the formula IVc or into a different salt.

**11.** A process according to claim 10 for the manufacture of a compound of the formula IVc in which $R_1$ represents carboxy, lower alkoxycarbonyl, carbamoyl, N-lower alkylcarbamoyl or N,N-di-lower alkylcarbamoyl, $R_3$ represents hydrogen or $C_1$-$C_7$alkyl, alk represents lower alkylene or lower alkylidene, the ring A is unsubstituted or is mono- or poly-substituted by hydroxy, lower alkoxy, $C_2$-$C_5$alkanoyloxy, halogen having an atomic number of up to and including 53, $C_1$-$C_7$alkyl and/or by trifluoromethyl, X represents an oxygen atom or a methylene group, Y represents an oxygen atom and n represents 1, with the proviso that in compounds of the formula IVc in which the ring A is unsubstituted, each of X and Y represents oxygen, n represents 1 and $R_3$ represents hydrogen, alk is other than methylene if $R_1$ represents carbamoyl or N-methyl-, N-ethyl-, N,N-dimethyl- or N,N-diethylcarbamoyl, radicals designated "lower" containing from 1 up to and including 4 carbon atoms, or a salt thereof.

**12.** A process according to claim 10 for the manufacture of a compound of the formula IVc in which $R_1$ represents carboxy, lower alkoxycarbonyl, carbamoyl, N-lower alkylcarbamoyl, N,N-di-lower alkylcarbamoyl, hydroxymethyl, $C_2$-$C_5$alkanoyloxymethyl, lower alkanesulphonyloxymethyl, benzoyloxymethyl or pyridoyloxymethyl, $R_3$ represents hydrogen or $C_1$-$C_7$alkyl, alk represents lower alkylene that links the ring system with the NH group shown in formula IVc by up to and including 3 carbon atoms, or alk represents lower alkylidene, the ring A is unsubstituted or is mono-, di- or poly-substituted by hydroxy, lower alkoxy, $C_2$-$C_5$alkanoyloxy, halogen having an atomic number of up to and including 53, $C_1$-$C_7$alkyl and/or by trifluoromethyl, and either each of X and Y represents an oxygen atom and n represents 1, or X represents a methylene group, Y represents an oxygen atom and n represents 1, or X represents an oxygen atom, Y represents a methylene group and n represents 1, or X represents a direct bond, Y represents an oxygen atom and n represents 2, with the proviso that in compounds of the formula IVc in which the ring A is unsubstituted, each of X and Y represents oxygen, n represents 1 and $R_3$ represents hydrogen, alk is other than methylene if $R_1$ represents carbamoyl or N-methyl-, N-ethyl-, N,N-dimethyl- or N,N-diethyl-carbamoyl, radicals designated "lower" containing from 1 up to and including 4 carbon atoms, or a salt thereof.

**13.** A process according to claim 10 for the manufacture of a compound of the formula IVc in which $R_1$ represents carboxy, lower alkoxycarbonyl, carbamoyl, N-lower alkylcarbamoyl or N,N-di-lower alkylcarbamoyl, $R_3$ represents hydrogen or $C_1$-$C_7$alkyl, alk represents lower alkylene that links the ring system with the NH group shown in formula IVc by up to and including 3 carbon atoms, or alk represents lower alkylidene, the ring A is unsubstituted or is mono-, di- or poly-substituted by hydroxy, lower alkoxy, $C_2$-$C_5$alkanoyloxy, halogen having an atomic number of up to and including 53, $C_1$-$C_7$alkyl and/or by trifluoromethyl, X represents an oxygen atom or a methylene group, Y represents an oxygen atom and n represents 1, with the proviso that in compounds of the formula IVc in which the ring A is unsubstituted, each of X and Y represents oxygen, n represents 1 and $R_3$ represents hydrogen, alk is other than methylene if $R_1$ represents carbamoyl or N-methyl-, N-ethyl-, N,N-dimethyl- or N,N-diethylcarbamoyl, radicals designated "lower" containing from 1 up to and including 4 carbon atoms, or a salt thereof.

**14.** A process according to claim 10 for the manufacture of a compound of the formula IVc in which $R_1$ represents carboxy, hydroxymethyl, C-$C_5$alkanoyloxymethyl, $C_1$-$C_4$-alkoxycarbonyl or carbamoyl, $R_3$ represents hydrogen or $C_1$-$C_4$alkyl, alk represents $C_1$-$C_4$alkylene that links the ring system with the NH group shown in formula IVc by up to and including 3 carbon atoms, the ring A is unsubstituted or is substituted, especially in the 7-position, by $C_1$-$C_4$alkoxy, and either each of X and Y represents an oxygen atom and n represents 1, or X represents a methylene group, Y represents an oxygen atom and n represents 1, or X represents an oxygen atom, Y represents a methylene group and n represents 1, or X represents a direct bond, Y represents an oxygen atom and n represents 2, with the proviso that in compounds of the formula IVc in which the ring A is unsubstituted, each of X and Y represents oxygen, n represents 1 and $R_3$ represents hydrogen, alk is other than methylene if $R_1$ represents carbamoyl, or a salt thereof.

**15.** A process according to claim 10 for the manufacture of a compound of the formula IVc in which $R_1$ represents $C_1$-$C_4$alkoxycarbonyl, $R_3$ represents hydrogen or $C_1$-$C_4$-alkyl, alk represents $C_1$-$C_4$alkylene that links the ring system with the NH group shown in formula IVc by up to and including 3 carbon atoms, the ring A is unsubstituted, X represents an oxygen atom or a methylene group, Y represents an oxygen atom and n represents 1, or a salt thereof.

**16.** A process according to claim 10 for the manufacture of a compound of the formula IVc in which $R_1$ represents $C_1$-$C_4$alkoxycarbonyl, $R_3$ represents hydrogen, alk represents methylene or ethylene, the ring A is unsubstituted, X represents an oxygen atom or a methylene group, Y represents an oxygen atom and n represents 1, or a salt thereof.

**17.** A process for the manufacture of a compound of the formula

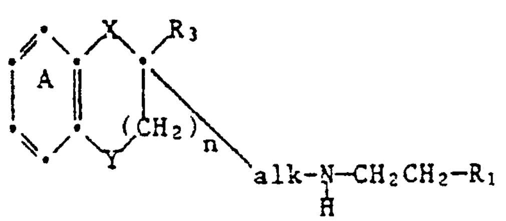

(IVc),

in which $R_1$ represents carboxy, lower alkoxycarbonyl, carbamoyl, N-lower alkylcarbamoyl or N,N-di-lower alkylcarbamoyl, $R_3$ represents hydrogen or $C_1$-$C_7$alkyl, alk represents lower alkylene or lower alkylidene, the ring A is unsubstituted or is mono- or poly-substituted by hydroxy, lower alkoxy, $C_2$-$C_5$alkanoyloxy, halogen having an atomic number of up to and including 53, $C_1$-$C_7$alkyl and/or by trifluoromethyl, X represents an oxygen atom or a methylene group, Y represents an oxygen atom and n represents 1, with the proviso that in compounds of the formula IVc in which the ring A is unsubstituted, each of X and Y represents oxygen, n represents 1 and $R_3$ represents hydrogen, alk is other than methylene if $R_1$ represents carbamoyl or N-methyl-, N-ethyl-, N,N-dimethyl- or N,N-diethyl-carbamoyl, radicals designated "lower" containing from 1 up to and including 4 carbon atoms, or a salt thereof, characterised in that

h) compounds of the formulae

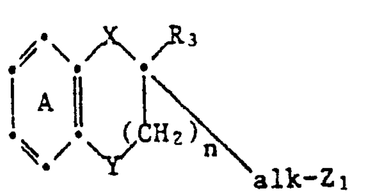

and $Z_2$-$CH_2$-$CH(Z_3)$-$R_1$

(VIIIa) (VIIIb),

in which one of the radicals $Z_1$ and $Z_2$ represents reactive esterified hydroxy and the other

represents amino and $Z_3$ represents hydrogen, or $Z_1$ represents amino and $Z_2$ and $Z_3$ together represent an additional bond, or optionally salts of these compounds, are reacted with one another, and, if desired, a compound of the formula IVc obtainable in accordance with the process or by other means is converted into a different compound of the formula IVc, an isomeric mixture obtainable in accordance with the process is separated into the components, an enantiomeric or diastereoisomeric mixture obtainable in accordance with the process is separated into the enantiomers or diastereoisomers, respectively, and/or a free compound of the formula IVc obtainable in accordance with the process is converted into a salt or a salt obtainable in accordance with the process is converted into the free compound of the formula IVc or into a different salt.

**18.** A process according to claim 17 for the manufacture of a compound of the formula IVc in which $R_1$ represents carboxy, lower alkoxycarbonyl, carbamoyl, N-lower alkylcarbamoyl or N,N-di-lower alkylcarbamoyl, $R_3$ represents hydrogen or $C_1$-$C_7$alkyl, alk represents lower alkylene that links the ring system with the NH group shown in formula IVc by up to and including 3 carbon atoms, or alk represents lower alkylidene, the ring A is unsubstituted or is mono-, di- or poly-substituted by hydroxy, lower alkoxy, $C_2$-$C_5$alkanoyloxy, halogen having an atomic number of up to and including 53, $C_1$-$C_7$alkyl and/or by trifluoromethyl, X represents an oxygen atom or a methylene group, Y represents an oxygen atom and n represents 1, with the proviso that in compounds of the formula IVc in which the ring A is unsubstituted, each of X and Y represents oxygen, n represents 1 and $R_3$ represents hydrogen, alk is other than methylene if $R_1$ represents carbamoyl or N-methyl-, N-ethyl-, N,N-dimethyl- or N,N-diethylcarbamoyl, radicals designated "lower" containing from 1 up to and including 4 carbon atoms, or a salt thereof.

**19.** A process according to claim 17 for the manufacture of a compound of the formula IVc in which $R_1$ represents $C_1$-$C_4$alkoxycarbonyl, $R_3$ represents hydrogen or $C_1$-$C_4$-alkyl, alk represents $C_1$-$C_4$alkylene that links the ring system with the NH group shown in formula IVc by up to and including 3 carbon atoms, the ring A is unsubstituted, X represents an oxygen atom or a methylene group, Y represents an oxygen atom and n represents 1, or a salt thereof.

**20.** A process according to claim 17 for the manufacture of a compound of the formula IVc in which $R_1$ represents $C_1$-$C_4$alkoxycarbonyl, $R_3$ represents hydrogen, alk represents methylene or ethylene, the ring A is unsubstituted, X represents an oxygen atom or a methylene group, Y represents an oxygen atom and n represents 1, or a salt thereof.

**21.** A process according to either claim 10 or claim 17 for the manufacture of N-[2-(chroman-3-yl)-ethyl]-N-(2-methoxycarbonylethyl)-amine or a salt thereof.

**22.** A process according to either claim 10 or claim 17 for the manufacture of N-(2-methoxycarbonylethyl)-N-(benzo-1,4-dioxan-2-ylmethyl)-amine or a salt thereof.

**23.** A process according to either claim 10 or claim 17 for the manufacture of N-(2-methoxycarbonylethyl)-N-(chroman-3-ylmethyl)-amine, N-[2-(benzo-1,4-dioxan-2-yl)-ethyl]-N-(2-methoxycarbonylethyl)-amine or N-(2-methoxycarbonylethyl)-N-(2-methylbenzo-1,4-dioxan-2-ylmethyl)-amine or, respectively, a salt thereof.

**24.** A process according to claim 10 for the manufacture of N-[2-(chroman-3-yl)-ethyl]-N-(2-carboxyethyl)-amine, N-[2-(chroman-2-yl)-ethyl]-N-(2-methoxycarbonylethyl)-amine or N-[2-(chroman-4-yl)-ethyl]-N-(2-methoxycarbonylethyl)-amine or, respectively, a salt thereof.

**25.** A process for the manufacture of a pharmaceutical preparation, characterised in that
a) a compound of the formula

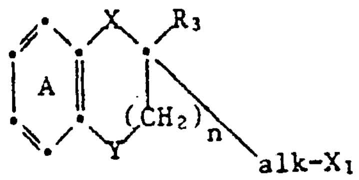

(IIa)

or a salt thereof, in which $X_1$ represents hydroxy or reactive esterified hydroxy, is reacted with a compound of the formula

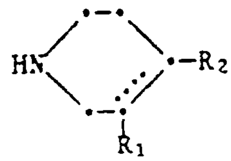

(IIb)

or a tautomer and/or salt thereof, or

b) in a compound of the formula

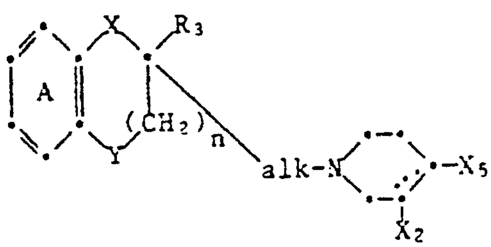

(III)

or a tautomer and/or salt thereof, in which $X_2$ represents a radical that can be converted into $R_1$ that is other than hydrogen, and $X_5$ represents a radical $R_a$, and $R_a$ represents hydrogen, hydroxy, lower alkoxy, benzyloxy, $C_2$-$C_5$alkanoyloxy, lower alkanesulphonyloxy, benzoyloxy, pyridoyloxy, amino, $C_2$-$C_5$alkanoylamino, lower alkanesulphonylamino, benzoylamino or pyridoylamino, $X_2$ is converted into $R_1$ that is other than hydrogen, or in a compound of the formula III in which $X_2$ represents hydrogen and $X_5$ represents a radical that can be converted into $R_b$, and $R_b$ represents a radical $R_2$ other than a radical $R_a$, $X_5$ is converted into $R_b$, or

c) for the manufacture of a compound of the formula I or a tautomer and/or salt thereof, in which $R_2$ represents hydroxy or amino and $R_1$ is other than hydrogen and represents especially lower alkoxycarbonyl, a compound of the formula

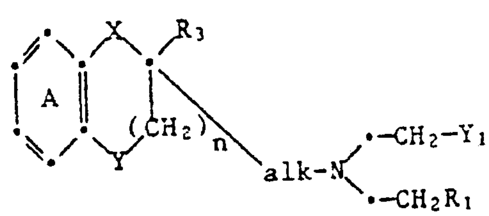

(IV),

in which $Y_1$ represents a group of the formula $-CH=R_2'$, $-C(Y_2)=R_2'$, $-CH(Y_2)-R_2$ or cyano, wherein $R_2'$ represents oxo or imino and $Y_2$ represents a removable radical, or a salt thereof, is cyclised, or

d) for the manufacture of a compound of the formula I' or a tautomer and/or salt thereof, in which $R_2'$ represents oxo or imino and $R_1$ is other than hydrogen and represents especially lower alkoxycarbonyl, a compound of the formula

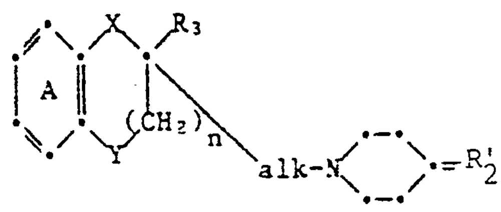

(Va)

or a tautomer, or in each case a salt thereof, is reacted with a compound of the formula

$X_3$-$R_1$ (Vb)

or a salt thereof, in which $R_1$ is other than hydrogen and $X_3$ represents halogen or lower alkoxy, or
e) for the manufacture of a compound of the formula I or a tautomer and/or salt thereof, in which $R_2$ represents hydroxy, lower alkoxy, benzyloxy, $C_2$-$C_5$alkanoyloxy, lower alkanesulphonyloxy, benzoyloxy, pyridoyloxy, amino, $C_2$-$C_5$alkanoylamino, lower alkanesulphonylamino, benzoylamino or pyridoylamino, in a compound of the formula

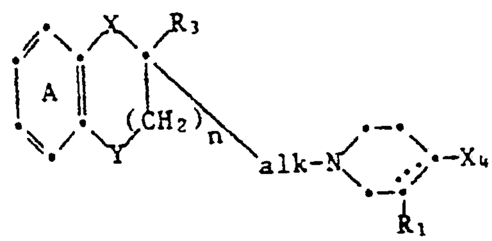

(VI)

or in a salt thereof, in which $X_4$ represents a radical that can be converted into $R_2$, $X_4$ is converted into $R_2$, or
f) especially for the manufacture of a compound of the formula I or a tautomer and/or salt thereof, in which $R_2$ is hydrogen, hydroxy, amino, carboxy, lower alkoxycarbonyl, N-lower alkylcarbamoyl, N,N-di-lower alkylcarbamoyl or hydroxymethyl, in a compound of the formula

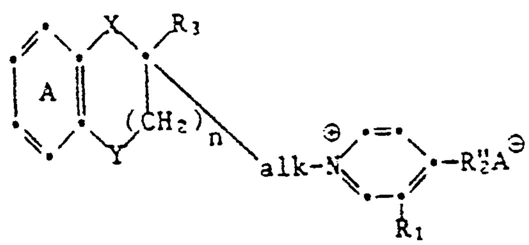

(VII),

in which $A^{\ominus}$ represents the anion of an acid and $R_2''$ represents hydrogen, lower alkoxy, benzyloxy, $C_2$-$C_5$-alkanoyloxy, lower alkanesulphonyloxy, benzoyloxy, pyridoyloxy, protected hydroxy, $C_2$-$C_5$-alkanoylamino, lower alkanesulphonylamino, benzoylamino, pyridoylamino, protected amino, carboxy, lower alkoxycarbonyl, N-lower alkylcarbamoyl, N,N-di-lower alkylcarbamoyl, $C_2$-$C_5$-alkanoyloxymethyl, lower alkanesulphonyloxymethyl, benzoyloxymethyl, pyridoyloxymethyl or etherified or protected hydroxymethyl, the excess double bonds are reduced to single bonds, or
g) for the manufacture of a compound of the formula I or a tautomer and/or salt thereof, in which $R_2$ represents carboxy, N-lower alkylcarbamoyl, N,N-di-lower alkylcarbamoyl or especially lower alkoxycarbonyl, a compound of the formula

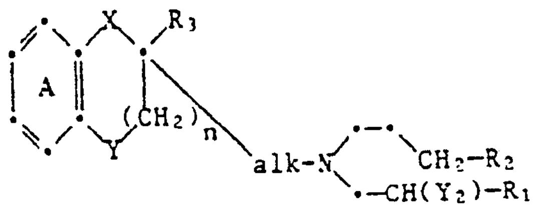

(VIII),

in which $Y_2$ represents a removable radical, or a salt thereof, is cyclised, and in the case of each of processes a) to g), a protecting group which may be present is removed, and, if desired, a compound of the formula I obtainable in accordance with the process or by other means is converted into a different compound of the formula I, an isomeric mixture obtainable in accordance with the process is separated into the components, an enantiomeric or diastereoisomeric mixture obtainable in accordance with the process is separated into the enantiomers or diastereoisomers, respectively, and/or a free compound of the formula I obtainable in accordance with the process is converted into a salt or a salt obtainable in accordance with the process is converted into the free compound of the formula I or into a different salt, and a resulting compound of the formula

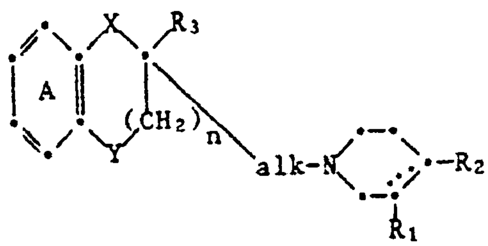

(I),

in which either $R_1$ represents carboxy, lower alkoxycarbonyl, carbamoyl, N-lower alkylcarbamoyl, N,N-di-lower alkylcarbamoyl, hydroxymethyl, $C_2$-$C_5$alkanoyloxymethyl, lower alkanesulphonyloxymethyl, benzoyloxymethyl or pyridoyloxymethyl and $R_2$ represents hydrogen, hydroxy, lower alkoxy, benzyloxy, $C_2$-$C_5$alkanoyloxy, lower alkanesulphonyloxy, benzoyloxy, pyridoyloxy, amino, $C_2$-$C_5$-alkanoylamino, lower alkanesulphonylamino, benzoylamino or pyridoylamino, or $R_1$ represents hydrogen and $R_2$ represents carboxy, lower alkoxycarbonyl, N-lower alkylcarbamoyl, N,N-di-lower alkylcarbamoyl, hydroxymethyl, $C_2$-$C_5$alkanoyloxymethyl, lower alkanesulphonyloxymethyl, benzoyloxymethyl or pyridoyloxymethyl, $R_3$ represents hydrogen or $C_1$-$C_7$alkyl, alk represents lower alkylene that bridges the two ring systems by up to and including 3 carbon atoms, or alk represents lower alkylidene, the ring A is unsubstituted or is mono-, di-or poly-substituted by hydroxy, lower alkoxy, $C_2$-$C_5$-alkanoyloxy, halogen having an atomic number of up to and including 53, $C_1$-$C_7$alkyl and/or by trifluoromethyl, the dotted line is intended to indicate the presence of a single or a double bond between the carbon atoms carrying the radicals $R_1$ and $R_2$, and either each of X and Y represents an oxygen atom and n represents 1, or X represents a methylene group, Y represents an oxygen atom and n represents 1, or X represents an oxygen atom, Y represents a methylene group and n represents 1, or X represents a direct bond, Y represents an oxygen atom and n represents 2, or a resulting tautomer and/or a resulting pharmaceutically acceptable salt thereof, is mixed with customary pharmaceutical adjuncts, radicals designated "lower" containing from 1 up to and including 4 carbon atoms.

**26.** A process for the manufacture of a pharmaceutical preparation, characterised in that

h) compounds of the formulae

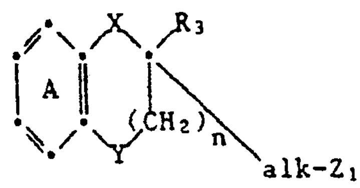

and $Z_2$-$CH_2$-$CH(Z_3)$-$R_1$

(VIIIa) (VIIIb),

in which one of the radicals $Z_1$ and $Z_2$ represents reactive esterified hydroxy and the other represents amino and $Z_3$ represents hydrogen, or $Z_1$ represents amino and $Z_2$ and $Z_3$ together represent an additional bond, or optionally salts of these compounds, are reacted with one another, or

i) in a compound of the formula

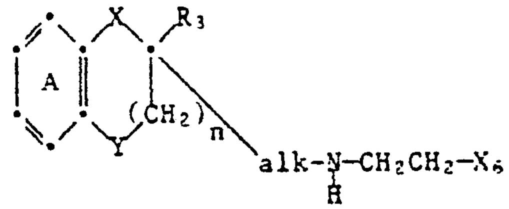

(IX),

in which $X_6$ represents a radical that can be converted into $R_1$, or in a salt thereof, $X_6$ is converted into $R_1$, and, if desired, in the case of each of processes h) and i), a compound of the formula IVc obtainable in accordance with the process or by other means is converted into a different compound of the formula IVc, an isomeric mixture obtainable in accordance with the process is separated into the components, an enantiomeric or diastereoisomeric mixture obtainable in accordance with the process is separated into the enantiomers or diastereoisomers, respectively, and/or a free compound of the formula IVc obtainable in accordance with the process is converted into a salt or a salt obtainable in accordance with the process is converted into the free compound of the formula IVc or into a different salt, and a resulting compound of the formula

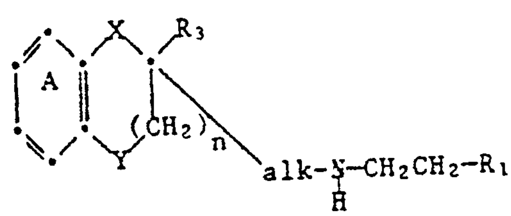

(IVc),

in which $R_1$ represents carboxy, lower alkoxycarbonyl, carbamoyl, N-lower alkylcarbamoyl, N,N-di-lower alkylcarbamoyl, hydroxymethyl, $C_2$-$C_5$alkanoyloxymethyl, lower alkanesulphonyloxymethyl, benzoyloxymethyl or pyridoyloxymethyl, $R_3$ represents hydrogen or $C_1$-$C_7$alkyl, alk represents lower alkylene or lower alkylidene, the ring A is unsubstituted or is mono- or poly-substituted by hydroxy, lower alkoxy, $C_2$-$C_5$alkanoyloxy, halogen having an atomic number of up to and including 53, $C_1$-$C_7$alkyl and/or by trifluoromethyl, and either each of X and Y represents an oxygen atom and n represents 1, or X represents a methylene group, Y represents an oxygen atom and n represents 1, or X represents an oxygen atom, Y represents a methylene group and n represents 1, or X represents a direct bond, Y represents an oxygen atom and n represents 2, or a resulting pharmaceutically acceptable salt thereof, is mixed with customary pharmaceutical adjuncts, radicals designated "lower" containing from 1 up to and including 4 carbon atoms.

**27.** A process for the manufacture of a pharmaceutical preparation, characterised in that

h) compounds of the formulae

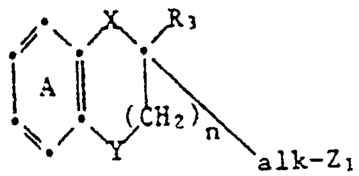 and $Z_2-CH_2-CH(Z_3)-R_1$

(VIIIa) (VIIIb),

in which one of the radicals $Z_1$ and $Z_2$ represents reactive esterified hydroxy and the other represents amino and $Z_3$ represents hydrogen, or $Z_1$ represents amino and $Z_2$ and $Z_3$ together represent an additional bond, or optionally salts of these compounds, are reacted with one another, and, if desired, a compound of the formula IVc obtainable in accordance with the process or by other means is converted into a different compound of the formula IVc, an isomeric mixture obtainable in accordance with the process is separated into the components, an enantiomeric or diastereoisomeric mixture obtainable in accordance with the process is separated into the enantiomers or diastereoisomers, respectively, and/or a free compound of the formula IVc obtainable in accordance with the process is converted into a salt or a salt obtainable in accordance with the process is converted into the free compound of the formula IVc or into a different salt, and a resulting compound of the formula

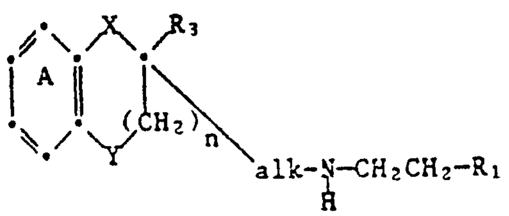 (IVc),

in which $R_1$ represents carboxy, lower alkoxycarbonyl, carbamoyl, N-lower alkylcarbamoyl or N,N-di-lower alkylcarbamoyl, $R_3$ represents hydrogen or $C_1$-$C_7$alkyl, alk represents lower alkylene or lower alkylidene, the ring A is unsubstituted or is mono- or poly-substituted by hydroxy, lower alkoxy, $C_2$-$C_5$alkanoyloxy, halogen having an atomic number of up to and including 53, $C_1$-$C_7$alkyl and/or by trifluoromethyl, X represents an oxygen atom or a methylene group, Y represents an oxygen atom and n represents 1, or a resulting pharmaceutically acceptable salt thereof, is mixed with customary pharmaceutical adjuncts, radicals designated "lower" containing from 1 up to and including 4 carbon atoms.

**28.** A process for the manufacture of a pharmaceutical preparation, characterised in that a compound obtainable in accordance with any one of claims 1 to 24, or if appropriate a tautomer and/or a pharmaceutically acceptable salt thereof, is mixed with customary pharmaceutical adjuncts.

**29.** A process for the manufacture of a pharmaceutical preparation, characterised in that a compound obtainable in accordance with any one of claims 2, 4, 6, 8 and 17 to 23, or if appropriate a tautomer and/or a pharmaceutically acceptable salt thereof, is mixed with customary pharmaceutical adjuncts.

**30.** A process for the manufacture of a pharmaceutical preparation, characterised in that a compound of the formula IVc in which $R_1$ represents carbamoyl or N-methyl-, N-ethyl-, N,N-dimethyl- or N,N-diethylcarbamoyl, $R_3$ represents hydrogen, alk represents methylene, the ring A is unsubstituted, each of X and Y represents an oxygen atom and n represents 1, or a pharmaceutically acceptable salt thereof, is mixed with customary pharmaceutical adjuncts.

**31.** The use of a compound obtainable in accordance with any one of claims 1 to 24, or if appropriate a tautomer and/or a pharmaceutically acceptable salt thereof, for the manufacture of a pharmaceutical preparation, for example a nootropic.

**Revendications**

**Revendications pour les Etats contractants suivants : BE, CH, DE, FR, GB, IT, LI, LU, NL, SE**

1. Composé de formule

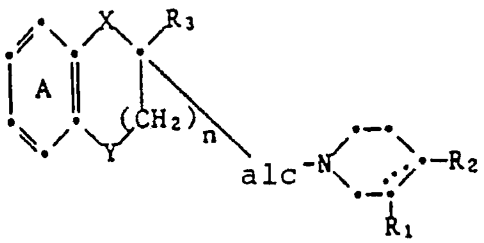

(I),

où ou bien $R_1$ représente un carboxy, alcoxy-inférieur-carbonyle, carbamyle, N-alcoyle inférieur carbamyle, N,N-dialcoyle inférieur carbamyle, hydroxyméthyle, alcanoyloxy en $C_{2-5}$-méthyle, alcane inférieur sulfonyloxyméthyle, benzoyloxyméthyle ou pyridoyloxyméthyle et $R_2$ représente un hydrogène, hydroxy, alcoxy inférieur, benzyloxy, alcanoyloxy en $C_{2-5}$, alcane inférieur sulfonyloxy, benzoyloxy, pyridoyloxy, amino, alcanoyle en $C_{2-5}$-amino, alcane inférieur sulfonylamino, benzoylamino ou pyridoylamino ou $R_1$ représente un hydrogène et $R_2$ représente un carboxy, alcoxy inférieur carbonyle, N-alcoyle inférieur carbamyle, N,N-dialcoyle inférieur carbamyle, hydroxyméthyle, alcanoyloxy en $C_{2-5}$-méthyle, alcane inférieur sulfonyloxyméthyle, benzoyloxyméthyle ou pyridoyloxyméthyle, $R_3$ représente un hydrogène ou un alcoyle en $C_{1-7}$, alc représente un alcoylène inférieur qui relie par un point les deux systèmes cycliques par jusqu'à trois atomes de carbone compris, ou un alcoylidène inférieur, le noyau A est non substitué ou est mono-, di- ou polysubstitué par un hydroxy, alcoxy inférieur, alcanoyloxy en $C_{2-5}$, halogène ayant un numéro atomique allant juqu'à 53 compris, alcoyle en $C_{1-7}$ et/ou trifluorométhyle, la ligne pointillée exprime le fait qu'entre les atomes de carbone que portent les radicaux $R_1$ et $R_2$ se trouve une liaison simple ou une liaison double et ou bien X et Y représentent chacun un atome d'oxygène et n vaut 1, ou bien x représente un groupe méthylène, Y représente un atome d'oxygène et n vaut 1, ou X représente un atome d'oxygène, Y représente un groupe méthylène et n vaut 1 ou X représente une liaison directe, Y représente un atome d'oxygène et n vaut 2, où les radicaux décrits comme "inférieurs" peuvent présenter de 1 à 4 atomes de carbone compris, ou un de ses tautomères et/ou sels.

2. Composé de formule I selon la revendication 1, où $R_1$ représente un carboxy, alcoxy inférieur carbonyle, carbamyle, N-alcoyle inférieur carbamyle, ou N,N-dialcoyle inférieur carbamyle, $R_2$ représente hydrogène, hydroxy, alcoxy inférieur, benzyloxy, alcanoyloxy en $C_{2-5}$, alcane inférieur sulfonyloxy, benzoyloxy, pyridoyloxy, amino, alcanoylamino en $C_{2-5}$, benzoylamino pyridoylamino, $R_3$ représente un hydrogène ou un alcoyle en $C_{1-7}$, alc représente un alcoylène inférieur, qui relie par un pont les deux systèmes cycliques par jusqu'à trois atomes de carbone compris, ou représente un alcoylidène inférieur, le noyau A est non substitué ou mono-, di- ou polysubstitué par un hydroxy, alcoxy inférieur, alcanoyloxy en $C_{2-5}$, halogène ayant un numéro atomique allant jusqu'à 53 compris, alcoyle en $C_{1-7}$ et/ou trifluorométhyle, la ligne pointillée exprime le fait qu'entre les atomes de carbone que portent les radicaux $R_1$ et $R_2$ se trouve une liaison simple ou une liaison double, X représente un atome d'oxygène ou un groupe méthlène, Y représente un atome d'oxygène et n vaut 1, les radicaux décrits comme "inférieurs" pouvant présenter de 1 à 4 atomes de carbone compris ou un de ses tautomères et/ou sels.

3. Composé de formule I, selon la revendication 1, où ou bien $R_1$ représente un alcoxy en $C_{1-4}$-carbonyle, carbamyle, hydroxyméthyle, ou alcanoyle en $C_{2-5}$-oxyméthyle et $R_2$ représente un hydrogène, ou un hydroxy ou bien $R_1$ représente un hydrogène et $R_2$ représente un alcoxy en $C_{1-4}$-carbonyle, $R_3$ représente un hydrogène ou un alcoyle en $C_{1-4}$, alc représente un alcoylène en $C_{1-4}$ qui relie les deux systèmes cycliques par jusqu'à trois atomes de carbone compris, le noyau A est non substitué, ou est substitué, en particulier en position 7, par un alcoxy en $C_{1-4}$, la ligne pointillée exprime le fait qu'entre les atomes de carbone que portent les radicaux $R_1$ et $R_2$ se trouve une liaison simple ou une liaison double, et ou bien X et Y représentent chacun un atome d'oxygène, n vaut 1 ou

bien X représente un groupe méthylène, Y représente un atome d'oxygène et n vaut 1 ou bien X représente un atome d'oxygène, Y représente un groupe méthylène et n vaut 1, ou bien X représente une liaison directe, Y représente un atome d'oxygène et n vaut 2, ou un de ses tautomères ou sels.

**4.** Composé de formule I selon la revendication 1, où $R_1$ représente un alcoxy en $C_{1-4}$-carbonyle, $R_2$ représente un hydrogène ou un hydroxy, $R_3$ représente un hydrogène ou un alcoyle en $C_{1-4}$, alc représente un alcoylène en $C_{1-4}$ qui relie les deux systèmes cycliques par jusqu'à trois atomes de carbone compris, le noyau A est non substitué, la ligne pointillée exprime le fait qu'entre les atomes de carbone que portent les radicaux $R_1$ et $R_2$ se trouve une liaison simple ou une liaison double, X représente un atome d'oxygène ou un groupe méthyle, Y représente un atome d'oxygène et n vaut 1 ou de ses tautomères et/ou sels.

**5.** Composé de formule I selon la revendication 1, où $R_1$ représente un alcoxy en $C_{1-4}$-carbonyle, $R_2$ représente un hydrogène ou un hydroxy, $R_3$ est un hydrogène, alc représente un méthylène ou un éthylène, le noyau A est non substitué, la ligne pointillée exprime le fait qu'entre les atomes de carbone que portent les radicaux $R_1$ et $R_2$ se trouve une liaison simple ou une liaison double, X représente un atome d'oxygène ou un groupe méthylène, Y représente un atome d'oxygène et n vaut 1, ou un de ses tautomères et/ou sels.

**6.** Ester méthylique de l'acide 4-hydroxy-1-(chroman-3-ylméthyl)-1,2,5,6-tétrahydro-pyridine-3-carboxylique ou ester méthylique de l'acide 1-(chroman-3-ylméthyl)-4-oxo-pipéridine-3-carboxylique, ester méthylique de l'acide 1-[2-(chroman-3-yl)éthyl]-1,2,5,6-tétrahydro-pyridine-3-carboxylique, ester méthylique de l'acide 1-(benzo-1,4-dioxan-2-yl-méthyl)-pipéridin-3-carboxylique ou ester méthylique de l'acide 4-hydroxy-1-(benzo-1,4-dioxan-2-ylméthyl)-1,2,5,6-tétrahydro-pyridin-3-carboxylique, ou ester méthylique de l'acide 1-(benzo-1,4-dioxan-2-ylméthyl)-4-oxo-pipéridin-3-carboxylique ou à chaque fois un de leurs sels.

**7.** Ester éthylique de l'acide 1-[2-(chroman-3-yl)éthyl]-1,2,5,6-tétrahydro-pyridin-3-carboxylique ou un de ses sels.

**8.** Ester méthylique de l'acide 1-(chroman-3-ylméthyl)-1,2,5,6-tétrahydro-pyridin-3-carboxylique, ester méthylique de l'acide 1-[2-(chroman-3-yl)éthyl]-4-hydroxy-1,2,5,6-tétrahydro-pyridin-3-carboxylique, ou ester méthylique de l'acide 1-[2-(chroman-3-yl)éthyl]-4-oxo-pipéridin-3-carboxylique, ester méthylique de l'aoide 1-(benzo-1,4-dioxan-2-ylméthyl)-1,2,5,6-tétrahydro-pyridin-3-carboxylique, ester méthylique de l'acide 4-hydroxy-1-(2-méthyl-benzo-1,4-dioxan-2-ylméthyl)-1,2,5,6-tétrahydro-pyridin-3-carboxylique ou ester méthylique de l'acide 1-(2-méthyl-benzo-1,4-dioxan-2-ylméthyl)1-4-oxo-pipéridin-3-carboxylique, ester méthylique de l'acide 1-[2-(benzo-1,4-dioxan-2-yl)éthyl]-4-hydroxy-1,2,5,6-tétrahydropyridin-3-carboxylique, ou ester méthylique de l'acide 1-[2-(benzo-1,4-dioxan-2-yl)éthyl]-4-oxo-pipéridin-3-carboxylique, ester méthylique de l'acide cis-1-[2-(benzo-1,4-dioxan-2-yl)éthyl]-4-hydroxy-pipéridin-3-carboxylique, ester méthylique de l'acide trans-1-[2-(benzo-1,4-dioxan-2-yl)éthyl]-4-hydroxy-pipéridin-3-carboxylique ou ester méthylique de l'acide 1-[2-(benzo-1,4-dioxan-2-yl)éthyl]-1,2,5,6-tétrahydro-pyridin-3-carboxylique ou à chaque fois un de leurs sels.

**9.** Ester éthylique de l'acide 1-[2-(chroman-3-yl)éthyl]-pipéridin-3-carboxylique, ester éthylique de l'acide 1-[2-(chroman-3-yl)éthyl]-pipéridin-4-carboxylique, ester éthylique de l'acide 1-[2-(benzo-1,4-dioxan-2-yl)éthyl]-pipéridin-3-carboxylique, ester éthylique de l'acide 1-[2-(benzo-1,4-dioxan-2-yl)éthyl]-pipéridin-4-carboxylique, ester éthylique de l'acide 1-[2-(chroman-2-yl)éthyl]-pipéridin-3-carboxylique, ester éthylique de l'acide 1-[2-(chroman-2-yl)éthyl]-pipéridin-4-carboxylique, ester méthylique de l'acide cis-1-[2-(benzo-1,4-dioxan-2-yl)éthyl]-4-benzyloxy-pipéridin-3-carboxylique, ester méthylique de l'acide trans-1-[2-(benzo-1,4-dioxan-2-yl)éthyl]-4-benzyloxy-pipéridin-3-carboxylique, amide de l'acide cis-4-amino-1-[2-(benzo-1,4-dioxan-2-yl)éthyl]-pipéridin-3-carboxylique, amide de l'acide trans-4-amino-1-[2-(benzo-1,4-dioxan-2-yl)éthyl]-pipéridin-3-carboxylique, ester méthylique de l'acide 1-[2-(chroman-4-yl)éthyl]-1,2,5,6-tétrahydro-pyridin-3-carboxylique ou ester éthylique de l'acide 1-[2-(chroman-4-yl)éthyl]-pipéridin-3-carboxylique ou à chaque fois un de ses sels.

**10.** Procédé de préparation d'un composé de formule I ou d'un de ses tautomères et/ou sels selon l'une des revendications 1 à 9, caractérisé en ce que

a) on fait réagir un composé de formule

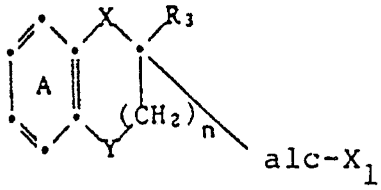

(IIa)

ou un de ses sels, où $X_1$ représente un hydroxy ou un hydroxy estérifié réactif, avec un composé de fomrule

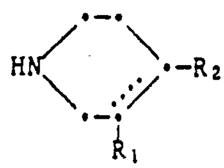

(IIb)

ou un de ses tautomères et/ou sels ou
b) dans un composé de formule

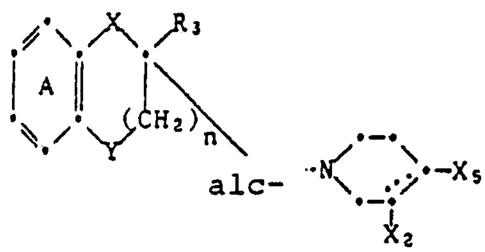

(III)

ou un de ses tautomères et/ou sels, où $X_2$ représente un radical transformable en $R_1$ différent d'un hydrogène et $X_5$ représente un radical $R_a$ où $R_a$ représente un hydrogène, hydroxy, alcoxy inférieur, benzyloxy, alcoyloxy en $C_{2-5}$, alcane inférieur sulfonyloxy, benzoyloxy, pyridoyloxy, amino, alcanoyle en $C_{2-5}$-amino, alcane inférieur sulfonylamino, benzoylamino ou pyridoylamino, on transforme $X_2$ en un radical $R_1$ différent d'un hydrogène ou dans un composé de formule III où $X_2$ est un hydrogène et $X_5$ représente un radical transformable en $R_b$, où $R_b$ représente un radical $R_2$ différent d'un radical $R_a$, on transforme $X_5$ et $R_b$, ou
c) pour préparer un composé de formule I ou un de ses tautomères et/ou sels où $R_2$ représente un hydroxy ou un amino et $R_1$ est différent d'un hydrogène, et représente en particulier un alcoxy inférieur carbonyle, on cyclise un composé de formule

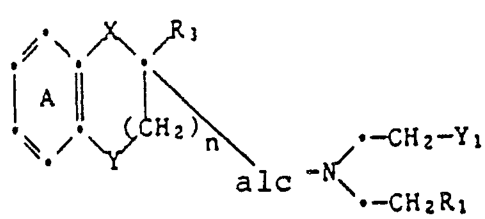

(IV),

où $Y_1$ représente un groupe de formule - $CH=R_2'$, $-C(Y_2)=R_2'$, $-CH(Y_2)-R_2$ ou un cyano où $R_2'$ représente un oxy ou un imino et $Y_2$ représente un radical séparable ou un de ses sels, ou
d) pour préparer un composé de formule I( ou un de ses tautomères et/ou sels où $R_2'$ représente un oxo ou un imino ou $R_1$ est différent d'un hydrogène et représente en particulier un alcoxy inférieur

carbonyle, on fait réagir un composé de formule

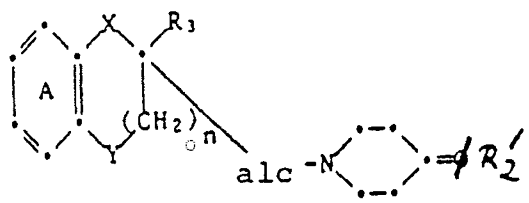

(Va)

ou un de ses tautomères ou un de ses sels avec un composé de formule

$X_3$-$R_1$ (Vb)

où $R_1$ est différent d'un hydrogène et $X_3$ représente un halogène ou un alcoxy inférieur, ou
e) pour préparer un composé de formule I et un de ses tautomères et/ou sels où $R_2$ est un hydroxy, alcoxy inférieur, benzyloxy, alcanoyloxy en $C_{2-5}$, alcane inférieur-sulfonyloxy, benzoyloxy, pyridoyloxy, amino, alcanoylamino en $C_{2-5}$, alcane inférieur-sulfonylamino, benzoylamino ou pyridoylamino, dans un composé de formule

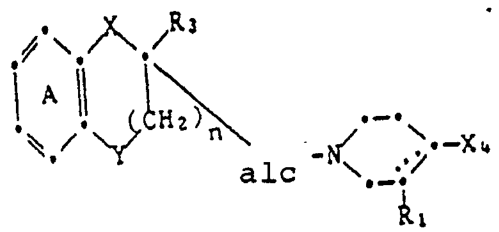

(VI)

ou un de ses sels où $X_4$ représente un radical transformable en $R_2$, on transforme $X_4$ en $R_2$ ou
f) en particulier pour préparer un composé de formule I et un de ses tautomères et/ou sels où $R_2$ est un hydrogène, hydroxy, amino, carboxy, alcoxy inférieur carbonyle, N-alcoyle inférieur-carbamoyle, N,N-dialcoyle inférieur-carbamyle ou hydroxyméthyle, dans un composé de formule

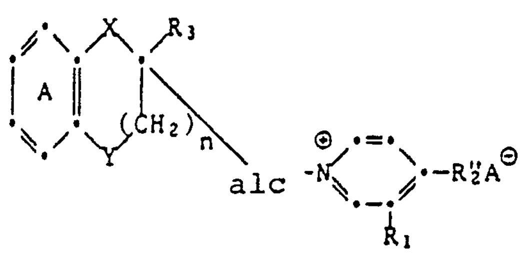

(VII),

ou $A^{\ominus}$ représente l'anion d'un acide et $R_2$" représente un hydrogène, alcoxy inférieur, benzyloxy, alcanoyloxy en $C_{2-5}$, alcane inférieur sulfonyloxy, benzoyloxy, pyridoyloxy, hydroxy protégé, alcanoylamino en $C_{2-5}$, alcane inférieur sulfonylamino, benzoylamino, pyridoylamino, carboxy, alcoxy inférieur carbonyle, N-alcoyle inférieur carbamyle, N,N-dialcoyle inférieur carbamyle, alcanoyloxy en $C_{2-5}$-méthyle, alcane inférieur sulfonyloxyméthyle, benzoyloxyméthyle, pyridoyloxyméthyle ou hydroxyméthyle éthérifié ou protégé, on réduit les doubles liaisons en excès en liaisions simples, ou
g) pour préparer un composé de formule I ou un de ses tautomères et:ou sels où $R_2$ représente un carboxy, N-alcoyle inférieur-carbamyle, N,N-dialcoyle inférieur carbamyle et en particulier un alcoxy inférieur carbonyle, on cyclise un composé de formule

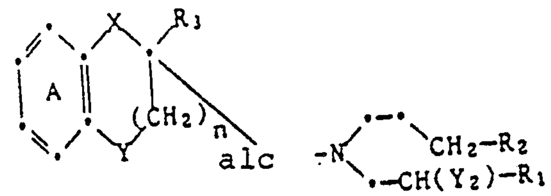

(VIII),

où $Y_2$ représente un radical séparable, ou un de ses sels, et à chaque fois on sépare un groupe protecteur éventuellement présent et à chaque fois on transforme si on le désire, un composé de formule I que l'on peut obtenir selon le procédé ou d'une autre manière en un autre composé de formule I, et chaque fois on dédouble un mélange d'isomères que l'on peut obtenir selon le procédé en ses composants, à chaque fois on sépare un mélange d'énantiomères dans les cas de diastéréomères que l'on peut obtenir selon le procédé en les énantiomères ou selon les cas diastéréomères, et/ou à chaque fois on transforme un composé de formule I libre que l'on peut obtenir selon le procédé en un sel ou un sel que l'on peut obtenir selon le procédé en le composé de formule I libre ou en un autre sel, où les radicaux décrits comme "inférieurs" peuvent présenter de 1 à 4 atomes de carbone compris.

**11.** Composé de formule

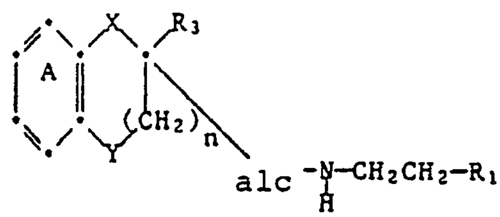

(IVc),

où $R_1$ représente un carboxy, alcoxy inféireur carbonyle, carbamyle, N-alcoyle inférieur carbamyle, N,N-dialcoyle inférieur carbamyle, hydroxyméthyle, alcanoyloxy en $C_{2-5}$-méthyle, alcane inférieur sulfonyloxyméthyle, benzoyloxyméthyle ou pyridoyloxyméthyle, $R_3$ représente un hydrogène ou un alcoyle en $C_{1-4}$, alc représente un alcoylène inférieur ou un alcoylidène inférieur, le noyau A est non substitué ou mono- ou polysubstitué par un hydroxy, alcoxy inférieur, alcanoyloxy en $C_{2-5}$, halogène de numéro atomique allant jusqu'à 53 compris, alcoyle en $C_{1-7}$ et/ou trifluorométhyle et ou bien X et Y représentent chacun un atome d'oxygène et n vaut 1 ou bien X représente un groupe méthylène, Y représente un atome d'oxygène et n vaut 1, ou bien X représente un atome d'oxygène, Y représente un groupe méthylène et n vaut 1, ou bien X représente une liaison directe, Y représente un atome d'oxygène et n vaut 2, avec cette précision que dans les composés de formule IVc où le noyau A est non substitué, X et Y représentent à chaque fois un oxygène, n vaut 1 et $R_3$ représente un atome d'hydrogène, alc est différent d'un méthylène lorsque $R_1$ représente un carbamyle ou un N-méthyl-, N-éthyl-, N,N-diméthyl-ou N,N-diéthylcarbamyle, ou les radicaux décrits comme "inférieurs" peuvent présenter de 1 à 4 atomes de carbone compris, ou un de ses sels.

**12.** Composé de formule IVc selon la revendication 11, où $R_1$ représente un carboxy, alcoxy inférieur carbonyle, carbamyle, N-alcoyle inférieur carbamyle, ou N,N-dialcoyle inférieur carbamyle, $R_3$ représente un hydrogène ou un alcoyle en $C_{1-4}$, alc représente un alcoylène inférieur ou un alcoylidène inférieur, le noyau A est non substitué ou mono-ou polysubstitué par un hydroxy, alcoxy inférieur, alcanoyloxy en $C_{2-5}$, halogène de numéro atomique allant jusqu'à 53 compris, alcoyle en $C_{1-7}$ et/ou trifluorométhyle, X représente un atome d'oxygène ou un groupe méthylène, Y représente un atome d'oxygène et n vaut 1, avec cette précision que dans les composés de formule IVc où le noyau A est non substitué, X et Y représentent à chaque fois un oxygène, n vaut 1 et $R_3$ représente un hydrogène, alc est différent d'un méthylène lorsque $R_1$ représente un carbamyle, ou un N-méthyl-, N-éthyl-, N,N-diméthyl- ou N,N-diéthylcarbamyle, où les radicaux décrits comme "inférieurs" peuvent présenter de 1 à 4 atomes de carbone compris, ou un de ses sels.

13. Composé de formule IVc selon la revendication 11, où $R_1$ représente un carboxy, alcoxy inférieur carbonyle, carbamyle, N-alcoyle inférieur carbamyle, N,N-dialcoyle inférieur-carbamyle, hydroxyméthyle, alcanoyloxy en $C_{2-5}$-méthyle, alcane inférieur sulfonyloxyméthyle, benzoyloxyméthyle ou pyridoyloxyméthyle, $R_3$ représente un hydrogène ou un alcoyle en $C_{1-4}$, alc représente un alcoylène inférieur qui relie le système cyclique avec le groupe NH représenté dans la formule IVc par de 1 à 3 atomes de carbone compris, ou représente un alcoylidène inférieur, le noyau A est non substitué ou est mono-, di- ou polysubstitué par un hydroxy, alcoxy inférieur, alcanoyloxy en $C_{2-5}$, halogène de numéro atomique allant jusqu'à 53 compris, alcoyle en $C_{1-7}$ et/ou trifluorométhyle et ou bien X et Y représentent chacun un atome d'oxygène et n vaut 1, ou bien X représente un groupe méthylène, Y représente un atome d'oxygène et n vaut 1 ou bien X représente un atome d'oxygène, Y représente un groupe méthylène et n vaut 1, ou bien X représente une liaison directe, Y représente un atome d'oxygène et n vaut 2, avec cette précision que dans les composés de formule IVc où le noyau A est non substitué, X et représentent chacun un oxygène, n vaut 1 et $R_3$ représente un hydrogène, alc est différent d'un méthylène lorsque $R_1$ représente un carbamyle ou un N-méthyl-, N-éthyl-, N,N-diméthyl- ou N,N-diéthylcarbamyle, où les radicaux décrits comme "inférieurs" peuvent présenter de 1 à 4 atomes de carbone compris, ou un de ses sels.

14. Composé de formule IVc selon la revendication 11, où $R_1$ représente un carboxy, alcoxy inférieur carbonyle, carbamyle, N-alcoyle inférieur carbamyle, ou N,N-dialcoyle inférieur carbamyle, $R_3$ représente un hydrogène ou un alcoyle en $C_{1-4}$, alc représente un alcoylène inférieur qui relie les systèmes cycliques avec le groupe NH représenté dans la formule IVc par jusqu'à trois atomes de carbone compris, ou représente un alcoylidène inférieur, le noyau A est non substitué ou mono-, di- ou polysubstitué par un hydroxy, alcoxy inférieur, alcanoyloxy en $C_{2-5}$, halogène de numéro atomique allant jusqu'à 53 compris, alcoyle en $C_{1-7}$ et/ou trifluorométhyle, X représente un atome d'oxygène ou un groupe méthylène, Y représente un atome d'oxygène et n vaut 1, avec cette précision que dans les composés de formule IVc où le noyau A est non substitué, X et Y représentent à chaque fois un oxygène, n vaut 1 et $R_3$ représente un hydrogène, alc est différent d'un méthylène lorsque $R_1$ représente un carbamyle ou N-méthyl-, N-éthyl-, N,N-diméthyl- ou N,N-diéthylcarbamyle où les radicaux décrits comme "inférieurs" peuvent présenter de 1 à 4 atomes de carbone compris ou un de ses sels.

15. Composé de formule IVc selon la revendication 11, où $R_1$ représente un carboxy, hydroxyméthyle, alcanoyloxy en $C_{2-5}$-méthyle, alcoxy en $C_{1-4}$-carbonyle ou carbamyle, $R_3$ représente un hydrogène ou un alcoyle en $C_{1-4}$, alc représente un alcoylène en $C_{1-4}$ qui relie le système cyclique avec le groupe NH représenté dans la formule IVc par jusqu'à 3 atomes de carbone compris, le noyau A est non substitué ou, en particulier en position 7, par un alcoxy en $C_{1-4}$ et ou bien X et Y représentent à chaque fois un atome d'oxygène et n vaut 1, ou bien X représente un groupe méthylène, Y représente un atome d'oxygène et n vaut 1, ou bien X représente un atome d'oxygène, Y représente un groupe méthylène et n vaut 1, ou bien X représente une liaison directe, Y représente un atome d'oxygène et n vaut 2, avec cette précision que dans les composés de formule IVc où le noyau A est non substitué, X et Y représentent à chaque fois un oxygène, n vaut 1 et $R_3$ représente un hydrogène, alc est différent d'un oxygène lorsque $R_1$ représente un carbamyle, ou un de ses sels.

16. Composé de formule IVc selon la revendication 11, où $R_1$ représente un alcoxy en $C_{1-4}$-carbonyle, $R_3$ représente un hydrogène ou un alcoyle en $C_{1-4}$, alc représente un alcoylène en $C_{1-4}$ qui relie le système cyclique avec le groupe NH représenté dans la formule IVc par jusqu'à trois atomes de carbone compris, le noyau A est non substitué, X représente un atome d'oxygène ou un groupe méthylène, Y représente un atome d'oxygène et n vaut 1, ou un de ses sels.

17. Composé de formule IVc selon la revendication 11, où $R_1$ représente un alcoxy en $C_{1-4}$-carbonyle, $R_3$ est un hydrogène, alc représente un méthylène ou un éthylène, le noyau A est non substitué, X représente un atome d'oxygène ou un groupe méthylène, Y représente un atome d'oxygène et n vaut 1, ou un de ses sels.

18. N-12-(chroman-3-yl)éthyl]-N-(2-méthoxycarbonyléthyl)-amine ou un de ses sels.

19. N-(2-méthoxycarbonyléthyl)-N-(benzo-1,4-dioxan-2-ylméthyl)-amineou un de ses sels.

**20.** N-(2-méthoxycarbonyléthyl)-N-(chroman-3-ylméthyl)-amine, N-[2-(benzo-1,4-dioxan-2-yl)éthyl]-N-(2-méthoxycarbonyléthyl)-amine ou N-(2-méthoxycarbonyléthyl)-N-(2-méthyl-benzo-1,4-dioxan-2-ylméthyl)-amine ou à chaque fois un de leurs sels.

**21.** N-[2-(chroman-3-yl)éthyl]-N-(2-carboxyéthyl)-amine,
N-[2-(chroman-2-yl)éthyl]-N-(2-méthoxycarbonyléthyl)-amine ou
N-[2-(chroman-4-yl)éthyl]-N-(2-méthoxycarbonyléthyl)-amine ou à chaque fois un de leurs sels.

**22.** Procédé de préparation d'un composé de formule IVc ou d'un de ses sels selon l'une des revendications 11 à 21, caractérisé en ce que

h) on fait réagir les composés de formules

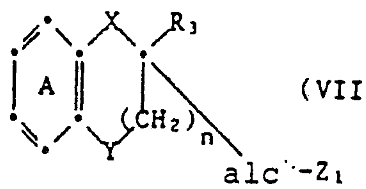 (VIIIa) et $Z_2—CH_2—CH(Z_3)—R_1$ (VIIIb),

où l'un des radicaux $Z_1$ et $Z_2$ représente un hydroxy estérifié réactif, l'autre un amino et $Z_3$ représente un hydrogène ou bien $Z_1$ est un amino et $Z_2$ ainsi que $Z_3$ représentent ensemble une liaison supplémentaire, ou éventuellement des sels de ces composés entre eux, ou

i) dans un composé de formule

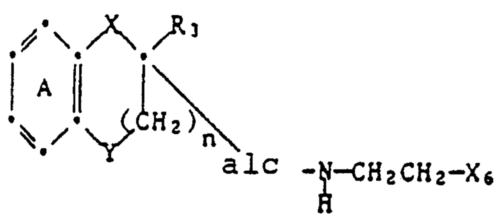 (IX)

où $X_6$ représente un radical transformable en $R_1$ ou un de ses sels, on transforme $X_6$ en $R_1$ et, si on le désire, on transforme à chaque fois un composé de formule IVc que l'on peut obtenir selon le procédé ou d'une autre manière en un autre composé de formule IVc, à chaque fois on dédouble un mélange d'isomères que l'on peut obtenir selon le procédé en ses composants, à chaque fois on sépare un mélange d'énantiomères ou selon les cas de diastéréomères que l'on peut obtenir selon le procédé en les énantiomères ou selon les cas diastéréomères, et/ou à chaque fois on transforme un composé de formule IVc libre que l'on peut obtenir selon le procédé en un sel ou on transforme un sel que l'on peut obtenir selon le procédé en le composé de formule IVc libre ou en un autre sel.

**23.** Composé de formule

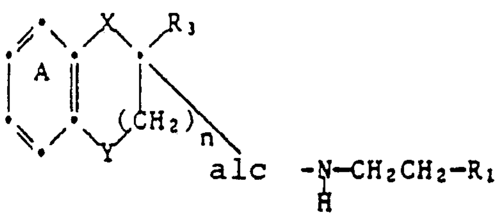 (IVc),

où $R_1$ représente un carboxy, alcoxy inférieur carbonyle, carbamyle, N-alcoyle inférieur carbamyle, N-alcoyle inférieur carbamyle, N,N-dialcoyle inférieur carbamyle, hydroxyméthyle, alcanoyloxy en $C_{2-5}$-méthyle, alcane inférieur sulfonyloxyméthyle, benzoyloxyméthyle ou pyridoyloxyméthyle, $R_3$ représente un hydrogène ou un alcoyle inférieur en $C_{1-7}$, alc représente un alcoylène inférieur ou un alcoylidène inférieur, le noyau A est non substitue ou mono- ou polysubstitué par un hydroxy, alcoxy inférieur, alcanoyloxy en $C_{2-5}$, halogène de numéro atomique allant jusqu'à 53 compris, alcoyle en $C_{1-7}$ et/ou trifluorométhyle, et ou bien X et Y représentent chacun un atome d'oxygène et n vaut 1, ou bien X représente un groupe méthylène, Y représente un atome d'oxygène et n vaut 1, ou bien X représente un atome d'oxygène, Y représente un groupe méthylène et n vaut 1, ou bien X représente une liaison directe, Y représente un atome d'oxygène et n vaut 2, où les radicaux décrits comme "inférieurs" peuvent présenter de 1 à 4 atomes de carbone compris, ou un de ses sels pharmaceutiquement acceptables aux fins d'application dans un procédé de traitement thérapeutique et/ou prophylactique du corps humain ou animal.

**24.** Procédé de formule

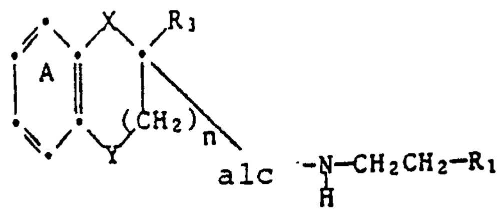

(IVc),

où $R_1$ représente un carboxy, alcoxy inférieur carbonyle, carbamyle, N-alcoyle inférieur carbamyle, ou N,N-diacoyle inférieur carbamyle, $R_3$ représente un hydrogène ou un alcoyle en $C_{1-7}$, alc représente un alcoylène inférieur ou un alcoylidène inférieur, un noyau A est non substitué, mono- ou polysubstitué par un hydroxy, alcoxy inférieur, alcanoyloxy en $C_{2-5}$, halogène de numéro atomique allant jusqu'à 53 compris, alcoyle en $C_{1-7}$ et/ou trifluorométhyle, X représente un atome d'oxygène ou un groupe méthylène, Y représente un atome d'oxygène et n vaut 1, où les radicaux décrits comme "inférieurs" peuvent présenter de 1 à 4 atomes de carbone compris, ou un de ses sels pharmaceutiquement acceptables aux fins d'application dans un procédé de traitement thérapeutique et/ou prophylactique du corps humain ou animal.

**25.** Composé selon l'une des revendications 1 à 9 et 11 à 21 ou éventuellement un de ses tautomères et/ou sels pharmaceutiquement acceptables aux fins d'application dans un procédé de traitement thérapeutique et/ou prophylactique du corps humain ou animal.

**26.** Composé selon l'une des revendications 2, 4, 6, 8, 12, 14, et 16 à 20 ou éventuellement un de ses tautomères et/ou un de ses sels pharmaceutiquement acceptables aux fins d'application dans un procédé de traitement thérapeutique et/ou prophylactique du corps humain ou animal.

**27.** Composé de formule IVc selon la revendication 23, où $R_1$ représente un carbamyle ou un N-méthyl-, N-éthyl-, N,N-diméthyl- ou N,N-diéthylcarbamyle, $R_3$ représente un hydrogène, alc représente un méthylène, le noyau est non substitué, X et Y représentent a chaque fois un atome d'oxygène et n vaut 1, ou un de ses sels pharmaceutiquement acceptables aux fins d'application dans un procédé de traitement thérapeutique et/ou prophylactique du corps humain ou animal.

**28.** Préparation pharmaceutique contenant comme substance active un composé selon l'une des revendications 1 à 9, 11 à 21, 23 et 25 et éventuellement un de ses tautomères et/ou un de ses sels pharmaceutiquement acceptables outre des additifs pharmaceutiques habituels.

**29.** Préparation pharmaceutique contenant comme substance active un composé selon l'une des revendications 2, 4, 6, 8, 12, 14, 16 à 20, 24, 26 et 27 ou éventuellement un de ses tautomères et/ou un de ses sels pharmaceutiquement acceptables outre les supports pharmaceutiques habituels.

**30.** Application d'un composé selon l'une des revendications 1 à 9, 11 à 21, 23, et 25 ou éventuellement

d'un de ses tautomères et/ou sels pharmaceutiquement acceptables à la préparation d'une préparation pharmaceutique, p. ex. d'un agent nootropique.

**Revendications pour les Etats contractants suivants: AT, ES, GR**

**1.** Procédé de préparation d'un composé de formule

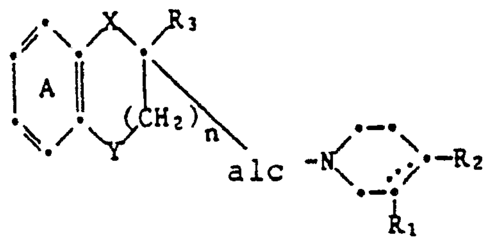

(I),

où ou bien $R_1$ représente un carboxy, alcoxy-inférieur-carbonyle, carbamyle, N-alcoyle inférieur carbamyle, N,N-dialcoyle inférieur carbamyle, hydroxyméthyle, alcanoyloxy en $C_{2-5}$-méthyle, alcane inférieur sulfonyloxyméthyle, benzoyloxyméthyle ou pyridoyloxyméthyle et $R_2$ représente un hydrogène, hydroxy, alcoxy inférieur, benzyloxy, alcanoyloxy en $C_{2-5}$, alcane inférieur sulfonyloxy, benzoyloxy, pyridoyloxy, amino, alcanoyle en $C_{2-5}$-amino, alcane inférieur sulfonylamino, benzoylamino ou pyridoylamino ou $R_1$ représente un hydrogène et $R_2$ représente un carboxy, alcoxy inférieur carbonyle, N-alcoyle inférieur carbamyle, N,N-dialcoyle inférieur carbamyle, hydroxyméthyle, alcanoyloxy en $C_{2-5}$-méthyle, alcane inférieur sulfonyloxyméthyle, benzoyloxyméthyle ou pyridoyloxyméthyle, $R_3$ représente un hydrogène ou un alcoyle en $C_{1-7}$, alc représente un alcoylène inférieur qui relie par un point les deux systèmes cycliques par jusqu'à trois atomes de carbone compris, ou un alcoylidène inférieur, le noyau A est non substitué ou est mono-, di- ou polysubstitué par un hydroxy, alcoxy inférieur, alcanoyloxy en $C_{2-5}$, halogène ayant un numéro atomique allant juqu'à 53 compris, alcoyle en $C_{1-7}$ et/ou trifluorométhyle, la ligne pointillée exprime le fait qu'entre les atomes de carbone que portent les radicaux $R_1$ et $R_2$ se trouve une liaison simple ou une liaison double et ou bien X et Y représentent chacun un atome d'oxygène et n vaut 1, ou bien X représente un groupe méthylène, Y représente un atome d'oxygène et n vaut 1, ou X représente un atome d'oxygène, Y représente un groupe méthylène et n vaut 1 ou X représente une liaison directe, Y représente un atome d'oxygène et n vaut 2, où les radicaux décrits comme "inférieurs" peuvent présenter de 1 à 4 atomes de carbone compris ou un de ses tautomères et/ou sels caractérisé en ce que

a) on fait réagir un composé de formule

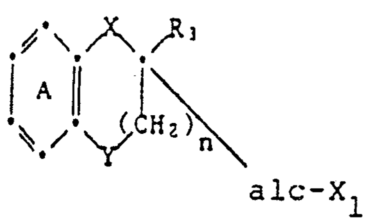

(IIa)

ou un de ses sels, où $X_1$ représente un hydroxy ou un hydroxy estérifié réactif, avec un composé de fomrule

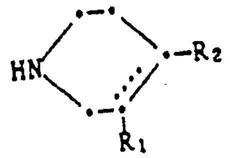

(IIb)

ou un de ses tautomères et/ou sels ou

b) dans un composé de formule

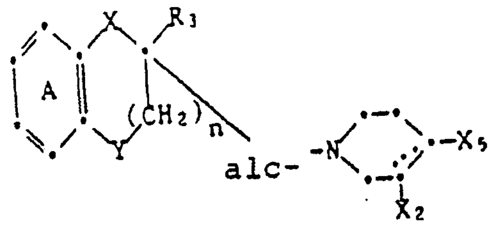

(III)

ou un de ses tautomères et/ou sels, où $X_2$ représente un radical transformable en $R_1$ différent d'un hydrogène et $X_5$ représente un radical $R_a$ où $R_a$ représente un hydrogène, hydroxy, alcoxy inférieur, benzyloxy, alcoyloxy en $C_{2-5}$, alcane inférieur sulfonyloxy, benzoyloxy, pyridoyloxy, amino, alcanoyle en $C_{2-5}$-amino, alcane inférieur sulfonylamino, benzoylamino ou pyridoylamino, on transforme $X_2$ en un radical $R_1$ différent d'un hydrogène ou dans un composé de formule III où $X_2$ est un hydrogène et $X_5$ représente un radical transformable en $R_b$ où $R_b$ représente un radical $R_2$ différent d'un radical $R_a$, on transforme $X_5$ et $R_b$, ou

c) pour préparer un composé de formule I ou un de ses tautomères et/ou sels où $R_2$ représente un hydroxy ou un amino et $R_1$ est différent d'un hydrogène, et représente en particulier un alcoxy inférieur carbonyle, on cyclise un composé de formule

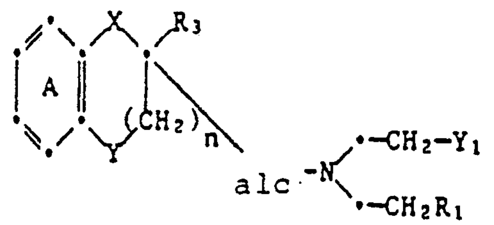

(IV),

où $Y_1$ représente un groupe de formule - $CH=R_2'$, $-C(Y_2)=R_2'$, $-CH(Y_2)-R_2$ ou un cyano où $R_2'$ représente un oxy ou un imino et $Y_2$ représente un radical séparable ou un de ses sels, ou

d) pour préparer un composé de formule I( ou un de ses tautomères et/ou sels où $R_2'$ représente un oxo ou un imino ou $R_1$ est différent d'un hydrogène et représente en particulier un alcoxy inférieur carbonyle, on fait réagir un composé de formule

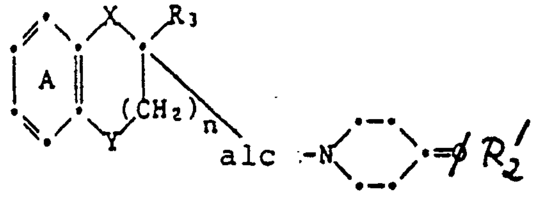

(Va)

ou un de ses tautomères ou un de ses sels avec un composé de formule

$X_3$-$R_1$ (Vb)

où $R_1$ est différent d'un hydrogène et $X_3$ représente un halogène ou un alcoxy inférieur, ou
e) pour préparer un composé de formule I et un de ses tautomères et/ou sels où $R_2$ est un hydroxy, alcoxy inférieur, benzyloxy, alcanoyloxy en $C_{2-5}$, alcane inférieur-sulfonyloxy, benzoyloxy, pyridoyloxy, amino, alcanoylamino en $C_{2-5}$, alcane inférieur-sulfonylamino, benzoylamino ou pyridoylamino, dans un composé de formule

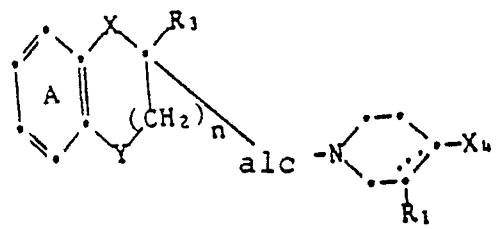

(VI)

ou un de ses sels où $X_4$ représente un radical transformable en $R_2$, on transforme $X_4$ en $R_2$ ou
f) en particulier pour préparer un composé de formule I et un de ses tautomères et/ou sels où $R_2$ est un hydrogène, hydroxy, amino, carboxy, alcoxy inférieur carbonyle, N-alcoyle inférieur-carbamoyle, N,N-dialcoyle inférieur-carbamyle ou hydroxyméthyle, dans un composé de formule

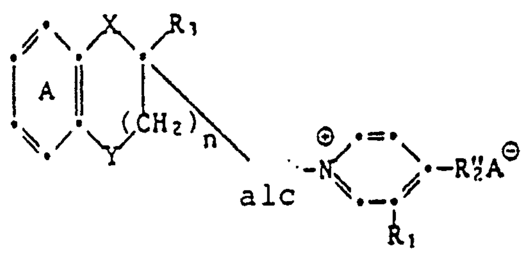

(VII),

où $A^{\ominus}$ représente l'anion d'un acide et $R_2$" représente un hydrogène, alcoxy inférieur, benzyloxy, alcanoyloxy en $C_{2-5}$, alcane inférieur sulfonyloxy, benzoyloxy, pyridoyloxy, hydroxy protégé alcanoylamino en $C_{2-5}$, alcane inférieur sulfonylamino, benzoylamino, pyridoylamino, carboxy, alcoxy inférieur carbonyle, N-alcoyle inférieur carbamyle, N,N-dialcoyle inférieur carbamyle, alcanoyloxy en $C_{2-5}$-méthyle, alcane inférieur sulfonyloxyméthyle, benzoyloxyméthyle, pyridoyloxyméthyle ou hydroxyméthyle éthérifié ou protégé, on réduit les doubles liaisons en excès en liaisons simples, ou
g) pour préparer un composé de formule I ou un de ses tautomères et:ou sels où $R_2$ représente un carboxy, N-alcoyle inférieur-carbamyle, N,N-dialcoyle inférieur carbamyle et en particulier un alcoxy inférieur carbonyle, on cyclise un composé de formule

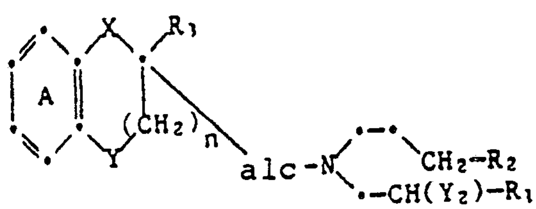

(VIII),

où $Y_2$ représente un radical séparable, ou un de ses sels, et à chaque fois on sépare un groupe protecteur éventuellement présent et à chaque fois on transforme si on le désire, un composé de formule I que l'on peut obtenir selon le procédé ou d'une autre manière en un autre composé de formule I, et chaque fois on dédouble un mélange d'isomères que l'on peut obtenir selon le procédé

en ses composants, à chaque fois on sépare un mélange d'énantiomères dans les cas de diastéréomères que l'on peut obtenir selon le procédé en les énantiomères ou selon les cas diastéréomères, et/ou à chaque fois on transforme un composé de formule I libre que l'on peut obtenir selon le procédé en un sel ou un sel que l'on peut obtenir selon le procédé en le composé de formule I libre ou en un autre sel, où les radicaux décrits comme "inférieurs" peuvent présenter de 1 à 4 atomes de carbone compris.

2. Procédé selon la revendication 1 de préparation d'un composé de formule I où $R_1$ représente un carboxy, alcoxy inférieur carbonyle, carbamyle, N-alcoyle inférieur carbamyle, ou N,N-dialcoyle inférieur carbamyle, $R_2$ représente hydrogène, hydroxy, alcoxy inférieur, benzyloxy, alcanoyloxy en $C_{2-5}$, alcane inférieur sulfonyloxy, benzoyloxy, pyridoyloxy, amino, alcanoylamino en $C_{2-5}$, benzoylamino pyridoylamino, $R_3$ représente un hydrogène ou un alcoyle en $C_{1-7}$, alc représente un alcoylène inférieur, qui relie par un pont les deux systèmes cycliques par jusqu'à trois atomes de carbone compris, ou représente un alcoylidène inférieur, le noyau A est non substitué ou mono-, di- ou polysubstitué par un hydroxy, alcoxy inférieur, alcanoyloxy en $C_{2-5}$, halogène ayant un numéro atomique allant jusqu'à 53 compris alcoyle en $C_{1-7}$ et/ou trifluorométhyle, la ligne pointillée exprime le fait qu'entre les atomes de carbone que portent les radicaux $R_1$ et $R_2$ se trouve une liaison simple ou une liaison double, X représente un atome d'oxygène ou un groupe méthlène, Y représente un atome d'oxygène et n vaut 1, les radicaux décrits comme "inférieurs" pouvant présenter de 1 à 4 atomes de carbone compris ou un de ses tautomères et/ou sels.

3. Procédé selon la revendication 1, de préparation d'un composé de formule I où $R_1$ représente un alcoxy en $C_{1-4}$-carbonyle, carbamyle, hydroxyméthyle, ou alcanoyle en $C_{2-5}$-oxyméthyle et $R_2$ représente un hydrogène, ou un hydroxy ou bien $R_1$ représente un hydrogène et $R_2$ représente un alcoxy en $C_{1-4}$-carbonyle, $R_3$ représente un hydrogène ou un alcoyle en $C_{1-4}$, alc représente un alcoylène en $C_{1-4}$ qui relie les deux systèmes cycliques par jusqu'à trois atomes de carbone compris, le noyau A est non substitué, ou est substitué, en particulier en position 7, par un alcoxy en $C_{1-4}$, la ligne pointillée exprime le fait qu'entre les atomes de carbone que portent les radicaux $R_1$ et $R_2$ se trouve une liaison simple ou une liaison double, et ou bien X et Y représentent chacun un atome d'oxygène, n vaut 1 ou bien X représente un groupe méthylène, Y représente un atome d'oxygène et n vaut 1 ou bien X représente un atome d'oxygène, Y représente un groupe méthylène et n vaut 1 ou bien X représente une liaison directe, Y représente un atome d'oxygène et n vaut 2, ou un de ses tautomères ou sels.

4. Procédé selon la revendication 1 de préparation d'un composé de formule I, où $R_1$ représente un alcoxy en $C_{1-4}$-carbonyle, $R_2$ représente un hydrogène ou un hydroxy, $R_3$ représente un hydrogène ou un alcoyle en $C_{1-4}$, alc représente un alcoylène en $C_{1-4}$ qui relie les deux systèmes cycliques par jusqu'à trois atomes de carbone compris, le noyau A est non substitué, la ligne pointillée exprime le fait qu'entre les atomes de carbone que portent les radicaux $R_1$ et $R_2$ se trouve une liaison simple ou une liaison double, X représente un atome d'oxygène ou un groupe méthyle, Y représente un atome d'oxygène et n vaut 1 ou de ses tautomères et/ou sels.

5. Procédé selon la revendication 1, de préparation d'un composé de formule I, où $R_1$ représente un alcoxy en $C_{1-4}$-carbonyle, $R_2$ représente un hydrogène ou un hydroxy, $R_3$ est un hydrogène, alc représente un méthylène ou un éthylène, le noyau A est non substitué, la ligne pointillée exprime le fait qu'entre les atomes de carbone que portent les radicaux $R_1$ et $R_2$ se trouve une liaison simple ou une liaison double, X représente un atome d'oxygène ou un groupe méthylène, Y représente un atome d'oxygène et n vaut 1, ou un de ses tautomères et/ou sels.

6. Procédé selon la revendication 1 ou 2, de préparation d'un : Ester méthylique de l'acide 4-hydroxy-1-(chroman-3-ylméthyl)-1, 2, 5, 6-tétrahydro-pyridine-3-carboxylique ou ester méthylique de l'acide 1-(chroman-3-ylméthyl)-4-oxo-pipéridine-3-carboxylique, ester méthylique de l'acide 1-[2-(chroman-3-yl)-éthyl]-1,2,5,6-tétrahydro-pyridine-3-carboxylique, ester méthylique de l'acide 1-(benzo-1,4-dioxan-2-yl-méthyl)-pipéridin-3-carboxylique ou ester méthylique de l'acide 4-hydroxy-1-(benzo-1,4-dioxan-2-ylméthyl)-1,2,5,6-tétrahydro-pyridin-3-carboxylique, ou ester méthylique de l'acide 1-(benzo-1,4-dioxan-2-ylméthyl)-4-oxo-pipéridin-3-carboxylique ou à chaque fois un de leurs sels.

7. Procédé selon la revendication 1, de préparation d'un Ester éthylique de l'acide 1-[2-(chroman-3-yl)-

éthyl]-1,2,5,6-tétrahydro-pyridin-3-carboxylique ou un de ses sels.

8. Procédé selon la revendication 1 ou 2 de préparation d'un Ester méthylique de l'acide 1-(chroman-3-ylméthyl)-1,2,5,6-tétrahydro-pyridin-3-carboxylique, ester méthylique de l'acide 1-[2-(chroman-3-yl)-éthyl]-4-hydroxy-1,2,5,6-tétrahydro-pyridin-3-carboxylique, ou ester méthylique de l'acide 1-[2-(chroman-3-yl)éthyl]-4-oxo-pipéridin-3-carboxylique, ester méthylique de l'acide 1-(benzo-1,4-dioxan-2-ylméthyl)-1,2,5,6-tétrahydro-pyridin-3-carboxylique, ester méthylique de l'acide 4-hydroxy-1-(2-méthyl-benzo-1,4-dioxan-2-ylméthyl)-1,2,5,6-tétrahydro-pyridin-3-carboxylique ou ester méthylique de l'acide 1-(2-méthyl-benzo-1,4-dioxan-2-ylméthyl)-4-oxo-pipéridin-3-carboxylique, ester méthylique de l'acide 1-[2-(benzo-1,4-dioxan-2-yl)éthyl]-4-hydroxy-1,2,5,6-tétrahydropyridin-3-carboxylique, ou ester méthylique de l'acide 1-[2-(benzo-1,4-dioxan-2-yl)éthyl]-4-oxo-pipéridin-3-carboxylique, ester méthylique de l'acide cis-1-[2-(benzo-1,4-dioxan-2-yl)éthyl]-4-hydroxy-pipéridin-3-carboxylique, ester méthylique de l'acide trans-1-[2-(benzo-1,4-dioxan-2-yl)éthyl]-4-hydroxy-pipéridin-3-carboxylique ou ester méthylique de l'acide 1-[2-(benzo-1,4-dioxan-2-yl)éthyl]-1,2,5,6-tétrahydro-pyridin-3-carboxylique ou à chaque fois un de leurs sels.

9. Procédé selon la revendication 1 de préparation de Ester éthylique de l'acide 1-[2-(chroman-3-yl)éthyl]-pipéridin-3-carboxylique, ester éthylique de l'acide 1-(2-(chroman-3-yl)éthyl]-pipéridin-4-carboxylique, ester éthylique de l'acide 1-[2-(benzo-1,4-dioxan-2-yl)éthyl]-pipéridin-3-carboxylique, ester éthylique de l'acide 1-[2-(benzo-1,4-dioxan-2-yl)éthyl]-pipéridin-4-carboxylique, ester éthylique de l'acide 1[2-(chroman-2-yl)éthyl]-pipéridin-3-carboxylique, ester éthylique de l'acide 1-[2-(chroman-2-yl)éthyl]-pipéridin-4-carboxylique, ester méthylique de l'acide cis-1-[2-(benzo-1,4-dioxan-2-yl)éthyl]-4-benzyloxy-pipéridin-3-carboxylique, ester méthylique de l'acide trans-1-[2-(benzo-1,4-dioxan-2-yl)éthyl]-4-benzyloxy-pipéridin-3-carboxylique, amide de l'acide cis-4-amino-1-[2-(benzo-1,4-dioxan-2-yl)éthyl]-pipéridin-3-carboxylique, amide de l'acide trans-4-amino-1-[2-(benzo-1,4-dioxan-2-yl)éthyl]-pipéridin-3-carboxylique, ester méthylique de l'acide 1-[2-(chroman-4-yl)éthyl]-1,2,5,6-tétrahydro-pyridin-3-carboxylique ou ester éthylique de l'acide 1-[2-(chroman-4-yl)éthyl]-pipéridin-3-carboxylique ou à chaque fois un de ses sels.

10. Procédé de préparation d'un composé de formule

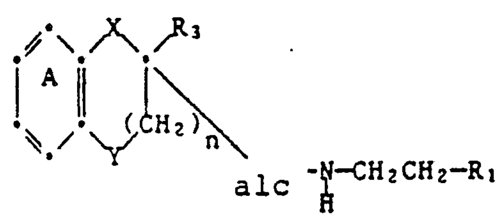

(IVc),

où $R_1$ représente un carboxy, alcoxy inféireur carbonyle, carbamyle, N-alcoyle inférieur carbamyle, N,N-dialcoyle inférieur carbamyle, hydroxyméthyle, alcanoyloxy en $C_{2-5}$-méthyle, alcane inférieur sulfonyloxyméthyle, benzoyloxyméthyle ou pyridoyloxyméthyle, $R_3$ représente un hydrogène ou un alcoyle en $C_{1-4}$, alc représente un alcoylène inférieur ou un alcoylidène inférieur, le noyau A est non substitué ou mono- ou polysubstitué par un hydroxy, alcoxy inférieur, alcanoyloxy en $C_{2-5}$, halogène de numéro atomique allant jusqu'à 53 compris, alcoyle en $C_{1-7}$ et/ou trifluorométhyle et ou bien X et Y représentent chacun un atome d'oxygène et n vaut 1 ou bien X représente un groupe méthylène, Y représente un atome d'oxygène et n vaut 1, ou bien X représente un atome d'oxygène, Y représente un groupe méthylène et n vaut 1, ou bien X représente une liaison directe, Y représente un atome d'oxygène et n vaut 2, avec cette précision que dans les composés de formule IVc où le noyau A est non substitué, X et Y représentent à chaque fois un oxygène, n vaut 1 et $R_3$ représente un atome d'hydrogène, alc est différent d'un méthylène lorsque $R_1$ représente un carbamyle ou un N-méthyl-, N-éthyl-, N,N-diméthyl-ou N,N-diéthylcarbamyle, ou les radicaux décrits comme "inférieurs" peuvent présenter de 1 à 4 atomes de carbone compris, ou un de ses sels,
caractérisé en ce que

h) on fait réagir les composés de formules

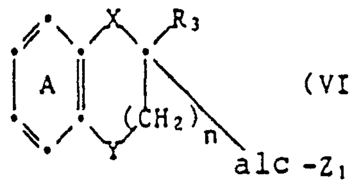 (VIIIa) et $Z_2-CH_2-CH(Z_3)-R_1$ (VIIIb),

où l'un des radicaux $Z_1$ et $Z_2$ représente un hydroxy estérifié réactif, l'autre un amino et $Z_3$ représente un hydrogène ou bien $Z_1$ est un amino et $Z_2$ ainsi que $Z_3$ représentent ensemble une liaison supplémentaire, ou éventuellement des sels de ces composés entre eux, ou

i) dans un composé de formule

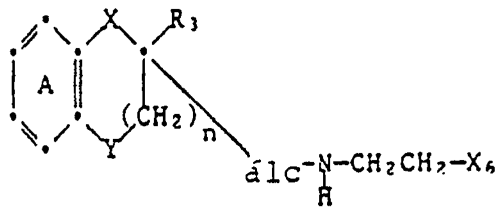 (IX),

où $X_6$ représente un radical transformable en $R_1$ ou un de ses sels, on transforme $X_6$ en $R_1$ et, si on le désire, on transforme à chaque fois un composé de formule IVc que l'on peut obtenir selon le procédé ou d'une autre manière en un autre composé de formule IVc, à chaque fois on dédouble un mélange d'isomères que l'on peut obtenir selon le procédé en ses composants, à chaque fois on sépare un mélange d'énantiomères ou selon les cas de diastéréomères que l'on peut obtenir selon le procédé en les énantiomères ou selon les cas diastéréomères, et/ou à chaque fois on transforme un composé de formule IVc libre que l'on peut obtenir selon le procédé en un sel ou on transforme un sel que l'on peut obtenir selon le procédé en le composé de formule IVc libre ou en un autre sel.

**11.** Procédé de préparation selon la revendication 10 de préparation d'un composé de formule IVc où $R_1$ représente un carboxy, alcoxy inférieur carbonyle, carbamyle, N-alcoyle inférieur carbamyle, ou N,N-dialcoyle inférieur carbamyle, $R_3$ représente un hydrogène ou un-alcoyle en $C_{1-4}$, alc représente un alcoylène inférieur ou un alcoylidène inférieur, le noyau A est non substitué ou mono-ou polysubstitué par un hydroxy, alcoxy inférieur, alcanoyloxy en $C_{2-5}$, halogène de numéro atomique allant jusqu'à 53 compris, alcoyle en $C_{1-7}$ et/ou trifluorométhyle, X représente un atome d'oxygène ou un groupe méthylène, Y représente un atome d'oxygène et n vaut 1, avec cette précision que dans les composés de formule IVc où le noyau A est non substitué, X et Y représentent à chaque fois un oxygène, n vaut 1 et $R_3$ représente un hydrogène, alc est différent d'un méthylène lorsque $R_1$ représente un carbamyle, ou un N-méthyl-, N-éthyl-, N,N-diméthyl- ou N,N-diéthylcarbamyle, où les radicaux décrits comme "inférieurs" peuvent présenter de 1 à 4 atomes de carbone compris, ou un de ses sels.

**12.** Procédé selon la revendication 10, de préparation d'un composé de formule IVc où $R_1$ représente un carboxy, alcoxy inférieur carbonyle, carbamyle, N-alcoyle inférieur carbamyle, N,N-dialcoyle inférieur-carbamyle, hydroxyméthyle, alcanoyloxy en $C_{2-5}$-méthyle, alcane inférieur sulfonyloxyméthyle, benzoyloxyméthyle ou pyridoyloxyméthyle, $R_3$ représente un hydrogène ou un alcoyle en $C_{1-4}$,' alc représente un alcoylène inférieur qui relie le système cyclique avec le groupe NH représenté dans la formule IVc par de 1 à 3 atomes de carbone compris, ou représente un alcoylidène inférieur, le noyau A est non substitué ouest mono-, di- ou polysubstitué par un hydroxy, alcoxy inférieur, alcanoyloxy en $C_{2-5}$, halogène de numéro atomique allant jusqu'à 53 compris, alcoyle en $C_{1-7}$ et/ou trifluorométhyle et ou bien X et Y représentent chacun un atome d'oxygène et n vaut 1, ou bien X représente un groupe méthylène, Y représente un atome d'oxygène et n vaut 1 ou bien X représente un atome d'oxygène, Y représente un groupe méthylène et n vaut 1, ou bien X représente une liaison directe, Y représente un atome d'oxygène et n vaut 2, avec cette précision que dans les composés de formule IVc où le noyau A est non substitué, X et représentent chacun un oxygène, n vaut 1 et $R_3$ représente un hydrogène, alc est différent d'un méthylène lorsque $R_1$ représente un carbamyle ou un N-méthyl-, N-éthyl-, N,N-diméthyl-ou N,N-diéthylcarbamyle, où les radicaux décrits comme "inférieurs" peuvent présenter de 1

à 4 atomes de carbone compris, ou un de ses sels.

**13.** Procédé selon la revendication 10, de préparation d'un composé de formule IVc où $R_1$ représente un carboxy, alcoxy inférieur carbonyle, carbamyle, N-alcoyle inférieur carbamyle, ou N,N-dialcoyle inférieur carbamyle, $R_3$ représente un hydrogène ou un alcoyle en $C_{1-4}$, alc représente un alcoylène inférieur qui relie les systèmes cycliques avec le groupe NH représenté dans la formule IVc par jusqu'à trois atomes de carbone compris, ou représente un alcoylidène inférieur, le noyau A est non substitué ou mono-, di- ou polysubstitué par un hydroxy, alcoxy inférieur, alcanoyloxy en $C_{2-5}$, halogène de numéro atomique allant jusqu'à 53 compris, alcoyle en $C_{1-7}$ et/ou trifluorométhyle, X représente un atome d'oxygène ou un groupe méthylène, Y représente un atome d'oxygène et n vaut 1, avec cette précision que dans les composés de formule IVc où le noyau A est non substitué X et Y représentent à chaque fois un oxygène, n vaut 1 et $R_3$ représente un hydrogène, alc est différent d'un méthylène lorsque $R_1$ représente un carbamyle ou N-méthyl-, N-éthyl-, N,N-diméthyl- ou N,N-diéthylcarbamyle où les radicaux décrits comme "inférieurs" peuvent présenter de 1 à 4 atomes de carbone compris ou un de ses sels.

**14.** Procédé selon la revendication 10, de préparation d'un composé de formule IVc, où $R_1$ représente un carboxy, hydroxyméthyle, alcanoyloxy en $C_{2-5}$-méthyle, alcoxy en $C_{1-4}$-carbonyle ou carbamyle, $R_3$ représente un hydrogène ou un alcoyle en $C_{1-4}$, alc représente un alcoylène en $C_{1-4}$ qui relie le système cyclique avec le groupe NH représenté dans la formule IVc par jusqu'à 3 atomes de carbone compris, le noyau A est non substitué ou, en particulier en position 7, par un alcoxy en $C_{1-4}$ et ou bien X et Y représentent à chaque fois un atome d'oxygène et n vaut 1, ou bien X représente un groupe méthylène, y représente un atome d'oxygène et n vaut 1, ou bien X représente un atome d'cxygène, Y représente un groupe méthylène et n vaut 1, ou bien X représente une liaison directe, Y représente un atome d'oxygène et n vaut 2, avec cette précision que dans les composés de formule IVc où le noyau A est non substitué, X et Y représentent à chaque fois un oxygène, n vaut 1 et $R_3$ représente un hydrogène, alc est différent d'un oxygène lorsque $R_1$ représente un carbamyle, ou un de ses sels.

**15.** Procédé selon la revendication 10, de préparation d'un composé de formule IVc où $R_1$ représente un alcoxy en $C_{1-4}$-carbonyle, $R_3$ représente un hydrogène ou un alcoyle en $C_{1-4}$, alc représente un alcoylène en $C_{1-4}$ qui relie le système cyclique avec le groupe NH représenté dans la formule IVc par jusqu'à trois atomes de carbone compris, le noyau A est non substitué, X représente un atome d'oxygène ou un groupe méthylène, Y représente un atome d'oxygène et n vaut 1, ou un de ses sels.

**16.** Procédé selon la revendication 10, de préparation d'un composé de formule IVc où $R_1$ représente un alcoxy en $C_{1-4}$-carbonyle, $R_3$ est un hydrogène, alc représente un méthylène ou un éthylène, le noyau A est non substitué, X représente un atome d'oxygène ou un groupe méthylène, Y représente un atome d'oxygène et n vaut 1, ou un de ses sels.

**17.** Procédé de préparation d'un composé de formule

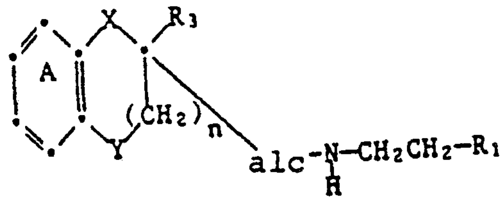

(IVc),

où $R_1$ représente un carboxy, alcoxy inférieur carbonyle, carbamyle, N-alcoyle inférieur carbamyle, ou N,N-diacoyle inférieur carbamyle, $R_3$ représente un hydrogène ou un alcoyle en $C_{1-7}$, alc représente un alcoylène inférieur ou un alcoylidène inférieur, un noyau A est non substitué, mono- ou polysubstitué par un hydroxy alcoxy inférieur, alcanoyloxy en $C_{2-5}$, halogène de numéro atomique allant jusqu'à 53 compris, alcoyle en $C_{1-7}$ et/ou trifluorométhyle, X représente un atome d'oxygène ou un groupe méthylène, Y représente un atome d'oxygène et n vaut 1, avec cette précision que dans les composés de formule IVc, le noyau A est non substitué, X et Y représentent chacun un oxygène, n vaut 1 et $R_3$

représente un hydrogène, alc est différent d'un méthylène lorsque $R_1$ représente un carbamyle ou un N-méthyl-, N-éthyl-, N,N-diméthyl- ou N,N-diéthylcarbamyle, où les radicaux décrits comme "inférieurs" peuvent présenter de 1 à 4 atomes de carbone compris ou d'un de ses sels, caractérisé en qu'on fait des composés de formules

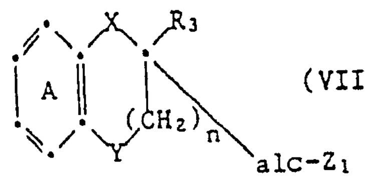 (VIIIa) et $Z_2-CH_2-CH(Z_3)-R_1$ (VIIIb),

où l'un des radicaux $Z_1$ et $Z_2$ représente un hydroxy estérifié réactif, l'autre représente un amino et $Z_3$ représente un hydrogène, ou $Z_1$ est un amino et $Z_2$ ainsi que $Z_3$ représentent ensemble une liaison supplémentaire, ou éventuellement des sels de ces composés entre eux et si on le désire on transforme un composé de formule IVc que l'on peut obtenir selon le procédé ou d'une autre manière en un composé de formule IVc, on dédouble un mélange d'isomères que l'on peut obtenir selon le procédé en ses composants, on sépare un mélange d'énantiomères ou selon les cas de diastéréomères que l'on peut obtenir selon le procédé en les énantiomères ou selon les cas diastéréomères, et/ou on transforme un composé de formule IVc libre que l'on peut obtenir selon le procédé en un sel ou on transforme un sel que l'on peut obtenir selon le procédé en le composé de formule IVc libre ou en un autre sel.

**18.** Procédé selon la revendication 17, de préparation d'un composé de formule IVc où $R_1$ représente un carboxy, alcoxy inférieur carbonyle, carbamyle, N-alcoyle inférieur carbamyle, ou N,N-dialcoyle inférieur carbamyle, $R_3$ représente un hydrogène ou un alcoyle en $C_{1-7}$, alc représente un alcoylène inférieur qui relie le système cyclique avec le groupe NH représenté dans la formule IVc par jusqu'à trois atomes de carbone compris ou un alcoylidène inférieur, le noyau A est non substitué, mono-, di- ou polysubstitué par un l'hydroxy, alcoxy inférieur, alcanoyloxy en $C_{2-5}$, halogène de numéro atomique allant jusqu'à 53 compris, alcoyle en $C_{1-7}$ et/ou trifluorométhyle, X représente un atome d'oxygène ou un groupe méthyle, Y représente un atome d'oxygène et n vaut 1, avec cette précision que dans les composés de formule IVc où le noyau A est non substitué, X et Y représentent chacun un oxygène, n vaut 1 et $R_3$ représente un hydrogène, alc est différent d'un méthylène lorsque $R_1$ représente un carbamyle ou un N-méthyl-, N-éthyl-, N,N-diméthyl-ou N,N-diéthylcarbamyle, où les radicaux décrits comme "inférieurs" peuvent présenter de 1 à 4 atomes de carbone compris ou d'un de ses sels.

**19.** Procédé selon la revendication 17, de préparation d'un composé de formule IVc où $R_1$ représente un alcoxy en $C_{1-4}$-carbonyle, $R_3$ représente un hydrogène ou un alcoyle en $C_{1-4}$, alc représente un alcoylène en $C_{1-4}$ qui relie le sytème cyclique avec le groupe NH représenté dans la formule IVc par jusqu'à trois atomes de carbone compris, le noyau A est non substitué X représente un atome d'oxygène ou un groupe méthylène, Y représente un atome d'oxygène et n vaut 1, ou d'un de ses sels.

**20.** Procédé selon la revendication 17, de préparation d'un composé de formule IVc où $R_1$ représente un alcoxy en $C_{1-4}$-carbonyle, $R_3$ représente un hydrogène, alc représente un méthylène ou un éthylène, le noyau A est non substitué, X représente un atome d'oxygène ou un groupe méthylène, Y représente un atome d'oxygène et n vaut 1 ou d'un de ses sels.

**21.** Procédé selon la revendication 10 ou 17 de préparation d'un N-[2-(chroman-3-yl)éthyl]-N-(2-méthoxycarbonyléthyl)-amine ou d'un de ses sels

**22.** Procédé selon la revendication 10 ou 17 de préparation d'un N-(2-méthoxycarbonyléthyl)-N-(benzo-1,4-dioxan-2-ylméthyl)-amine ou d'un de ses sels.

**23.** Procédé selon la revendication 10 ou 17, de préparation de N-(2-méthoxycarbonyléthyl)-N-(chroman-3-ylméthyl)-amine, N-[2-(benzo-1,4-dioxan-2-yl)éthyl]-N-(2-méthoxycarbonyléthyl)-amine ou N-(2-méthoxycarbonyléthyl)-N-(2-méthyl-benzo-1,4-dioxan-2-ylméthyl)-amine ou à chaque fois d'un de ses sels.

**24.** Procédé selon la revendication 10, de préparation de N-[2-(chroman-3-yl)éthyl]-N-(2-carboxyéthyl)-amine, N-[2-(chroman-2-yl)éthyl]-N-(2-méthoxycarbonyléthyl)-amine ou N-[2-(chroman-4-yl)éthyl]-N-(2-méthoxycarbonyléthyl)-amine ou à chaque fois d'un de ses sels.

**25.** Procédé de préparation d'une préparation pharmaceutique, caractérisé en ce que
a) on fait réagir un composé de formule

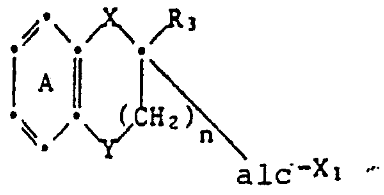

(IIa)

ou un de ses sels, où $X_1$ représente un hydroxy ou un hydroxy estérifié réactif, avec un composé de formule

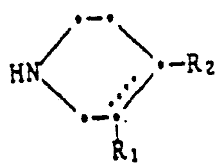

(IIb)

ou un de ses tautomères et/ou sels ou
b) dans un composé de formule

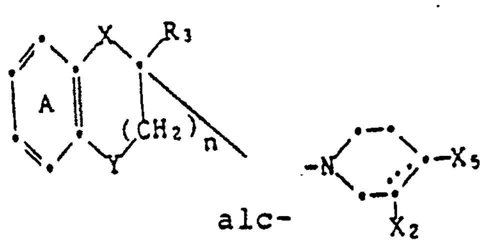

(III)

ou un de ses tautomères et/ou sels, où $X_2$ représente un radical transformable en $R_1$ différent d'un hydrogène et $X_5$ représente un radical $R_a$ où $R_a$ représente un hydrogène, hydroxy, alcoxy inférieur, benzyloxy, alcoyloxy en $C_{2-5}$, alcane inférieur sulfonyloxy, benzoyloxy, pyridoyloxy, amino, alcanoyle en $C_{2-5}$-amino, alcane inférieur sulfonylamino, benzoylamino ou pyridoylamino, on transforme $X_2$ en un radical $R_1$ différent d'un hydrogène ou dans un composé de formule III où $X_2$ est un hydrogène et $X_5$ représente un radical transformable en $R_b$ où $R_b$ représente un radical $R_2$ différent d'un radical $R_a$, on transforme $X_5$ et $R_b$, ou
c) pour préparer un composé de formule I ou un de ses tautomères et/ou sels où $R_2$ représente un hydroxy ou un amino et $R_1$ est différent d'un hydrogène, et représente en particulier un alcoxy inférieur carbonyle, on cyclise un composé de formule

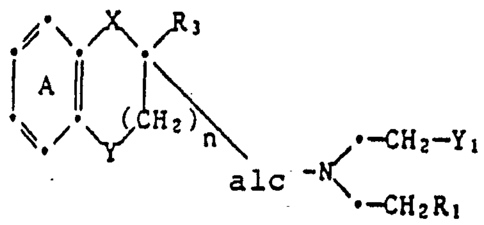

(IV),

où $Y_1$ représente un groupe de formule - $CH=R_2'$, $-C(Y_2)=R_2'$, $-CH(Y_2)-R_2$ ou un cyano où $R_2'$ représente un oxy ou un imino et $Y_2$ représente un radical séparable ou un de ses sels, ou

d) pour préparer un composé de formule I( ou un de ses tautomères et/ou sels où $R_2'$ représente-un oxo ou un imino ou $R_1$ est différent d'un hydrogène et représente en particulier un alcoxy inférieur carbonyle, on fait réagir un composé de formule

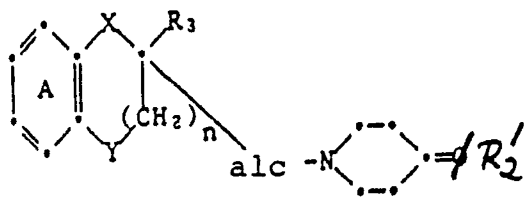

(Va)·

ou un de ses tautomères ou un de ses sels avec un composé de formule

$X_3-R_1$ (Vb)

où $R_1$ est différent d'un hydrogène et $X_3$ représente un halogène ou un alcoxy inférieur, ou

e) pour préparer un composé de formule I et un de ses tautomères et/ou sels où $R_2$ est un hydroxy, alcoxy inférieur, benzyloxy, alcanoyloxy en $C_{2-5}$, alcane inférieur-sulfonyloxy, benzoyloxy, pyridoyloxy, amino, alcanoylamino en $C_{2-5}$, alcane inférieur-sulfonylamino, benzoylamino ou pyridoylamino, dans un composé de formule

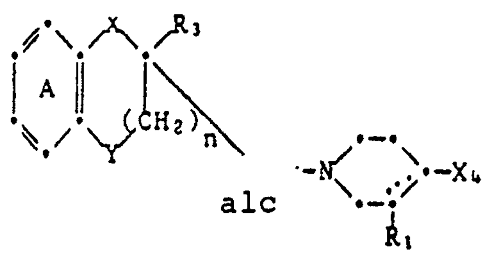

(VI),

ou un de ses sels où $X_4$ représente un radical transformable en $R_2$, on transforme $X_4$ en $R_2$ ou

f) en particulier pour préparer un composé de formule I et un de ses tautomères et/ou sels où $R_2$ est un hydrogène, hydroxy, amino, carboxy, alcoxy inférieur carbonyle, N-alcoyle inférieur-carbamoyle, N,N-dialcoyle inférieur-carbamyle ou hydroxyméthyle, dans un composé de formule

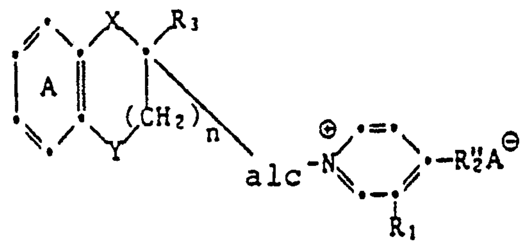

(VII),

où $A^{\ominus}$ représente l'anion d'un acide et $R_2''$ représente un hydrogène, alcoxy inférieur, benzyloxy, alcanoyloxy en $C_{2-5}$, alcane inférieur sulfonyloxy, benzoyloxy, pyridoyloxy, hydroxy protégé, alcanoylamino en $C_{2-5}$, alcane inférieur sulfonylamino, benzoylamino, pyridoylamino, carboxy, alcoxy inférieur carbonyle, N-alcoyle inférieur carbamyle, N,N-dialcoyle inférieur carbamyle, alcanoyloxy en $C_{2-5}$-méthyle, alcane inférieur sulfonyloxyméthyle, benzoyloxyméthyle, pyridoyloxyméthyle ou hydroxyméthyle éthérifié ou protégé, on réduit les doubles liaisons en excès en liaisions simples, ou
g) pour préparer un composé de formule I ou un de ses tautomères et:ou sels où $R_2$ représente un carboxy, N-alcoyle inférieur-carbamyle, N,N-dialcoyle inférieur carbamyle et en particulier un alcoxy inférieur carbonyle, on cyclise un composé de formule

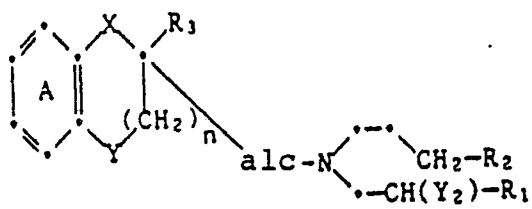

(VIII),

où $Y_2$ représente un radical séparable, ou un de ses sels, et à chaque fois on sépare un groupe protecteur éventuellement présent et à chaque fois on transforme si on le désire un composé de formule I que l'on peut obtenir selon le procédé ou d'une autre manière en un autre composé de formule I, et chaque fois on dédouble un mélange d'isomères que l'on peut obtenir selon le procédé en ses composants, à chaque fois on sépare un mélange d'énantiomères dans les cas de diastéréomères que l'on peut obtenir selon le procédé en les énantiomères ou selon les cas diastéréomères, et/ou à chaque fois on transforme un composé de formule I libre que l'on peut obtenir selon le procédé en un sel ou un sel que l'on peut obtenir selon le procédé en le composé de formule I libre ou en un autre sel, où les radicaux décrits comme "inférieurs" peuvent présenter de 1 à 4 atomes de carbone compris.

**26.** Procédé de préparation d'une préparation pharmaceutique, caractérisé en ce que
h) on fait réagir les composés de formules

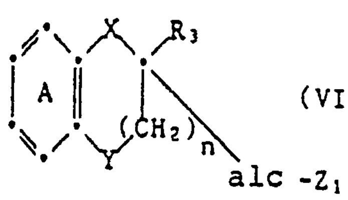

(VIIIa) et $Z_2-CH_2-CH(Z_3)-R_1$ (VIIIb),

où l'un des radicaux $Z_1$ et $Z_2$ représente un hydroxy estérifié réactif, l'autre un amino et $Z_3$ représente un hydrogène ou bien $Z_1$ est un amino et $Z_2$ ainsi que $Z_3$ représentent ensemble une liaison supplémentaire, ou éventuellement des sels de ces composés entre eux, ou
i) dans un composé de formule

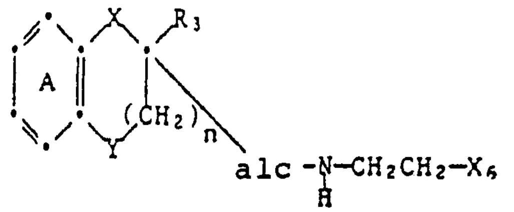

(IX),

où $X_6$ représente un radical transformable en $R_1$ ou un de ses sels, on transforme $X_6$ en $R_1$ et, si on le désire, on transforme à chaque fois un composé de formule IVc que l'on peut obtenir selon le procédé ou d'une autre manière en un autre composé de formule IVc, à chaque fois on dédouble un mélange d'isomères que l'on peut obtenir selon le procédé en ses composants, à chaque fois on sépare un mélange d'énantiomères ou selon les cas de diastéréomères que l'on peut obtenir selon le procédé en les énantiomères ou selon les cas diastéréomères, et/ou à chaque fois on transforme un composé de formule Ivc libre que l'on peut obtenir selon le procédé en un sel ou on transforme un sel que l'on peut obtenir selon le procédé en le composé de formule IVc libre ou en un autre sel,et on mélange un composé à chaque fois obtenu de formule

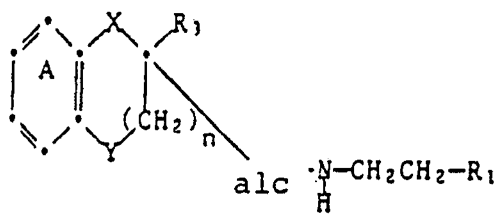

(IVc),

où $R_1$ représente un carboxy, alcoxy inférieur carbonyle, carbamyle, N-alcoyle inférieur carbamyle, N-alcoyle inférieur carbamyle, N,N-dialcoyle inférieur carbamyle, hydroxyméthyle, alcanoyloxy en $C_{2-5}$-méthyle, alcane inférieur sulfonyloxyméthyle, benzoyloxyméthyle ou pyridoyloxyméthyle $R_3$ représente un hydrogène ou un alcoyle inférieur en $C_{1-7}$, alc représente un alcoylène inférieur ou un alcoylidène inférieur, le noyau A est non substitué ou mono- ou polysubstitué par un hydroxy, alcoxy inférieur, alcanoyloxy en $C_{2-5}$, halogène de numéro atomique allant jusqu'à 53 compris, alcoyle en $C_{1-7}$ et/ou trifluorométhyle, et ou bien X et Y représentent chacun un atome d'oxygène et n vaut 1, ou bien X représente un groupe méthylène, Y représente un atome d'oxygène et n vaut 1, ou bien X représente un atome d'oxygène, Y représente un groupe méthylène et n vaut 1, ou bien X représente une liaison directe, Y représente un atome d'oxygène et n vaut 2, ou l'un de ses sels pharmaceutiquement acceptables à chaque fois obtenu, avec des additifs pharmaceutiques habituels.

**27.** Procédé de préparation d'une préparation pharmaceutique, caractérisé en ce qu'on fait réagir des composés de fromule

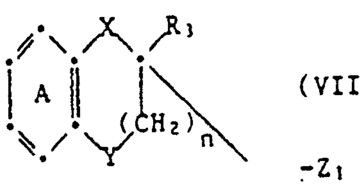

(VIIIa) et $Z_2-CH_2-CH(Z_3)-R_1$ (VIIIb),

où l'un des radicaux $Z_1$ et $Z_2$ représente un hydroxy estérifié réactif, l'autre représente un amino et $Z_3$ représente un hydrogène, ou $Z_1$ est un amino et $Z_2$ ainsi que $Z_3$ représentent ensemble une liaison supplémentaire, ou éventuellement des sels de ces composés entre eux et si on le désire on transforme un composé de formule IVc que l'on peut obtenir selon le procédé ou d'une autre manière en un composé de formule IVc, on dédouble un mélange d'isomères que l'on peut obtenir selon le procédé en ses composants, on sépare un mélange d'énantiomères ou selon les cas de diastéréomères que l'on peut obtenir selon le procédé en les énantiomères ou selon les cas diastéréomères, et/ou

on transforme un composé de formule IVc libre que l'on peut obtenir selon le procédé en un sel ou on transforme un sel que l'on peut obtenir selon le procédé en le composé de formule IVc libre ou en un autre sel ou en ce qu'on mélange un composé à chaque fois obtenu de formule

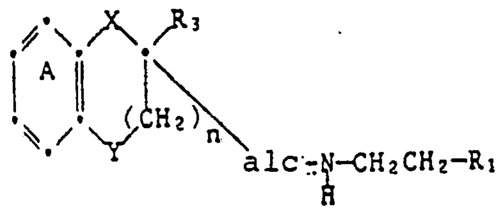

(IVc),

où $R_1$ représente un carboxy, alcoxy inférieur carbonyle, carbamyle, N-alcoyle inférieur carbamyle, N-alcoyle inférieur carbamyle, N,N-dialcoyle inférieur carbamyle, $R_3$ représente un hydrogène ou un alcoyle en $C_{1-7}$, alc représente un alcoylène inférieur ou un alcoylidène inférieur, le noyau A est non substitué ou mono- ou polysubstitué par un hydroxy, alcoxy inférieur, alcanoyloxy en $C_{2-5}$, halogène de numéro atomique allant jusqu'à 53 compris, alcoyle en $C_{1-7}$ et/ou trifluorométhyle, X représente un atome d'oxygène ou un groupe méthylène, Y représente un atome d'oxygène et n vaut 1, ou un de ses sels pharmaceutiquement acceptables à chaque fois obtenus avec des additifs pharmaceutiques habituels où les radicaux décrits comme "inférieurs" peuvent présenter de 1 à 4 atomes de carbone compris.

**28.** Procédé de préparation d'une préparation pharmaceutique, caractérisé en ce qu'on mélange un composé que l'on peut obtenir selon l'une des revendications 1 à 24, ou éventuellement un de ses tautomères et/ou un de ses sels pharmaceutiquement acceptables, avec des supports pharmaceutiques habituels.

**29.** Procédé de préparation d'une préparation pharmaoeutique, caractérisé en ce qu'on mélange un composé que l'on peut obtenir selon l'une des revendications 2, 4, 6, 8 et 17 à 23, ou éventuellement un de ses tautomères et/ou un de ses sels pharmaceutiquement acceptables, avec des additifs pharmaceutiques habituels.

**30.** Procédé de préparation d'une préparation pharmaceutique, caractérisé en ce qu'on mélange un composé de formule IVc où $R_1$ représente un carbamyle ou un N-méthyl-, N-éthyl-, N,N-diméthyl- ou N,N-diéthylcarbamyle, $R_3$ représente un hydrogène alc représente un méthylène, le noyau A est non substitué, X et Y représentent à chaque fois un atome d'oxygène et n vaut 1, ou un de ses sels pharmaceutiquement acceptables avec des additifs pharmaceutiques habituels.

**31.** Application d'un composé que l'on peut obtenir selon l'une des revendications 1 à 24, ou éventuellement d'un de ses tautomères et/ou de ses sels pharmaceutiquement acceptables, à la préparation d'une préparation pharmaceutique, p. ex. d'un agent nootropique.